(19) Europäisches Patentamt
European Patent Office
Office européen des brevets

(11) EP 4 471 030 A1

(12) EUROPEAN PATENT APPLICATION
published in accordance with Art. 153(4) EPC

(43) Date of publication:
**04.12.2024 Bulletin 2024/49**

(21) Application number: **23746391.4**

(22) Date of filing: **28.01.2023**

(51) International Patent Classification (IPC):
***C07D 471/04*** *(2006.01)* ***C07D 487/04*** *(2006.01)*
***A61K 31/437*** *(2006.01)* ***A61K 31/5025*** *(2006.01)*
***A61P 35/00*** *(2006.01)*

(52) Cooperative Patent Classification (CPC):
**A61K 31/4188; A61K 31/427; A61K 31/437; A61K 31/4985; A61K 31/5025; A61K 31/506; A61K 31/519; A61K 31/5377; A61P 35/00; A61P 35/02; C07D 417/04; C07D 471/04; C07D 487/04;** Y02P 20/55

(86) International application number:
**PCT/CN2023/073557**

(87) International publication number:
**WO 2023/143514 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.01.2022 CN 202210107872**
**20.05.2022 CN 202210557434**
**31.08.2022 CN 202211065464**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222069 (CN)**

(72) Inventors:
- **ZENG, Mi**
  **Shanghai 201203 (CN)**
- **GAO, Peng**
  **Shanghai 201203 (CN)**
- **XU, Peng**
  **Shanghai 201203 (CN)**
- **ZENG, Minggao**
  **Shanghai 201203 (CN)**
- **YU, Wensheng**
  **Shanghai 201203 (CN)**

(74) Representative: **Cabinet Beau de Loménie**
**158, rue de l'Université**
**75340 Paris Cedex 07 (FR)**

# (54) PROPYLENE KETONE-CONTAINING BIOINHIBITOR, PREPARATION METHOD THEREFOR, AND USE THEREOF

(57) The present invention relates to a propylene ketone-containing bioinhibitor, a preparation method therefor, and a use thereof. In particular, the present invention relates to the compound represented by each of the general formulas or a stereoisomer thereof, a preparation method therefor, a pharmaceutical composition containing the compound, and a use thereof in the preparation of a drug for treating cancer and other related diseases.

EP 4 471 030 A1

## Description

The present application claims the priority to Chinese patent application 202210107872.2 filed on January 28, 2022, Chinese patent application 202210557434.6 filed on May 20, 2022, and Chinese patent application 202211065464.1, and August 31, 2022. The aforementioned Chinese patent applications are incorporated in the present application by reference in their entirety.

### Technical Field

The present invention belongs to the field of biomedicine, particularly relates to a propylene ketone-containing bioinhibitor, a preparation method therefor, and the use thereof, and further relates to the use of a pharmaceutically acceptable salt and pharmaceutical composition thereof in the treatment of cancer, and other related diseases.

### Background Art

FGFRs (Fibroblast Growth Factor Receptors) belong to tyrosine kinases, including four isoforms, i.e., FGFR1, FGFR2, FGFR3 and FGFR4. Upon ligand binding, FGFRs dimerize and autophosphorylate, thus activating the downstream signaling pathways: RAS-RAF-MAPK, PI3K-AKT, STAT, and PLCy. FGFR-mediated signal transduction plays an important role in cell proliferation, migration, differentiation, and survival.

Over-activation caused by various mutations in FGFR widely exists in various tumors, and inhibition of FGFR is a potential targeted approach for the treatment of various cancers. In a study in Clinical Cancer Research (Clin Cancer Res; 22(1) January 1, 2016) published in 2015, sequencing of 4853 various solid tumors shows that FGFR aberrations exist in about 7.1% of cancers. FGFR1 amplification aberrations exist in about 20% of squamous cell lung carcinoma and about 20% of breast cancer. FGFR2 rearrangement aberrations exist in about 15% of cholangiocarcinoma, FGFR2 point mutations exist in about 10% of endometrial carcinoma, and FGFR2b amplification exists in about 10% of gastric cancer. FGFR3 point mutations exist in about 20% of metastatic urothelial carcinoma.

FGFR inhibitors, as drugs, have a good application prospect in the pharmaceutical industry, and are expected to become a new choice for first-line therapy for cholangiocarcinoma and targeted treatment of all types of cancers, and are expected to become useful for patients with cancers with various FGFR aberrations. However, at present, the standard therapy for cholangiocarcinoma is chemotherapy, which has a poor prognosis, and there is no second-line therapy. At present, the main problem of FGFR inhibitors is that patients all develop resistance after about 7 months since administration. In addition, drugs related to the FGFR1 target have hyperphosphatemia toxicity.

At present, there have been two pan-FGFR inhibitors on the market in the world. In April 2019, Balversa from Johnson & Johnson (erdafitinib, JNJ-42756493) was approved for patients with locally metastatic or metastatic bladder cancer with FGFR3 or FGFR2 point mutations. In April 2020, Pemazyre from Incyte (pemigatinib, INCB054828) was approved for treated patients with advanced cholangiocarcinoma with FGFR2 gene fusion or rearrangement.

Although it has been reported that FGFR inhibitors have therapeutic effects on various cancers, no potent and highly selective FGFR inhibitors have been found. The present invention aims to provide novel compounds having FGFR inhibitory activity and being useful as anticancer agents.

### Summary of the Invention

An object of the present invention is to provide a compound represented by general formula (I), or a stereoisomer or pharmaceutically acceptable salt thereof. The structure thereof is as follows:

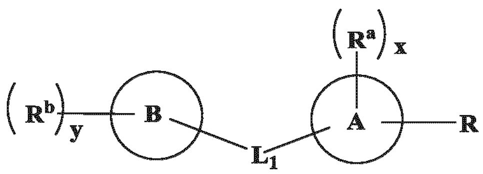

(I)

wherein:

$L_1$ is selected from a bond, alkylene, alkenylene, alkynylene, cycloalkylene, heterocyclylene, arylene, heteroarylene, -(C=C)-, $-(CR_{a1}=CR_{b1})_{n1}-$, $-(CR_{a1}R_{b1})_{n1}-$, $-O(CR_{a1}R_{b1})_{n1}-$, $-S(CR_{a1}R_{b1})_{n1}-$, $-(CR_{a1}R_{b1})_{n1}NR_{c1}-$, $-(CH_2)_{n1}C(O)$

$NR_{a1}$-, $-(CH_2)_{n1}NR_{a1}C(O)$-, $-(CH_2)_{n1}S(O)_{m1}$-, $-(CH_2)_{n1}NR_{a1}S(O)_{m1}$-, or - $(CH_2)_{n1}S(O)_{m1}NR_{a1}$-, wherein the alkylene, alkenylene, alkynylene, cycloalkylene, heterocyclylene, arylene, and heteroarylene can be optionally further substituted;

ring A is selected from cycloalkyl, heterocyclyl, aryl, or heteroaryl, wherein the cycloalkyl, heterocyclyl, aryl, and heteroaryl can be optionally further substituted;

ring B is selected from cycloalkyl, heterocyclyl, aryl, or heteroaryl, wherein the cycloalkyl, heterocyclyl, aryl, and heteroaryl can be optionally further substituted;

R is selected from hydrogen, deuterium, halogen, amino, nitro, hydroxyl, cyano, alkyl, deuteroalkyl, haloalkyl, hydroxyalkyl, alkoxy, haloalkoxy, heterocyclylalkylene, alkenyl, alkynyl, cycloalkyl, heterocyclyl, aryl, heteroaryl, $-(CH_2)_nNR_{a2}C(O)CR_{b2}=CR_{c2}$, $-O(CR_{a2}R_{b2})_{n2}R_{c2}$, - $S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}C(O)R_{c2}$, $-(CH_2)_{n2}S(O)_{m2}R_{c2}$, - $(CH_2)_{n2}NR_{a2}S(O)_{m2}R_{c2}$, or $-(CH_2)_{n2}S(O)_{m2}NR_{a2}R_{c2}$, wherein the amino, alkyl, deuteroalkyl, haloalkyl, hydroxyalkyl, alkoxy, haloalkoxy, alkenyl, alkynyl, cycloalkyl, heterocyclyl, aryl, and heteroaryl can be optionally further substituted;

$R^a$ is selected from hydrogen, deuterium, halogen, amino, nitro, hydroxyl, cyano, mercapto, oxo, thio, alkyl, deuteroalkyl, haloalkyl, hydroxyalkyl, alkoxy, haloalkoxy, alkenyl, alkynyl, alkylacyl, aminocarbonyl, alkylaminocarbonyl, alkylamino, cycloalkyl, heterocyclyl, aryl, and heteroaryl, wherein the amino, alkyl, deuteroalkyl, haloalkyl, hydroxyalkyl, alkoxy, haloalkoxy, alkenyl, alkynyl, alkylacyl, aminocarbonyl, alkylaminocarbonyl, alkylamino, cycloalkyl, heterocyclyl, aryl, and heteroaryl can be optionally further substituted;

alternatively, any two $R^a$ are connected to form cycloalkyl, heterocyclyl, aryl, or heteroaryl, wherein the cycloalkyl, heterocyclyl, aryl, and heteroaryl can be optionally further substituted; $R^b$ is selected from hydrogen, deuterium, halogen, amino, nitro, hydroxyl, cyano, mercapto, oxo, thio, alkyl, deuteroalkyl, haloalkyl, hydroxyalkyl, alkoxy, haloalkoxy, alkenyl, alkynyl, alkylacyl, aminocarbonyl, alkylaminocarbonyl, alkylamino, cycloalkyl, heterocyclyl, aryl, heteroaryl, - $O(CR_{a2}R_{b2})_{n2}R_{c2}$, $-S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}$-$C(O)R_{c2}$, - $(CH_2)_{n2}S(O)_{m2}R_{c2}$, $-(CH_2)_{n2}NR_{a2}S(O)_{m2}R_{c2}$, or $-(CH_2)_{n2}S(O)_{m2}NR_{a2}R_{c2}$, wherein the amino, alkyl, deuteroalkyl, haloalkyl, hydroxyalkyl, alkoxy, haloalkoxy, alkenyl, alkynyl, alkylacyl, aminocarbonyl, alkylaminocarbonyl, alkylamino, cycloalkyl, heterocyclyl, aryl, and heteroaryl can be optionally further substituted;

alternatively, any two $R^b$ are connected to form cycloalkyl, heterocyclyl, aryl, or heteroaryl, wherein the cycloalkyl, heterocyclyl, aryl, and heteroaryl can be optionally further substituted; $R_{a1}$, $R_{b1}$, and $R_{c1}$ are each independently selected from hydrogen, deuterium, halogen, amino, nitro, hydroxyl, cyano, alkyl, deuteroalkyl, haloalkyl, hydroxyalkyl, alkoxy, haloalkoxy, alkenyl, alkynyl, cycloalkyl, heterocyclyl, aryl, or heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl, and heteroaryl can be optionally further substituted;

alternatively, any two of $R_{a1}$, $R_{b1}$, and $R_{c1}$ are connected to form cycloalkyl, heterocyclyl, aryl, or heteroaryl, wherein the cycloalkyl, heterocyclyl, aryl, and heteroaryl can be optionally further substituted;

$R_{a2}$, $R_{b2}$, $R_{c2}$, and $R_{d2}$ are each independently selected from hydrogen, deuterium, halogen, amino, nitro, hydroxyl, cyano, alkyl, deuteroalkyl, haloalkyl, hydroxyalkyl, alkoxy, haloalkoxy, alkenyl, alkynyl, cycloalkyl, heterocyclyl, aryl, or heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl, and heteroaryl can be optionally further substituted;

alternatively, any two of $R_{a2}$, $R_{b2}$, $R_{c2}$, and $R_{d2}$ are connected to form cycloalkyl, heterocyclyl, aryl, or heteroaryl, wherein the cycloalkyl, heterocyclyl, aryl, and heteroaryl can be optionally further substituted;

x is 0, 1, 2, 3, 4, or 5;

y is 0, 1, 2, 3, 4, or 5;

n1 and n2 are 0, 1, 2, 3, 4, or 5; and

m1 and m2 are 0, 1, or 2.

In certain embodiments of the present invention, the compound is further as represented by general formula (I-1):

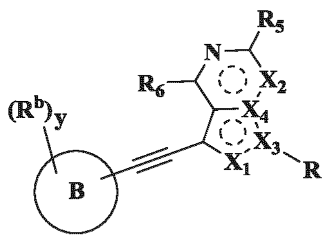

(I-1)

wherein $X_1$ is N or $CR_3$;

$X_2$ is N or $CR_4$; preferably, $X_2$ is $CR_4$;
$X_3$ is N or C;
$X_4$ is N or C;
$R_3$, $R_4$, $R_5$, and $R_6$ are each independently selected from hydrogen, deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more R'''; wherein the R''' is selected from deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5-to 14-membered heteroaryl, optionally substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;
preferably, $R_3$, $R_4$, $R_5$, and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3-to 12-membered heterocyclyl-carbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5-to 14-membered heteroaryl;
$R^b$, y, ring B, and R are the same as above;
more preferably, $R_4$ is not hydrogen.

In a further preferred embodiment of the present invention, the compound is further as represented by general formula (II-1):

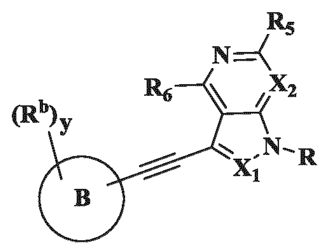

( II-1)

wherein $X_1$ is N or $CR_3$;
$X_2$ is N or $CR_4$;
$R_3$, $R_4$, $R_5$, and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$

hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;

preferably, $R_3$, $R_4$, $R_5$, and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R^b$, y, ring B, and R are the same as above;

preferably provided that when ring B is

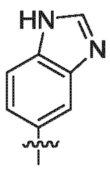 , 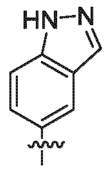 , 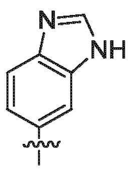 , 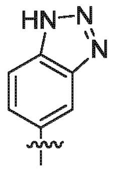 , 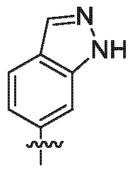 ,

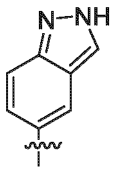 , 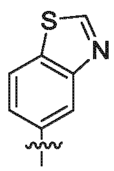 , 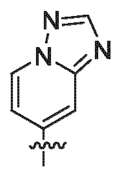 , 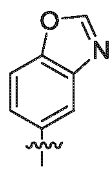 , or 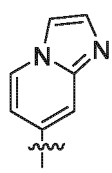 ,

$R_4$ is not hydrogen.

In a further preferred embodiment of the present invention, the R is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, 3- to 12-membered heterocyclyl $C_{1-6}$ alkylene, $C_{3-12}$ cycloalkyl, 3- to 12-membered heterocyclyl, $C_{6-14}$ aryl, 5- to 14-membered heteroaryl, $-(CH_2)_{n2}NR_{a2}C(O)CR_{b2}=CR_{c2}$, $-O(CR_{a2}R_{b2})_{n2}R_{c2}$, $-S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}C(O)R_{c2}$, $-(CH_2)_{n2}S(O)_{m2}R_{c2}$, $-(CH_2)_{n2}NR_{a2}S(O)_{m2}R_{c2}$, or $-(CH_2)_{n2}S(O)_{m2}NR_{a2}R_{c2}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more R';

In a further preferred embodiment of the present invention, the R is selected from 3- to 8-membered heterocyclyl $C_{1-6}$ alkylene, 3- to 8-membered heterocyclyl, or $-(CH_2)_{n2}NR_{a2}C(O)CR_{b2}=CR_{c2}$, wherein the 3- to 8-membered heterocyclyl $C_{1-6}$ alkylene and 3- to 8-membered heterocyclyl are optionally further substituted with one or more R';

R' is selected from deuterium, halogen, amino, hydroxyl, cyano, mercapto, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, 5- to 14-membered heteroaryl, $-C(O)CR_a=CR_b(CH_2)_nR_c$, $-C(O)CR_a=CR_b(CH_2)_nNR_cR_d$, $-(CH_2)_nR_a$, $-(CR_aR_b)_nR_c$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, mercapto, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_{a2}$, $R_{b2}$, $R_{c2}$, and $R_{d2}$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$

alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

n and n2 are 0, 1, 2, 3, 4, or 5; and

m and m2 are 0, 1, or 2.

In a further preferred embodiment of the present invention, the compound is further as represented by general formula (I-2):

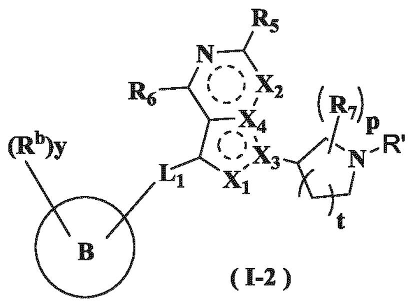

(I-2)

wherein $X_1$ is N or $CR_3$;

$X_2$ is N or $CR_4$; preferably, $X_2$ is $CR_4$;

$X_3$ is N or C;

$X_4$ is N or C;

$R_3$, $R_4$, $R_5$, and $R_6$ are each independently selected from hydrogen, deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more R‴; wherein the R‴ is selected from deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5-to 14-membered heteroaryl, optionally substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;

ring B is selected from $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R^b$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$

deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

alternatively, any two $R^b$ and adjacent atoms form $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_7$ is independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3-to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

R' is selected from deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, 5- to 14-membered heteroaryl, $-C(O)CR_a=CR_b(CH_2)_nR_c$, $-C(O)CR_a=CR_b(CH_2)_nNR_cR_d$, $-(CH_2)_nR_a$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$L_1$ is selected from a bond, $C_{1-3}$ alkylene, $C_{2-3}$ alkenylene, $C_{2-6}$ alkynylene, $C_{3-8}$ cycloalkylene, 3- to 8-membered heterocyclylene, -(C=C)-, $-(CR_{a1}=CR_{b1})_{n1}-$, $-O(CR_{a1}R_{b1})_{n1}-$ $-(CR_{a1}R_{b1})_{n1}(CO)-$, $-(CR_{a1}R_{b1})_{n1}NR_{c1}-$, or $-(CH_2)_{n1}C(O)NR_{a1}-$, wherein the $C_{1-3}$ alkylene, $C_{2-3}$ alkenylene, $C_{2-6}$ alkynylene, $C_{3-8}$ cycloalkylene, and 3- to 8-membered heterocyclylene can be optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-8}$ cycloalkyl, 3- to 8-membered heterocyclyl, $C_{6-10}$ aryl, and 5- to 10-membered heteroaryl;

$R_{a1}$, $R_{b1}$, and $R_{c1}$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

n1 is 0, 1, 2, 3, 4, or 5;
y is 0, 1, 2, 3, 4, or 5;
p is 0, 1, 2, 3, 4, or 5;
n is 0, 1, 2, 3, 4, or 5;
m is 0, 1, or 2; and
t is 0, 1, 2, 3, or 4;
provided that when ring B is

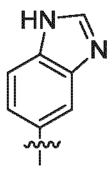, 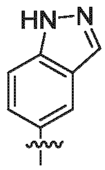, 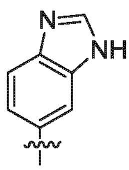, 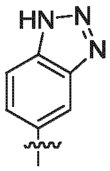, 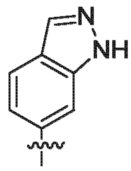, 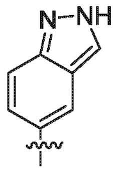,

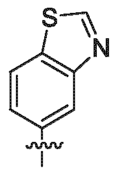, 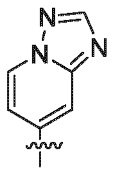, 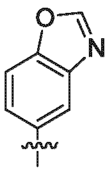, or 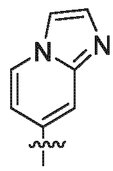,

$R_4$ is not hydrogen.

In a further preferred embodiment of the present invention, the compound or the stereoisomer or pharmaceutically acceptable salt thereof is further as represented by general formula (IV):

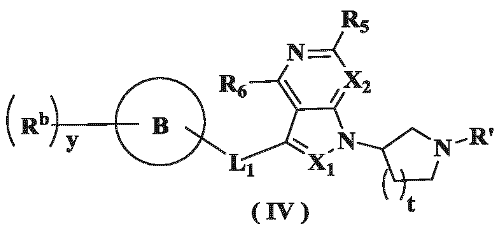

(IV)

wherein:

wherein $X_1$ is N or $CR_3$;
$X_2$ is N or $CR_4$;
$R_3$, $R_4$, $R_5$, and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, $C_{1-3}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;
preferably, $R_3$ and $R_4$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3-to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered

heterocyclyl, $C_{6-14}$ aryl, and 5- to 14-membered heteroaryl;

preferably, $R_5$ and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3-to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

R' is selected from deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, 5- to 14-membered heteroaryl, $-C(O)CR_a=CR_b(CH_2)_nR_c$, $-C(O)CR_a=CR_b(CH_2)_nNR_cR_d$, $-(CH_2)_nR_a$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

n is 0, 1, 2, 3, 4, or 5;

m is 0, 1, or 2; and

t is 0, 1, 2, 3, or 4.

In a preferred embodiment of the present invention, $L_1$ is selected from a bond, $C_{1-6}$ alkylene, $C_{2-6}$ alkenylene, $C_{2-6}$ alkynylene, $C_{3-12}$ cycloalkylene, 3- to 12-membered heterocyclylene, $C_{6-14}$ arylene, 6-14-membered heteroarylene, $-(C\equiv C)-$, $-(CR_{a1}=CR_{b1})_{n1}-$, $-(CR_{a1}R_{b1})_{n1}-$, $-O(CR_{a1}R_{b1})_{n1}-$, $-S(CR_{a1}R_{b1})_{n1}-$, $-(CR_{a1}R_{b1})_{n1}NR_{c1}-$, $-(CH_2)_{n1}C(O)NR_{a1}-$, $-(CH_2)_{n1}NR_{a1}C(O)-$, $-(CH_2)_{n1}S(O)_{m1}-$, $-(CH_2)_{n1}NR_{a1}S(O)_{m1}-$, or $-(CH_2)_{n1}S(O)_{m1}NR_{a1}-$, wherein the $C_{1-6}$ alkylene, $C_{2-6}$ alkenylene, $C_{1-6}$ alkynylene, $C_{3-12}$ cycloalkylene, 3- to 12-membered heterocyclylene, $C_{6-14}$ arylene, and 6-14-membered heteroarylene can be optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

In a further preferred embodiment of the present invention, $L_1$ is selected from a bond, $C_{1-3}$ alkylene, $C_{2-3}$ alkenylene, $C_{2-6}$ alkynylene, $C_{3-8}$ cycloalkylene, 3- to 8-membered heterocyclylene, $-(C=C)-$, $-(CR_{a1}=CR_{b1})_{n1}-$, $-O(CR_{a1}R_{b1})_{n1}-$, $-(CR_{a1}R_{b1})_{n1}NR_{c1}-$, or $-(CH_2)_{n1}C(O)NR_{a1}-$, wherein the $C_{1-3}$ alkylene, $C_{2-3}$ alkenylene, $C_{2-6}$ alkynylene, $C_{3-8}$ cycloalkylene, and 3- to 8-membered heterocyclylene can be optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-8}$ cycloalkyl, 3- to 8-membered heterocyclyl, $C_{6-10}$ aryl, and 5- to 10-membered heteroaryl;

$R_{a1}$, $R_{b1}$, and $R_{c1}$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

n1 is 0, 1, 2, 3, 4, or 5; and

m1 is 0, 1, or 2.

In certain embodiments of the present invention, $L_1$ is a bond, -(C≡C)-, -CH=CH-, -NHCO-, $-CH_2CH_2-$,

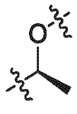, 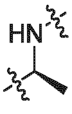, 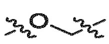, 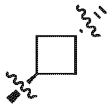, 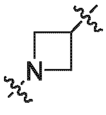, 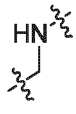, 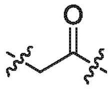, 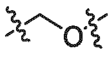, 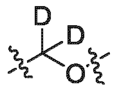,

or

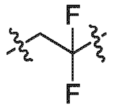,

and preferably, $L_1$ is -(C≡C)-.

In certain embodiments of the present invention, $L_1$ is a bond, -(C≡C)-, -CH=CH-, -NHCO-, $-CH_2CH_2-$,

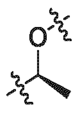, 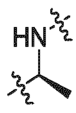, 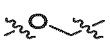, 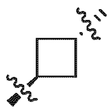, 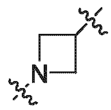, or 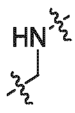,

and preferably, $L_1$ is -(C≡C)-.

In a preferred embodiment of the present invention, ring A is selected from $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, oxo, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl.

In a further preferred embodiment of the present invention, ring A is selected from 5-membered heteroaryl, 8- to 10-membered bicyclic nitrogen-containing heterocyclyl, or 8- to 10-membered bicyclic nitrogen-containing heteroaryl.

In a further preferred embodiment of the present invention, ring A is selected from 8- to 10-membered bicyclic nitrogen-containing heterocyclyl or 8- to 10-membered bicyclic nitrogen-containing heteroaryl.

In a further preferred embodiment of the present invention, ring A is selected from five-ring-fused five-membered nitrogen-containing heterocyclyl, five-ring-fused five-membered nitrogen-containing heteroaryl, five-ring-fused six-membered nitrogen-containing heterocyclyl, five-ring-fused six-membered nitrogen-containing heteroaryl, six-ring-fused six-membered nitrogen-containing heterocyclyl, or six-ring-fused six-membered nitrogen-containing heteroaryl.

In a further preferred embodiment of the present invention, ring A is selected from the following groups:

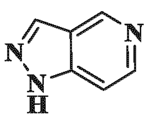, 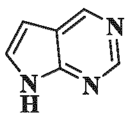, 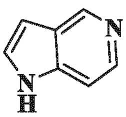, 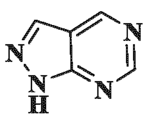, 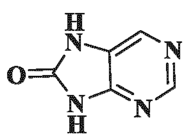,

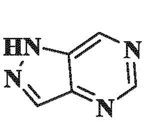, 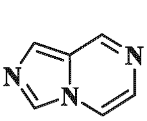, 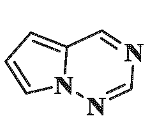, 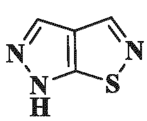, 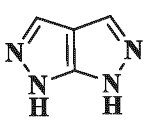, 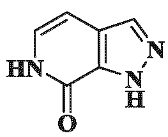,

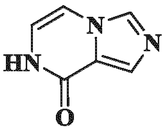, 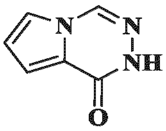, 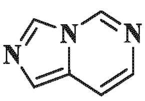, 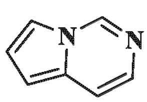, 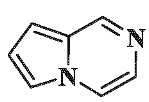, 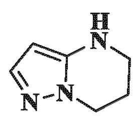,

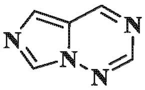, 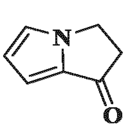, 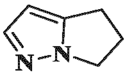, 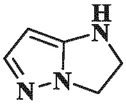, 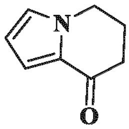, 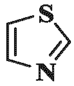, 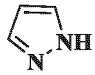, 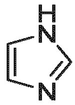, 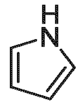,

and

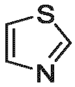

In a further preferred embodiment of the present invention, ring A is selected from the following groups:

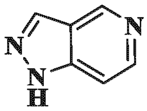, 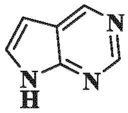, 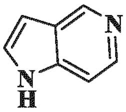, 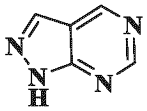, 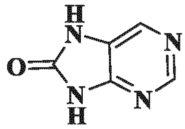,

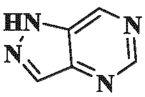, 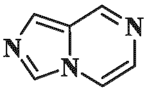, 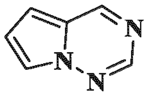, 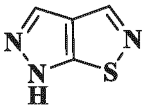, 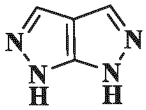, 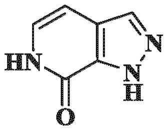,

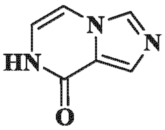, 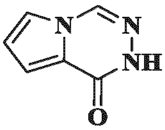, 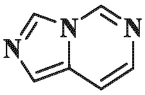, 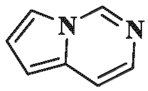, 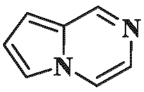, 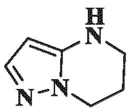,

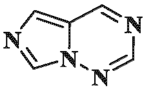, 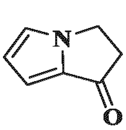, 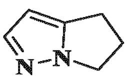, 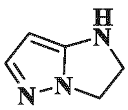, 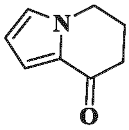, 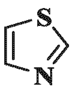, or 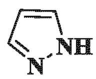

In certain embodiments of the present invention, ring A is selected from the following groups:

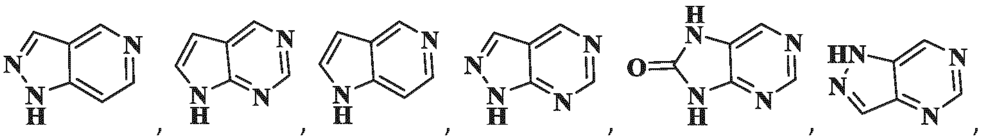

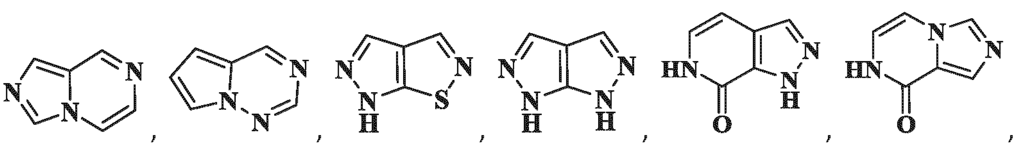

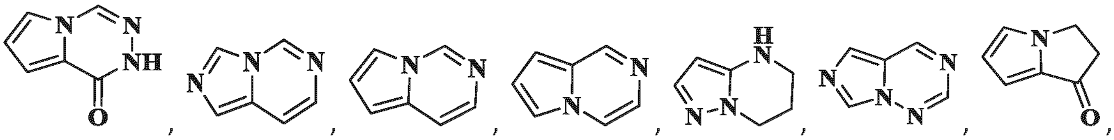

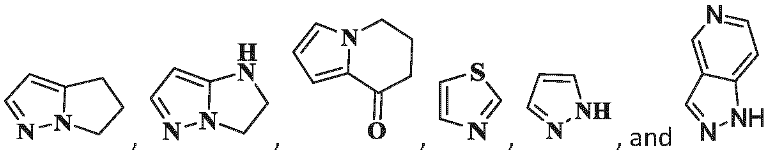

In a preferred embodiment of the present invention, ring B is selected from $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl.

In a further preferred embodiment of the present invention, ring B is selected from monocyclic aryl, 5- to 6-membered monocyclic heteroaryl, $C_{8-10}$ bicyclic aryl, or 8- to 10-membered bicyclic heteroaryl.

In a further preferred embodiment of the present invention, ring B is selected from phenyl, pyridyl, benzo 5- to 6-membered heterocyclyl, benzo 5- to 6-membered heteroaromatic ring group, pyrido 5- to 6-membered heterocyclyl, and pyrido 5- to 6-membered heteroaromatic ring group.

In certain embodiments of the present invention, ring B is selected from the following groups:

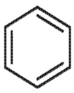, 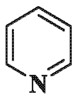, 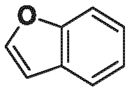, 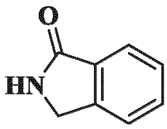, 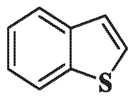, 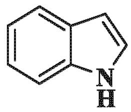, 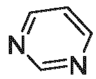, 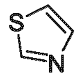,

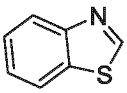, 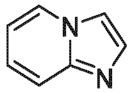, 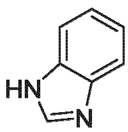, 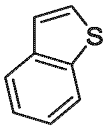, 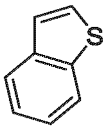, 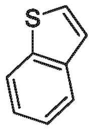, 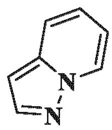, 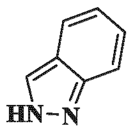,

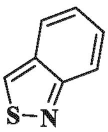, 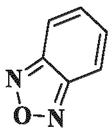, 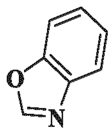, and 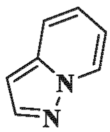

In a further preferred embodiment of the present invention, ring B is selected from the following groups:

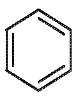, 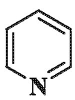, 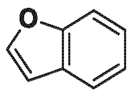, 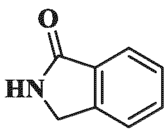, 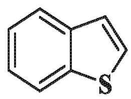, 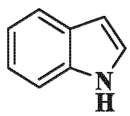, 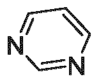,

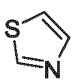, 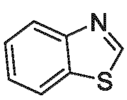, 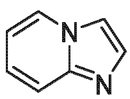, 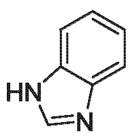, 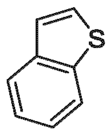, 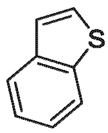, and 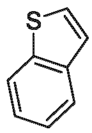

In a further preferred embodiment of the present invention, ring B is selected from the following groups:

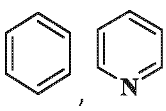, 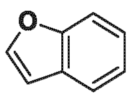, 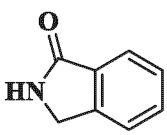, 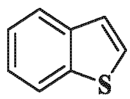, or 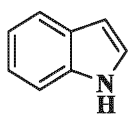

In a further preferred embodiment of the present invention, ring B is selected from the following groups:

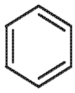, 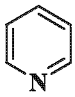, 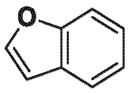, 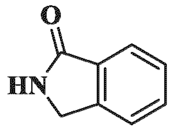, 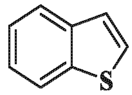, 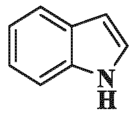, 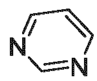,

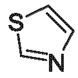, 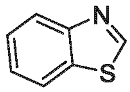, and 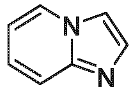

In a preferred embodiment of the present invention, $R^a$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclylcarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclylcarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, $C_{1-3}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5-to 14-membered heteroaryl.

In a preferred embodiment of the present invention, the $R^a$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

alternatively, any two $R^a$ are connected to form $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl.

In a preferred embodiment of the present invention, $R^a$ is selected from hydrogen, deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 3- to 8-membered heterocyclylcarbonyl, $C_{1-3}$ alkylamino, $C_{3-8}$ cycloalkyl, 3- to 8-membered heterocyclyl, $C_{6-10}$ aryl, or 5- to 10-membered heteroaryl, wherein the $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 3- to 8-membered heterocyclylcarbonyl, $C_{1-3}$ alkylamino, $C_{3-8}$ cycloalkyl, 3-to 8-membered heterocyclyl, $C_{6-10}$ aryl, and 5- to 10-membered heteroaryl are optionally further substituted with one or more substituents of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-8}$ cycloalkyl, 3- to 8-membered heterocyclyl, $C_{1-3}$ alkyl-substituted 3- to 8-membered heterocyclyl, $C_{6-10}$ aryl, and 5- to 10-membered heteroaryl.

In certain embodiments of the present invention, $R^a$ is independently selected from hydrogen, deuterium, halogen, amino, $C_{1-3}$ alkyl, $C_{1-3}$ alkoxy, $C_{1-3}$ haloalkyl, $C_{3-6}$ cycloalkyl, cyano, $C_{1-3}$ deuteroalkyl, $C_{1-3}$ haloalkoxy, $C_{1-3}$ haloalkyl, aminocarbonyl, or $C_{1-3}$ alkylamino, wherein the amino, $C_{1-3}$ alkyl, $C_{1-3}$ deuteroalkyl, $C_{1-3}$ haloalkyl, $C_{1-3}$ alkoxy, $C_{1-3}$ haloalkoxy, aminocarbonyl, $C_{1-3}$ alkylamino, $C_{1-3}$ haloalkyl, $C_{1-3}$ alkylamino, and $C_{3-6}$ cycloalkyl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl.

In certain embodiments of the present invention, $R^a$ is independently selected from hydrogen, deuterium, fluorine, chlorine, bromine, iodine, amino, cyano, methyl, ethyl, 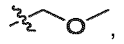, methoxy, trifluoromethoxy, cyclopropyl, trifluoromethyl, methylamino, cyclopropylamino, aminocarbonyl, cyclopropylmethyl,

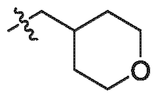,

isopropyl,

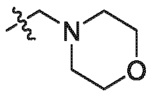, 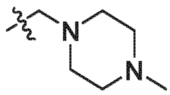,

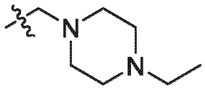, 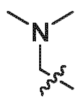, 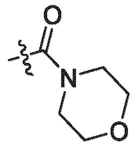,

$-CH_2CN$, $-CH_2OH$, $-COCH_3$,

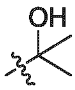, 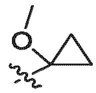, 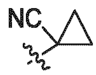,

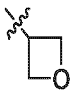, 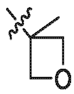,

$-CH_2CH_2OH$, $-CH_2CH_2OCH_3$, and $-CH_2OCH_2CH_3$.

In certain embodiments of the present invention, $R^a$ is independently selected from hydrogen, deuterium, fluorine, chlorine, bromine, iodine, amino, cyano, methyl, ethyl,

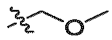,

methoxy, trifluoromethoxy, cyclopropyl, trifluoromethyl, methylamino, cyclopropylamino, and aminocarbonyl.

In a preferred embodiment of the present invention, the $R^b$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl, $-O(CR_{a2}R_{b2})_{n2}R_{c2}$, $-S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}C(O)R_{c2}$, $-(CH_2)_{n2}S(O)_{m2}R_{c2}$, $-(CH_2)_{n2}NR_{a2}S(O)_{m2}R_{c2}$, or $-(CH_2)_{n2}S(O)_{m2}NR_{a2}R_{c2}$ are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

alternatively, any two $R^b$ are connected to form $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl.

In certain embodiments of the present invention, the $R^b$ is independently selected from hydrogen, deuterium, halogen, cyano, $C_{1-3}$ alkoxy, $C_{1-3}$ alkyl, $-O(CR_{a2}R_{b2})_{n2}R_{c2}$, and aminocarbonyl, wherein the $C_{1-3}$ alkoxy, $C_{1-3}$ alkyl, and aminocarbonyl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl;

alternatively, any two $R^b$ are connected to form $C_{3-6}$ cycloalkyl, 3- to 6-membered heterocyclyl, $C_{6-10}$ aryl, or 5- to 6-membered heteroaryl, wherein the $C_{3-6}$ cycloalkyl, 3- to 6-membered heterocyclyl, $C_{6-10}$ aryl, and 5- to 6-membered heteroaryl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl.

In certain embodiments of the present invention, the $R^b$ is independently selected from hydrogen, deuterium, fluorine, chlorine, methoxy, cyano, methyl,

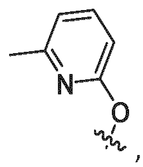

and

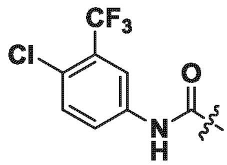

In certain embodiments of the present invention, $R_3$, $R_4$, $R_5$, and $R_6$ are each independently selected from hydrogen, deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 3- to 8-membered heterocyclyl-carbonyl, $C_{1-3}$ alkylamino, $C_{3-6}$ cycloalkyl, 3- to 8-membered heterocyclyl, $C_{6-10}$ aryl, or 5- to 10-membered heteroaryl, wherein the $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 3- to 8-membered heterocyclyl-carbonyl, $C_{1-3}$ alkylamino, $C_{3-6}$ cycloalkyl, 3- to 8-membered heterocyclyl, $C_{6-10}$ aryl, and 5- to 10-membered heteroaryl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 8-membered heterocyclyl, $C_{1-3}$ alkyl-substituted 3- to 8-membered heterocyclyl, $C_{6-10}$ aryl, and 5-to 10-membered heteroaryl.

In certain embodiments of the present invention, $R_3$, $R_4$, $R_5$, and $R_6$ are each independently selected from hydrogen, deuterium, halogen, amino, hydroxyl, cyano, aldehyde group, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{1-4}$ alkylcarbonyl, aminocarbonyl, $C_{1-4}$ alkylaminocarbonyl, $C_{1-4}$ alkylcarbonylamino, 3- to 8-membered cycloalkyl-carbonyl, 3- to 8-membered heterocyclyl-carbonyl, $C_{1-4}$ alkylamino, amino $C_{1-4}$ alkyl, $C_{3-8}$ cycloalkyl, 3- to 8-membered heterocyclyl, $C_{6-10}$ aryl, or 5- to 10-membered heteroaryl, wherein the amino, aldehyde group, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{1-4}$ alkylcarbonyl, aminocarbonyl, $C_{1-4}$ alkylaminocarbonyl, $C_{1-4}$ alkylcarbonylamino, 3- to 8-membered heterocyclyl-carbonyl, $C_{1-4}$ alkylamino, amino $C_{1-4}$ alkyl, $C_{3-8}$ cycloalkyl, 3- to 8-membered heterocyclyl, $C_{6-10}$ aryl, and 5- to 10-membered heteroaryl are optionally further substituted with one or more R‴; wherein the R‴ is selected from deuterium, halogen, amino, $C_{1-4}$ alkyl-substituted amino, hydroxyl, cyano, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{3-8}$ cycloalkyl, 3- to 8-membered heterocyclyl, $C_{1-4}$ alkyl-substituted 3- to 8-membered heterocyclyl, $C_{6-10}$ aryl, and 5- to 10-membered heteroaryl, optionally substituted with one or more substituents of deuterium, halogen, amino, $C_{1-4}$ alkyl-substituted amino, hydroxyl, cyano, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{3-8}$ cycloalkyl, 3- to 8-membered heterocyclyl, $C_{1-4}$ alkyl-substituted 3-to 8-membered heterocyclyl, $C_{6-10}$ aryl, and 5- to 10-membered heteroaryl.

In certain embodiments of the present invention, $R_3$, $R_4$, $R_5$, and $R_6$ are each independently selected from hydrogen, deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 4- to 6-membered heterocyclyl-carbonyl containing 1-3 atoms selected from N, O, or S, $C_{1-3}$ alkylamino, $C_{3-6}$ cycloalkyl, 4- to 6-membered heterocyclyl containing 1-3 atoms selected from N, O, or S, $C_{6-10}$ aryl, or 5- to 6-membered heteroaryl containing 1-3 atoms selected from N, O, or S, wherein the $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 4- to 6-membered heterocyclyl-carbonyl containing 1-3 atoms selected from N,

O, or S, $C_{1-3}$ alkylamino, $C_{3-6}$ cycloalkyl, 4- to 6-membered heterocyclyl containing 1-3 atoms selected from N, O, or S, $C_{6-10}$ aryl, and 5- to 6-membered heteroaryl containing 1-3 atoms selected from N, O, or S are optionally further substituted with one or more substituents of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 8-membered heterocyclyl, $C_{1-3}$ alkyl-substituted 3- to 8-membered heterocyclyl, $C_{6-10}$ aryl, and 5-to 10-membered heteroaryl.

In certain embodiments of the present invention, $R_3$, $R_4$, $R_5$, and $R_6$ are each independently selected from hydrogen, deuterium, halogen, amino, $C_{1-3}$ alkyl, $C_{1-3}$ alkoxy, $C_{1-3}$ haloalkyl, $C_{3-6}$ cycloalkyl, cyano, $C_{1-3}$ deuteroalkyl, $C_{1-3}$ haloalkoxy, $C_{1-3}$ haloalkyl, aminocarbonyl, or $C_{1-3}$ alkylamino, wherein the amino, $C_{1-3}$ alkyl, $C_{1-3}$ deuteroalkyl, $C_{1-3}$ haloalkyl, $C_{1-3}$ alkoxy, $C_{1-3}$ haloalkoxy, aminocarbonyl, $C_{1-3}$ alkylamino, $C_{1-3}$ haloalkyl, $C_{1-3}$ alkylamino, and $C_{3-6}$ cycloalkyl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl.

In certain embodiments of the present invention, $R_3$, $R_4$, $R_5$, and $R_6$ are each independently selected from hydrogen, deuterium, fluorine, chlorine, bromine, iodine, amino, cyano, methyl, ethyl,

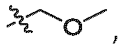,

methoxy, trifluoromethoxy, cyclopropyl, trifluoromethyl, methylamino, cyclopropylamino, aminocarbonyl, cyclopropylmethyl,

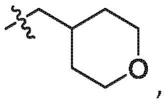,

isopropyl,

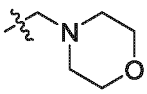, 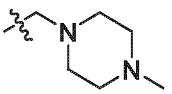, 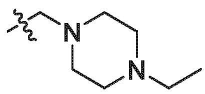, 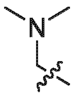, 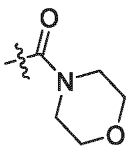,

$-CH_2CN$, $-CH_2OH$, $-COCH_3$,

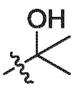, 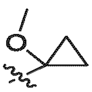, 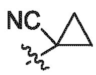, 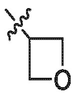, 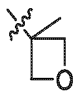,

$-CH_2CH_2OH$, $-CH_2CH_2OCH_3$, $-CH_2OCH_2CH_3$,

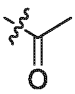,

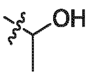, 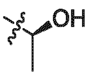, 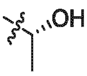, 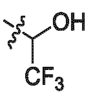, 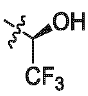, 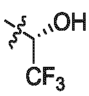, 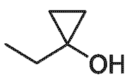, 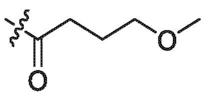,

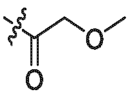, 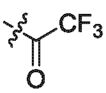, 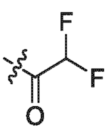, 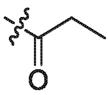, 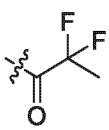, 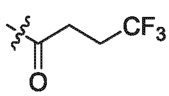, 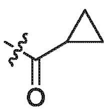, 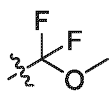,

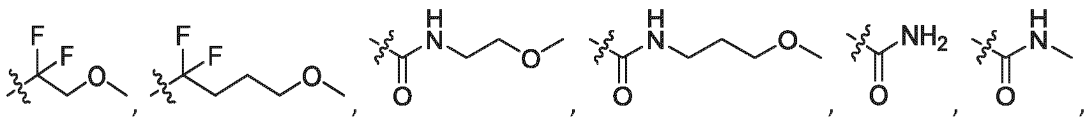

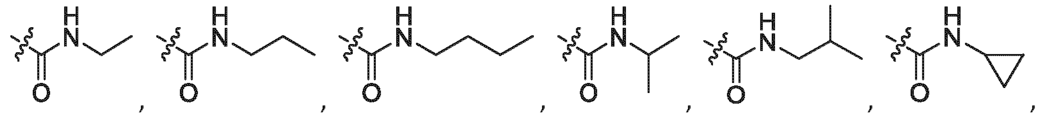

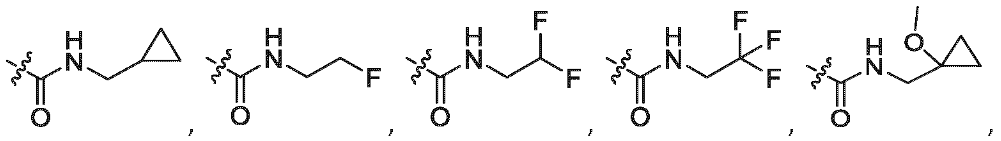

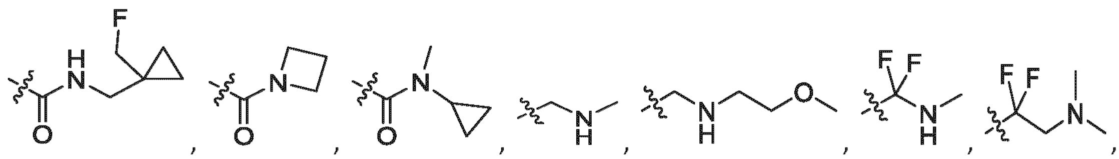

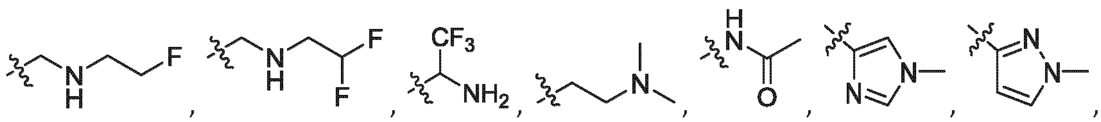

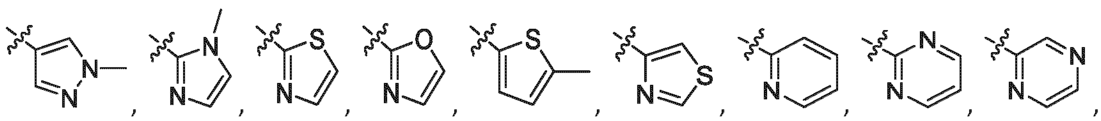

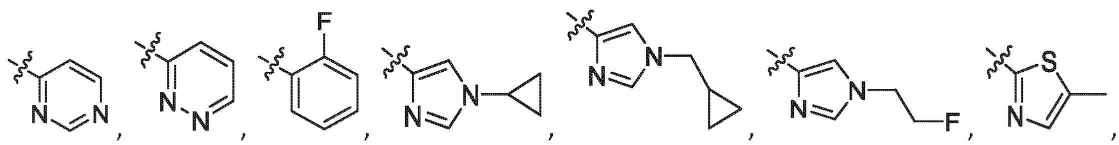

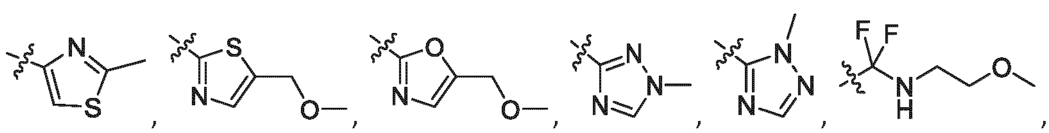

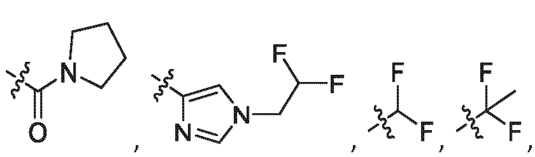 and 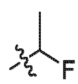

In certain embodiments of the present invention, $R_3$, $R_4$, $R_5$, and $R_6$ are each independently selected from hydrogen, deuterium, fluorine, chlorine, bromine, iodine, amino, cyano, methyl, ethyl,

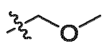,

methoxy, trifluoromethoxy, cyclopropyl, trifluoromethyl, methylamino, cyclopropylamino, aminocarbonyl, cyclopropylmethyl,

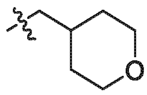,

isopropyl,

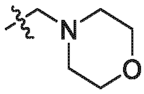, 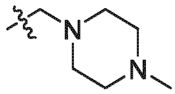, 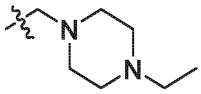, 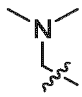, 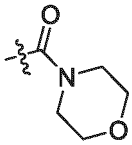,

$-CH_2CN$, $-CH_2OH$, $-COCH_3$,

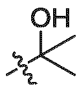, 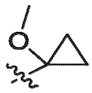, 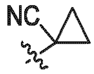, 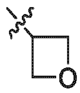, 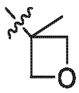,

$-CH_2CH_2OH$, $-CH_2CH_2OCH_3$, and $-CH_2OCH_2CH_3$.

In certain embodiments of the present invention, $R_3$, $R_4$, $R_5$, and $R_6$ are each independently selected from hydrogen, deuterium, fluorine, chlorine, bromine, iodine, amino, cyano, methyl, ethyl,

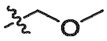,

methoxy, trifluoromethoxy, cyclopropyl, trifluoromethyl, methylamino, cyclopropylamino, and aminocarbonyl.

In certain embodiments of the present invention, the $R_1$ is independently selected from hydrogen, deuterium, halogen, cyano, $C_{1-3}$ alkoxy, $C_{1-3}$ alkyl,

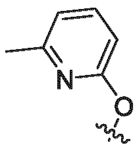,

and aminocarbonyl, wherein the $C_{1-3}$ alkoxy, $C_{1-3}$ alkyl, and aminocarbonyl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl.

In certain embodiments of the present invention, $R_1$ is independently selected from hydrogen, deuterium, fluorine, chlorine, methoxy, cyano, methyl,

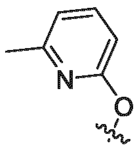,

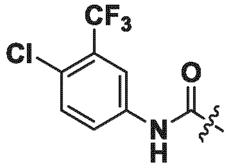, and 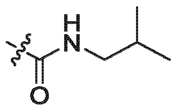

In certain embodiments of the present invention, $R_1$ is independently selected from hydrogen, deuterium, fluorine, chlorine, methoxy, cyano, methyl,

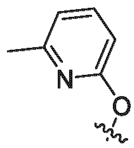

,

and

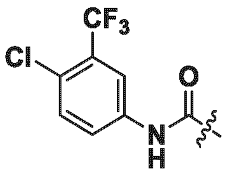

In certain embodiments of the present invention, the R is hydrogen, deuterium, halogen, amino, $C_{1-6}$ alkyl, $C_{1-6}$ deuteroalkyl, $C_{1-6}$ haloalkyl, $C_{1-6}$ hydroxyalkyl, $C_{1-6}$ alkoxy, $C_{1-6}$ haloalkoxy, 3-to 12-membered heterocyclyl $C_{1-6}$ alkylene, $C_{2-6}$ alkenyl, $C_{2-6}$ alkynyl, $C_{3-12}$ cycloalkyl, 3- to 12-membered heterocyclyl, $C_{6-14}$ aryl, 5- to 14-membered heteroaryl, $-(CH_2)_nNR_{a2}C(O)CR_{b2}=CR_{c2}$, - $O(CR_{a2}R_{b2})_{n2}R_{c2}$, $-S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}C(O)R_{c2}$, - $(CH_2)_{n2}S(O)_{m2}R_{c2}$, $-(CH_2)_{n2}NR_{a2}S(O)_{m2}R_{c2}$, or $-(CH_2)_{n2}S(O)_{m2}NR_{a2}R_{c2}$, wherein the amino, $C_{1-6}$ alkyl, $C_{1-6}$ deuteroalkyl, $C_{1-6}$ haloalkyl, $C_{1-6}$ hydroxyalkyl, $C_{1-6}$ alkoxy, $C_{1-6}$ haloalkoxy, $C_{2-6}$ alkenyl, $C_{2-6}$ alkynyl, $C_{3-12}$ cycloalkyl, 3-12-membered heterocyclyl, $C_{6-14}$ aryl, and 5- to 14-membered heteroaryl can be optionally further substituted with one or more R'; R' is as described above.

In certain embodiments of the present invention, the R is

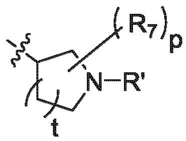

;

preferably, the R is

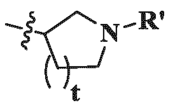

,

wherein $R_7$, R', and t are as described as above.

In certain embodiments of the present invention, R' is selected from deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, 5- to 6-membered heteroaryl, $-C(O)CR_a=CR_b(CH_2)_nR_c$, - $C(O)CR_a=CR_b(CH_2)_nNR_cR_d$, $-C(O)C{\equiv}C(CH_2)_nR_c$, $-(CH_2)_nR_a$, $-(CH_2)_nNR_aOR_b$, $-(CH_2)_nC(O)NR_aR_b$, - $(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or$-(CH_2)_nNR_a(CH_2)_{n2}R_b$, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, or 5- to 6-membered heteroaryl, wherein the $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl;

n is 0, 1, 2, 3, 4, or 5;
m is 0, 1, or 2; and
t is 0, 1, 2, 3, or 4.

In certain embodiments of the present invention, $R_7$ is independently selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, $C_{3-6}$ cycloalkyl, 3- to 6-membered heterocyclyl, $C_{6-10}$ aryl, or 5- to 10-membered heteroaryl, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, $C_{3-6}$ cycloalkyl, 3- to 6-membered heterocyclyl, $C_{6-10}$ aryl, and 5- to 10-membered heteroaryl are optionally further substituted with one or more substituents of deuterium, halogen, amino, hydroxyl, cyano, $C_{1-3}$ alkyl, $C_{2-4}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 10-membered heteroaryl.

In certain embodiments of the present invention, the R is

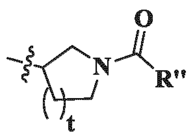

, \

wherein R'' is $-CR_a=CR_b(CH_2)_nR_c$, $-C\equiv C(CH_2)_nR_c$, or - $CR_a=CR_b(CH_2)_nNR_cR_d$, wherein n, $R_b$, $R_c$, and $R_d$ are as described above. Further preferably, R'' is selected from

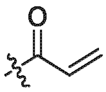, 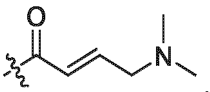,

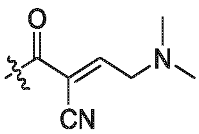, 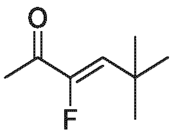, 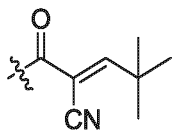, 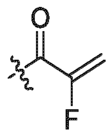, 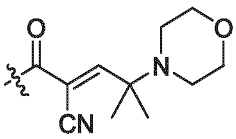,

and

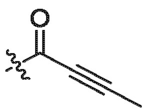;

t is preferably 0 or 1.

In certain embodiments of the present invention, the R is

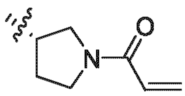,

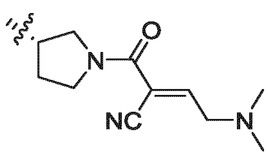, 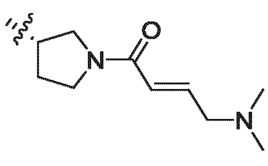, 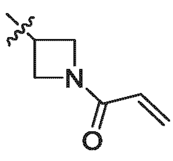, 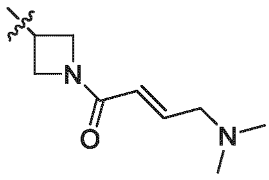,

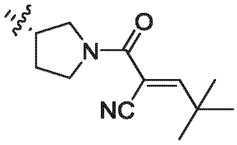, 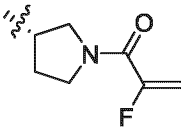, 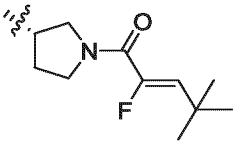, 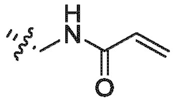, 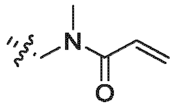,

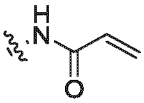, 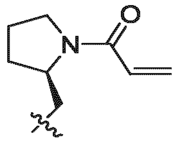, 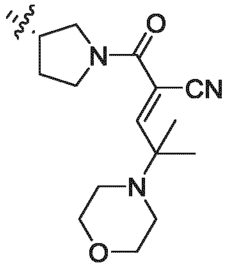, 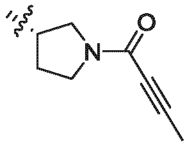, 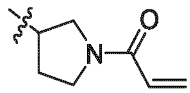,

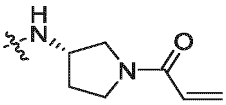, 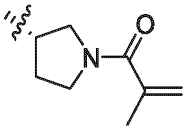, 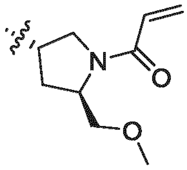, and 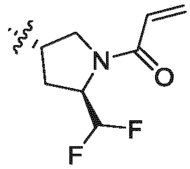

In certain embodiments of the present invention, the R is

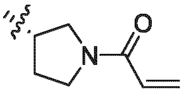,

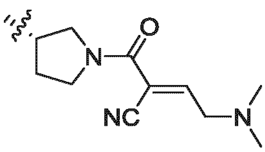, 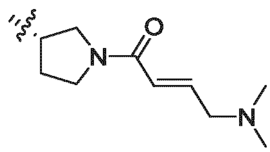, 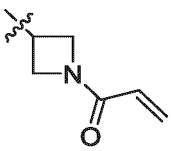, 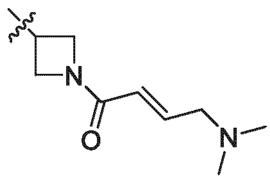,

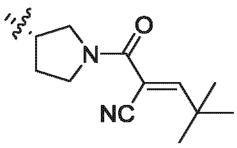, 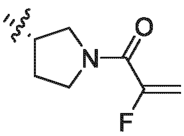, 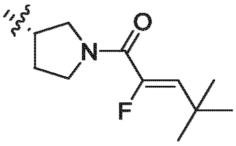, 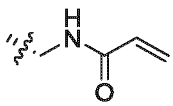, 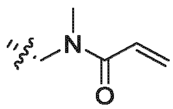,

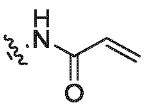, 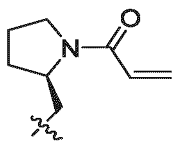, 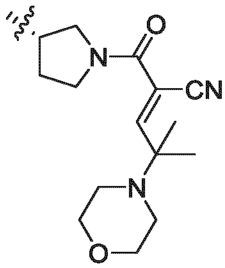, 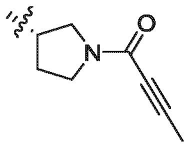, 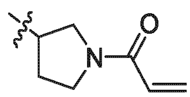,

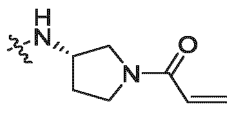, and 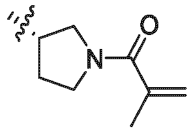

In certain embodiments of the present invention, the compound is further as represented by general formula (II-3):

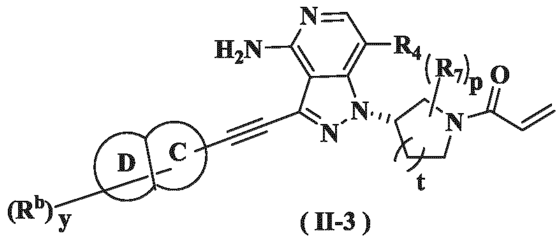

(II-3)

wherein

ring C is phenyl or 6-membered heteroaryl;
ring D is five-membered heteroaryl;
$R_4$ is selected from deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl; preferably, $R_4$ is selected from deuterium, halogen, amino, hydroxyl, cyano, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, or 5- to 10-membered heteroaryl, wherein the amino, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, and 5- to 10-membered heteroaryl are optionally further substituted with one or more of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, and $C_{3-6}$ cycloalkyl; more preferably, $R_4$ is selected from aldehyde group, $C_{1-3}$ alkylcarbonyl, $C_{1-3}$ alkyl, $C_{3-6}$ cycloalkyl, halogen, 5- to 6-membered heteroaryl containing 1-3 atoms selected from N, O, or S, or $C_{1-3}$ alkyl-substituted 5- to 6-membered heteroaryl containing 1-3 atoms selected from N, O, or S, wherein optionally, the aldehyde group is further substituted with one or more of $C_{1-6}$ alkyl and $C_{3-6}$ cycloalkyl; more further preferably, $R_4$ is selected from methyl, ethyl, cyclopropyl, chlorine, methylcarbonyl, isopropylcarbonyl, cyclobutylcarbonyl, ethylcarbonyl, cyclopropylcarbonyl, tert-butylcarbonyl,

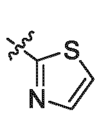, 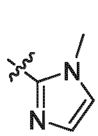, 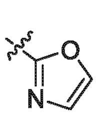, 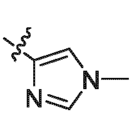, 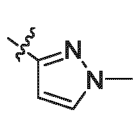, 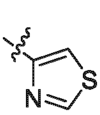, 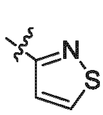,

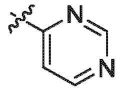, or 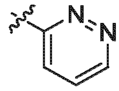;

$R_7$ is independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, and $C_{1-6}$ alkylamino are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, and $C_{1-6}$ haloalkoxy; preferably, $R_7$ is independently selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{3-3}$ alkylamino, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, and $C_{1-3}$ alkylamino are optionally further substituted with one or more substituents of deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, and $C_{1-3}$ haloalkoxy;
more preferably, $R_7$ is independently selected from hydrogen, $-CH_2OCH_3$, or $-CHF_2$;
$R^b$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl;
p is 0, 1, 2, 3, 4, or 5;
t is 0, 1, 2, or 3.

In a further preferred embodiment of the present invention, the compound is further as represented by general formula (VII-1), (VII-2), (VII-3), (VII-4), (VII-5), (VII-6), or (VII-7):

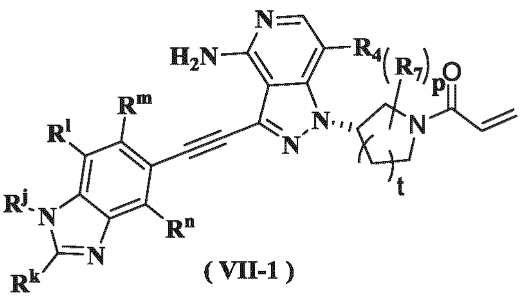
( VII-1 )

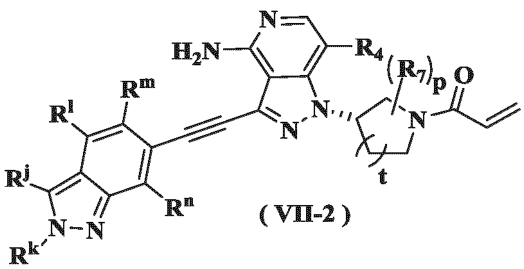
( VII-2 )

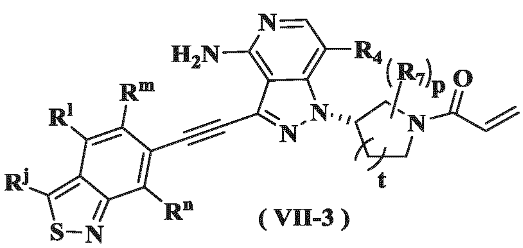
( VII-3 )

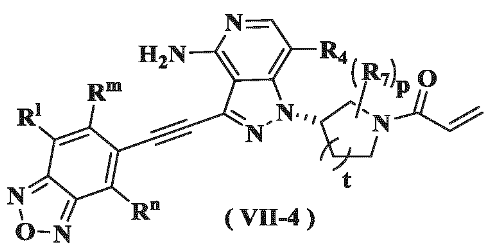
( VII-4 )

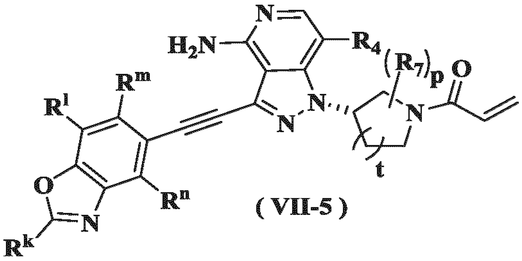
( VII-5 )

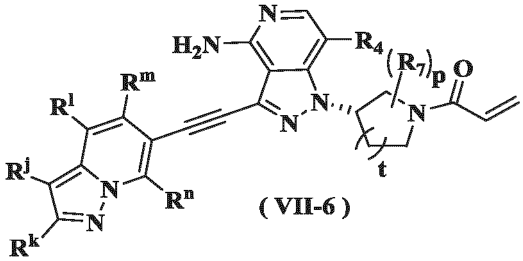
( VII-6 )

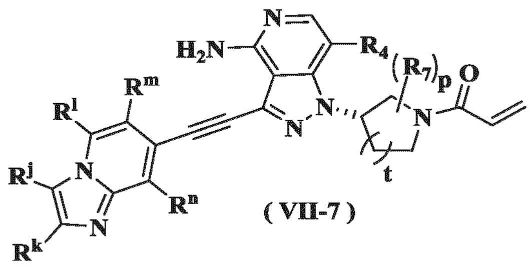

( VII-7 )

wherein

$R_4$ is selected from deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl; preferably, $R_4$ is selected from deuterium, halogen, amino, hydroxyl, cyano, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, or 5- to 10-membered heteroaryl, wherein the amino, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, and 5- to 10-membered heteroaryl are optionally further substituted with one or more of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, and $C_{3-6}$ cycloalkyl; more preferably, $R_4$ is selected from aldehyde group, $C_{1-3}$ alkylcarbonyl, $C_{1-3}$ alkyl, $C_{3-6}$ cycloalkyl, halogen, 5- to 6-membered heteroaryl containing 1-3 atoms selected from N, O, or S, or $C_{1-3}$ alkyl-substituted 5- to 6-membered heteroaryl containing 1-3 atoms selected from N, O, or S, wherein optionally, the aldehyde group is further substituted with one or more of $C_{1-6}$ alkyl and $C_{3-6}$ cycloalkyl; more further preferably, $R_4$ is selected from $C_{1-3}$ alkylcarbonyl, $C_{1-3}$ alkyl, $C_{3-6}$ cycloalkyl, halogen, 5- to 6-membered heteroaryl containing 1-3 atoms selected from N, O, or S, or $C_{1-3}$ alkyl-substituted 5- to 6-membered heteroaryl containing 1-3 atoms selected from N, O, or S; still more further preferably, $R_4$ is selected from methyl, ethyl, cyclopropyl, chlorine, methylcarbonyl, isopropylcarbonyl, cyclobutylcarbonyl, ethylcarbonyl, cyclopropylcarbonyl, tert-butylcarbonyl,

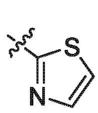, 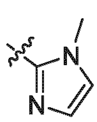, 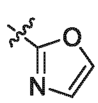, 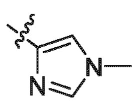, 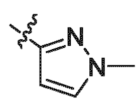, 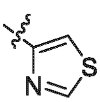, 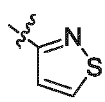,

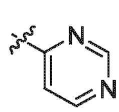, or 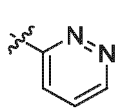;

or still yet more further preferably, $R_4$ is selected from methyl, ethyl, cyclopropyl, chlorine, methylcarbonyl,

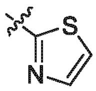, 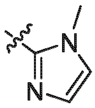, 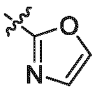, 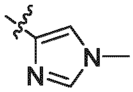, 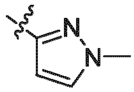,

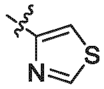, 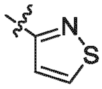, 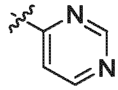, or 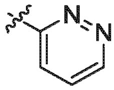;

$R_7$ is independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, and $C_{1-6}$ alkylamino are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, and $C_{1-6}$ haloalkoxy; preferably, $R_7$ is independently selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{3-3}$ alkylamino, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, and $C_{1-3}$ alkylamino are optionally further substituted with one or more substituents of deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, and $C_{1-3}$ haloalkoxy; more preferably, $R_7$ is independently selected from hydrogen, $-CH_2OCH_3$, or $-CHF_2$;

$R^j$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl; preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, or $C_{3-6}$ cycloalkyl; further preferably selected from hydrogen, deuterium, halogen, $C_{1-3}$ alkyl, $C_{1-3}$ deuteroalkyl, or $C_{3-6}$ cycloalkyl; more further preferably selected from hydrogen, chlorine, methyl, ethyl, deuteromethyl, or cyclopropyl; still more further preferably selected from methyl, ethyl, deuteromethyl, or cyclopropyl;

$R^m$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino; preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{1-3}$ alkylamino; further preferably selected from hydrogen, deuterium, or halogen; more further preferably fluorine;

$R^n$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino; preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{1-3}$ alkylamino; further preferably selected from hydrogen, deuterium, or halogen; more further preferably fluorine;

$R^k$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl; preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, or $C_{3-6}$ cycloalkyl; further preferably selected from hydrogen, deuterium, halogen, $C_{1-3}$ alkyl, $C_{1-3}$ deuteroalkyl, or $C_{3-6}$ cycloalkyl; more further preferably hydrogen, methyl, ethyl, or cyclopropyl; still more further preferably cyclopropyl;

$R^l$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl; preferably hydrogen;

p is 0, 1, 2, 3, 4, or 5;

t is 0, 1, 2, or 3.

In certain embodiments of the present invention, $R_4$ is selected from acetyl or propionyl.

In certain embodiments of the present invention, $R_4$ is acetyl.

In certain embodiments of the present invention, $R_4$ is propionyl.

The present invention further provides a method for preparing the compound represented by general formula (!!-3) above. The method is method ! or method II:

method I: the method comprises the following steps:

step I: under acidic conditions, a compound represented by formula (II-3-1) is subjected to a deprotection reaction as shown below;
step II: in an organic solvent, under alkaline conditions, a compound represented by formula (II-3-2) obtained in step I and acryloyl chloride are subjected to a condensation reaction,

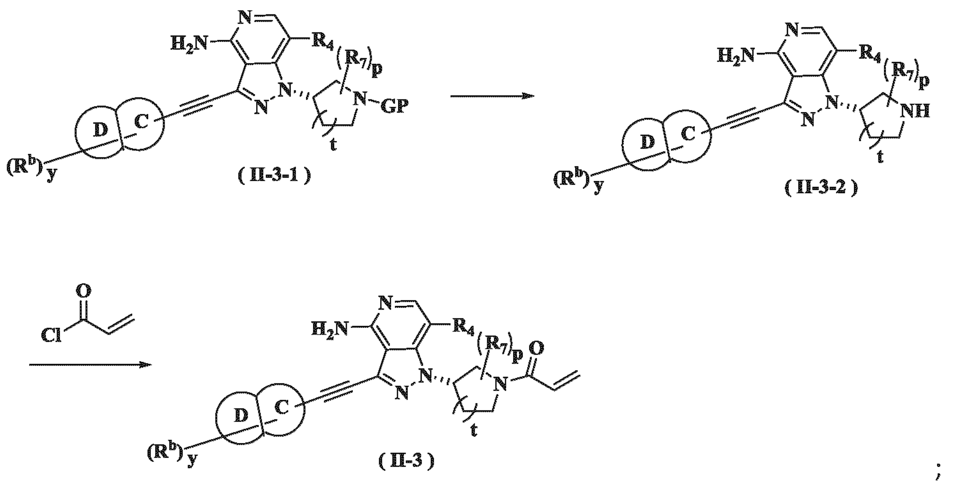

(II-3-1) (II-3-2) (II-3)

;

method II comprises the following steps:

step I: under acidic conditions, a compound represented by formula (II-3-3) is subjected to a reaction as shown below to obtain a compound represented by formula (II-3-4);
step II: in an organic solvent, under alkaline conditions, the compound represented by formula (II-3-4) obtained in step I and acryloyl chloride are subjected to a condensation reaction,

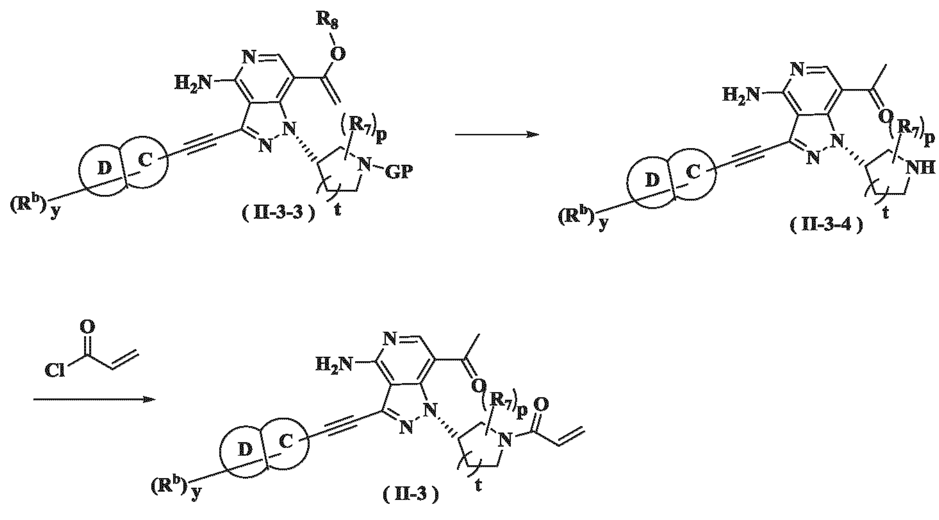

(II-3-3) (II-3-4) (II-3)

wherein GP is an amino protecting group, preferably Boc;
$R_8$ is $C_{1-6}$ alkyl, preferably n-butyl.

The present invention further provides a compound represented by general formula (II-3-1), (II-3-2), (II-3-3), or

(II-3-4), or a stereoisomer or pharmaceutically acceptable salt thereof,

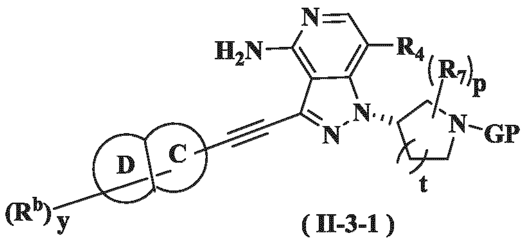

( II-3-1 )

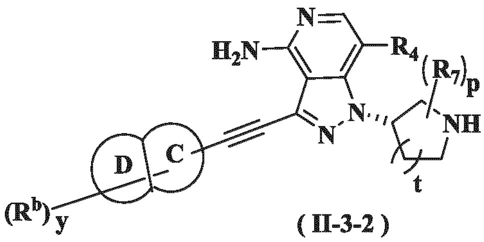

( II-3-2 )

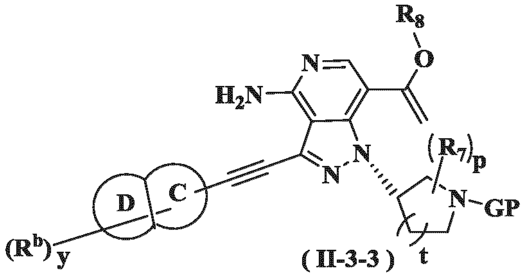

( II-3-3 )

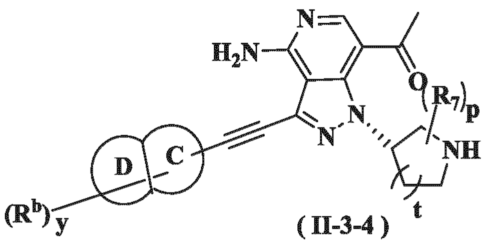

( II-3-4 )

wherein ring C, ring D, $R^b$, $R_4$, y, t, $R_7$, p, $R_8$, and GP are as defined above.

The present invention further relates to a pharmaceutical composition comprising a therapeutically effective dose of the above compound or the stereoisomer or pharmaceutically acceptable salt thereof, and one or more pharmaceutically acceptable carriers, diluents, or excipients. Furthermore, the weight percentage of the compound or the stereoisomer or pharmaceutically acceptable salt thereof in the composition is 0.1-95%, preferably 0.5-85%, more preferably 1-60%, further preferably 10-50%, more further preferably 15-40%, more further preferably 20-30%, and more further preferably 20-25% (based on the total weight of the pharmaceutical composition). In another aspect, the object of the present invention is also to provide the use of the above compound, the stereoisomer or pharmaceutically acceptable salt thereof, or the pharmaceutical composition thereof in the preparation of a drug for treating and/or preventing FGFR inhibitor-related diseases.

In another aspect, the object of the present invention is also to provide the use of the above compound, the stereoisomer or pharmaceutically acceptable salt thereof, or the pharmaceutical composition thereof in the preparation of a drug for treating and/or preventing FGFR1-4 kinase inhibitor-related diseases.

In another aspect, the object of the present invention is also to provide the use of the above compound, a stereoisomer or pharmaceutically acceptable salt thereof, or a pharmaceutical composition thereof in the preparation of a drug for treating and/or preventing cancer, bone dysplasia, and other related diseases.

The present invention further relates to a method for treating and/or preventing cancer, achondroplasia, and other related diseases. In another aspect, the object of the present invention is also to provide the use of the above compound, a stereoisomer or pharmaceutically acceptable salt thereof, or a pharmaceutical composition thereof for treating and/or preventing cancer, achondroplasia, and other related diseases.

In the above technical solution, the related disease-cancer is selected from solid tumor, colorectal cancer, bladder cancer, gastric cancer, thyroid cancer, esophageal cancer, head and neck cancer, brain cancer, glioma, glioblastoma, hepatocellular carcinoma, lung cancer, melanoma, myeloma, pancreatic cancer, renal cell cancer, cervical cancer, urothelial cancer, prostate cancer, ovarian cancer, breast cancer, leukemia, or lymphoma.

After structural optimization, the above compound of each general formula, a stereoisomer or pharmaceutically acceptable salt thereof, or a pharmaceutical composition thereof, as described in the present invention, has a non-hydrogen substituent added at a specific position of the mother nucleus, which can greatly decrease the enzyme activity of FGFR2 V564F and improve the inhibitory activity against resistant mutations in cells, etc., thereby solving the problems of current drugs on the market showing V564F resistance and there being no cure.

## Detailed Description of the Invention

Unless stated to the contrary, the terms used in the specification and claims have the following meanings.

The term "alkyl" refers to a saturated aliphatic hydrocarbon group, which is a linear or branched chain group containing 1 to 20 carbon atoms, preferably alkyl containing 1 to 12 carbon atoms, more preferably alkyl containing 1 to 8 carbon atoms, further preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl,

1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 4-heptyl, 1-propylbutyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, their respective branched chain isomers, etc. Lower alkyl groups containing 1 to 6 carbon atoms are more preferred, and non-limiting examples thereof include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, 4-heptyl, 1-propylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituents may be at any available point of attachment. The substituents are preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, heterocycloalkoxy, cycloalkylthio, heterocycloalkylthio, oxo, carboxyl or carboxylate group, and methyl, ethyl, isopropyl, tert-butyl, haloalkyl, deuteroalkyl, alkoxy-substituted alkyl and hydroxy-substituted alkyl are preferred in the present invention.

The term "alkylene" refers to bivalent alkyl formed by further replacing one hydrogen atom of alkyl, for example: "methylene" refers to $-CH_2-$, "ethylene" refers to $-(CH_2)_2-$, "propylene" refers to $-(CH_2)_3-$, and "butylene" refers to $-(CH_2)_4-$, etc. The point of attachment of an alkylene chain to the rest of a molecule and to a group can be via one carbon or any two carbons in the chain. Alkylene may be substituted or unsubstituted, and when it is substituted, the substituents may be at any available point of attachment. The substituents are preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, heterocycloalkoxy, cycloalkylthio, heterocycloalkylthio, oxo, carboxyl or carboxylate group, and methyl, ethyl, isopropyl, tert-butyl, haloalkyl, deuteroalkyl, alkoxy-substituted alkyl and hydroxy-substituted alkyl are preferred in the present invention.

The term "alkenyl" refers to an alkyl group as defined above consisting of at least two carbon atoms and at least one carbon-carbon double bond, such as vinyl, 1-propenyl, 2-propenyl, 1-, 2- or 3 -butenyl, etc. Alkenyl can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "alkenylene" refers to bivalent alkenyl formed by further replacing one hydrogen atom of alkenyl, for example, vinylidene, propenylidene, and butenylidene. Alkenylene can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "alkynyl" refers to an alkyl group as defined above consisting of at least two carbon atoms and at least one carbon-carbon triple bond, e.g., ethynyl, propynyl, and 1-, 2- or 3-butynyl. Alkynyl can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "alkynylene" refers to a bivalent alkynyl group formed by further replacing one hydrogen atom of alkynyl, e.g., ethynylene, propynylene, and butynylene. Alkynylene can be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The term "cycloalkyl" refers to a saturated or partially unsaturated monocyclic or polycyclic cyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro, fused and bridged cycloalkyl, preferably cyclopropyl, cyclobutyl, cyclohexyl, cyclopentyl and cycloheptyl.

The term "spirocycloalkyl" refers to a 5- to 20-membered polycyclic group with monocyclic rings sharing one carbon atom (called a spiro atom). It may contain one or more double bonds, but no ring has a completely conjugated $\pi$ electron system. Preferably, it is 6- to 14-membered and more preferably 7- to 10-membered. According to the number of shared spiro atoms between rings, the spirocycloalkyl is divided into monospirocycloalkyl, bispirocycloalkyl or polyspirocycloalkyl, preferably monospirocycloalkyl and bispirocycloalkyl. More preferably, it is 3-membered/6-membered, 3-membered/5-membered, 4-membered/4-membered, 4-membered/5-membered, 4-membered/6-membered, 5-membered/5-membered or 5-membered/6-membered monospirocycloalkyl. Non-limiting examples of spirocycloalkyl include:

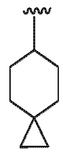 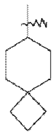 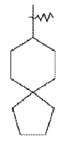 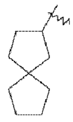 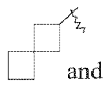 and 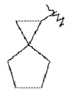,

etc.; and also included are spirocycloalkyl in which monospirocycloalkyl and heterocycloalkyl share a spiro atom. Non-limiting examples include:

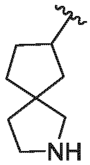 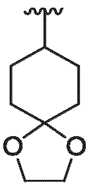 and 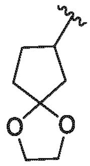,

etc.

The term "fused cycloalkyl" refers to a 5- to 20-membered all-carbon polycyclic group in which each ring in the system shares a pair of carbon atoms neighboring another ring in the system, one or more ring of which may contain one or more double bonds, but no ring has a fully conjugated π electron system. Preferably, it is 6- to 14-membered and more preferably 7-to 10-membered. According to the number of constituent rings, it can be divided into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include:

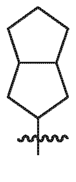 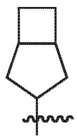 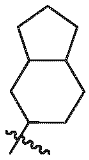 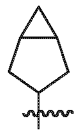 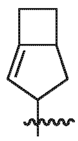 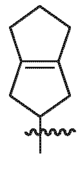 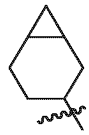 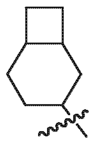, 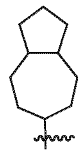,

etc.

The term "bridged cycloalkyl" refers to a 5- to 20-membered all-carbon polycyclic group with any two rings sharing two carbon atoms that are not directly connected. It may contain one or more double bonds, but no ring has a fully conjugated π electron system. Preferably, it is 6- to 14-membered and more preferably 7- to 10-membered. According to the number of constituent rings, it can be divided into bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

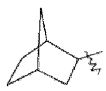 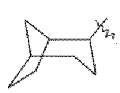 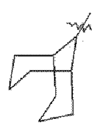 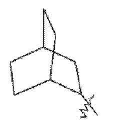 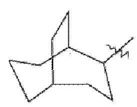

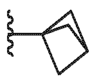 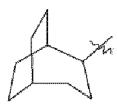 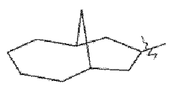 and 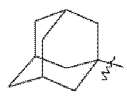

The cycloalkyl ring can be fused to an aryl, heteroaryl or heterocycloalkyl ring, where the ring connected to the parent structure is cycloalkyl, and non-limiting examples include indanyl, tetrahydronaphthyl, benzocycloheptanyl, and the like. Cycloalkyl may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group.

The term "cycloalkylene" refers to bivalent cycloalkyl formed by further replacing one hydrogen atom of cycloalkyl, wherein the cycloalkylene is optionally substituted or unsubstituted, and the cycloalkyl is defined as above.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen, C(O) or $S(O)_m$ (wherein m is an integer of 0 to 2), but excluding ring moieties of -O-O-, -O-S- or -S-S-, and the remaining ring atoms are carbon. Preferably, it comprises 3 to 12 ring atoms, of which 1-4 are heteroatoms; more preferably it contains 3 to 8 ring atoms; most preferably, it comprises 3 to 8 ring atoms; and further preferably, it is a 3- to 8-membered heterocyclyl containing 1-3 nitrogen atoms, optionally substituted with 1-2 oxygen atoms, sulfur atoms, or oxo, including nitrogen-containing monocyclic heterocyclyl, nitrogen-containing spirocyclic heterocyclyl or nitrogen-containing fused heterocyclyl.

Non-limiting examples of monocyclic heterocyclyl include oxetanyl, azetidinyl, thietanyl, pyrrolidinyl, imidazolidinyl, tetrahydrofuryl, tetrahydrothienyl, tetrahydropyranyl, dihydroimidazolyl, dihydrofuryl, dihydropyrazolyl, dihydropyrrolyl, piperidinyl, piperazinyl, morpholinyl, thiomorpholinyl, homopiperazinyl, azepanyl, 1,4-diazacycloheptyl, pyranyl or tetrahydrothiopyranyl dioxide group, *etc.*; preferably, oxetanyl, azetidinyl, thietanyl, tetrahydrofuryl, tetrahydropyranyl, tetrahydrothienyl, tetrahydrothiopyranyl, tetrahydrothiopyranyl dioxide group, pyrrolidinyl, morpholinyl, piperidinyl, piperazinyl, hexahydropyrazinyl, hexahydropyrimidinyl, azepanyl, 1,4-diazacycloheptyl and piperazinyl; More preferred are piperidinyl, piperazinyl, pyrrolidinyl, morpholinyl, oxetanyl, or azetidinyl. Polycyclic heterocyclyl includes spiro, fused and bridged heterocyclyl; the involved spiro, fused and bridged heterocyclyl are optionally connected to other groups through a single bond, or further fused to other cycloalkyl, heterocyclyl, aryl and heteroaryl through any two or more atoms on the ring.

The term "spiroheterocyclyl" refers to a 5- to 20-membered polycyclic heterocyclyl with monocyclic rings sharing one atom (called a spiro atom), wherein one or more ring atoms are heteroatoms selected from nitrogen, oxygen, or $S(O)_m$ (wherein m is an integer of 0 to 2), and the remaining ring atoms are carbon. Spiroheterocyclyl may contain one or more double bonds, but no ring has a fully conjugated $\pi$ electron system. Preferably, it is 6- to 14-membered and more preferably 7- to 10-membered. According to the number of shared spiro atoms between rings, the spiro heterocyclyl is divided into monospiroheterocyclyl, bispiroheterocyclyl or polyspiroheterocyclyl, preferably monospiroheterocyclyl and bispiroheterocyclyl. More preferably, it is 3-membered/5-membered, 3-membered/6-membered, 4-membered/4-membered, 4-membered/5-membered, 4-membered/6-membered, 5-membered/5-membered, or 5-membered/6-membered monospiro heterocyclyl. Non-limiting examples of spiro heterocyclyl include:

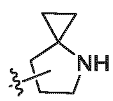 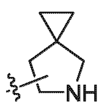 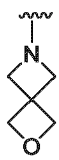 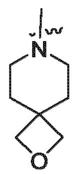 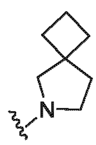 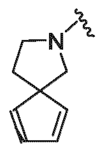 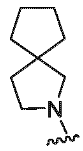 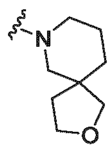

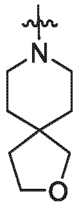 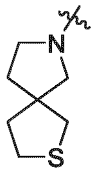 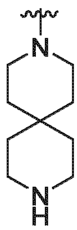 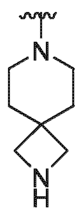 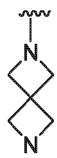 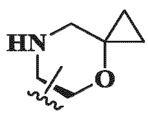

etc.

The term "fused heterocyclyl" refers to a 5- to 20-membered polycyclic heterocyclyl with each ring in the system sharing a pair of atoms neighboring other rings in the system, and one or more rings may contain one or more double bonds, but no ring has a fully conjugated $\pi$ electron system, wherein one or more ring atoms are heteroatoms selected from nitrogen, oxygen, or $S(O)_m$ (wherein m is an integer of 0 to 2), and the remaining ring atoms are carbon. Preferably, it is 6- to 14-membered and more preferably 7- to 10-membered. According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include:

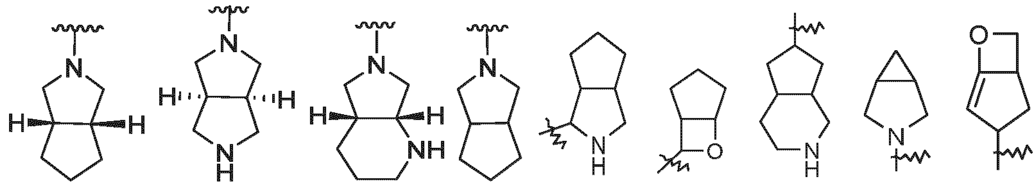

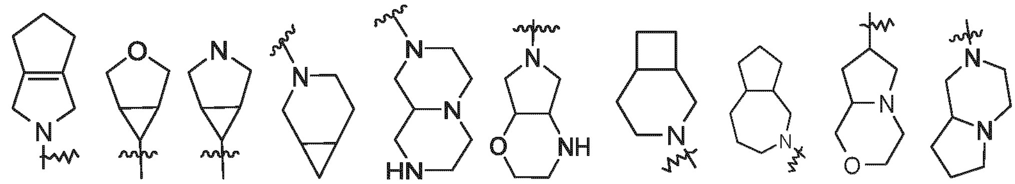

,

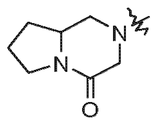

,

etc.

The term "bridged heterocyclyl" refers to a 5- to 14-membered polycyclic heterocyclyl with any two rings sharing two atoms that are not directly connected. It may contain one or more double bonds, but no ring has a fully conjugated π electron system, wherein one or more ring atoms are heteroatoms selected from nitrogen, oxygen, or $S(O)_m$ (wherein m is an integer of 0 to 2), and the remaining ring atoms are carbon. Preferably, it is 6- to 14-membered and more preferably 7- to 10-membered. According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include:

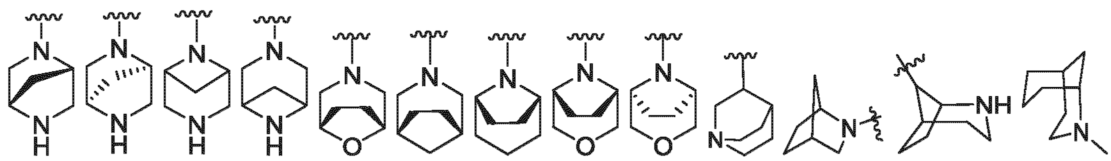

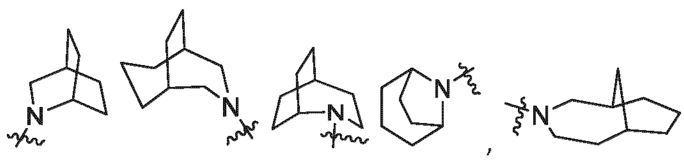

, ,

etc.

The heterocyclyl ring can be fused to an aryl, heteroaryl or cycloalkyl ring, wherein the ring connected to the parent structure is heterocyclyl, and the non-limiting examples thereof include:

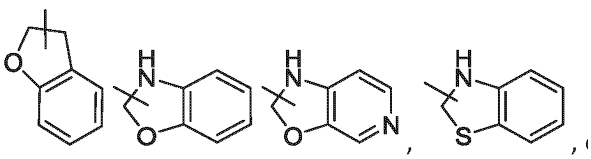

, ,

etc.

Heterocyclyl may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group.

The term "heterocyclylalkylene" refers to heterocyclyl connected to alkylene to form "heterocyclyl-alkylene-",

wherein the heterocyclyl or alkyl may be optionally substituted or unsubstituted, and the heterocyclyl and alkylene are as defined above.

The term "heterocyclylene" refers to bivalent heterocyclyl formed by further replacing one hydrogen atom of cycloalkyl, wherein the heterocyclylene may be optionally substituted or unsubstituted, and the heterocyclyl is defined as above.

The term "aryl" refers to a 6- to 14-membered all-carbon monocyclic or fused polycyclic (i.e., rings sharing an adjacent pair of carbon atoms) group having a conjugated $\pi$ electron system, preferably 6- to 12-membered aryl, such as phenyl and naphthyl. Phenyl is more preferred. An aryl ring can be fused to heteroaryl, heterocyclyl or cycloalkyl ring, including benzo 5- to 10-membered heteroaryl, benzo 3- to 8-membered cycloalkyl and benzo 3- to 8-membered heteroalkyl, preferably benzo 5- to 6-membered heteroaryl, benzo 3- to 6-membered cycloalkyl and benzo 3- to 6-membered heteroalkyl, wherein the heterocyclyl is heterocyclyl containing 1-3 nitrogen atoms, oxygen atoms, or sulfur atoms; or further including a three-membered nitrogen-containing fused ring containing a benzene ring,
wherein the ring connected to the parent structure is an aryl ring, and the non-limiting examples thereof include:

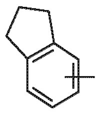 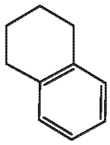 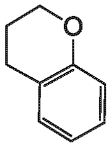 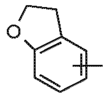 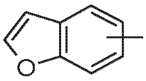 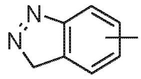 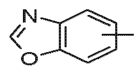 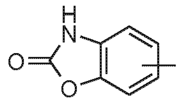

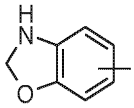 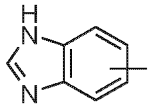 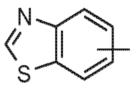 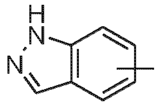 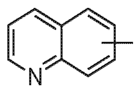 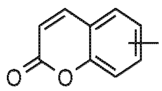

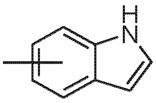 , 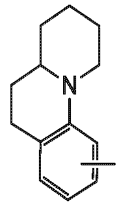 ,

etc.

Aryl may be substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

The term "arylene" refers to bivalent aryl formed by further replacing one hydrogen atom of cycloalkyl, wherein the arylene is optionally substituted or unsubstituted, and the aryl is defined as above.

The term "heteroaryl" refers to a heteroaromatic system containing 1 to 4 heteroatoms and 5 to 14 ring atoms, where the heteroatoms are selected from oxygen, sulfur and nitrogen. The heteroaryl is preferably 5- to 12-membered, more preferably 5- to 8-membered, and further preferably 5-membered or 6-membered, e.g., imidazolyl, furyl, thienyl, thiazolyl, pyrazolyl, oxazolyl, pyrrolyl, triazolyl, tetrazolyl, pyridyl, pyrimidinyl, thiadiazolyl, pyrazinyl, etc., preferably pyridyl, oxadiazolyl, triazolyl, thienyl, imidazolyl, pyrazolyl, oxazolyl, thiazolyl, pyrimidinyl, or thiazolyl; tetrazolyl, pyridyl, oxadiazolyl, pyrazolyl, pyrrolyl, thiazolyl, and oxazolyl are more preferred. The heteroaryl ring can be fused to an aryl, heterocyclyl or cycloalkyl ring, wherein the ring connected to the parent structure is heteroaryl ring, and the non-limiting examples thereof include:

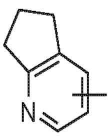 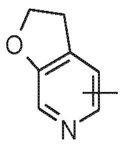 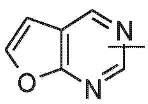 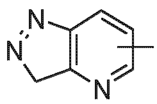 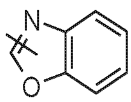 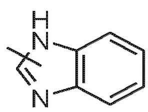 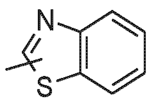

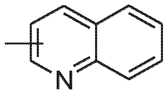 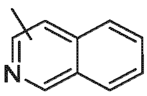 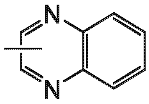 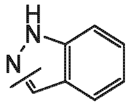, 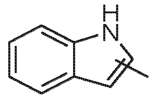,

etc.

Heteroaryl may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "heteroarylene" refers to bivalent heteroaryl formed by further replacing one hydrogen atom of cycloalkyl, wherein the heteroarylene is optionally substituted or unsubstituted, and the heteroaryl is defined as above.

The term "alkoxy" refers to -O-(alkyl) and -O-(unsubstituted cycloalkyl), where the alkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

"Haloalkyl" refers to alkyl substituted with one or more halogens, wherein the alkyl is as defined above.

"Haloalkoxy" refers to alkoxy substituted with one or more halogens, wherein the alkoxy is as defined above.

"Hydroxyalkyl" refers to alkyl substituted with hydroxy, wherein the alkyl is as defined above.

The term "alkenylcarbonyl" refers to -C(O)-(alkenyl), wherein the alkenyl is as defined above. Non-limiting examples of alkenylcarbonyl include: vinylcarbonyl, propenylcarbonyl, and butenylcarbonyl. Alkenylcarbonyl can be optionally substituted or unsubstituted. When it is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Aminocarbonyl" refers to $NH_2$-C(O)-.

"Alkylaminocarbonyl" means that one or both of two hydrogens on aminocarbonyl ($NH_2$-C(O)-) are substituted by alkyl, wherein the alkyl is as described above.

"Alkylamino" means that one or both of two hydrogens on the amino are substituted by alkyl, wherein the alkyl is as described above.

"Alkylcarbonyl" or "acyl" refers to (alkyl)-C(O)-, wherein the alkyl is as described above.

"Hydroxyl" refers to a -OH group.

"Halogen" refers to fluorine, chlorine, bromine, or iodine.

"Amino" refers to $-NH_2$.

"Cyano" refers to -CN.

"Nitro" refers to $-NO_2$.

"Carbonyl" refers to -C(O)-.

"Carboxyl" refers to -C(O)OH.

"THF" refers to tetrahydrofuran.

"Ethyl acetate" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"TEA" refers to triethylamine.

"MeCN" refers to acetonitrile.

"DMA" refers to N,N-dimethylacetamide.

"$Et_2O$" refers to diethyl ether.

"DCM" refers to dichloromethane.

"DMAP" refers to 4-dimethylaminopyridine.

"DCC" refers to dicyclohexylcarbodiimide.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"$Pd_2(dba)_3$" refers to tris(dibenzylideneacetone)dipalladium.

"Dppf" refers to 1,1'-bisdiphenylphosphine ferrocene.

"HATU" refers to 2-(7-oxidobenzotriazole)-N,N,N',N'-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to n-butyl lithium.

"NaBH(OAc)3" refers to sodium triacetoxyborohydride.

Different expressions such as "X is selected from A, B, or C", "X is selected from A, B, and C", "X is A, B, or C", and "X is A, B, and C" all express the same meaning, i.e., X can be any one or more of A, B, and C.

The hydrogen atoms described in the present invention can be replaced by its isotope deuterium, and any hydrogen atom in the example compounds involved in the present invention can also be replaced by deuterium atom.

"Optional" or "optionally" means that the event or circumstance subsequently described may but need not to occur, and the description includes the occasions where the events or circumstances occur or do not occur. For example, "heterocyclyl optionally substituted with alkyl" means the alkyl may but need not be present, the description includes the case where the heterocyclyl is substituted with alkyl and the case where the heterocyclyl is not substituted with alkyl.

"Substituted" refers to one or more hydrogen atoms in the group, preferably at most 5, more preferably 1-3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort. For example, amino having a free hydrogen or a hydroxy group may be unstable when combined the carbon atoms having an unsaturated (e.g., olefinic) bond.

"Pharmaceutical composition" denotes a mixture containing one or more of the compounds as described herein or physiologically/pharmaceutically acceptable salts or prodrug thereof and other chemical components, as well as other components, such as a physiologically/pharmaceutically acceptable carrier and an excipient. The purpose of pharmaceutical compositions is to facilitate administration to living organisms and facilitate the absorption of active ingredients to exert biological activity.

"Pharmaceutically acceptable salt" refers to a salt of the compound of the present invention, which are safe and effective when used in mammals, and have appropriate biological activity.

Detailed Description of Embodiments

The present invention will be further described below with reference to examples, but these examples do not limit the scope of the present invention.

<u>Examples</u>

The structure of the compound of the present invention was determined by nuclear magnetic resonance (NMR) or/and liquid mass spectrometry (LC-MS). NMR chemical shift (δ) was given in parts per million (ppm) unit. NMR was determined using a Bruker AVANCE-400 nuclear magnetic instrument. The solvents for determination were deuterated dimethyl sulfoxide (DMSO-$d_6$), deuterated methanol ($CD_3OD$) and deuterated chloroform ($CDCl_3$), and the internal standard was tetramethylsilane (TMS).

Agilent 1200 Infinity Series mass spectrometer was used for Liquid chromatography-mass spectrometry LC-MS determination. HPLC determination used Agilent 1200 DAD high-pressure liquid chromatograph instrument (Sunfire C18 150 × 4.6 mm chromatographic column) and Waters 2695-2996 high-pressure liquid chromatograph instrument (Gimini $C_{18}$ 150 × 4.6 mm chromatographic column).

Yantai Huanghai HSGF254 or Qingdao GF254 silica gel plate was used as a thin layer chromatography silica plate, and the specification used for the TLC was 0.15-0.20 mm, and the specification when separating and purifying a product by thin layer chromatography is 0.4-0.5 mm. For the column chromatography, Yantai Huanghai silica gel of 200-300 mesh silica gel was generally used as a carrier.

The starting materials in the examples of the present invention were known and can be purchased on the market, or can be synthesized using or according to methods known in the art.

Unless otherwise specified, all reactions of the present invention were carried out under continuous magnetic stirring in a dry nitrogen or argon atmosphere, the solvent was a dry solvent, and the reaction temperature unit was degrees Celsius.

Intermediates

Step I: Preparation of 2-amino-4-chloronicotinaldehyde

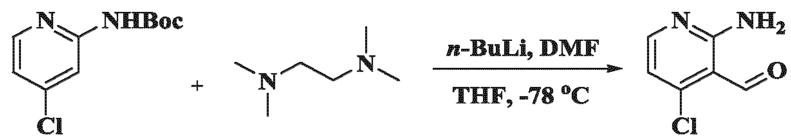


Under nitrogen protection, tert-butyl 4-chloropyridine-2-carboxylate (30 g, 131.19 mmol) and N,N,N',N'-tetramethylethylenediamine (38.11 g, 327.98 mmol) were dissolved in THF (600 mL), an n-hexane solution of n-butyllithium (2.5M, 131.19 mL) was added dropwise at -78°C, and after the dropwise addition was completed, the reaction was stirred for 2 hours. DMF (28.77 g, 393.57 mmol, 30.47 mL) was then dropwise added, and the reaction was continued for 1 hour. After the reaction was complete, a saturated ammonium chloride solution (500 mL) was added to quench the reaction, the reaction liquid was extracted with ethyl acetate (250 mL × 3) and washed with a saturated aqueous sodium chloride solution (250 mL × 3), and the organic phases were collected, dried over anhydrous sodium sulfate, and filtered, and the organic solvent was then concentrated under reduced pressure. A solution of hydrogen chloride in dioxane (4.0M, 50 mL) was then added to the product obtained by concentration, and the mixture was stirred at room temperature for 1 h. The reaction liquid was neutralized to alkalinity with a sodium hydroxide solution, and then extracted with ethyl acetate (250 mL × 3) and washed with saturated aqueous sodium chloride solution (250 mL × 3). The organic phases were collected, dried over anhydrous sodium sulfate, filtered and then subjected to separation by column chromatography (PE : EA = 1 : 4) to obtain the title compound (6.16 g, 30%).
MS *m*/*z* (ESI): 157.1 [M+H]$^+$.

Step II: Preparation of 2-amino-5-bromo-4-chloronicotinaldehyde

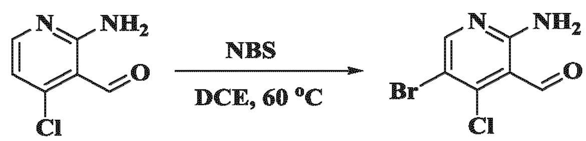


Under nitrogen protection, 2-amino-4-chloronicotinaldehyde (6.16 g, 39.34 mmol) and N-bromosuccinimide (7.70 g, 43.28 mmol) were dissolved in dichloroethane (100 mL) and heated to 60°C, and the reaction was stirred for 2 hours. After the reaction was complete, the reaction liquid was cooled and then filtered, and the filter cake was washed with dichloroethane (15 mL × 3) and water (15 mL × 3) to obtain the title compound (7.36 g, 80%).
MS *m*/*z* (ESI): 236.1 [M+H]$^+$.

Step III: Preparation of 7-bromo-1H-pyrazolo[4, 3-c]pyridine-4-amine

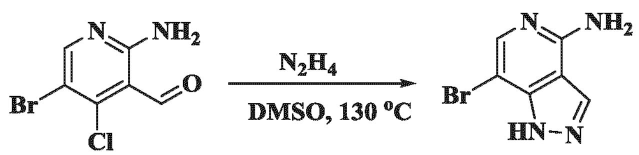


Under nitrogen protection, 2-amino-5-bromo-4-chloronicotinaldehyde (7.36 g, 31.26 mmol) and 85% hydrazine hydrate (3.68 g, 62.52 mmol) were dissolved in dimethyl sulfoxide (40 mL), and the reaction was stirred at 130°C for 4 hours. After the reaction was complete, the reaction liquid was extracted with ethyl acetate (150 mL × 3) and washed with a saturated aqueous sodium chloride solution (150 mL × 3), and the organic phases were collected, dried over anhydrous sodium sulfate, and then concentrated to obtain the title compound (4.18 g, 63%).
MS *m*/*z* (ESI): 213.1, 215.1 [M+H]$^+$.

Step IV: Preparation of 1-(7-bromo-1H-pyrazolo[4,3-c]pyridin-4-yl)pyrrolidine-2,5-dione

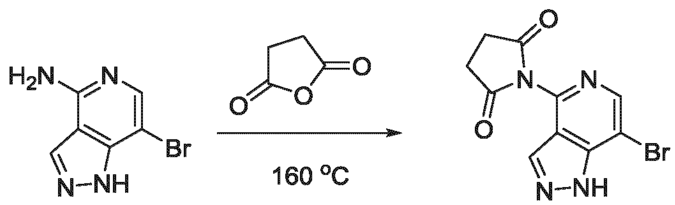


At room temperature, 7-bromo-1H-pyrazolo[4,3-c]pyridine-4-amine (1.5 g, 7.04 mmol) and succinic anhydride (2.11 g, 21.12 mmol) were mixed, then gradually warmed to 160°C, and then reacted for 30 minutes. After the reaction was complete, the reaction liquid was cooled, the reaction liquid was then extracted with ethyl acetate (50 mL × 3) and washed with a saturated aqueous sodium chloride solution (50 mL × 3), and the organic phases were dried over anhydrous sodium sulfate, filtered, concentrated, and separated by column chromatography (DCM : MeOH = 95 : 5) to obtain the title compound (1.5 g, 72%).

MS *m/z* (ESI): 295.0 $[M+H]^+$.

Step V: Preparation of 1-(7-bromo-3-iodo-1H-pyrazolo[4,3-c]pyridin-4-yl)pyrrolidine-2,5-dione

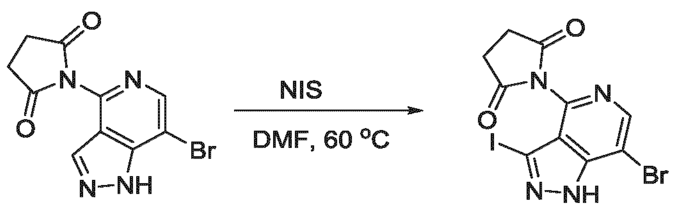


Under nitrogen protection, 1-(7-bromo-1H-pyrazolo[4,3-c]pyridin-4-yl)pyrrolidine-2,5-dione (1.5 g, 5.08 mmol) and N-iodosuccinimide (1.37 g, 6.10 mmol) were dissolved in DMF (20 mL), and the reaction was stirred at 60°C for 2 hours. After the reaction was complete, the reaction liquid was cooled and then filtered, the filtrate was extracted with ethyl acetate (50 mL × 3) and washed with a saturated aqueous sodium chloride solution (50 mL × 3), and the organic phases were collected, dried over anhydrous sodium sulfate, then concentrated, and subjected to column chromatography (DCM : MeOH = 95 : 5) to obtain the title compound (1.5 g, 70%).

MS *m/z* (ESI): 421.1 $[M+H]^+$.

Step VI: Preparation of tert-butyl-(S)-3-(4-amino-7-bromo-3-iodo-1H-pyrazolo[4,3-c]pyridin-1-yl)pyrrolidine-1-carboxylate

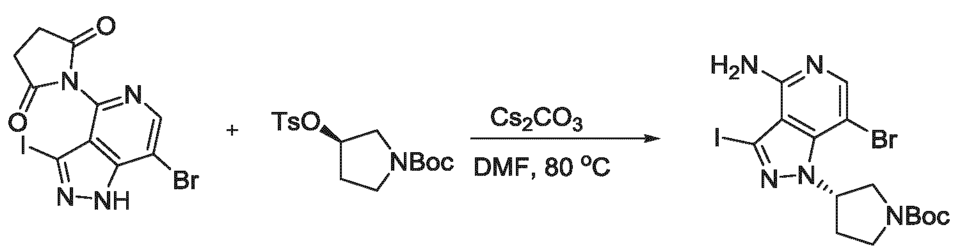


Under nitrogen protection, 1-(7-bromo-3-iodo-1H-pyrazolo[4,3-c]pyridin-4-yl)pyrrolidine-2,5-dione (1.5 g, 3.56 mmol), tert-butyl-(R)-3-(toluenesulfonyloxo)pyrrolidine-1-carboxylate (1.46 g, 4.28 mmol), and cesium carbonate (2.32 g, 7.13 mmol) were suspended in DMF (50 mL) and heated to 80°C, and the reaction was stirred overnight. After the reaction was complete, the reaction liquid was extracted with ethyl acetate (50 mL × 3) and washed with a saturated aqueous sodium chloride solution (50 mL × 3), and the organic phases were dried over anhydrous sodium sulfate, filtered,

concentrated, and separated by column chromatography (PE : EA = 1 : 1) to obtain the title compound (560 mg, 31%).
MS *m/z* (ESI): 508.1 $[M+H]^+$.

**Reference Example 1**

(*S*)-1-(3-(4-amino-3-((2,6-dimethoxypyridin-4-yl)ethynyl)-1H-pyrazolo[3,4-d]pyrimidin-1-yl)pyrrolidin-1-yl)prop-2-en-1-one

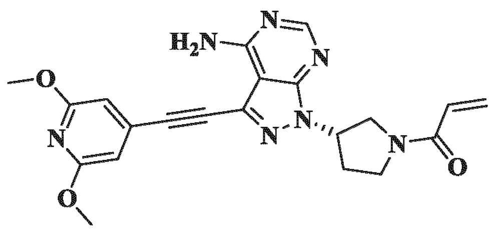

Step I: Preparation of 4-iodo-2,6-dimethoxypyridine

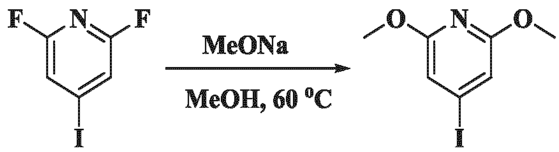


Under nitrogen protection, 2,6-difluoro-4-iodo-pyridine (1 g, 4.15 mmol) and sodium methoxide (896.35 mg, 16.60 mmol) were suspended in methanol (40 mL), and the reaction was stirred at 60°C under reflux overnight. After the reaction was complete, the reaction liquid was cooled and then filtered, the filtrate was extracted with ethyl acetate (30 mL × 3) and washed with a saturated aqueous sodium chloride solution (30 mL × 3), and the organic phases were collected, dried over anhydrous sodium sulfate, filtered and then separated by column chromatography (100% PE) to obtain the title compound (450 mg, 41%).
MS *m/z* (ESI): 266.1 $[M+H]^+$.

Step II: Preparation of 2,6-dimethoxy-4-((trimethylsilyl)ethynyl)pyridine

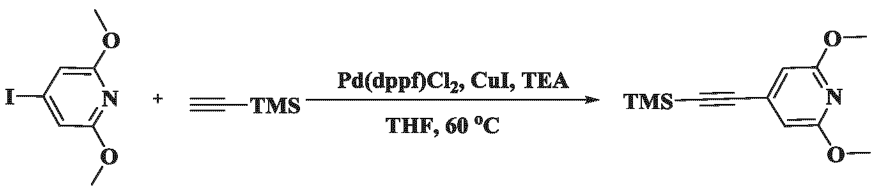


Under nitrogen protection, 4-iodo-2,6-dimethoxypyridine (450 mg, 1.7 mmol), ethynyltrimethylsilane (250 mg, 2.55 mmol), [1,1'-bis(diphenylphosphino)ferrocene]palladium dichloride (124 mg, 0.17 mmol), triethylamine (343 mg, 3.4 mmol), and cuprous iodide (64.6 mg, 0.34 mmol) were suspended in anhydrous tetrahydrofuran (10 mL), and the reaction was stirred at 60°C for 2 hours. After the reaction was complete, the reaction liquid was cooled and then filtered, the filtrate was extracted with ethyl acetate (30 mL × 3) and washed with a saturated aqueous sodium chloride solution (30 mL × 3), and the organic phases were collected, dried over anhydrous sodium sulfate, filtered, and then subjected to column chromatography (PE : EA = 10 : 1) to obtain the title compound (300 mg, 75%).
MS *m/z* (ESI): 236.1 $[M+H]^+$.

Step III: Preparation of 4-ethynyl-2,6-dimethoxypyridine

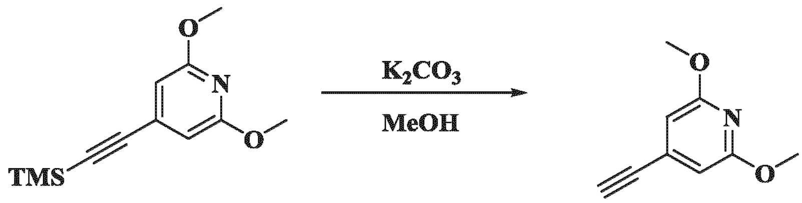


Under nitrogen protection, 2,6-dimethoxy-4-((trimethylsilyl)ethynyl)pyridine (300 mg, 1.27 mmol) and potassium carbonate (351.81 mg, 2.55 mmol) were suspended in methanol (10 mL), and the reaction was stirred at room temperature for 2 hours. After the reaction was complete, the reaction liquid was extracted with ethyl acetate (10 mL × 3) and washed with a saturated aqueous sodium chloride solution (10 mL × 3), and the organic phases were collected, dried over anhydrous sodium sulfate, filtered and then subjected to column chromatography (100% PE) to obtain the title compound (50 mg, 24%).

MS *m*/*z* (ESI): 164.1 [M+H]+.

Step IV: Preparation of tert-butyl (3S)-3-(4-amino-3-iodo-pyrazolo[3,4-d]pyrimidin-1-yl)pyrrolidine-1-carboxylate

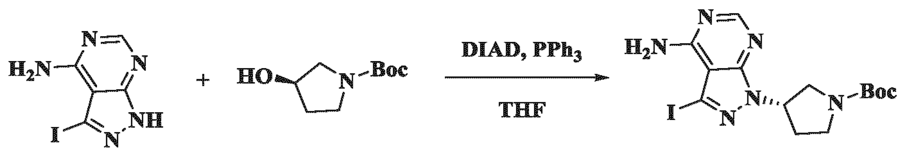


3-Iodo-1H-pyrazolo[3,4-d]pyrimidin-4-amine (5.22 g, 20.00 mmol), tert-butyl (3R)-3-hydroxypyrrolidine-1-carboxylate (5.62 g, 30.00 mmol), and triphenylphosphine (9.44 g, 36.00 mmol) were added to a dry reaction flask, the reaction flask was protected by dry nitrogen, anhydrous tetrahydrofuran (50 mL) was added, the reaction liquid was cooled to 0°C in an ice-water bath, and DIAD (8.09 g, 40.00 mmol) was added dropwise under stirring condition. After the dropwise addition was complete, the reaction liquid was transferred to room temperature, stirred for 1 hour, and concentrated under reduced pressure to remove the solvent, and the residue was separated by silica gel column chromatography to obtain the title compound (3.78 g, 44%).

MS *m*/*z* (ESI): 431.1 [M+H]+.

Step V: Preparation of tert-butyl-(S)-3-(4-amino-3-((2,6-dimethoxypyridin-4-yl)ethynyl)-1H-pyrazolo[3, 4-d]pyrimidin-1-yl)pyrrolidine-1-carboxylate

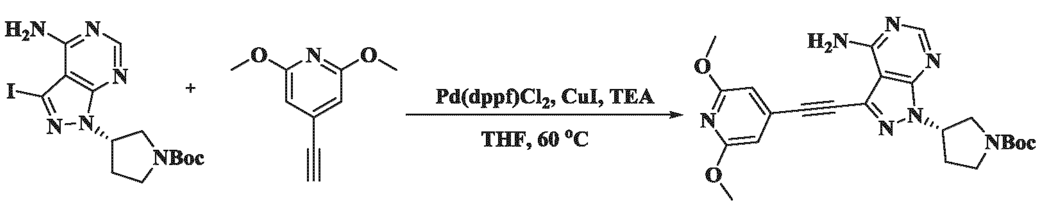


Under nitrogen protection, tert-butyl-(S)-3-(4-amino-3-iodo-1H-pyrazolo[3, 4-d]pyrimidin-1-yl)pyrrolidine-1-carboxylate (120 mg, 0.28 mmol), 4-ethynyl-2,6-dimethoxypyridine (50 mg, 0.31 mmol), [1, 1'-bis(diphenylphosphino) ferrocene]palladium dichloride (20.4 mg, 0.028 mmol), triethylamine (56.4 mg, 0.56 mmol), and cuprous iodide (10.6 mg, 0.056 mmol) were suspended in anhydrous tetrahydrofuran (11.22 mL), and the reaction was stirred at 60°C for 2 hours. After the reaction was complete, the reaction liquid was cooled and then filtered, the filtrate was extracted with ethyl acetate (30 mL × 3) and washed with a saturated aqueous sodium chloride solution (30 mL × 3), and the organic phases were collected, dried over anhydrous sodium sulfate, filtered, and then subjected to column chromatography (DCM :

MeOH = 95 : 5) to obtain the title compound (35 mg, 27%).
MS *m/z* (ESI): 466.1 [M+H]+.

Step VI: Preparation of (S)-3-((2,6-dimethoxypyridin-4-yl)ethynyl)-1-(pyrrolidin-3-yl)-1H-pyrazolo[3, 4-d]pyrimidin-4-amine

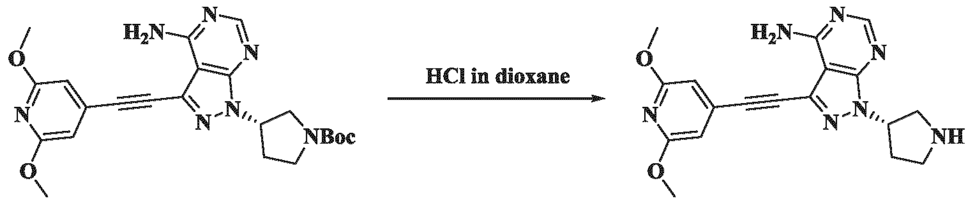


Under nitrogen protection, tert-butyl-(S)-3-(4-amino-3-((2, 6-dimethoxypyridin-4-yl)ethynyl)-1H-pyrazolo[3,4-d]pyrimidin-1-yl)pyrrolidine-1-carboxylate (35 mg, 0.075 mmol) was dissolved in a solution of hydrogen chloride in dioxane (4M, 1 mL), and the reaction was stirred at room temperature for 1 hour. After the reaction was complete, the reaction liquid was dried and concentrated to obtain the title compound (25 mg, 91%).
MS *m/z* (ESI): 366.1 [M+H]+.

Step VII: Preparation of (*S*)-1-(3-(4-amino-3-((2,6-dimethoxypyridin-4-yl)ethynyl)-1H-pyrazolo[3, 4-d]pyrimidin-1-yl)pyrrolidin-1-yl)prop-2-en-1-one

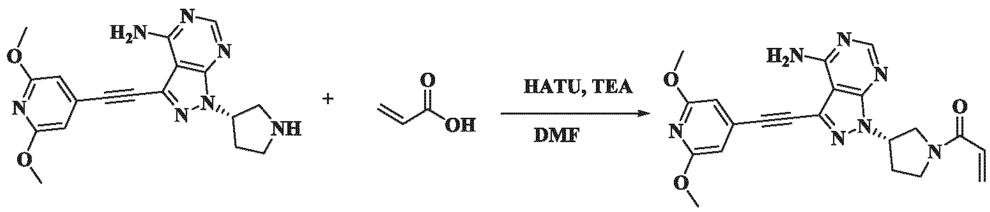


Under nitrogen protection, (S)-3-((2,6-dimethoxypyridin-4-yl)ethynyl)-1-(pyrrolidin-3-yl)-1H-pyrazolo[3, 4-d]pyrimidin-4-amine (25 mg, 68.49 μmol), acrylic acid (7.4 mg, 103 μmol), 2-(7-azobenzotriazole)-N, N, N', N'-tetramethyluronium hexafluorophosphate (38.8 mg, 103 μmol), and triethylamine (13.83 mg, 136.98 μmol) were dissolved in DMF (5 mL), and the reaction was stirred at room temperature for 2 hours. After the reaction was complete, the reaction liquid was extracted with dichloromethane (10 mL × 3) and washed with a saturated aqueous sodium chloride solution (10 mL × 3), the organic phases were collected, dried over anhydrous sodium sulfate, filtered, and then concentrated under reduced pressure, and the resulting product was subjected to prep-HPLC to obtain the title compound (0.7 mg, 2%).
MS *m/z* (ESI): 420.1 [M+H]+.

**Reference Example 2**

(S)-1-(3-(4-amino-7-chloro-3-((3,5-difluoro-2,6-dimethoxypyridin-4-yl)ethynyl)-1H-pyrazolo[4,3-c]pyridin-1-yl)pyrrolidin-1-yl)prop-2-en-1-one

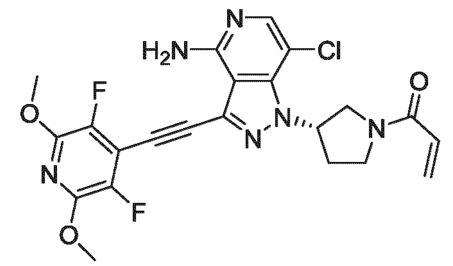

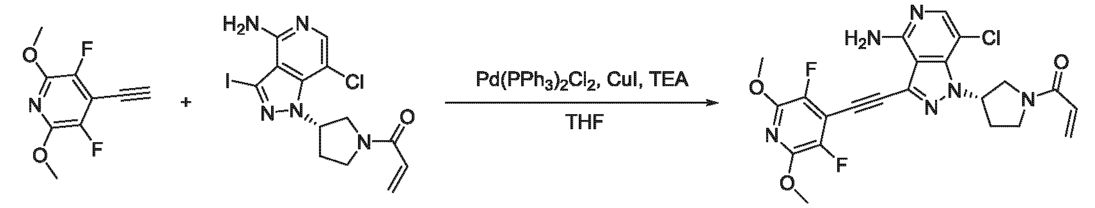


At room temperature, CuI (9.58 mg, 50.28 μmol), $Pd(PPh_3)_2Cl_2$ (17.65 mg, 25.14 μmol), and TEA (76.32 mg, 754.25 μmol, 105.20 μL) were respectively added to a solution of (5)-1-(3-(4-amino-7-chloro-3-iodo-1H-pyrazolo[4,3-c]pyridin-1-yl)pyrrolidin-1-yl)prop-2-en-1-one (0.105 g, 251.42 μmol) and 4-ethynyl-3,5-difluoro-2,6-dimethoxypyridine (75.12 mg, 377.13 μmol) in THF (2.0 mL), and after displacement with nitrogen three times, the solution was warmed to 60°C and stirred for 2 hours. The reaction liquid was concentrated under reduced pressure, and the residue was separated and purified by silica gel column chromatography (DCM : MeOH = 100 : 1 to 10 : 1), and then separated by prep-HPLC to obtain the title compound as a white solid (25 mg, 20%).

MS *m/z* (ESI): 489.1 $[M+H]^+$.

$^1H$ NMR (400 MHz, $CDCl_3$) $\delta$ 7.82-7.74 (m, 1H), 6.57-6.36 (m, 3H), 6.27 (s, 1H), 6.07-5.91 (m, 1H), 5.78-5.68 (m, 1H), 4.15-3.93 (m, 9H), 3.89-3.76 (m, 1H), 2.87-2.43 (m, 2H).

**Reference Example 3**

(S)-1-(3-(4-amino-3-((2,6-difluoro-3,5-dimethoxyphenyl)ethynyl)-7-methyl-1H-pyrazolo[4,3-c]pyridin-1-yl)pyrrolidin-1-yl)prop-2-en-1-one

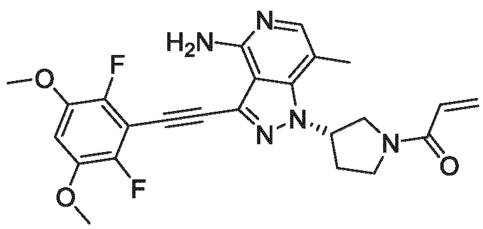

Step I: Preparation of (S)-tert-butyl 3-(4-amino-7-bromo-3-((2,6-difluoro-3,5-dimethoxyphenyl)ethynyl)-1H-pyrazolo[4,3-c]pyridin-1-yl)pyrrolidine-1-carboxylate

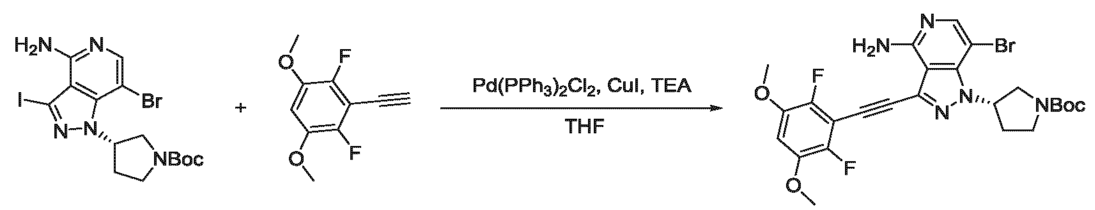


$Pd(PPh_3)_2Cl_2$ (68.98 mg, 98.40 μmol) and CuI (28.11 mg, 147.59 μmol) were respectively added to a solution of

(S)-tert-butyl 3-(4-amino-7-bromo-3-iodo-1H-pyrazolo[4,3-c]pyridin-1-yl)pyrrolidine-1-carboxylate (0.5 g, 983.96 μmol), 3-ethynyl-2,4-difluoro-1,5-dimethoxybenzene (234.29 mg, 1.18 mmol), triethylamine (298.70 mg, 2.95 mmol) in THF (10 mL), and after displacement with nitrogen three times, the solution was warmed to 60°C and stirred for 1 hour. The reaction liquid was concentrated under reduced pressure, and the residue was separated in water (10 mL) and DCM (10 mL). The organic layer was dried over anhydrous sodium sulfate and filtered, and the filtrate was concentrated under reduced pressure. The residue was separated and purified by silica gel column chromatography (DCM : MeOH = 100 : 1 to 10 : 1) to obtain the title compound as a light yellow gel (0.538 g, 95%).

MS *m/z* (ESI): 578.1 $[M+H]^+$.

Step II: Preparation of (S)-tert-butyl 3-(4-amino-3-((2,6-difluoro-3,5-dimethoxyphenyl)ethynyl)-7-methyl-1H-pyrazolo [4,3-c]pyridin-1-yl)pyrrolidine-1-carboxylate

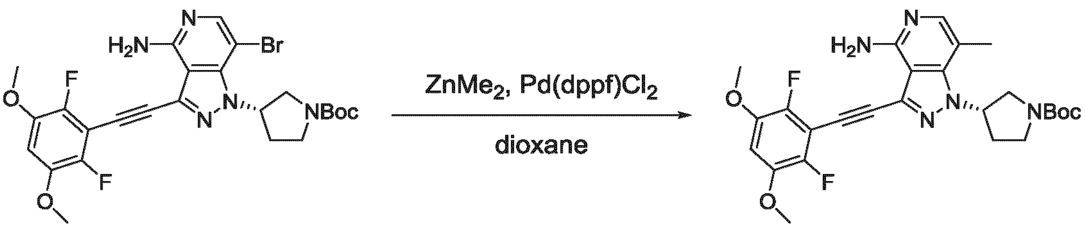


At room temperature and under nitrogen protection, dimethyl zinc (7.2 mL, 1 M toluene solution) was dropwise added to a solution of (S)-tert-butyl 3-(4-amino-7-bromo-3-((2,6-difluoro-3,5-dimethoxyphenyl)ethynyl)-1H-pyrazolo[4,3-c]pyridin-1-yl)pyrrolidine-1-carboxylate (1.5 g, 2.59 mmol), $Pd(dppf)Cl_2$(190 mg, 2.60 μmol) in 1,4-dioxane (15 mL), whereupon gas evolution occurred. The reaction liquid was subjected to displacement with nitrogen three times, then warmed to 100°C, and stirred for 1 hour. After cooling, the reaction liquid was quenched with MeOH (5 mL) and diluted with ethyl acetate (100 mL), and a saturated aqueous sodium bicarbonate solution (20 mL) was then added with stirring, whereupon a solid was generated. After filtration with a diatomaceous earth pad, the filtrate was separated, the organic layer was washed with a saturated aqueous sodium chloride solution (10 mL), dried over anhydrous sodium sulfate, and concentrated under reduced pressure, and the residue was separated by silica gel column chromatography (DCM : MeOH = 100 : 1~10 : 1) to obtain the title compound as a light brown oil (585 mg, 44%).

MS *m/z* (ESI): 514.2 $[M+H]^+$.

Step III: Preparation of (S)-3-((2,6-difluoro-3,5-dimethoxyphenyl)ethynyl)-7-methyl-1-(pyrrolidin-3-yl)-1H-pyrazolo[4,3-c]pyridine-4-amine

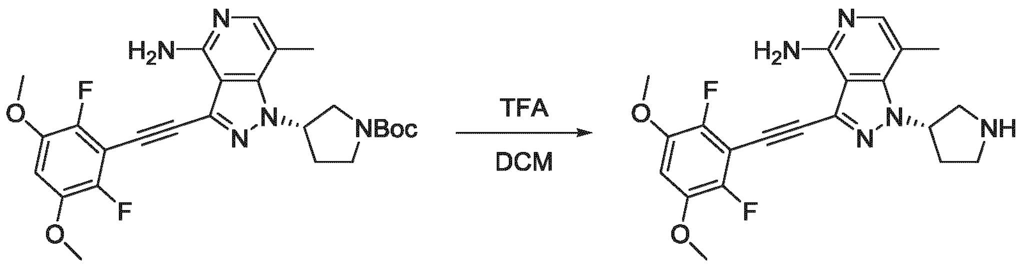


At room temperature, TFA (3 mL) was added to a solution of (S)-tert-butyl 3-(4-amino-3-((2,6-difluoro-3,5-dimethoxyphenyl)ethynyl)-7-methyl-1H-pyrazolo[4,3-c]pyridin-1-yl)pyrrolidine-1-carboxylate (0.524 g, 1.02 mmol) in DCM (12 mL) and stirred at room temperature for 0.5 hours. The reaction liquid was concentrated under reduced pressure, and the residue was then diluted with DCM (10 mL) and concentrated under reduced pressure to obtain the title compound (1.02 mmol, 100%) as a brown oil.

MS *m/z* (ESI): 414.2 $[M+H]^+$.

Step IV: Preparation of (S)-1-(3-(4-amino-3-((2,6-difluoro-3,5-dimethoxyphenyl)ethynyl)-7-methyl-1H-pyrazolo[4,3-c]pyridin-1-yl)pyrrolidin-1-yl)prop-2-en-1-one

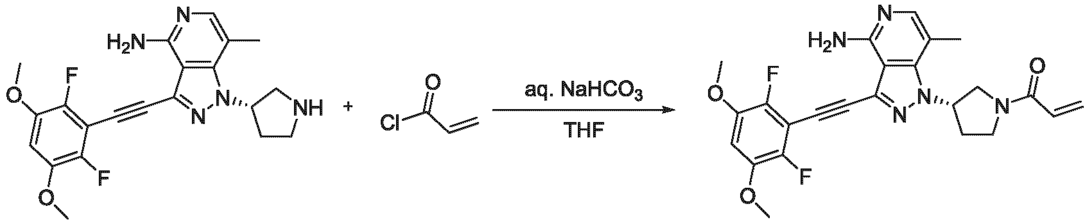


In an ice bath, a solution of acryloyl chloride (92.32 mg, 1.02 mmol, 82.43 μL) in THF (0.5 mL) was dropwise added to a solution of (S)-3-((2,6-difluoro-3,5-dimethoxyphenyl)ethynyl)-7-methyl-1-(pyrrolidin-3-yl)-1H-pyrazolo[4,3-c]pyridine-4-amine(1.02 mmol), a saturated aqueous $NaHCO_3$ solution (4.76 g) and THF (10 mL) and stirred at room temperature for 10 minutes. The reaction liquid was concentrated under reduced pressure, the residue was diluted with DCM (10 mL), washed with water (10 mL) and then with a saturated aqueous sodium chloride solution (10 mL), dried over anhydrous sodium sulfate, and concentrated under reduced pressure, and the residue was separated and purified by silica gel column chromatography (DCM : MeOH = 100 : 1 to 10 : 1) and then separated by prep-HPLC (0.5% aqueous ammonia) to obtain the title compound (249 mg, 52%).

MS *m/z* (ESI): 468.2 $[M+H]^+$.

$^1H$ NMR (400 MHz, $CDCl_3$) $\delta$ 7.60-7.53 (m, 1H), 6.75-6.66 (m, 1H), 6.58-6.47 (m, 1H), 6.45-6.39 (m, 1H), 5.93-5.74 (m, 2H), 5.75-5.67 (m, 1H), 5.58-5.44 (m, 1H), 4.19-3.97 (m, 3H), 3.92 (s, 6H), 3.85-3.75 (m, 1H), 2.90-2.62 (m, 1H), 2.56-2.51 (m, 3H), 2.48-2.37 (m, 1H).

**Reference Example 4**

((*S*)-1-(3-(4-amino-7-bromo-3-((3,5-dimethoxyphenyl)ethynyl)-1H-pyrazolo[4,3-c]pyridin-1-yl)pyrrolidin-1-yl)prop-2-en-1-one

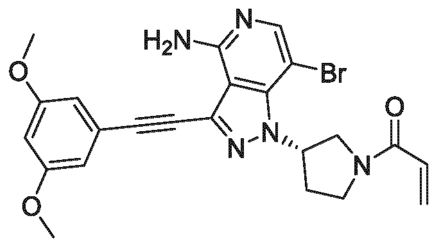

Step I: Preparation of 1-(7-bromo-1H-pyrazolo[4,3-c]pyridin-4-yl)pyrrolidine-2,5-dione

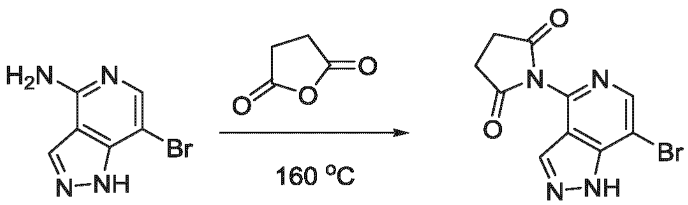


At room temperature, 7-bromo-1H-pyrazolo[4,3-c]pyridine-4-amine (1.5 g, 7.04 mmol) and succinic anhydride (2.11 g, 21.12 mmol) were mixed, then gradually warmed to 160°C, and then reacted for 30 minutes. After the reaction was complete, the reaction liquid was cooled, the reaction liquid was then extracted with ethyl acetate (50 mL × 3) and washed with a saturated aqueous sodium chloride solution (50 mL × 3), and the organic phases were collected, dried over

anhydrous sodium sulfate, and then separated by column chromatography (DCM : MeOH = 95 : 5) to obtain the title compound (1.5 g, 72%).

MS *m/z* (ESI): 295.0 $[M+H]^+$.

Step II: Preparation of 1-(7-bromo-3-iodo-1H-pyrazolo[4,3-c]pyridin-4-yl)pyrrolidine-2,5-dione

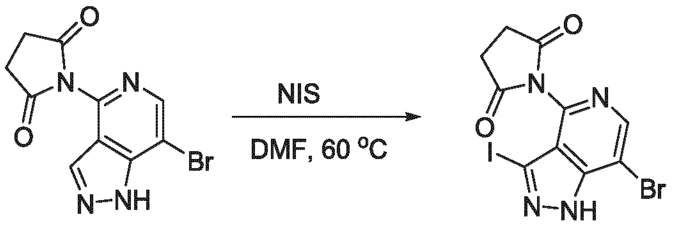


Under nitrogen protection, 1-(7-bromo-1H-pyrazolo[4,3-c]pyridin-4-yl)pyrrolidine-2,5-dione (1.5 g, 5.08 mmol) and N-iodosuccinimide (1.37 g, 6.10 mmol) were dissolved in N,N-dimethylformamide (20 mL), and the reaction was stirred at 60°C for 2 hours. After the reaction was complete, the reaction liquid was cooled and then filtered, the filtrate was extracted with ethyl acetate (50 mL × 3) and washed with a saturated aqueous sodium chloride solution (50 mL × 3), and the organic phases were collected, dried over anhydrous sodium sulfate, then concentrated, and subjected to column chromatography (DCM : MeOH = 95 : 5) to obtain the title compound (1.5 g, 70%).

MS *m/z* (ESI): 421.1 $[M+H]^+$.

Step III: Preparation of tert-butyl-(S)-3-(4-amino-7-bromo-3-iodo-1H-pyrazolo[4,3-c]pyridin-1-yl)pyrrolidine-1-carboxylate

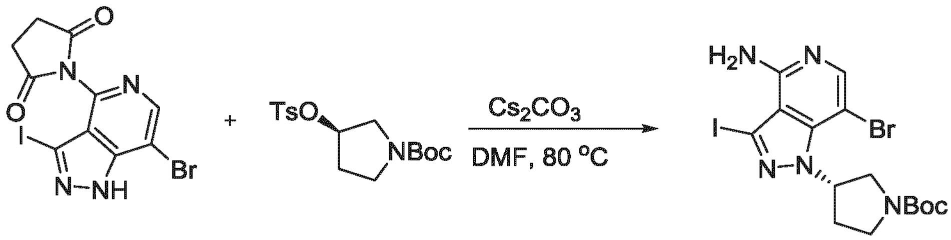


Under nitrogen protection, 1-(7-bromo-3-iodo-1H-pyrazolo[4,3-c]pyridin-4-yl)pyrrolidine-2,5-dione (1.5 g, 3.56 mmol), tert-butyl-(R)-3-(toluenesulfonyloxo)pyrrolidine-1-carboxylate (1.46 g, 4.28 mmol), and cesium carbonate (2.32 g, 7.13 mmol) were suspended in N,N-dimethylformamide (50 mL), and heated to 80°C, and the reaction liquid was stirred overnight. After the reaction was complete, the reaction liquid was extracted with ethyl acetate (50 mL × 3) and washed with a saturated aqueous sodium chloride solution (50 mL × 3), and the organic phases were collected, dried over anhydrous sodium sulfate, and then separated by column chromatography (PE : EA = 1 : 1) to obtain the title compound (560 mg, 31%).

MS *m/z* (ESI): 508.1 $[M+H]^+$.

Step IV: Preparation of tert-butyl-(*S*)-3-(4-amino-7-bromo-3-((3,5-dimethoxyphenyl)ethynyl)-1H-pyrazolo[4,3-c]pyridin-1-yl)pyrrolidine-1-carboxylate

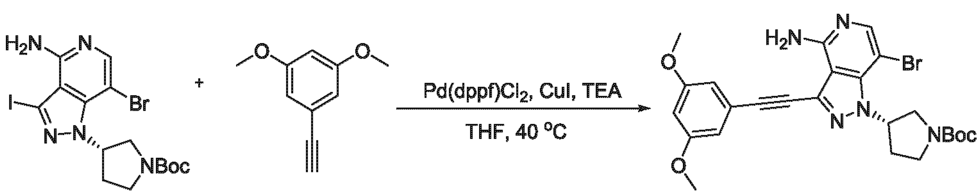

Under nitrogen protection, tert-butyl-(5)-3-(4-amino-7-bromo-3-iodo-1H-pyrazolo[4,3-c]pyridin-1-yl)pyrrolidine-1-carboxylate (560 mg, 1.1 mmol), 3,5-dimethoxyphenylacetylene (196.6 mg, 1.21 mmol), [1,1'-bis(diphenylphosphino)ferrocene]palladium dichloride (80.6 mg, 0.11 mmol), triethylamine (223 mg, 2.2 mmol), and cuprous iodide (42 mg, 0.22 mmol) were suspended in dry tetrahydrofuran (10 mL) and heated to 40°C, and the reaction was stirred for 2 hours. After the reaction was complete, the reaction liquid was cooled and then filtered, the filtrate was extracted with ethyl acetate (30 mL × 3) and washed with a saturated aqueous sodium chloride solution (20 mL × 3), and the organic phases were collected, dried over anhydrous sodium sulfate, and then subjected to column chromatography (PE : EA = 1 : 1) to obtain the title compound (35 mg, 6%).
MS *m/z* (ESI): 542.1 $[M+H]^+$.

Step V: Preparation of (S)-7-bromo-3-((3,5-dimethoxyphenyl)ethynyl)-1-(pyrrolidin-3-yl)-1H-pyrazolo[4,3-c]pyridine-4-amine

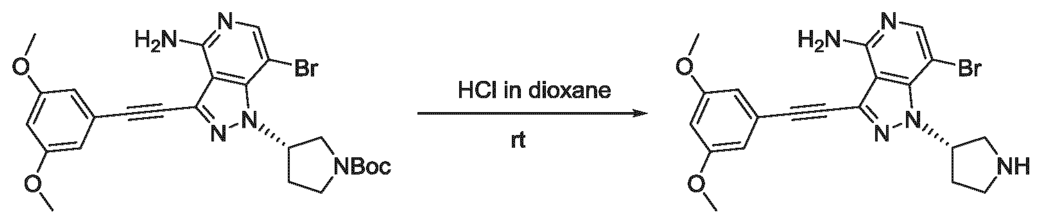


Under nitrogen protection, tert-butyl-(S)-3-(4-amino-7-bromo-3-((3,5-dimethoxyphenyl)ethynyl)-1H-pyrazolo [4,3-c]pyridin-1-yl)pyrrolidine-1-carboxylate (35 mg, 0.064 mmol) was dissolved in a solution of hydrogen chloride in dioxane (4 M, 2 mL), and the reaction was stirred at room temperature for 1 hour. After the reaction was complete, the reaction liquid was dried and concentrated to obtain the title compound (25 mg, 88%).
MS *m/z* (ESI): 442.1 $[M+H]^+$.

Step VI: Preparation of (*S*)-1-(3-(4-amino-7-bromo-3-((3,5-dimethoxyphenyl)ethynyl)-1H-pyrazolo[4,3-c]pyridin-1-yl) pyrrolidin-1-yl)pro p-2-en-1-one

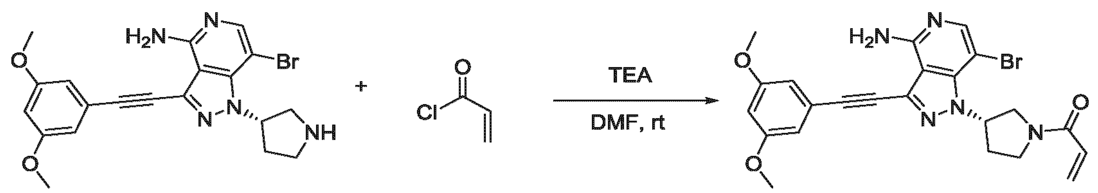


Under nitrogen protection, (5)-7-bromo-3-((3,5-dimethoxyphenyl)ethynyl)-1-(pyrrolidin-3-yl)-1H-pyrazolo[4,3-c]pyridine-4-amine(25 mg, 0.057 mmol) and triethylamine (11.4 mg, 0.114 mmol) were dissolved in N,N-dimethylformamide (2 mL), a solution of acryloyl chloride (5.6 mg, 0.062 mmol) in N,N-dimethylformamide (1 mL) was dropwise added in an ice-water bath, the solution was warmed to room temperature, and the reaction was stirred for 30 minutes. After the reaction was complete, the reaction liquid was extracted with dichloromethane (15 mL × 3) and washed with a saturated aqueous sodium chloride solution (10 mL × 3), the organic phases were collected, dried over anhydrous sodium sulfate, filtered, and then concentrated under reduced pressure, and the resulting product was subjected to prep-HPLC to obtain the title compound (8.3 mg, 29%).
MS *m/z* (ESI): 496.1 $[M+H]^+$.
$^1$H NMR (400 MHz, DMSO-$d_6$) $\delta$ = 7.91 (s, 1H), 6.86 (d, *J* = 1.8 Hz, 2H), 6.63 (t, *J* = 14.0 Hz, 4H), 6.21-6.12 (m, 1H), 5.74-5.63 (m, 1H), 4.12 (d, *J* = 6.8 Hz, 1H), 4.06 (s, 1H), 3.91 (d, *J* = 6.4 Hz, 1H), 3.79 (s, 6H), 3.64 (d, *J* = 7.2 Hz, 2H), 2.44-2.30 (m, 2H).

**Reference Example 5**

(*S*)-1-(1-acryloylpyrrolidin-3-yl)-4-amino-3-((3,5-difluoro-2,6-dimethoxypyridin-4-yl)ethynyl)-1H-pyrazolo[4,3-c]pyridine-7-carbonitrile

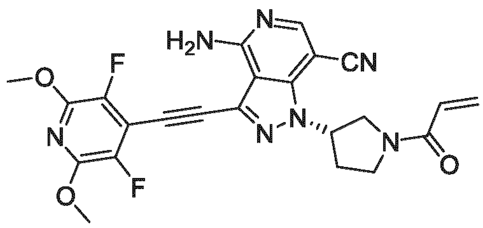

Preparation of (S)-1-(1-acryloylpyrrolidin-3-yl)-4-amino-3-((3,5-difluoro-2,6-dimethoxypyridin-4-yl)ethynyl)-1H-pyrazolo[4,3-c]pyridine-7-carbonitrile according to Reference Example 2.
MS *m*/*z* (ESI): 480.2 $[M+H]^+$.

**Reference Example 6**

(*S*)-1-(3-(4-amino-7-cyclopropyl-3-((2,6-difluoro-3,5-dimethoxyphenyl)ethynyl)-1H-pyrazolo[4,3-c]pyridin-1-yl)pyrrolidin-1-yl)prop-2-en-1-one

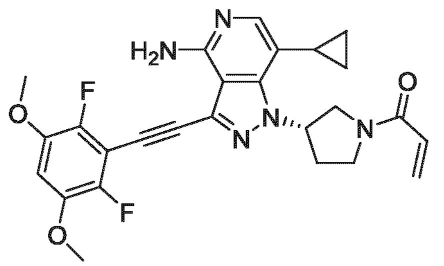

Step I: Preparation of (S)-tert-butyl 3-(4-amino-7-cyclopropyl-3-((2,6-difluoro-3,5-dimethoxyphenyl)ethynyl)-1H-pyrazolo[4,3-c]pyridin-1-yl)pyrrolidine-1-carboxylate

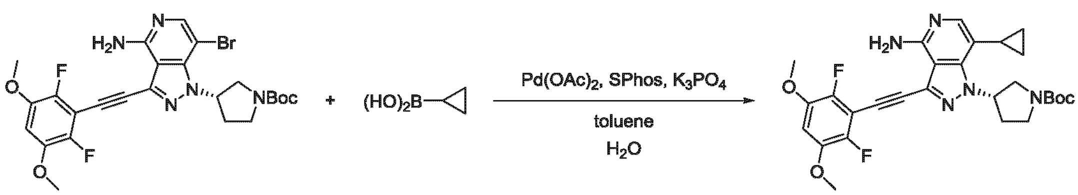


At room temperature, cyclopropylboronic acid (203.45 mg, 2.37 mmol) was added to a mixed solution of (S)-tert-butyl 3-(4-amino-7-bromo-3-((2,6-difluoro-3,5-dimethoxyphenyl)ethynyl)-1H-pyrazolo[4,3-c]pyridin-1-yl)pyrrolidine-1-carboxylate (0.685 g, 1.18 mmol), S-Phos (97.11 mg, 236.86 μmol), potassium phosphate (753.21 mg, 3.55 mmol), and $Pd(OAc)_2$ (53.06 mg, 236.86 μmol) in toluene (10 mL) and water (1 mL), and after displacement with nitrogen three times, the solution was then warmed to 90°C and stirred for 1.5 hours. The reaction liquid was separated, the organic layer was concentrated under reduced pressure, and the residue was separated and purified by silica gel column chromatography (DCM : MeOH = 100 : 1 to 10 : 1) to obtain the title compound as a light brown gel (228 mg, 36%).
MS *m*/*z* (ESI): 540.2 $[M+H]^+$.

Step II: Preparation of (S)-7-cyclopropyl-3-((2,6-difluoro-3,5-dimethoxyphenyl)ethynyl)-1-(pyrrolidin-3-yl)-1H-pyrazolo[4,3-c]pyridine-4-amine

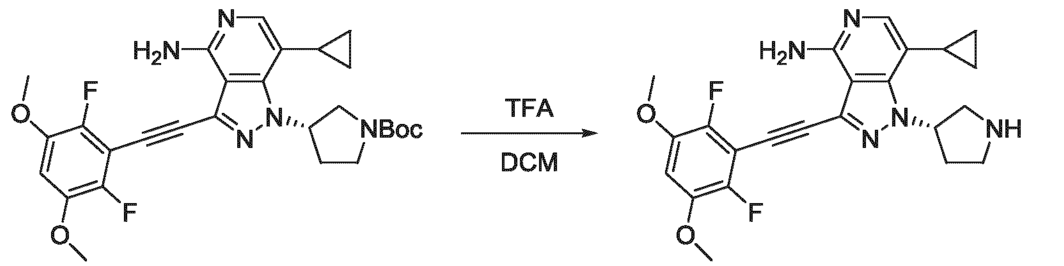


At room temperature, TFA (3 mL) was added to a solution of (S)-tert-butyl 3-(4-amino-7-cyclopropyl-3-((2,6-difluoro-3,5-dimethoxyphenyl)ethynyl)-1H-pyrazolo[4,3-c]pyridin-1-yl)pyrrolidine-1-carboxylate (500 mg, 926.66 μmol) in DCM (12 mL), and the mixture was stirred at room temperature for 0.5 hours. The reaction liquid was concentrated under reduced pressure, and the residue was then diluted with DCM (10 mL) and concentrated under reduced pressure to obtain the title compound (0.927 mmol, 100%) as a brown oil.

MS *m/z* (ESI): 440.2 $[M+H]^+$.

Step III: Preparation of (S)-1-(3-(4-amino-7-cyclopropyl-3-((2,6-difluoro-3,5-dimethoxyphenyl)ethynyl)-1H-pyrazolo[4,3-c]pyridin-1-yl)pyrrolidin-1-yl)prop-2-en-1-one

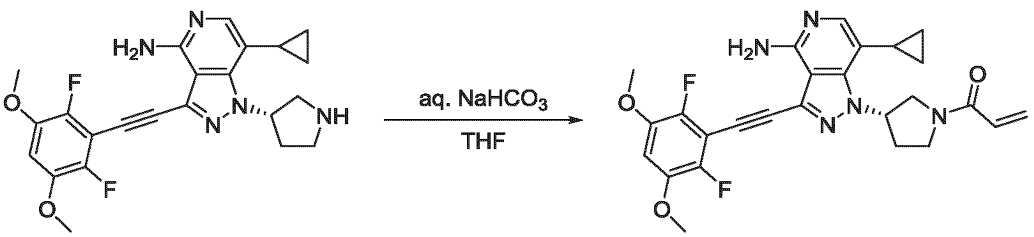


In an ice bath, a solution of acryloyl chloride (84.54 mg, 934.09 μmol, 75.48 μL) in THF (0.5 mL) was dropwise added to a solution of (S)-7-cyclopropyl-3-((2,6-difluoro-3,5-dimethoxyphenyl)ethynyl)-1-(pyrrolidin-3-yl)-1H-pyrazolo[4,3-c]pyridine-4-amine(0. 934 mmol), $NaHCO_3$ aqueous solution (6.10 g), and THF (5 mL), and the mixture was stirred at room temperature for 10 minutes. The reaction liquid was concentrated under reduced pressure, the residue was diluted with DCM (10 mL) and washed with water (10 mL), the organic layer was concentrated under reduced pressure, and the residue was separated and purified by silica gel column chromatography (DCM : MeOH = 100 : 1 to 10 : 1) and then separated by prep-HPLC (0.5% aqueous ammonia) to obtain the title compound (136 mg, 29%).

MS *m/z* (ESI): 494.2 $[M+H]^+$.

$^1H$ NMR (400 MHz, $CDCl_3$) $\delta$ 7.48-7.40 (m, 1H), 6.65 (t, $J$ = 8.2 Hz, 1H), 6.50-6.30 (m, 2H), 6.13-5.95 (m, 1H), 5.72-5.62 (m, 1H), 4.15-4.01 (m, 2H), 4.01-3.90 (m, 1H), 3.85 (s, 6H), 3.79-3.63 (m, 1H), 2.76-2.33 (m, 2H), 1.98-1.89 (m, 1H), 1.04-0.94 (m, 2H), 0.83-0.68 (m, 2H).

**Reference Example 7**

(S)-1-(3-(7-acetyl-4-amino-3-((2,6-difluoro-3,5-dimethoxyphenyl)ethynyl)-1H-pyrazolo[4,3-c]pyridin-1-yl)pyrrolidin-1-yl)prop-2-en-1-one

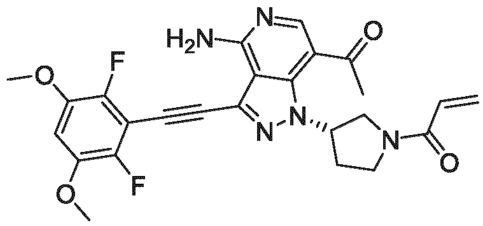

Step I: Preparation of tert-butyl (S)-3-(7-acetyl-4-amino-3-((2,6-difluoro-3,5-dimethoxyphenyl)ethynyl)-1H-pyrazolo [4,3-c]pyridin-1-yl)pyrrolidine-1-carboxylate

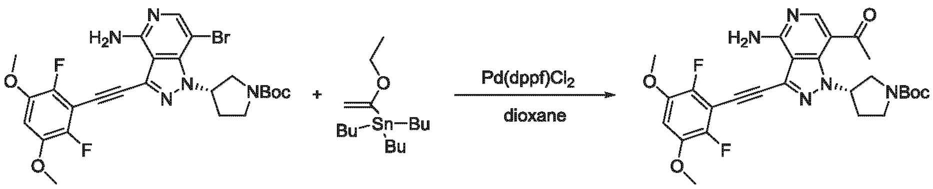


A solution of (*S*)-tert-butyl 3-(4-amino-7-bromo-3-((2,6-difluoro-3,5-dimethoxyphenyl)ethynyl)-1H-pyrazolo[4,3-c]pyridin-1-yl)pyrrolidine-1-carboxylate (1 g, 1.73 mmol), tributyl(1-ethoxyethylene)tin (749 mg, 2.07 mmol), and Pd(dppf) $Cl_2$ (126 mg, 172 μmol) in 1, 4-dioxane (25 mL) was warmed to 100°C under nitrogen protection and stirred for 16 hours. The reaction liquid was concentrated under reduced pressure, the residue was dissolved in THF (10 mL), 2M HCl (10 mL) was added, and the solution was stirred at room temperature for 0.5 hours. The mixture was adjusted to pH 7-8 with a saturated bicarbonate solution, DCM (30 mL) was added and stirred. After filtration with a diatomaceous earth pad, the filtrate was separated, the organic layer was concentrated under reduced pressure, and the residue was separated and purified by silica gel column chromatography to obtain the title compound (411 mg, 44%).

MS *m/z* (ESI): 542.2 $[M+H]^+$.

Step II: Preparation of (*S*)-1-(4-amino-3-((2,6-difluoro-3,5-dimethoxyphenyl)ethynyl)-1-(pyrrolidin-3-yl)-1H-pyrazolo [4,3-c]pyridin-7-yl)ethan-1-one

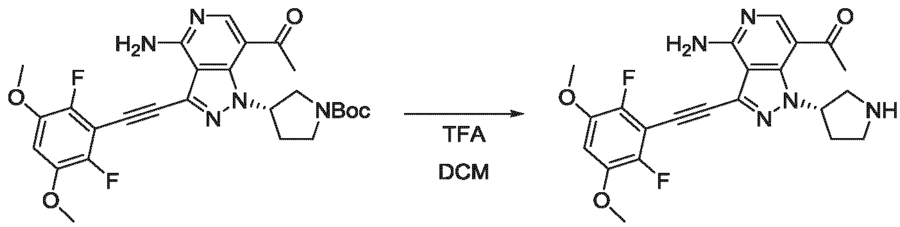


TFA (2 mL) was added to a solution of tert-butyl (*S*)-3-(7-acetyl-4-amino-3-((2,6-difluoro-3,5-dimethoxyphenyl) ethynyl)-1H-pyrazolo[4,3-c]pyridin-1-yl)pyrrolidine-1-carboxylate (311 mg, 574 μmol) in DCM (4 mL), and the mixture was stirred at room temperature for 0.5 hours. The reaction liquid was concentrated under reduced pressure to obtain the crude title compound (319 mg), which was used in the reaction of the next step directly.

Step III: Preparation of (*S*)-1-(3-(7-acetyl-4-amino-3-((2,6-difluoro-3,5-dimethoxyphenyl)ethynyl)-1H-pyrazolo[4,3-c] pyridin-1-yl)pyrrolidin-1-yl)prop-2-en-1-one

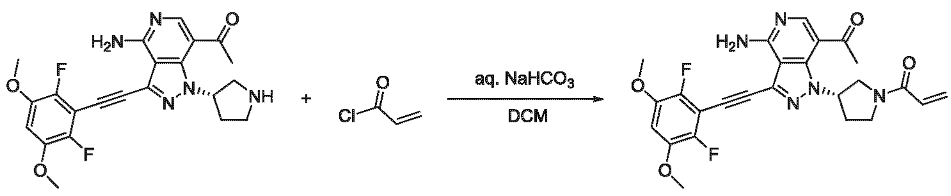


In an ice bath, a solution of acryloyl chloride (52 mg, 574 μmol) in DCM (0.5 mL) was dropwise added to a mixed solution of crude (*S*)-1-(4-amino-3-((2,6-difluoro-3,5-dimethoxyphenyl)ethynyl)-1-(pyrrolidin-3-yl)-1H-pyrazolo[4,3-c]pyridin-7-yl)ethan-1-one (319 mg, 574 μmol) in a saturated $NaHCO_3$ solution (3.2 g) and DCM (5 mL), and the mixture was stirred in an ice bath for 10 minutes. The reaction liquid was separated, the organic layer was concentrated under reduced pressure, and the residue was separated by prep-HPLC to obtain the title compound (27.6 mg, 10%).

MS *m*/*z* (ESI): 496.2 $[M+H]^+$.

$^1H$ NMR (400 MHz, $CDCl_3$) $\delta$ 8.49-8.36 (m, 1H), 7.17-6.80 (m, 2H), 6.73 (t, *J* = 8.1 Hz, 1H), 6.55-6.33 (m, 2H), 6.12-6.02 (m, 1H), 5.75-5.64 (m, 1H), 4.17-3.70 (m, 10H), 2.78-2.36 (m, 5H).

**Reference Example 8**

(*S*)-1-(3-(4-amino-3-((2,6-difluoro-3,5-dimethoxyphenyl)ethynyl)-7-(1-methyl-1H-pyrazol-3-yl)-1H-pyrazolo[4, 3-c]pyridin-1-yl)pyrrolidin-1-yl)prop-2-en-1-one

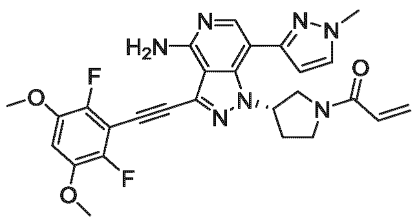

Step I: Preparation of tert-butyl (*S*)-3-(4-amino-3-((2,6-difluoro-3,5-dimethoxyphenyl)ethynyl)-7-(1-methyl-1H-pyrazol-3-yl)-1H-pyrazolo[4, 3-c]pyridin-1-yl)pyrrolidin-1-yl)pyrrolidine-1-carboxylate

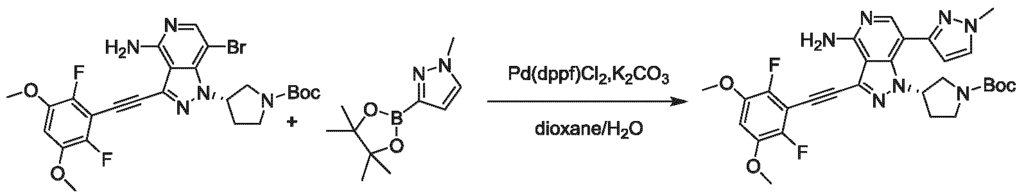


(*S*)-tert-butyl 3-(4-amino-7-bromo-3-((2,6-difluoro-3,5-dimethoxyphenyl)ethynyl)-1H-pyrazolo[4,3-c]pyridin-1-yl)pyrrolidine-1-carboxylate (100 mg, 173 μmol), [1, 1'-bis(diphenylphosphino)ferrocene]palladium dichloride (13 mg, 17 μmol), potassium carbonate (72 mg, 0.52 mmol), and 1-methylpyrazole-3-boronic acid pinacol ester (72 mg, 345 μmol) were suspended in a mixed solution of dioxane (5 mL) and water (1 mL), which was heated to 90°C, and the reaction was stirred for 2 hours. The reaction liquid was cooled and filtered, and the filtrate was concentrated and then subjected to silica gel column chromatography to obtain the title compound (40 mg, 40%).

MS *m*/*z* (ESI): 580.2 $[M+H]^+$.

Step II: Preparation of (S)-3-((2,6-difluoro-3,5-dimethoxyphenyl)ethynyl)-7-(1-methyl-1H-pyrazol-3-yl)-1-(pyrrolidin-3-yl)-1H-pyrazolo[4,3-c]pyridine-4-amino

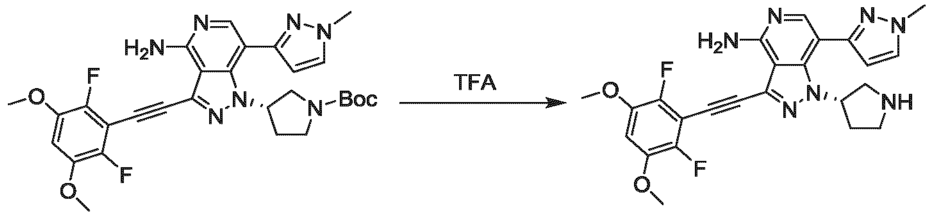


Tert-butyl (*S*)-3-(4-amino-3-((2,6-difluoro-3,5-dimethoxyphenyl)ethynyl)-7-(1-methyl-1H-pyrazol-3-yl)-1H-pyrazolo[4, 3-c]pyridin-1-yl)pyrrolidin-1-yl)pyrrolidine-1-carboxylate (40 mg, 69 umol) was dissolved in a trifluoroacetic acid solution (5 mL), and the mixture was stirred at room temperature for 0.5 hours. The reaction liquid was dried and concentrated to obtain the crude title compound (40 mg).
MS *m*/*z* (ESI): 480.2 $[M+H]^+$.

Step III: Preparation of (*S*)-1-(3-(4-amino-3-((2,6-difluoro-3,5-dimethoxyphenyl)ethynyl)-7-(1-methyl-1H-pyrazol-3-yl)-1H-pyrazolo[4, 3-c]pyridin-1-yl)pyrrolidin-1-yl)prop-2-en-1-one

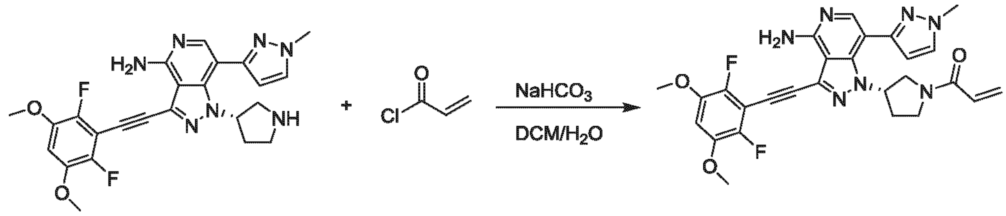


(*S*)-3-((2,6-difluoro-3,5-dimethoxyphenyl)ethynyl)-7-(1-methyl-1H-pyrazol-3-yl)-1-(pyrrolidin-3-yl)-1H-pyrazolo[4,3-c]pyridine-4-amino (35 mg, 73 μmol) and sodium bicarbonate (61 mg, 0.73 mmol) were suspended in a mixed solvent of dichloromethane and water (V/V = 1 : 1, 10 mL), acryloyl chloride (6.6 mg, 73 μmol) was added at 0°C, and the reaction was stirred for 0.2 hours. Methanol was added to quench the reaction. After liquid separation, the organic phase was washed with a saturated aqueous sodium chloride solution (10 mL), the organic phase was collected, dried over anhydrous sodium sulfate, filtered and then concentrated under reduced pressure, and the residue was subjected to prep-HPLC to obtain the title compound (17 mg, 44%).
MS *m*/*z* (ESI): 534.2 $[M+H]^+$.
$^1$H NMR (400 MHz, DMSO-*d*$_6$) δ 8.42 (s, 1H), 7.93 - 7.68 (m, 2H), 7.19 (t, *J* = 8.5 Hz, 1H), 6.77-6.51 (m, 2H), 6.50 (t, *J* = 7.3 Hz, 1H), 6.13 (d, *J* = 16.9 Hz, 1H), 5.67 (td, *J* = 10.2, 2.2 Hz, 1H), 5.53 (dd, *J* = 11.7, 6.0 Hz, 1H), 4.02-3.83 (m, 7H), 3.86-3.42 (m, 3H), 2.51 (s, 3H), 2.44-2.05 (m, 2H).

## Example 1

(*S*)-1-(1-acryloylpyrrolidin-3-yl)-4-amino-3-((4-methoxybenzofuran-6-yl)ethynyl)-1H-pyrazolo[4, 3-c]pyridine-7-carbonitrile

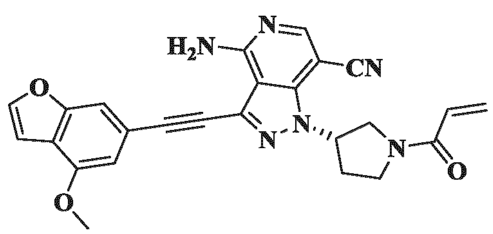

Preparation of (*S*)-1-(1-acryloylpyrrolidin-3-yl)-4-amino-3-((4-methoxybenzofuran-6-yl)ethynyl)-1H-pyrazolo[4, 3-c]pyridine-7-carbonitrile according to Reference Example 1.
MS *m*/*z* (ESI): 453.2 $[M+H]^+$.

**Example 2**

(*S*)-1-(1-acryloylpyrrolidin-3-yl)-4-amino-3-((7-methoxy-3-carbonylisoindolin-5-yl)ethynyl)-1H-pyrazolo[4, 3-c]pyridine-7-carbonitrile

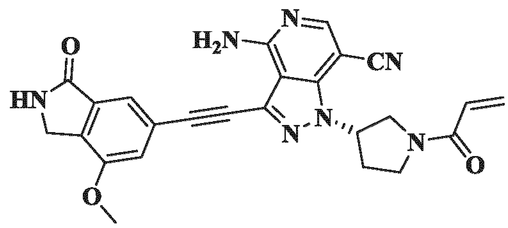

Preparation of (*S*)-1-(1-acryloylpyrrolidin-3-yl)-4-amino-3-((7-methoxy-3-carbonylisoindolin-5-yl)ethynyl)-1H-pyrazolo[4, 3-c]pyridine-7-carbonitrile according to Reference Example 1.
MS *m*/*z* (ESI): 468.2 $[M+H]^+$.

**Example 3**

(*S*)-1-(3-(4-amino-3-((7-methoxy-5-methylbenzo[b]thien-2-yl)ethynyl)-1H-pyrazolo[3, 4-d]pyrimidin-1-yl)pyrrolidin-1-yl) prop-2-en-1-one

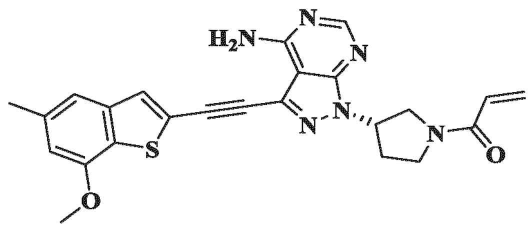

Step I: Preparation of 2-iodo-7-methoxy-5-methylbenzo[b]thiophene

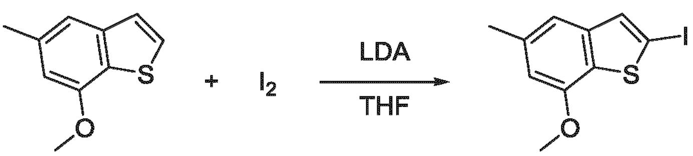


At -78°C, LDA (2 M, 308.55 μL) was dropwise added to a solution of 7-methoxy-5-methylbenzo[b]thiophene (100 mg, 561.01 μmol) in THF (2 mL), and the mixture was stirred for 10 minutes while the temperature was maintained. A solution of iodine (170.87 mg, 673.21 μmol) in THF (1 mL) was then dropwise added, and the resulting mixture was naturally warmed to room temperature and stirred for 0.5 hours. The reaction liquid was quenched with water, distributed between ethyl acetate (10 mL) and a saturated aqueous sodium bisulfite solution (5 mL), the organic layer was washed with a saturated aqueous sodium chloride solution, dried over anhydrous sodium sulfate, and filtered, and the filtrate was concentrated under reduced pressure to obtain the title compound as a light brown oil (105 mg, 62%).
MS *m*/*z* (ESI): 305.0 $[M+H]^+$.
$^1H$ NMR (400 MHz, $CDCl_3$) $\delta$ 7.42 (s, 1H), 7.13 (s, 1H), 6.56 (s, 1H), 3.95 (s, 3H), 2.45 (s, 3H).

Step II: Preparation of ((7-methoxy-5-methylbenzo[b]thien-2-yl)ethynyl)trimethylsilane

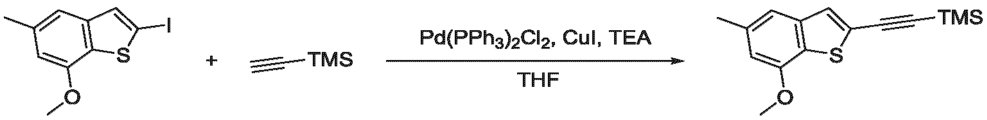


Trimethylethynylsilane (12.92 mg, 0.132 mmol) was added to a mixed solution of 2-iodo-7-methoxy-5-methylbenzo[b]thiophene (20 mg, 0.066 mmol), CuI (1.25 mg, 6.58 μmol), and $Pd(PPh_3)_2Cl_2$ (2.30 mg, 3.29 μmol) in TEA (28 μL) and THF (0.5 mL), and the mixture was stirred at room temperature under nitrogen protection for 0.5 hours. The reaction liquid was concentrated under reduced pressure, and the residue was separated and purified by silica gel column chromatography to obtain the title compound as a brown solid (18 mg, 100%).

MS *m/z* (ESI): 275.1 $[M+H]^+$.

$^1H$ NMR (400 MHz, $CDCl_3$) $\delta$ 7.36 (s, 1H), 7.14 (s, 1H), 6.61 (s, 1H), 3.95 (s, 3H), 2.44 (s, 3H), 0.27 (s, 9H).

Step III: Preparation of 2-ethynyl-7-methoxy-5-methylbenzo[b]thiophene

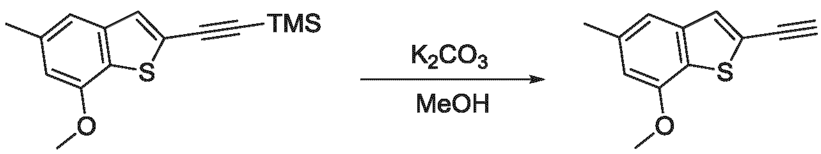


At room temperature, $K_2CO_3$(104 mg, 753.62 μmol) was added to a solution of ((7-methoxy-5-methylbenzo[b]thien-2-yl)ethynyl)trimethylsilane (72 mg, 262.34 μmol) in MeOH (2 mL), and the mixture was stirred at room temperature for 1 hour. The reaction liquid was filtered, the filtrate was concentrated under reduced pressure, and the residue was separated in MTBE(10 mL) and water (10 mL). The organic layer was dried over anhydrous sodium sulfate and filtered, and the filtrate was concentrated under reduced pressure to obtain the title compound as a colorless oil (53 mg, 100%).

MS *m/z* (ESI): 203.1 $[M+H]^+$.

Step IV: Preparation of (S)-tert-butyl 3-(4-amino-3-((7-methoxy-5-methylbenzo[b]thien-2-yl)ethynyl)-1H-pyrazolo[3,4-d]pyrimidin-1-yl)pyrrolidine-1-carboxylate

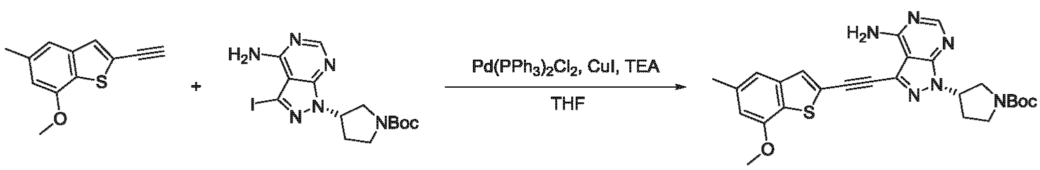


At room temperature, TEA (56.03 mg, 553.71 μmol, 77.23 μL), $Pd(PPh_3)_2Cl_2$ (19.41 mg, 27.69 μmol), and CuI (10.55 mg, 55.37 μmol) were respectively added to a solution of 2-ethynyl-7-methoxy-5-methylbenzo[b]thiophene (56 mg, 276.86 μmol), and (S)-tert-butyl 3-(4-amino-3-iodo-1H-pyrazolo[3,4-d]pyrimidin-1-yl)pyrrolidine-1-carboxylate (142.94 mg, 332.23 μmol) in THF (1 mL), and the mixture was stirred at room temperature under nitrogen protection for 1 hour. The reaction liquid was concentrated under reduced pressure, and the residue was separated and purified by silica gel column chromatography (DCM : MeOH = 100 : 1 to 10 : 1) to obtain the title compound as a light brown oil (139 mg, 100%).

MS *m/z* (ESI): 505.2 $[M+H]^+$.

Step V: Preparation of (*S*)-3-((7-methoxy-5-methylbenzo[b]thien-2-yl)ethynyl)-1-(pyrrolidin-3-yl)-1H-pyrazolo[3,4-d]pyrimidin-4-amine

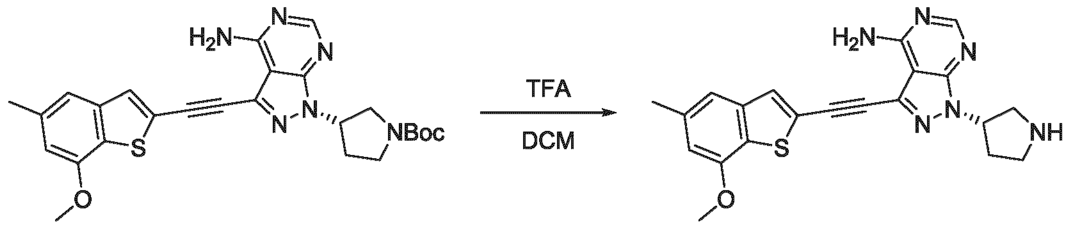


At room temperature, TFA (1 mL) was added to a solution of (*S*)-tert-butyl 3-(4-amino-3-((7-methoxy-5-methylbenzo[b]thien-2-yl)ethynyl)-1H-pyrazolo[3,4-d]pyrimidin-1-yl)pyrrolidine-1-carboxylate (140 mg, 277.45 μmol) in DCM (3 mL), and the mixture was stirred at room temperature for 1 hour. The reaction liquid was concentrated under reduced pressure. The residue was dissolved with DCM (10 mL), a saturated aqueous sodium bicarbonate solution (2 mL) was added with stirring to generate a brown solid, and after filtration, the filter cake was dried under reduced pressure to obtain the title compound as a brown solid (35 mg, 31%).

MS *m*/*z* (ESI): 405.2 [M+H]$^+$.

Step VI: Preparation of (*S*)-1-(3-(4-amino-3-((7-methoxy-5-methylbenzo[b]thien-2-yl)ethynyl)-1H-pyrazolo[3,4-d]pyrimidin-1-yl)pyrrolidin-1-yl)prop-2-en-1-one

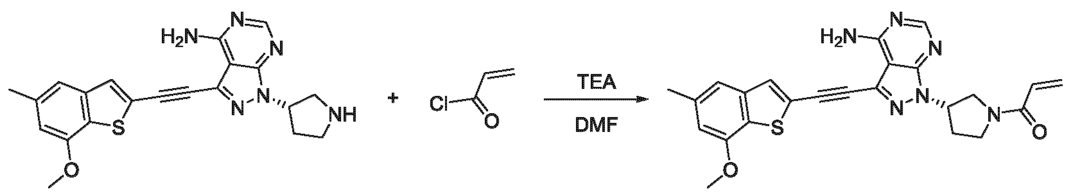


At room temperature, a solution of acryloyl chloride (7.83 mg, 86.53 μmol, 6.99 μL) in DCM (0.1 mL) was dropwise added to a solution of (S)-3-((7-methoxy-5-methylbenzo[b]thien-2-yl)ethynyl)-1-(pyrrolidin-3-yl)-1H-pyrazolo[3,4-d]pyrimidin-4-amine (35 mg, 86.53 μmol, TFA salt) in DMF (0.5 mL), and the mixture was stirred at room temperature for 10 minutes. The reaction liquid was filtered, and the filtrate was separated by prep-HPLC to obtain the title compound as a white solid (5.1 mg, 13%).

MS *m*/*z* (ESI): 459.2 [M+H]$^+$.

$^1$H NMR (400 MHz, DMSO-$d_6$) $\delta$ 8.29 (s, 1H), 7.91 (d, *J* = 1.7 Hz, 1H), 7.32 (s, 1H), 6.91 (s, 1H), 6.72-6.53 (m, 1H), 6.17 (ddd, *J* = 16.7, 7.2, 2.4 Hz, 1H), 5.76-5.63 (m, 1H), 5.59-5.43 (m, 1H), 4.17-3.76 (m, 6H), 3.75-3.57 (m, 1H), 2.44 (s, 3H), 2.43-2.30 (m, 2H).

**Example 4**

(*S*)-1-(3-(4-amino-3-((7-methoxy-5-methylbenzo[d]thiazol-2-yl)ethynyl)-1H-pyrazolo[3, 4-d]pyrimidin-1-yl)pyrrolidin-1-yl)prop-2-en-1-one

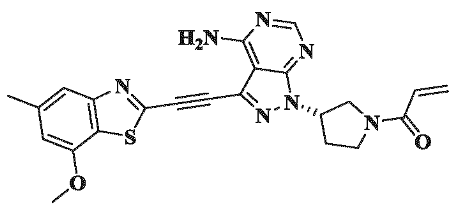

Preparation of (*S*)-1-(3-(4-amino-3-((7-methoxy-5-methylbenzo[d]thiazol-2-yl)ethynyl)-1H-pyrazolo[3, 4-d]pyrimidin-1-yl)pyrrolidin-1-yl)prop-2-en-1-one according to Reference Example 1.

MS *m*/*z* (ESI): 460.2 [M+H]$^+$.

**Example 5**

(*S*)-1-(3-(4-amino-3-((8-methoxy-6-methylimidazo[1, 2-a]pyridin-2-yl)ethynyl)-1H-pyrazolo[3, 4-d]pyrimidin-1-yl)pyrrolidin-1-yl)prop-2-en-1-one

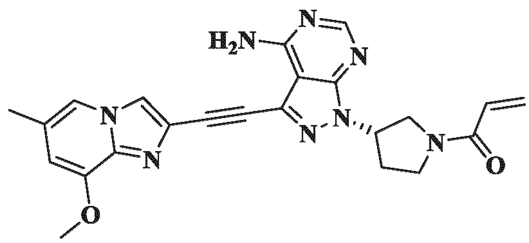

Preparation of (*S*)-1-(3-(4-amino-3-((8-methoxy-6-methylimidazo[1, 2-a]pyridin-2-yl)ethynyl)-1H-pyrazolo[3, 4-d]pyrimidin-1-yl)pyrrolidin-1-yl)prop-2-en-1-one according to Reference Example 1.
MS *m*/*z* (ESI): 443.2 $[M+H]^+$.

**Example 6**

(*S*)-1-(3-(4-amino-7-chloro-3-((7-methoxy-5-methylbenzo[b]thien-2-yl)ethynyl)-1H-pyrazolo[4,3-c]pyridin-1-yl)pyrrolidin-1-yl)prop-2-en-1-one

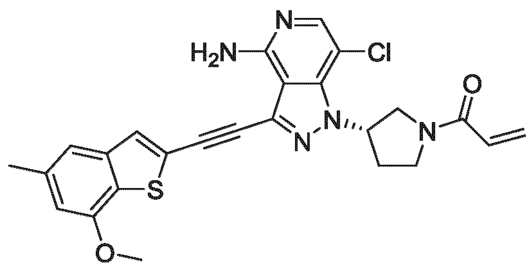

Step I: Preparation of (*S*)-7-chloro-3-iodo-1-(pyrrolidin-3-yl)-1H-pyrazolo[4,3-c]pyridine-4-amine

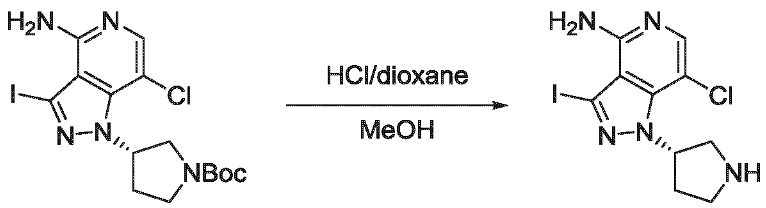


At room temperature, HCl/dioxane (4 M, 1 mL) was added to a solution of (S)-tert-butyl 3-(4-amino-7-chloro-3-iodo-1H-pyrazolo[4,3-c]pyridin-1-yl)pyrrolidine-1-carboxylate (317.6 mg, 684.92 μmol) in MeOH (1 mL), and the mixture was stirred at room temperature for 1 hour. The reaction liquid was concentrated under reduced pressure to obtain the title compound as a brown oil (274 mg, crude, hydrochloride).
MS *m*/*z* (ESI): 364.0 $[M+H]^+$.

Step II: Preparation of (S)-1-(3-(4-amino-7-chloro-3-iodo-1H-pyrazolo[4,3-c]pyridin-1-yl)pyrrolidin-1-yl)prop-2-en-1-one

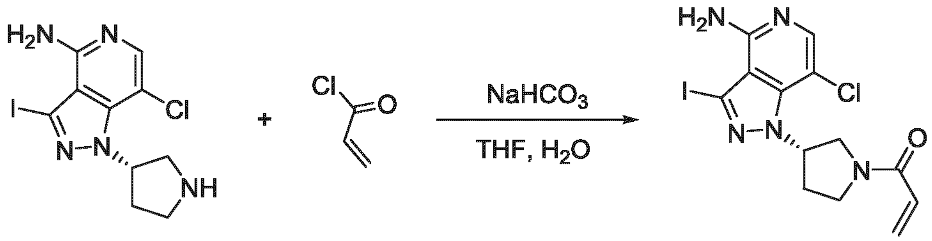


At room temperature, a solution of acryloyl chloride (49.59 mg, 547.94 μmol) in THF (0.5 mL) was dropwise added to a mixed solution of (S)-7-chloro-3-iodo-1-(pyrrolidin-3-yl)-1H-pyrazolo[4,3-c]pyridine-4-amine (0.274 g, 684.92 μmol, hydrochloride) in a saturated aqueous $NaHCO_3$ solution (3.2 mL) and THF (3 mL), and the mixture was stirred at room temperature for 5 minutes. The reaction liquid was separated in DCM (10 mL) and a saturated aqueous sodium chloride solution (10 mL), the organic layer was concentrated under reduced pressure, and the residue was separated and purified by silica gel column chromatography (DCM : MeOH = 100 : 1 to 10 : 1) to obtain the title compound as a brown solid (182 mg, 64% over two steps).

MS *m/z* (ESI): 418.0 $[M+H]^+$.

Step III: Preparation of (S)-1-(3-(4-amino-7-chloro-3-((7-methoxy-5-methylbenzo[b]thien-2-yl)ethynyl)-1H-pyrazolo[4,3-c]pyridin-1-yl)pyrrolidin-1-yl)prop-2-en-1-one

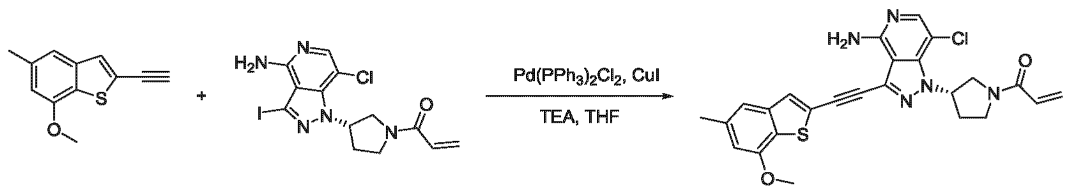


At room temperature, CuI (4.93 mg, 25.86 μmol), $Pd(PPh_3)_2Cl_2$ (9 mg, 12.93 μmol), and TEA (39.25 mg, 387.90 μmol, 54.10 μL) were respectively added to a solution of 2-ethynyl-7-methoxy-5-methylbenzo[b]thiophene (30 mg, 148.32 μmol) and (*S*)-1-(3-(4-amino-7-chloro-3-iodo-1H-pyrazolo[4,3-c]pyridin-1-yl)pyrrolidin-1-yl)prop-2-en-1-one (54 mg, 129.30 μmol) in THF (1 mL). After displacement with nitrogen three times, the solution was warmed to 50°C and stirred for 1 hour. The reaction liquid was concentrated under reduced pressure, and the residue was separated and purified by silica gel column chromatography (DCM : MeOH = 100 : 1 to 10 : 1) to obtain the title compound as a brown solid (5.8 mg, 9%).

MS *m/z* (ESI): 492.1 $[M+H]^+$.

$^1H$ NMR (400 MHz, $CDCl_3$) $\delta$ 7.77-7.69 (m, 1H), 7.59-7.52 (m, 2H), 6.85 (s, 1H), 6.71 (s, 1H), 6.62 (s, 1H), 6.58-6.45 (m, 1H), 6.45-6.38 (m, 1H), 6.06-5.92 (m, 1H), 5.78-5.69 (m, 1H), 4.17-3.95 (m, 6H), 3.89-3.78 (m, 1H), 2.86-2.78 (m, 1H), 2.69-2.63 (m, 3H), 2.59-2.49 (m, 1H).

**Example 7**

(*S*)-1-(3-(7-acetyl-4-amino-3-((1-cyclopropyl-6-fluoro-1H-benzo[d]imidazol-5-yl)ethynyl)-1H-pyrazolo[4,3-c]pyridin-1-yl)pyrrolidin-1-yl)prop-2-en-1-one

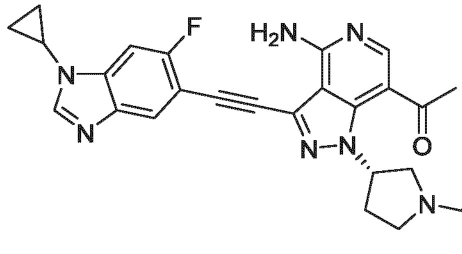

Preparation of (*S*)-1-(3-(7-acetyl-4-amino-3-((1-cyclopropyl-6-fluoro-1H-benzo[d]imidazol-5-yl)ethynyl)-1H-pyrazolo[4,3-c]pyridin-1-yl)pyrrolidin-1-yl)prop-2-en-1-one according to Reference Example 7.
Alternatively, it was synthesized according to the following method:

Step I: Preparation of 4-bromo-N-cyclopropyl-5-fluoro-2-nitroaniline

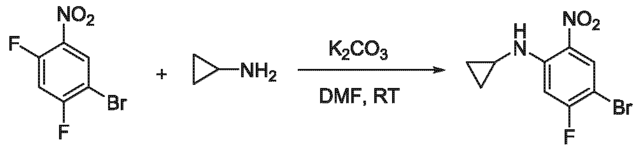


Under nitrogen protection, 1-bromo-2,4-difluoro-5-nitrobenzene (20 g, 84.04 mmol) and potassium carbonate (17.4 g, 126.06 mmol) were suspended in DMF (50 mL), cyclopropylamine (5.3 g, 92.44 mmol) was dropwise added under ice-water bath condition, and the solution was gradually warmed to room temperature and reacted under stirring for 1 hour. TLC monitored that the reaction was complete. The reaction liquid was cooled and then filtered, the filtrate was extracted with ethyl acetate (150 mLx 3) and washed with a saturated aqueous sodium chloride solution (300 mL × 3), the organic phases were collected, dried over anhydrous sodium sulfate, filtered, and concentrated, and the residue was dried to obtain the title compound (20 g, 87%).

Step II: Preparation of 4-bromo-N1-cyclopropyl-5-fluorobenzene-1,2-diamine

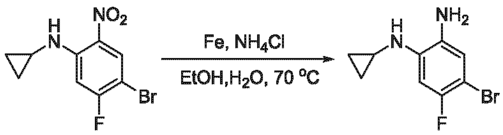


Under nitrogen protection, 4-bromo-N-cyclopropyl-5-fluoro-2-nitroaniline (20 g, 72.71 mmol), reduced iron powder (20.3 g, 363.54 mmol), and ammonium chloride (19.5 g, 363.54 mmol) were suspended in a mixed solution of ethanol (300 mL) and water (100 mL), which was warmed to 70°C, and the reaction was stirred for 2 hours. After the reaction was complete, the reaction liquid was cooled, then filtered, concentrated under reduced pressure to remove a majority of ethanol, then extracted with ethyl acetate (150 mL × 3), and washed with a saturated aqueous sodium chloride solution (100 mL × 3), the organic phases were collected, dried over anhydrous sodium sulfate, filtered, and concentrated, and the residue was dried to obtain the title compound (15 g, 84%).
MS *m*/*z* (ESI): 245.0 $[M+H]^+$.

Step III: Preparation of 5-bromo-1-cyclopropyl-6-fluoro-1H-benzo[d]imidazole

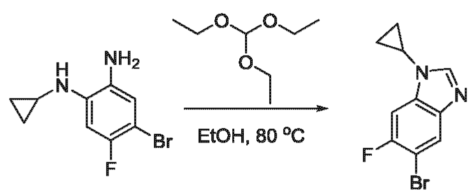


Under nitrogen protection, 4-bromo-N1-cyclopropyl-5-fluorobenzene-1,2-diamine (15 g, 61.2 mmol) and triethyl orthoformate (18.1 g, 122.4 mmol) were dissolved in ethanol (200 mL), which was warmed to 80°C, and the reaction was stirred for 4 hours. After the reaction was complete, the reaction liquid was concentrated under reduced pressure to remove a majority of ethanol, then extracted with ethyl acetate (150 mL × 3), and washed with a saturated aqueous sodium chloride solution (100 mL × 3), the organic phases were collected, dried over anhydrous sodium sulfate, filtered, and concentrated, and the residue was dried to obtain the title compound (12.4 g, 79%).
MS *m*/*z* (ESI): 255.0 $[M+H]^+$.

Step IV: Preparation of 1-cyclopropyl-6-fluoro-5-((trimethylsilyl)ethynyl)-1H-benzo[d]imidazole

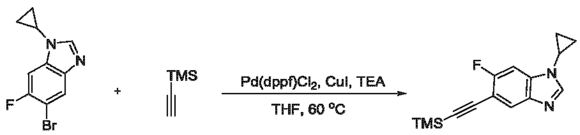


Under nitrogen protection, 5-bromo-1-cyclopropyl-6-fluoro-1H-benzo[d]imidazole (12.4 g, 48.61 mmol), trimethylethynylsilane (7.16 g, 72.92 mmol), Pd(dppf)$Cl_2$ (3.55 g, 4.86 mmol), CuI (1.85 g, 9.72 mmol), and TEA (9.84 g, 97.22 mmol) were suspended in THF (200 mL) and heated to 60°C, and the reaction was stirred for 2 hours. After the reaction was complete, the reaction liquid was cooled and then filtered, extracted with ethyl acetate (150 mL × 3), and washed with a saturated aqueous sodium chloride solution (100 mL × 3), the organic phases were collected, dried over anhydrous sodium sulfate, filtered, and concentrated, and the residue was separated and purified by silica gel column chromatography to obtain the title compound (9 g, 68%).
MS *m*/*z* (ESI): 273.1 $[M+H]^+$.

Step V: Preparation of 1-cyclopropyl-5-ethynyl-6-fluoro-1H-benzo[d]imidazole

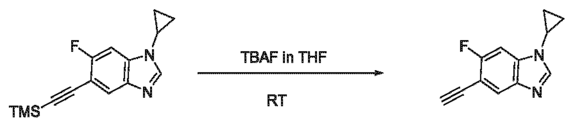


Under nitrogen protection, 1-cyclopropyl-6-fluoro-5-((trimethylsilyl)ethynyl)-1H-benzo[d]imidazole (9 g, 33.04 mmol) and TBAF(13 g, 49.56 mmol) were dissolved in tetrahydrofuran (100 mL), and the reaction was stirred at room temperature for 1 hour. After the reaction was complete, the reaction liquid was extracted with ethyl acetate (150 mL × 3), and washed with a saturated aqueous sodium chloride solution (100 mL × 3), the organic phases were collected, dried over anhydrous sodium sulfate, filtered, and concentrated, and the residue was separated and purified by silica gel column chromatography to obtain the title compound (6 g, 90.7%).
MS *m*/*z* (ESI): 201.0 $[M+H]^+$.

Step VI: Preparation of tert-butyl-(*S*)-3-(4-amino-7-bromo-3-((1-cyclopropyl-6-fluoro-1H-benzo[d]imidazol-5-yl)ethynyl)-1H-pyrazolo[4,3-c]pyridin-1-yl)pyrrolidine-1-carboxylate

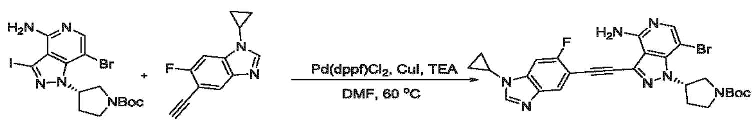


Under nitrogen protection, tert-butyl-(S)-3-(4-amino-5-iodo-7H-pyrrolo[2,3-d]pyrimidin-7-yl)pyrrolidine-1-carboxylate (2.5 g, 4.92 mmol), 1-cyclopropyl-5-ethynyl-6-fluoro-1H-benzo[d]imidazole (1.18 g, 5.90 mmol), Pd(dppf)$Cl_2$ (360 mg, 0.49 mmol), CuI (187 mg, 0.98 mmol), and TEA (996 mg, 9.84 mmol) were suspended in DMF (30 mL) and heated to 60°C, and the reaction was stirred for 2 hours. After the reaction was complete, the reaction liquid was cooled and then filtered, extracted with ethyl acetate (50 mL × 3), and washed with a saturated aqueous sodium chloride solution (50 mL × 3), the organic phases were collected, dried over anhydrous sodium sulfate, filtered, and concentrated, and the residue was separated and purified by silica gel column chromatography to obtain the title compound (1.7 g, 60%).
MS *m*/*z* (ESI): 580.1 $[M+H]^+$.

Step VII: Preparation of tert-butyl-(*S*)-3-(4-amino-7-(1-butoxyvinyl)-3-((1-cyclopropyl-6-fluoro-1H-benzo[d]imidazol-5-yl)ethynyl)-1H-pyrazolo[4,3-c]pyridin-1-yl)pyrrolidine-1-carboxylate

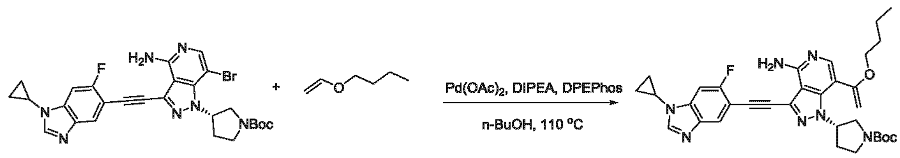


Under nitrogen protection, tert-butyl-(*S*)-3-(4-amino-7-bromo-3-((1-cyclopropyl-6-fluoro-1H-benzo[d]imidazol-5-yl)ethynyl)-1H-pyrazolo[4,3-c]pyridin-1-yl)pyrrolidine-1-carboxylate (650 mg, 1.12 mmol), 1-(vinyloxo)butane (336 mg, 3.36 mmol), palladium acetate (25 mg, 0.11 mmol), N,N-diisopropylethylamine (434 mg, 3.36 mmol), and bis(2-diphenylphosphorusphenyl)ether (121 mg, 0.22 mmol) were suspended in n-butanol (30 mL) and heated to 110°C, and the reaction was stirred for 2 hours. After the reaction was complete, the reaction liquid was cooled and then filtered, the filtrate was extracted with ethyl acetate (30 mL × 3), and washed with a saturated aqueous sodium chloride solution (20 mL × 3), the organic phases were collected, dried over anhydrous sodium sulfate, filtered, and concentrated, and the residue was separated and purified by silica gel column chromatography to obtain the title compound (500 mg, 74%).

MS *m/z* (ESI): 600.1 [M+H]+.

Step VIII: Preparation of (*S*)-1-(3-(7-acetyl-4-amino-3-((1-cyclopropyl-6-fluoro-1H-benzo[d]imidazol-5-yl)ethynyl)-1H-pyrazolo[4,3-c]pyridin-1-yl)pyrrolidin-1-yl)prop-2-en-1-one

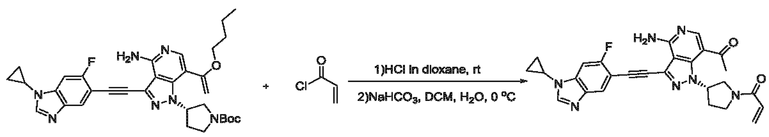


Under nitrogen protection, tert-butyl-(*S*)-3-(4-amino-7-(1-butoxyvinyl)-3-((1-cyclopropyl-6-fluoro-1H-benzo[d]imidazol-5-yl)ethynyl)-1H-pyrazolo[4,3-c]pyridin-1-yl)pyrrolidine-1-carboxylate (500 mg, 0.83 mmol) was dissolved in a solution of hydrogen chloride in dioxane (4 M, 20 mL), and the reaction was stirred at room temperature for 1 hour. The reaction liquid was dried and concentrated and then suspended, together with sodium bicarbonate (353 mg, 4.15 mmol), in a mixed solution of dichloromethane (30 mL) and water (60 mL), a solution of acryloyl chloride (90 mg, 1 mmol) in dichloromethane (20 mL) was dropwise added in an ice-water bath, and the reaction was stirred for 10 minutes. After the reaction was complete, the reaction liquid was extracted with dichloromethane (15 mL × 3) and washed with a saturated aqueous sodium chloride solution (10 mL × 3), the organic phases were collected, dried over anhydrous sodium sulfate, filtered, and concentrated, and the residue was separated and purified by silica gel column chromatography to obtain the title compound (213 mg, 51.6%).

$^1$H NMR (400 MHz, DMSO-$d_6$) $\delta$ 8.58 (d, *J* = 1.6 Hz, 1H), 8.38 (s, 1H), 8.09 (dd, *J* = 6.4, 2.0 Hz, 1H), 7.68 (d, *J* = 9.6 Hz, 2H), 6.70-6.51 (m, 1H), 6.16 (ddd, *J* = 16.8, 4.8, 2.4 Hz, 1H), 5.94 (dp, *J* = 33.2, 5.6 Hz, 1H), 5.69 (ddd, *J* = 16.4, 10.4, 2.4 Hz, 1H), 4.14-3.46 (m, 5H), 2.61 (s, 3H), 2.45 (d, *J* = 6.0 Hz, 1H), 2.34 (d, *J* = 6.4 Hz, 1H), 1.22 (s, 1H), 1.15-1.03 (m, 4H).

MS *m/z* (ESI): 498.2 [M+H]+.

**Example 8**

(*S*)-1-(3-(7-acetyl-4-amino-3-((6-fluoro-1-methyl-1H-benzo[d]imidazol-5-yl)ethynyl)-1H-pyrazolo[4,3-c]pyridin-1-yl)pyrrolidin-1-yl)prop-2-en-1-one

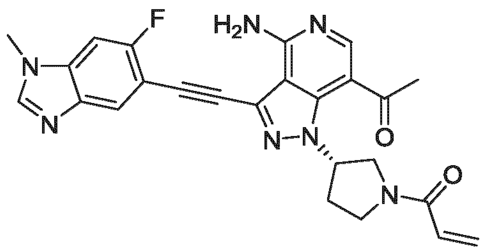

Preparation of (*S*)-1-(3-(7-acetyl-4-amino-3-((6-fluoro-1-methyl-1H-benzo[d]imidazol-5-yl)ethynyl)-1H-pyrazolo[4,3-c]pyridin-1-yl)pyrrolidin-1-yl)prop-2-en-1-one according to Reference Example 7.

It could also be prepared by the following steps:

Step I: Preparation of 6-fluoro-5-iodo-1-methyl-benzo[d]imidazole

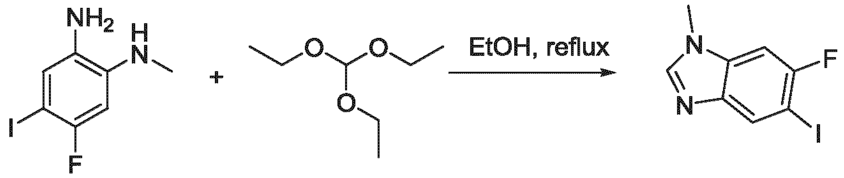


4-Fluoro-5-iodo-N2-methyl-1,2-benzenediamine (4.1 g, 15.41 mmol) was dissolved in anhydrous ethanol (40 mL), triethyl orthoformate (22.84 g, 154.10 mmol) was added, and the mixture was heated to reflux and reacted for 15 hours. The solvent was removed by concentration under reduced pressure, and the residue was washed with a small amount of ethyl acetate to obtain the title compound (3.63 g, 85%).

MS *m*/*z* (ESI): 277.2 $[M+H]^+$.

Step II: Preparation of 5-ethynyl-6-fluoro-1-methyl-benzo[d]imidazole

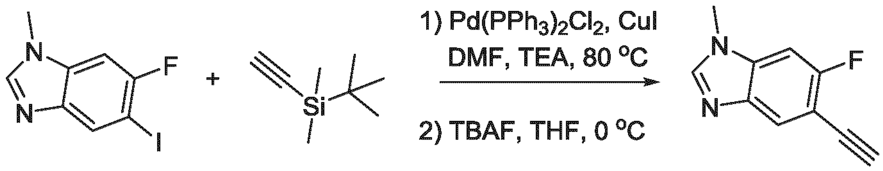


6-Fluoro-5-iodo-1-methyl-benzo[d]imidazole (3.63 g, 13.15 mmol), tert-butyl-ethynyl-dimethylsilane (7.38 g, 52.60 mmol), $Pd(PPh_3)_2Cl_2$ (1.85 g, 2.63 mmol), and cuprous iodide (501 mg, 2.63 mmol) were added to DMF (40 mL). After evacuation and nitrogen displacement three times, triethylamine (40 mL) was added, and after further evacuation and nitrogen displacement once, the solution was heated to 80°C, and the reaction was stirred for 10 hours. After cooling to room temperature, the solvent was removed by concentration under reduced pressure, the residue was dispersed in ethyl acetate, and washed with a dilute aqueous ammonia solution. After separation, the organic phase was collected, dried over anhydrous sodium sulfate, filtered, and concentrated, and the residue was separated by silica gel column chromatography. The resulting crude product was dissolved in THF (10 mL) and cooled to 0°C in an ice-water bath. Under nitrogen protection, a solution of 1M tetrabutylammonium fluoride in THF (20 mL) was dropwise added, and the reaction liquid was reacted under continued stirring for 20 minutes. The solvent was removed by concentration under reduced pressure, and the residue was separated by silica gel column chromatography to obtain the title compound (2.18 g, 95%).

MS *m*/*z* (ESI): 175.2 $[M+H]^+$.

Step III: Preparation of tert-butyl (3S)-3-[4-amino-7-bromo-3-[2-(6-fluoro-1-methyl-benzo[d]imidazol-5-yl)ethynyl]pyrazolo[4,3-c]pyridin-1-yl]pyrrolidine-1-carboxylate

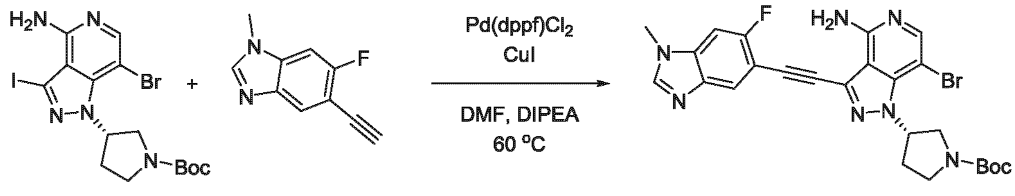


Tert-butyl (3S)-3-(4-amino-7-bromo-3-iodo-pyrazolo[4,3-c]pyridin-1-yl)pyrrolidine-1-carboxylate (508 mg, 1.00 mmol), 5-ethynyl-6-fluoro-1-methyl-benzo[d]imidazole (261 mg, 1.50 mmol), $Pd(dppf)Cl_2$ (73 mg, 0.10 mmol), and

cuprous iodide (38 mg, 0.20 mmol) were added to DMF (15 mL), the system was subjected to evacuation and nitrogen displacement three times, DIPEA (0.75 mL) was added, and after further evacuation and nitrogen displacement once, the solution was heated to 60°C and reacted for 10 hours. After cooling to room temperature, the solvent was removed by concentration under reduced pressure, the residue was dispersed in ethyl acetate, washed with a dilute aqueous ammonia solution, dried over anhydrous sodium sulfate, filtered, and concentrated, and the residue was separated by silica gel column chromatography to obtain the title compound (236 mg, 43%).

MS *m/z* (ESI): 554.2 $[M+H]^+$.

Step IV: Preparation of tert-butyl (3S)-3-[4-amino-7-(1-ethoxyvinyl)-3-[2-(6-fluoro-1-methyl-benzo[d]imidazol-5-yl)ethynyl]pyrazolo[4,3-c]pyridin-1-yl]pyrrolidine-1-carboxylate

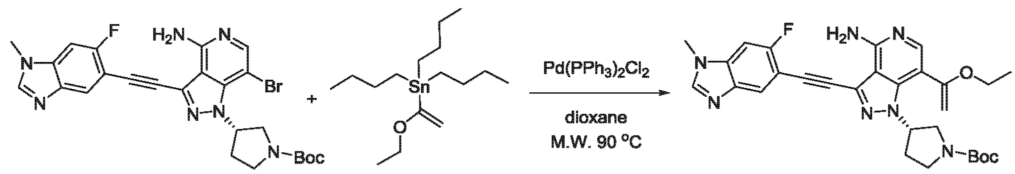


tert-butyl (3S)-3-[4-amino-7-bromo-3-[2-(6-fluoro-1-methyl-benzo[d]imidazol-5-yl)ethynyl]pyrazolo[4,3-c]pyridin-1-yl]pyrrolidine-1-carboxylate (600 mg, 1.08 mmol), tributyl(1-ethoxyvinyl)stannane (1.17 g, 3.25 mmol), and $Pd(PPh_3)_2Cl_2$ (76 mg, 0.11 mmol) were added to dioxane (20 mL), heated to 90°C by microwave and reacted for 3 hours, the solvent was removed by concentration under reduced pressure, and the residue was separated by silica gel column chromatography to obtain (488 mg, 83%).

MS m/z (ESI): 546.3 $[M+H]^+$.

Step V: Preparation of 1-[(3S)-3-[7-acetyl-4-amino-3-[2-(6-fluoro-1-methyl-benzo[d]imidazol-5-yl)ethynyl]pyrazolo[4,3-c]pyridin-1-yl]pyrrolidin-1-yl] -2-propen-1-one

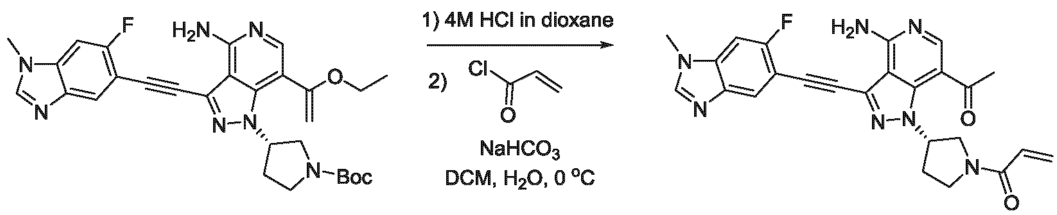


Tert-butyl (3S)-3-[4-amino-7-(1-ethoxyvinyl)-3-[2-(6-fluoro-1-methyl-benzo[d]imidazol-5-yl)ethynyl]pyrazolo [4,3-c]pyridin-1-yl]pyrrolidine-1-carboxylate (488 mg, 894.42 μmol) was dissolved in a small amount of DCM, 4M hydrogen chloride in dioxane (30 mL) was added, and the mixture was stirred, reacted for 30 minutes, and concentrated under reduced pressure. The residue was dispersed in DCM (30 mL). Under ice-water bath cooling, sodium bicarbonate (488 mg, 5.81 mmol) and water (30 mL) were added in order, and the reaction liquid was stirred at 0°C until no bubbles were generated. A solution of acryloyl chloride (81 mg, 894.42 μmol) in DCM (1 mL) was dropwise added slowly, and the reaction was kept at 0°C for several minutes. The reaction product was left to stand for layering to obtain an organic phase. The aqueous phase was extracted by DCM. The organic phases were combined, dried over anhydrous sodium sulfate, filtered, and concentrated, and the residue was separated by silica gel column chromatography and then purified by prep-HPLC to obtain the title compound (19.8 mg, 5%).

MS *m/z* (ESI): 472.2 $[M+H]^+$.

$^1H$ NMR (400 MHz, DMSO-$d_6$) $\delta$ 8.58 (d, $J$ = 2.1 Hz, 1H), 8.34 (s, 1H), 8.09 (dd, $J$ = 6.3, 2.4 Hz, 1H), 7.73 (d, $J$ = 9.8 Hz, 1H), 6.62 (ddd, $J$ = 31.4, 16.8, 10.3 Hz, 1H), 6.16 (ddd, $J$ = 17.0, 5.1, 2.4 Hz, 1H), 6.02-5.86 (m, 1H), 5.68 (ddd, $J$ = 16.8, 10.3, 2.4 Hz, 1H), 4.01 (ddd, $J$ = 15.3, 11.0, 5.3 Hz, 1H), 3.85 (s, 3H), 3.79-3.52 (m, 3H), 2.61 (s, 3H), 2.46-2.31 (m, 2H).

**Example 9**

(*S*)-1-(3-(4-amino-3-((6-fluoro-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one

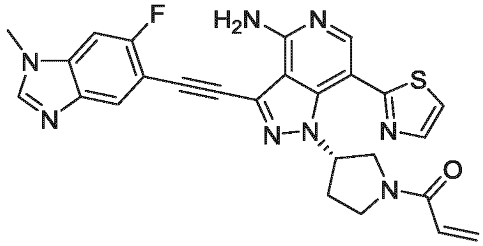

Preparation of (*S*)-1-(3-(4-amino-3-((6-fluoro-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one according to Reference Example 8.

It could also be prepared by the following steps:

Step I: Preparation of tert-butyl (3*S*)-3-[4-amino-3-[2-(6-fluoro-1methyl-benzo[d]imidazol-5-yl)ethynyl]-7-thiazol-2-yl-pyrazolo[4,3-c]pyridin-1-yl]pyrrolidine-1-carboxylate

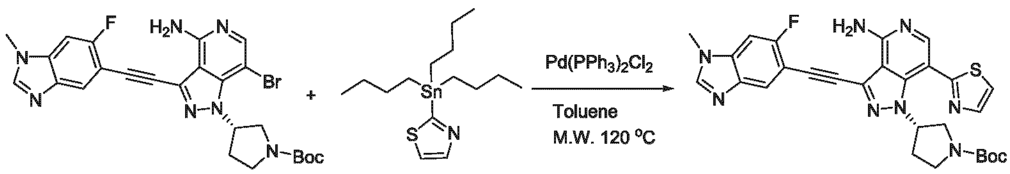


Tert-butyl (3S)-3-[4-amino-7-bromo-3-[2-(6-fluoro-1-methyl-benzo[d]imidazol-5-yl)ethynyl]pyrazolo[4,3-c]pyridin-1-yl]pyrrolidine-1-carboxylate (236 mg, 426 umol), tributyl(thiazol-2-yl)stannane (478 mg, 1.28 mmol), and $Pd(PPh_3)_2Cl_2$ (30 mg, 42.57 μmol) were added to toluene (10 mL). After evacuation and nitrogen displacement three times, the mixture was heated to 120°C by microwave and reacted for 4 hours. After cooling to room temperature, the reaction liquid was diluted with ethyl acetate, a saturated potassium fluoride solution was then added, the reaction was stirred for 30 minutes, the reaction was filtered by diatomaceous earth, and the filtrate was left to stand for layering. After liquid separation, the organic phase was taken, dried over anhydrous sodium sulfate, filtered, and concentrated, and the residue was separated by silica gel column chromatography to obtain the title compound (107 mg, 45%).

MS *m*/*z* (ESI): 559.2 $[M+H]^+$.

Step II: Preparation of 1-[(3S)-3-[4-amino-3-[2-(6-fluoro-1-methyl-benzo[d]imidazol-5-yl)ethynyl]-7-thiazol-2-yl-pyrazolo[4,3-c]pyridin-1-yl]pyrrolidin-1-yl] -2-propen-1-one

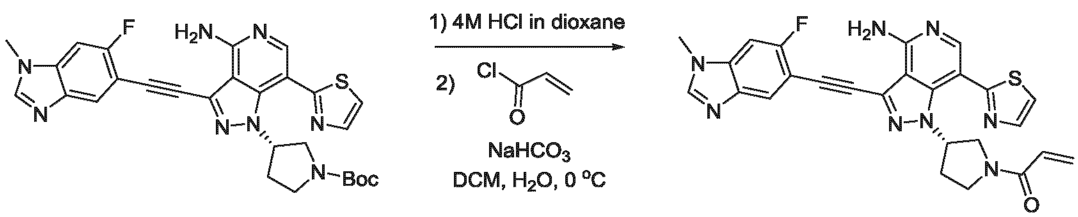


Tert-butyl (3*S*)-3-[4-amino-3-[2-(6-fluoro-1methyl-benzo[d]imidazol-5-yl)ethynyl]-7-thiazol-2-yl-pyrazolo[4,3-c]pyridin-1-yl]pyrrolidine-1-carboxylate (107 mg, 192 μmol) was dissolved in a solution of 4M hydrogen chloride in dioxane (20 mL), and the reaction was stirred for 20 minutes. The solvent was removed by concentration under reduced pressure, the residue is dispersed in DCM (20 mL), a solution of sodium bicarbonate (107 mg, 1.27 mmol) in water (20 mL) was added

at 0°C, and a solution of acryloyl chloride (17 mg, 191.54 μmol) in DCM (1 mL) was then dropwise added. After the reaction was complete, a small amount of methanol was added to quench the reaction, and DCM was added for dilution. After liquid separation, an organic phase was taken, dried over anhydrous sodium sulfate, filtered, and concentrated, and the residue was separated by silica gel column chromatography to obtain (35 mg, 35%).

MS *m/z* (ESI): 513.2 $[M+H]^+$.

$^1H$ NMR (400 MHz, DMSO-$d_6$) $\delta$ 8.33 (s, 1H), 8.09 (dd, *J* = 6.3, 1.6 Hz, 1H), 8.05 (d, *J* = 2.0 Hz, 1H), 8.00 (t, *J* = 3.1 Hz, 1H), 7.84 (dd, *J* = 3.3, 2.0 Hz, 1H), 7.73 (d, *J* = 9.8 Hz, 1H), 7.01 (s, 2H), 6.56 (ddd, *J* = 37.9, 16.7, 10.3 Hz, 1H), 6.17-6.08 (m, 1H), 5.80-5.62 (m, 2H), 3.99-3.82 (m, 4H), 3.79-3.51 (m, 3H), 2.42-2.09 (m, 2H).

## Example 10

(*S*)-1-(3-(4-amino-3-((6-fluoro-1-methyl-1H-benzo[d]imidazol-5-yl)ethynyl)-7-(1-methyl-1H-imidazol-2-yl)-1H-pyrazolo [4,3-c]pyridin-1-yl)pyrrolidin-1-yl)prop-2-en-1-one

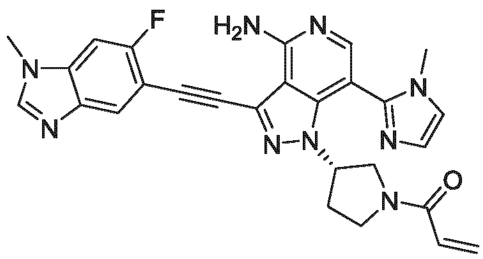

Preparation of (*S*)-1-(3-(4-amino-3-((6-fluoro-1-methyl-1H-benzo[d]imidazol-5-yl)ethynyl)-7-(1-methyl-1H-imidazol-2-yl)-1H-pyrazolo[4,3-c]pyridin-1-yl)pyrrolidin-1-yl)prop-2-en-1-one according to Reference Example 8.

MS *m/z* (ESI): 510.2 $[M+H]^+$.

## Example 11

(*S*)-1-(3-(4-amino-3-((6-fluoro-1-methyl-1H-benzo[d]imidazol-5-yl)ethynyl)-7-(oxazol-2-yl)-1H-pyrazolo[4,3-c]pyridin-1-yl)pyrrolidin-1-yl)prop-2-en-1-one

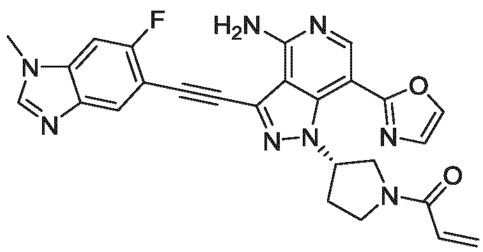

Preparation of (*S*)-1-(3-(4-amino-3-((6-fluoro-1-methyl-1H-benzo[d]imidazol-5-yl)ethynyl)-7-(oxazol-2-yl)-1H-pyrazolo[4,3-c]pyridin-1-yl)pyrrolidin-1-yl)prop-2-en-1-one according to Reference Example 8.

MS *m/z* (ESI): 497.2 $[M+H]^+$.

## Example 12

(*S*)-1-(3-(4-amino-3-((6-fluoro-1-methyl-1H-benzo[d]imidazol-5-yl)ethynyl)-7-(1-methyl-1H-imidazol-4-yl)-1H-pyrazolo [4,3-c]pyridin-1-yl)pyrrolidin-1-yl)prop-2-en-1-one

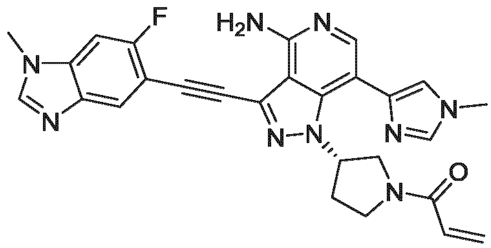

Preparation of (*S*)-1-(3-(4-amino-3-((6-fluoro-1-methyl-1H-benzo[d]imidazol-5-yl)ethynyl)-7-(1-methyl-1H-imidazol-4-yl)-1H-pyrazolo[4,3-c]pyridin-1-yl)pyrrolidin-1-yl)prop-2-en-1-one according to Reference Example 8.
MS *m/z* (ESI): 510.2 $[M+H]^+$.

**Example 13**

1-[(3*S*)-3-[4-amino-3-[2-(6-fluoro-1-methyl-benzo[d]imidazol-5-yl)ethynyl]-7-(1-methylpyrazol-3-yl)pyrazolo[4,3-c]pyridin-1-yl]pyrrolidin-1-yl]-2-propen-1-one

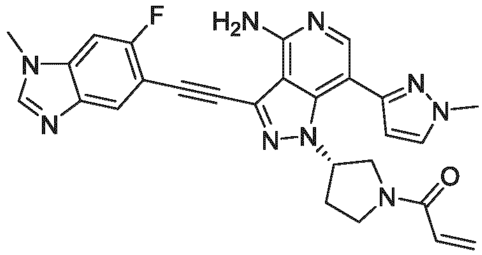

Step I: Preparation of tert-butyl (3*S*)-3-[4-amino-3-[2-(6-fluoro-1-methyl-benzo[d]imidazol-5-yl)ethynyl]-7-(1-methylpyrazol-3-yl)pyrazolo[4,3-c]pyridin-1-yl]pyrrolidine-1-carboxylate

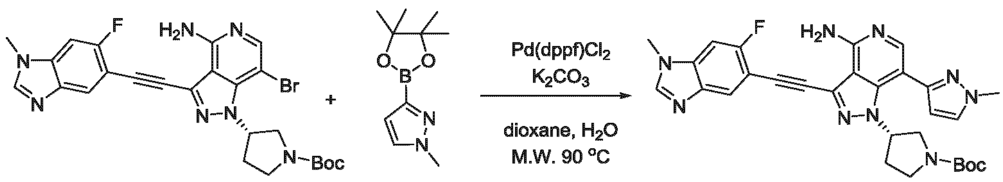


Tert-butyl (3*S*)-3-[4-amino-7-bromo-3-[2-(6-fluoro-1-methyl-benzo[d]imidazol-5-yl)ethynyl]pyrazolo[4,3-c]pyridin-1-yl]pyrrolidine-1-carboxylate (500 mg, 902 μmol), 1-methylpyrazole-3-boronic acid pinacol ester (563 mg, 2.71 mmol), and $Pd(dppf)Cl_2$ (66 mg, 90 μmol) were added to dioxane (18 mL), a solution of potassium carbonate (373 mg, 2.71 mmol) in water (3 mL) was added, and the system was subjected to evacuation and nitrogen displacement three times, heated to 90°C by microwave and reacted for 3 hours. The organic solvent was removed by concentration under reduced pressure. The residue was dispersed in ethyl acetate, washed with a saturated aqueous sodium chloride solution, dried over anhydrous sodium sulfate, filtered, and concentrated, and the residue was separated by silica gel column chromatography to obtain the title compound (205 mg, 41%).
MS *m/z* (ESI): 556.3 $[M+H]^+$.

Step II: Preparation of 1-[(3*S*)-3-[4-amino-3-[2-(6-fluoro-1-methyl-benzo[d]imidazol-5-yl)ethynyl]-7-(1-methylpyrazol-3-yl)pyrazolo[4,3-c]pyridin-1-yl]pyrrolidin-1-yl]-2-propen-1-one

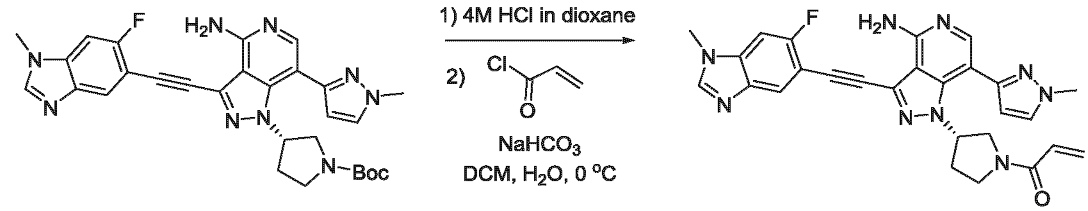


Tert-butyl (3*S*)-3-[4-amino-3-[2-(6-fluoro-1-methyl-benzo[d]imidazol-5-yl)ethynyl]-7-(1-methylpyrazol-3-yl)pyrazolo[4,3-c]pyridin-1-yl]pyrrolidine-1-carboxylate (205 mg, 369 μmol) was dissolved in a small amount of DCM, and a solution of 4M hydrogen chloride in dioxane (20 mL) was added under stirring and reacted for 30 minutes. The reaction product was concentrated under reduced pressure, and the residue was dispersed in water (20 mL). Under ice-water bath cooling, sodium bicarbonate (205 mg, 2.44 mmol) and DCM (20 mL) were added in order, and the reaction liquid was stirred at 0°C for several minutes. A solution of acryloyl chloride (34 mg, 369 μmol) in DCM (1 mL) was dropwise added slowly, and the stirred reaction was continued for several minutes. The reaction product was left to stand for layering to obtain an organic phase. The aqueous phase was extracted by DCM. The organic phases were combined, dried over anhydrous sodium sulfate, filtered, and concentrated, and the residue was separated by silica gel column chromatography and then purified by prep-HPLC to obtain the title compound (46.5 mg, 25%).
MS *m/z* (ESI): 510.2 $[M+H]^+$.

**Example 14**

1-[(3*S*)-3-[4-amino-3-[2-(6-fluoro-1-methyl-benzo[d]imidazol-5-yl)ethynyl]-7-thiazol-4-yl-pyrazolo[4,3-c]pyridin-1-yl]pyrrolidin-1-yl] -2-propen-1-one

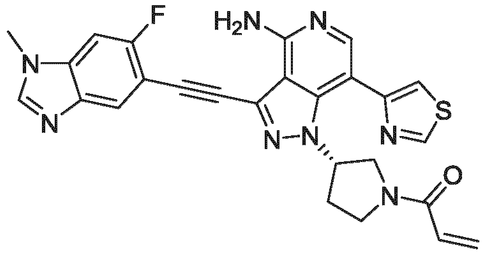

Step I: Preparation of tert-butyl (3*S*)-3-[4-amino-3-[2-(6-fluoro-1methyl-benzo[d]imidazol-5-yl)ethynyl]-7-thiazol-4-yl-pyrazolo[4,3-c]pyridin-1-yl]pyrrolidine-1-carboxylate

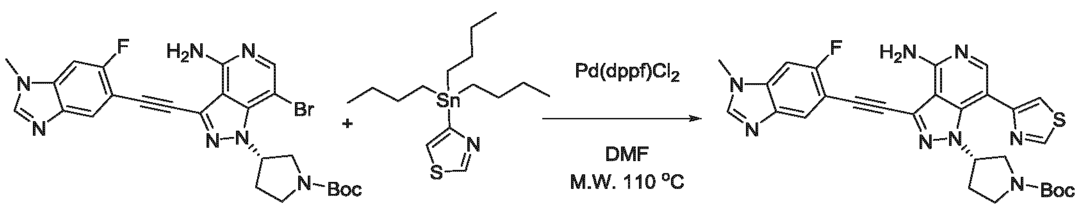


Tert-butyl (3*S*)-3-[4-amino-7-bromo-3-[2-(6-fluoro-1-methyl-benzo[d]imidazol-5-yl)ethynyl]pyrazolo[4,3-c]pyridin-1-yl]pyrrolidine-1-carboxylate (1.2 g, 2.16 mmol), tributyl(thiazol-4-yl)stannane (2.43 g, 6.49 mmol), and $Pd(PPh_3)_2Cl_2$ (151.92 mg, 216.45 μmol) were added to dry DMF (30 mL). The system was subjected to evacuation and nitrogen displacement for protection, heated to 110°C by microwave and reacted for 45 minutes. The solvent was removed by concentration under reduced pressure, the residue was dissolved in ethyl acetate, a saturated aqueous potassium fluoride solution was added, the obtained reaction liquid was stirred at room temperature and reacted for 30 minutes, and the reaction liquid was separated to obtain an organic phase, which was dried over anhydrous sodium sulfate, filtered, and concentrated, and the residue was separated by silica gel column chromatography to obtain (389 mg, 32%).

MS *m/z* (ESI): 559.2 $[M+H]^+$.

Step II: Preparation of 1-[(3*S*)-3-[4-amino-3-[2-(6-fluoro-1-methyl-benzo[d]imidazol-5-yl)ethynyl]-7-thiazol-4-yl-pyrazolo[4,3-c]pyridin-1-yl]pyrrolidin-1-yl] -2-propen-1-one

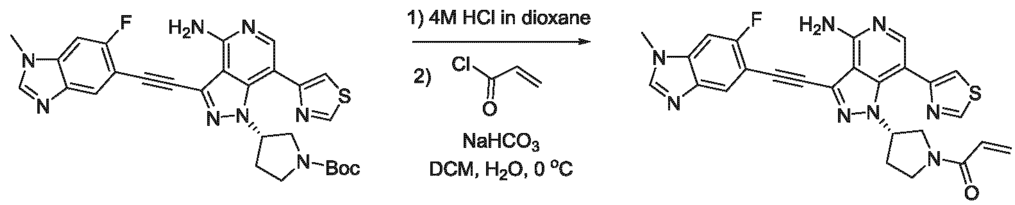


Tert-butyl (3*S*)-3-[4-amino-3-[2-(6-fluoro-1-methyl-benzo[d]imidazol-5-yl)ethynyl]-7-thiazol-4-yl-pyrazolo[4,3-c]pyridin-1-yl]pyrrolidine-1-carboxylate (389 mg, 696 μmol) was dissolved in a small amount of DCM, and 4M hydrogen chloride in dioxane (20 mL) was added under stirring. The reaction liquid was stirred for 30 minutes. The solvent was removed by concentration under reduced pressure, and the residue was dispersed in water (20 mL). Under ice-water bath cooling, sodium bicarbonate (400 mg, 4.76 mmol) and DCM (20 mL) were added in order, and stirring was continued until no bubbles were generated. A solution of acryloyl chloride (63 mg, 696.35 μmol) in DCM (1 mL) was dropwise added, and the reaction was stirred at 0°C for several minutes. The reaction liquid was diluted with DCM and left to stand for layering, and the organic phase was separated, dried over anhydrous sodium sulfate, filtered, and concentrated, and the residue was separated by silica gel column chromatography and then purified by prep-HPLC to obtain the title compound (81 mg, 23%).
MS m/z (ESI): 513.2 $[M+H]^+$.

**Example 15**

(*S*)-1-(3-(4-amino-3-((6-fluoro-1-methyl-1H-benzo[d]imidazol-5-yl)ethynyl)-7-(isothiazol-3-yl)-1H-pyrazolo[4,3-c]pyridin-1-yl)pyrrolidin-1-yl)prop-2-en-1-one

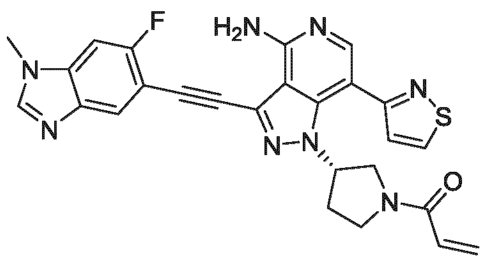

Preparation of (*S*)-1-(3-(4-amino-3-((6-fluoro-1-methyl-1H-benzo[d]imidazol-5-yl)ethynyl)-7-(isothiazol-3-yl)-1H-pyrazolo[4,3-c]pyridin-1-yl)pyrrolidin-1-yl)prop-2-en-1-one according to Reference Example 13.
MS *m/z* (ESI): 513.2 $[M+H]^+$.

**Example 16**

(*S*)-1-(3-(4-amino-3-((6-fluoro-1-methyl-1H-benzo[d]imidazol-5-yl)ethynyl)-7-(pyrimidin-4-yl)-1H-pyrazolo[4,3-c]pyridin-1-yl)pyrrolidin-1-yl)prop-2-en-1-one

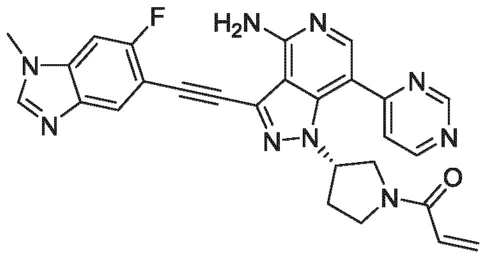

Preparation of (*S*)-1-(3-(4-amino-3-((6-fluoro-1-methyl-1H-benzo[d]imidazol-5-yl)ethynyl)-7-(pyrimidin-4-yl)-1H-pyrazolo[4,3-c]pyridin-1-yl)pyrrolidin-1-yl)prop-2-en-1-one according to Reference Example 13.
MS *m*/*z* (ESI): 508.2 $[M+H]^+$.

**Example 17**

(*S*)-1-(3-(4-amino-3-((6-fluoro-1-methyl-1H-benzo[d]imidazol-5-yl)ethynyl)-7-(pyridazin-3-yl)-1H-pyrazolo[4,3-c]pyridin-1-yl)pyrrolidin-1-yl)prop-2-en-1-one

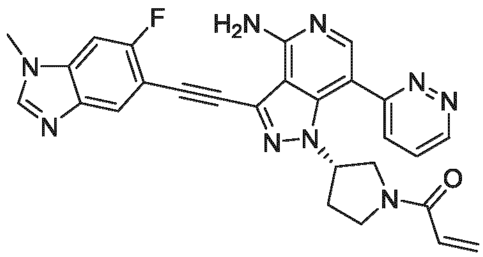

Preparation of (*S*)-1-(3-(4-amino-3-((6-fluoro-1-methyl-1H-benzo[d]imidazol-5-yl)ethynyl)-7-(pyridazin-3-yl)-1H-pyrazolo[4,3-c]pyridin-1-yl)pyrrolidin-1-yl)prop-2-en-1-one according to Reference Example 13.
MS m/z (ESI): 508.2 $[M+H]^+$.

**Example 18**

(*S*)-1-(3-(4-amino-3-((6-fluoro-1-methyl-1H-benzo[d]imidazol-5-yl)ethynyl)-7-methyl-1H-pyrazolo[4,3-c]pyridin-1-yl) pyrrolidin-1-yl)prop-2-en-1-one

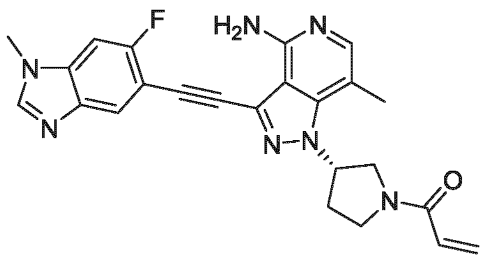

Preparation of (*S*)-1-(3-(4-amino-3-((6-fluoro-1-methyl-1H-benzo[d]imidazol-5-yl)ethynyl)-7-methyl-1H-pyrazolo[4,3-c]pyridin-1-yl)pyrrolidin-1-yl)prop-2-en-1-one according to Reference Example 13.
MS *m*/*z* (ESI): 444.2 $[M+H]^+$.

**Example 19**

(*S*)-1-(3-(4-amino-7-ethyl-3-((6-fluoro-1-methyl-1H-benzo[d]imidazol-5-yl)ethynyl)-1H-pyrazolo[4,3-c]pyridin-1-yl)pyrrolidin-1-yl)prop-2-en-1-one

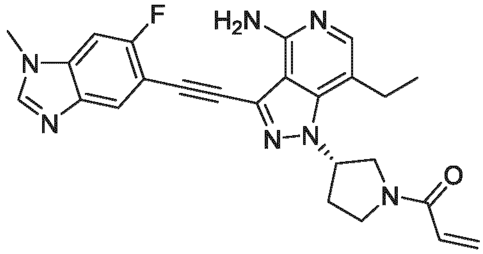

Preparation of (*S*)-1-(3-(4-amino-7-ethyl-3-((6-fluoro-1-methyl-1H-benzo[d]imidazol-5-yl)ethynyl)-1H-pyrazolo [4,3-c]pyridin-1-yl)pyrrolidin-1-yl)prop-2-en-1-one according to Reference Example 13.

MS *m*/*z* (ESI): 458.2 $[M+H]^+$.

**Example 20**

(*S*)-1-(3-(4-amino-7-cyclopropyl-3-((6-fluoro-1-methyl-1H-benzo[d]imidazol-5-yl)ethynyl)-1H-pyrazolo[4,3-c]pyridin-1-yl)pyrrolidin-1-yl)prop-2-en-1-one

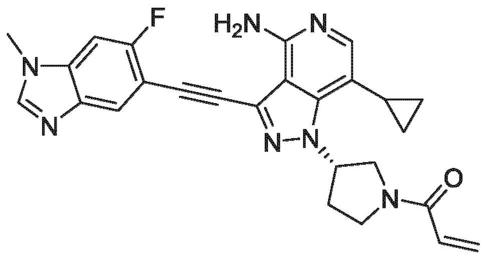

Preparation of (*S*)-1-(3-(4-amino-7-cyclopropyl-3-((6-fluoro-1-methyl-1H-benzo[d]imidazol-5-yl)ethynyl)-1H-pyrazolo[4,3-c]pyridin-1-yl)pyrrolidin-1-yl)prop-2-en-1-one according to Reference Example 13.

MS *m*/*z* (ESI): 470.2 $[M+H]^+$.

**Example 21**

(*S*)-1-(3-(4-amino-7-chloro-3-((6-fluoro-1-methyl-1H-benzo[d]imidazol-5-yl)ethynyl)-1H-pyrazolo[4,3-c]pyridin-1-yl)pyrrolidin-1-yl)prop-2-en-1-one

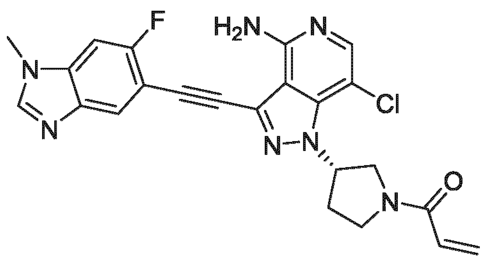

Preparation of (*S*)-1-(3-(4-amino-7-chloro-3-((6-fluoro-1-methyl-1H-benzo[d]imidazol-5-yl)ethynyl)-1H-pyrazolo[4,3-c]pyridin-1-yl)pyrrolidin-1-yl)prop-2-en-1-one according to Reference Example 13.
MS *m/z* (ESI): 464.2 [M+H]$^+$.

**Example 22**

(*S*)-1-(3-(4-amino-3-((1-ethyl-6-fluoro-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one

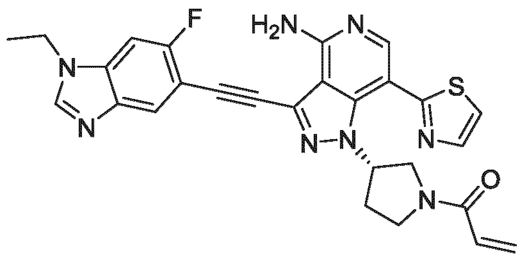

Preparation of (*S*)-1-(3-(4-amino-3-((1-ethyl-6-fluoro-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one according to Reference Example 9.
$^1$H NMR (400 MHz, DMSO-$d_6$) $\delta$ 8.41 (s, 1H), 8.09 (dd, *J* = 6.4, 1.5 Hz, 1H), 8.04 (d, *J* = 1.8 Hz, 1H), 8.00 (t, *J* = 3.1 Hz, 1H), 7.84 (dd, *J* = 3.3, 2.0 Hz, 1H), 7.79 (d, *J* = 9.9 Hz, 1H), 7.01 (s, 2H), 6.56 (ddd, *J* = 37.3, 16.8, 10.3 Hz, 1H), 6.13 (dd, *J* = 16.1, 2.8 Hz, 1H), 5.85-5.58 (m, 2H), 4.29 (q, *J* = 7.2 Hz, 2H), 4.00-3.84 (m, 1H), 3.81-3.64 (m, 2H), 3.57 (dt, *J* = 14.3, 7.4 Hz, 1H), 2.42-2.07 (m, 2H), 1.41 (t, *J* = 7.2 Hz, 3H).
MS *m/z* (ESI): 527.2 [M+H]$^+$.

**Example 23**

(*S*)-1-(3-(4-amino-3-((1-ethyl-6-fluoro-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one

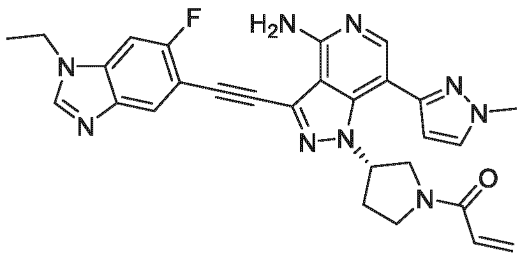

Preparation of (*S*)-1-(3-(4-amino-3-((1-ethyl-6-fluoro-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one according to Reference Example 13.
MS *m/z* (ESI): 524.2 [M+H]$^+$.

**Example 24**

(*S*)-1-(3-(7-acetyl-4-amino-3-((1-ethyl-6-fluoro-1H-benzo[d]imidazol-5-yl)ethynyl)-1H-pyrazolo[4,3-c]pyridin-1-yl)pyrrolidin-1-yl)prop-2-en-1-one

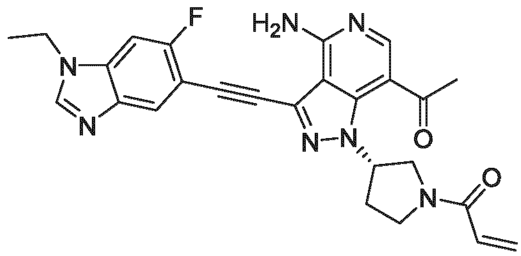

Preparation of (*S*)-1-(3-(7-acetyl-4-amino-3-((1-ethyl-6-fluoro-1H-benzo[d]imidazol-5-yl)ethynyl)-1H-pyrazolo[4,3-c]pyridin-1-yl)pyrrolidin-1-yl)prop-2-en-1-one according to Reference Example 8.

MS *m*/*z* (ESI): 486.2 $[M+H]^+$.

$^1$H NMR (400 MHz, DMSO) $\delta$ 8.51 (d, *J* = 2.0 Hz, 1H), 8.35 (s, 1H), 8.03 (dd, *J* = 6.4, 2.4 Hz, 1H), 7.73 (d, *J* = 9.9 Hz, 1H), 6.55 (dd, 16.7, 10.3 Hz, 1H), 6.09 (dd, *J* = 16.8, 5.0, 1H), 5.87 (dp, *J* = 33.2, 5.8 Hz, 1H), 5.62 (dd, *J* = 16.6, 10.3, 1H), 4.22 (q, *J* = 7.3 Hz, 2H), 3.91-3.45 (m,5H), 3.20 (s, 1H), 2.55 (s, 3H), 2.38 (q, *J* = 7.3 Hz, 1H), 1.35 (t, *J* = 7.2 Hz, 3H), 1.16 (s, 1H).

**Example 25**

(*S*)-1-(3-(4-amino-3-((6-fluoro-1-(methyl-d3)-1Hbenzo[d]imidazol-5-yl)ethynyl)-7-(thiazol-2-yl)-1Hpyrazolo[4,3-c]pyridin-1-yl)pyrrolidin-1-yl)prop-2-en-1-one

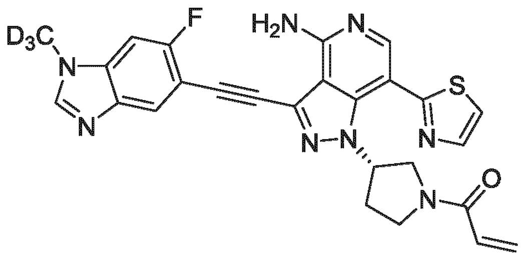

Preparation of (*S*)-1-(3-(4-amino-3-((6-fluoro-1-(methyl-d3)-1Hbenzo[d]imidazol-5-yl)ethynyl)-7-(thiazol-2-yl)-1Hpyrazolo[4,3-c]pyridin-1-yl)pyrrolidin-1-yl)prop-2-en-1-one according to Reference Example 9.

MS *m*/*z* (ESI): 516.2 $[M+H]^+$.

$^1$H NMR (400 MHz, DMSO-$d_6$) $\delta$ 8.34 (s, 1H), 8.10 (dd, *J* = 6.4, 1.6 Hz, 1H), 8.05 (d, *J* = 2.0 Hz, 1H), 8.03-7.98 (m, 1H), 7.85 (dd, *J* = 3.2, 2.0 Hz, 1H), 7.74 (d, *J* = 9.6 Hz, 1H), 7.02 (s, 2H), 6.57 (ddd, *J* = 37.6, 16.8, 10.4 Hz, 1H), 6.14 (dt, *J* = 16.8, 1.6 Hz, 1H), 5.80-5.62 (m, 1H), 4.01-3.85 (m, 1H), 3.81-3.64 (m, 2H), 3.58 (dt, *J* = 14.4, 7.2 Hz, 1H), 2.44-2.08 (m, 3H).

**Example 26**

(*S*)-1-(3-(4-amino-3-((6-fluoro-1-(methyl-d3)-1Hbenzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one

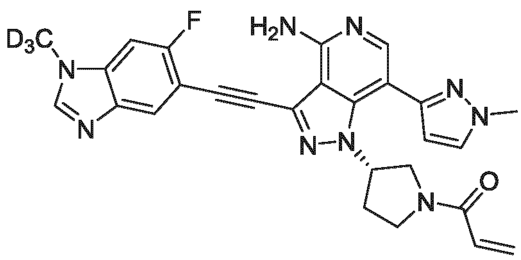

Preparation of (*S*)-1-(3-(4-amino-3-((6-fluoro-1-(methyl-d3)-1Hbenzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one according to Reference Example 13.
MS *m/z* (ESI): 513.2 [M+H]+.

**Example 27**

(*S*)-1-(3-(7-acetyl-4-amino-3-((6-fluoro-1-(methyl-d3)-1Hbenzo[d]imidazol-5-yl)ethynyl)-1Hpyrazolo[4,3-c]pyridin-1-yl)pyrrolidin-1-yl)prop-2-en-1-one

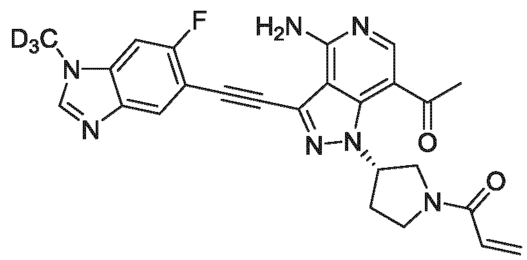

Preparation of (*S*)-1-(3-(7-acetyl-4-amino-3-((6-fluoro-1-(methyl-d3)-1Hbenzo[d]imidazol-5-yl)ethynyl)-1Hpyrazolo[4,3-c]pyridin-1-yl)pyrroldin-1-yl)prop-2-en-1-one according to Reference Example 8.
MS *m/z* (ESI): 475.2 [M+H]+.

**Example 28**

(*S*)-1-(3-(4-amino-3-((4,6-difluoro-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one

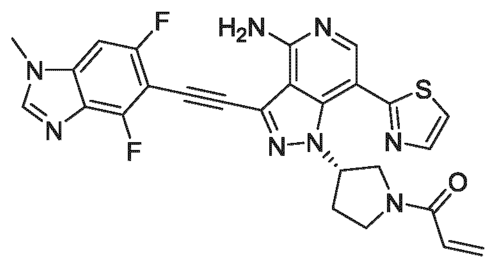

Preparation of (*S*)-1-(3-(4-amino-3-((4,6-difluoro-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one according to Reference Example 9.
MS *m/z* (ESI): 531.2 [M+H]+.
$^1$H NMR (400 MHz, DMSO-*d*$_6$) $\delta$ 8.40 (s, 1H), 8.03-7.92 (m, 1H), 7.92-7.70 (m, 2H), 7.67 (t, *J* = 15.0 Hz, 1H), 6.61 (dd, *J* = 16.6, 10.3 Hz, 1H), 6.55-6.37 (m, 1H), 6.13 (dd, *J* = 16.7, 2.1 Hz, 1H), 5.79-5.59 (m, 1H), 5.33 (t, *J* = 4.7 Hz, 1H), 5.11 (s, 2H), 4.00-3.79 (m, 3H), 2.35 (d, *J* = 12.8 Hz, 1H), 2.18 (d, *J* = 50.7 Hz, 2H), 2.00 (dd, *J* = 14.9, 6.7 Hz, 2H).

**Example 29**

(*S*)-1-(3-(4-amino-3-((4,6-difluoro-1-methyl-1H-benzo[d]imidazol-5-yl)ethynyl)-7-(thiazol-4-yl)-1H-pyrazolo[4,3-c]pyridin-1-yl)pyrrolidin-1-yl)prop-2-en-1-one

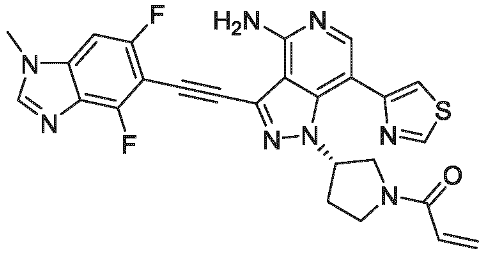

Preparation of (*S*)-1-(3-(4-amino-3-((4,6-difluoro-1-methyl-1H-benzo[d]imidazol-5-yl)ethynyl)-7-(thiazol-4-yl)-1H-pyrazolo[4,3-c]pyridin-1-yl)pyrrolidin-1-yl)prop-2-en-1-one according to Reference Example 14.

MS *m*/*z* (ESI): 531.2 $[M+H]^+$.

$^1$H NMR (400 MHz, DMSO-$d_6$) $\delta$ 8.33 (s, 1H), 7.99 (d, *J* = 2.0 Hz, 1H), 7.95 (t, *J* = 3.1 Hz, 1H), 7.79 (dd, *J* = 3.3, 2.6 Hz, 1H), 7.62 (d, *J* = 9.1 Hz, 1H), 6.60-6.40 (m, 2H),6.07 (dd, *J* = 16.8, 2.3 Hz, 1H), 5.69 (s, 1H), 5.67-5.50 (m, 1H), 3.84-3.71 (m, 3H), 3.74-3.55 (m, 2H), 3.55-3.40 (m, 2H), 2.30 (d, *J* = 25.9 Hz, 1H), 2.23-1.95 (m, 2H).

**Example 30**

(*S*)-1-(3-(4-amino-3-((4,6-difluoro-1-methyl-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one

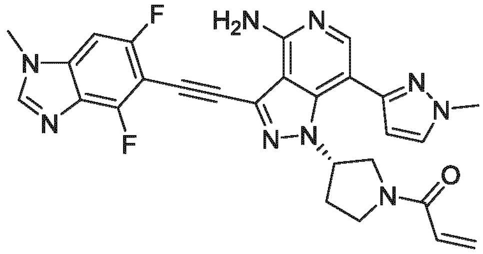

Preparation of (*S*)-1-(3-(4-amino-3-((4,6-difluoro-1-methyl-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one according to Reference Example 13.

MS *m*/*z* (ESI): 528.2 $[M+H]^+$.

**Example 31**

(*S*)-1-(3-(7-acetyl-4-amino-3-((4,6-difluoro-1-methyl-1H-benzo[d]imidazol-5-yl)ethynyl)-1H-pyrazolo[4,3-c]pyridin-1-yl) pyrrolidin-1-yl)prop-2-en-1-one

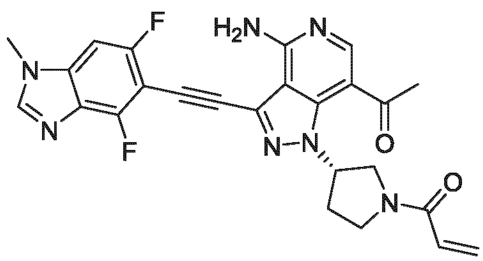

Preparation of (*S*)-1-(3-(7-acetyl-4-amino-3-((4,6-difluoro-1-methyl-1H-benzo[d]imidazol-5-yl)ethynyl)-1H-pyrazolo[4,3-c]pyridin-1-yl)pyrrolidin-1-yl)prop-2-en-1-one according to Reference Example 8.

MS *m*/*z* (ESI): 490.2 $[M+H]^+$.

$^1$H NMR (400 MHz, DMSO-$d_6$) $\delta$ 8.59 (d, *J* = 2.2 Hz, 1H), 7.68 (d, *J* = 9.2 Hz, 1H), 6.73-6.64 (m, 1H), 6.63-6.48 (m, 2H), 6.16 (dd, *J* = 16.9, 2.4 Hz, 1H), 5.94 (d, *J* = 30.9 Hz, 1H), 5.68 (dd, *J* = 16.3, 2.4 Hz, 1H), 5.33 (t, *J* = 4.6 Hz, 1H),

4.14-3.94 (m, 1H), 3.90-3.80 (m,3H), 3.73-3.55 (m, 1H), 2.71-2.55 (m, 2H), 2.34 (d, *J* = 7.4 Hz, 1H), 2.00 (dd, *J* = 15.1, 6.8 Hz,3H), 1.46 (s, 1H).

**Example 32**

(*S*)-1-(3-(4-amino-3-((4,6-difluoro-1-methyl-1H-benzo[d]imidazol-5-yl)ethynyl)-7-(oxazol-2-yl)-1H-pyrazolo[4,3-c]pyridin-1-yl)pyrrolidin-1-yl)prop-2-en-1-one

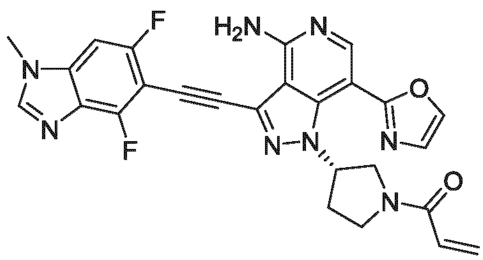

Preparation of (*S*)-1-(3-(4-amino-3-((4,6-difluoro-1-methyl-1H-benzo[d]imidazol-5-yl)ethynyl)-7-(oxazol-2-yl)-1H-pyrazolo[4,3-c]pyridin-1-yl)pyrrolidin-1-yl)prop-2-en-1-one according to Reference Example 13.
MS *m*/*z* (ESI): 515.2 $[M+H]^+$.

Example 33

(*S*)-1-(3-(4-amino-7-cyclopropyl-3-((4,6-difluoro-1-methyl-1H-benzo[d]imidazol-5-yl)ethynyl)-1H-pyrazolo[4,3-c]pyridin-1-yl)pyrrolidin-1-yl)prop-2-en-1-one

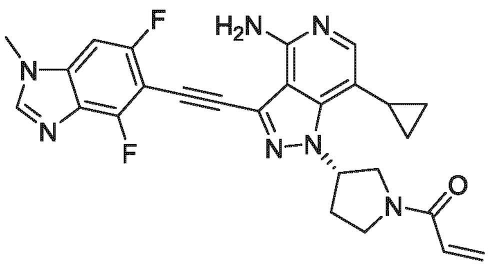

Preparation of (*S*)-1-(3-(4-amino-7-cyclopropyl-3-((4,6-difluoro-1-methyl-1H-benzo[d]imidazol-5-yl)ethynyl)-1H-pyrazolo[4,3-c]pyridin-1-yl)pyrrolidin-1-yl)prop-2-en-1-one according to Reference Example 13.
MS *m*/*z* (ESI): 488.2 $[M+H]^+$.

**Example 34**

(*S*)-1-(3-(4-amino-3-((1-cyclopropyl-6-fluoro-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one

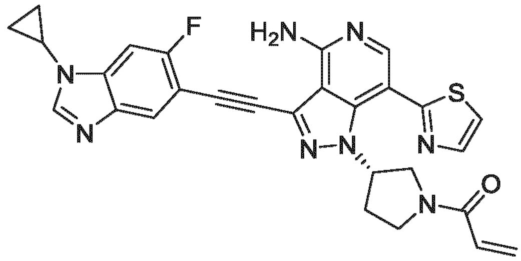

Preparation of (*S*)-1-(3-(4-amino-3-((1-cyclopropyl-6-fluoro-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one according to Reference Example 9.
MS *m/z* (ESI): 539.2 $[M+H]^+$.

**Example 35**

(*S*)-1-(3-(4-amino-3-((1-cyclopropyl-6-fluoro-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one

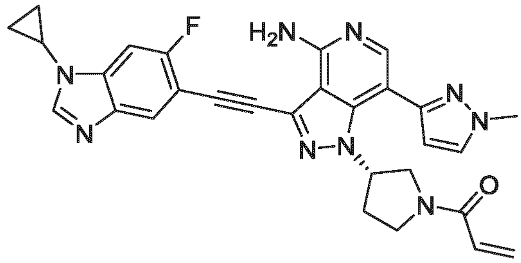

Preparation of (*S*)-1-(3-(4-amino-3-((1-cyclopropyl-6-fluoro-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one according to Reference Example 13.
MS m/z (ESI): 536.2 $[M+H]^+$.

**Example 36**

(*S*)-1-(3-(4-amino-3-((2-cyclopropyl-6-fluoro-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one

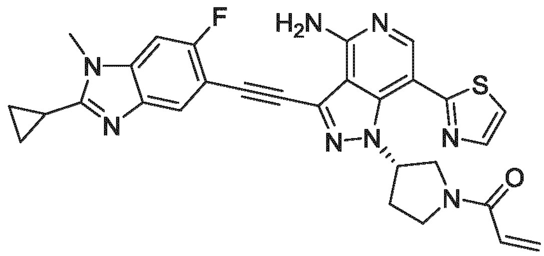

Preparation of (*S*)-1-(3-(4-amino-3-((2-cyclopropyl-6-fluoro-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one according to Reference Example 9.
MS *m/z* (ESI): 553.2 $[M+H]^+$.
$^1$H NMR (400 MHz, DMSO-$d_6$) $\delta$ 8.04 (d, *J* = 2.0 Hz, 1H), 8.00 (t, *J* = 3.2 Hz, 1H), 7.88-7.83 (m, 2H), 7.65 (d, *J* = 10.0 Hz, 1H), 7.02 (s, 2H), 6.56 (ddd, *J* = 38.0, 16.8, 10.4 Hz, 2H), 6.14 (dd, *J* = 16.8, 2.0 Hz, 1H), 5.77-5.64 (m, 1H), 3.87 (s, 3H), 3.78-3.67 (m, 2H), 3.60-3.55 (m, 2H), 2.26 (td, *J* = 8.2, 4.0 Hz, 2H), 1.17-0.98 (m, 5H).

**Example 37**

(*S*)-1-(3-(7-acetyl-4-amino-3-((1-cyclopropyl-1H-benzo[d]imidazol-5-yl)ethynyl)-1H-pyrazolo[4,3-c]pyridin-1-yl)pyrrolidin-1-yl)prop-2-en-1-one

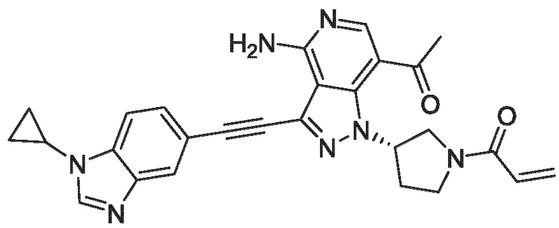

Step I: Preparation of N-cyclopropyl-4-iodo-2-nitroaniline

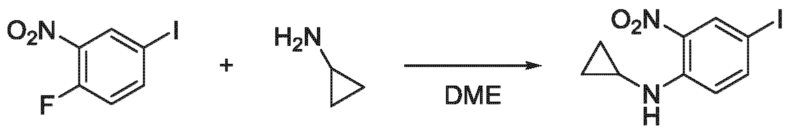


A solution of 2-fluoro-5-iodonitrobenzene (25 g, 93.63 mmol) and cyclopropylamine (10.69 g, 187.27 mmol) in glycol dimethyl ether (250 mL) was warmed to 80°C and stirred for 2 hours. The reaction liquid was concentrated under reduced pressure, and the residue was diluted with water (100 mL) and DCM (200 mL). After liquid separation, the organic layer was washed with water (100 mL), dried over anhydrous sodium sulfate, and filtered, and the filtrate was concentrated under reduced pressure to obtain the title compound (27.6 g, 97%).

MS *m*/*z* (ESI): 305.1 $[M+H]^+$.

Step II: Preparation of N1-cyclopropyl-4-iodobenzene-1,2-diamine

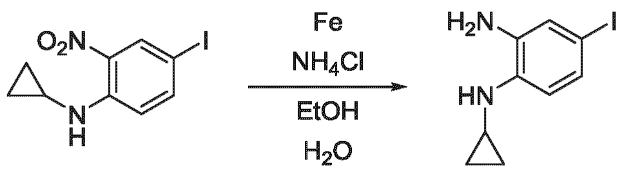


A suspension of N-cyclopropyl-4-iodo-2-nitroaniline (27.60 g, 90.76 mmol), iron powder (20.28 g, 363.06 mmol), ammonium chloride (19.42 g, 363.06 mmol) in EtOH (300 mL) and water (60 mL) was warmed to 80°C and stirred for 2 hours. The reaction liquid was filtered with a diatomaceous earth pad, and the filter cake was fully washed with ethyl acetate (100 mL). After the original filtrate was concentrated, the residue was combined with the washing filtrate, washed with a saturated sodium bicarbonate solution (50 mL), dried over anhydrous sodium sulfate, filtered, and concentrated to obtain the title compound (26 g), and the crude product was directly used in the next step.

Step III: Preparation of 1-cyclopropyl-5-iodo-1H-benzo[d]imidazole

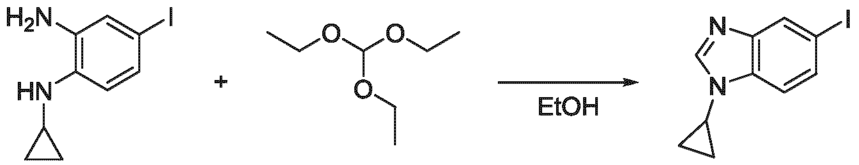


A solution of N1-cyclopropyl-4-iodobenzene-1,2-diamine (26 g) and triethyl orthoformate (28.12 g, 189.71 mmol, 31.6 mL) in ethanol (250 mL) was warmed to 70°C and stirred for 1 hour. The reaction liquid was concentrated under reduced pressure, and the residue was separated and purified by silica gel column chromatography to obtain the title compound (13 g, yield 50% over two steps).
MS *m/z* (ESI): 285.0 $[M+H]^+$.

Step IV: Preparation of 1-cyclopropyl-5-((trimethylsilyl)ethynyl)-1H-benzo[d]imidazole

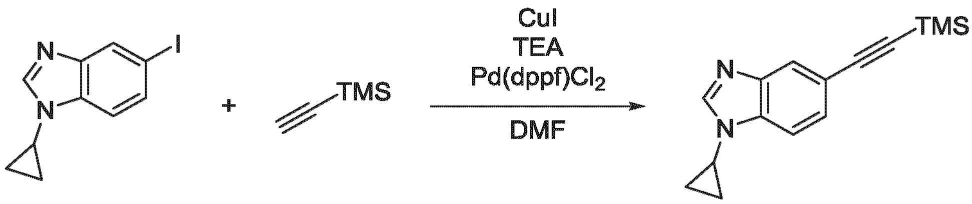


A solution of trimethylethynylsilane (6.74 g, 68.64 mmol, 9.7 mL), 1-cyclopropyl-5-iodo-1H-benzo[d]imidazole (13 g, 45.76 mmol), CuI (1.31 g, 6.86 mmol), TEA (23.15 g, 228.80 mmol, 31.9 mL), and $Pd(dppf)Cl_2$ (1.66 g, 2.29 mmol) in DMF (130 mL) was warmed to 60°C and stirred for 1 hour. The reaction liquid was separated in water (400 mL) and ethyl acetate (600 mL), the organic layer was washed with water (200 mL) and a saturated sodium chloride solution (200 mL), respectively, dried over anhydrous sodium sulfate, filtered, and concentrated under reduced pressure, and the residue was separated and purified by silica gel column chromatography to obtain the title compound (8.5 g, 73%).
MS *m/z* (ESI): 255.1 $[M+H]^+$.

Step V: Preparation of 1-cyclopropyl-5-ethynyl-1H-benzo[d]imidazole

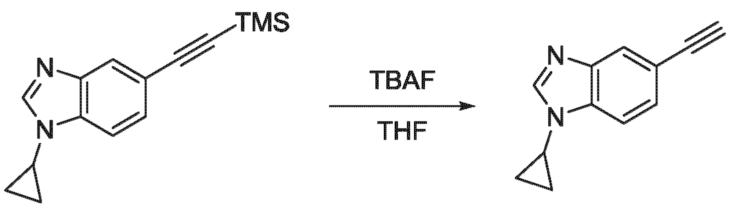


At room temperature, TBAF (1 M, 51.8 mL) was added to a solution of 1-cyclopropyl-5-((trimethylsilyl)ethynyl)-1H-benzo[d]imidazole (8.5 g, 33.41 mmol) in THF (100 mL), and the mixture was stirred at room temperature for 1 hour. The reaction liquid was concentrated under reduced pressure, and the residue was separated and purified by silica gel column chromatography to obtain the title compound (5.18 g, 85%).
MS *m/z* (ESI): 183.1 $[M+H]^+$.

Step VI: Preparation of (*S*)-tert-butyl 3-(4-amino-7-bromo-3-((1-cyclopropyl-1H-benzo[d]imidazol-5-yl)ethynyl)-1H-pyrazolo[4,3-c]pyridin-1-yl)pyrrolidine-1-carboxylate

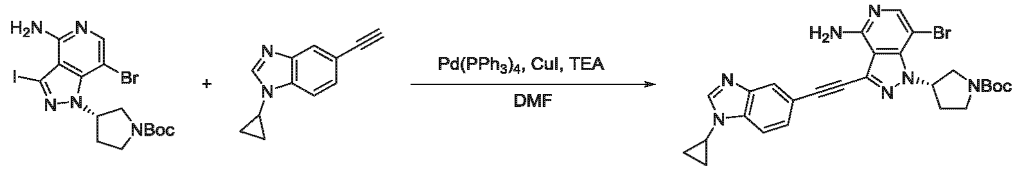


$Pd(PPh_3)_4$ (682 mg, 590 μmol) and CuI (169 mg, 886 μmol) were respectively added to a solution of (*S*)-tert-butyl 3-(4-amino-7-bromo-3-iodo-1H-pyrazolo[4,3-c]pyridin-1-yl)pyrrolidine-1-carboxylate (3 g, 5.90 mmol), 1-cyclopropyl-5-ethynyl-1H-benzo[d]imidazole (1.29 g, 7.08 mmol), and TEA (2.99 g, 29.52 mmol, 4.1 mL) in DMF (30 mL), and after displacement with nitrogen three times, the solution was warmed to 60°C and stirred for 1 hour. The reaction liquid was poured into 200 mL of ice water, whereupon a solid was precipitated out. After filtration, the solid was dissolved with DCM (50 mL), dried over anhydrous sodium sulfate, filtered, and concentrated under reduced pressure, and the residue was separated and purified by silica gel column chromatography to obtain the title compound (2.73 g, 82%).

MS *m*/*z* (ESI): 562.1 $[M+H]^+$.

Step VII: Preparation of (*S*)-tert-butyl 3-(4-amino-7-(1-butoxyvinyl)-3-((1-cyclopropyl-1H-benzo[d]imidazol-5-yl)ethynyl)-1H-pyrazolo[4,3-c]pyridin-1-yl)pyrrolidine-1-carboxylate

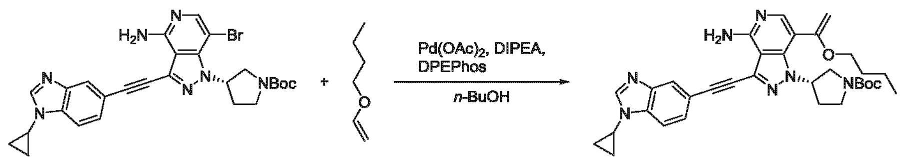


A suspension of (*S*)-tert-butyl 3-(4-amino-7-bromo-3-((1-cyclopropyl-1H-benzo[d]imidazol-5-yl)ethynyl)-1H-pyrazolo[4,3-c]pyridin-1-yl)pyrrolidine-1-carboxylate (1 g, 1.78 mmol), vinyl n-butyl ether (534 mg, 5.33 mmol), $Pd(OAc)_2$ (40 mg, 178 μmol), DIPEA (551 mg, 4.27 mmol, 743 μL), and DPEPhos (192 mg, 356 μmol) in n-butanol (30 mL) was warmed to 100°C under nitrogen protection and stirred for 1.5 hours. The reaction liquid was concentrated under reduced pressure, and the residue was separated in water (30 mL) and DCM (30 mL). The organic layer was dried over anhydrous sodium sulfate, filtered, and concentrated under reduced pressure, and the residue was separated and purified by silica gel column chromatography to obtain the title compound (837 mg, 81%).

MS *m*/*z* (ESI): 582.3 $[M+H]^+$.

Step VIII: Preparation of (*S*)-1-(4-amino-3-((1-cyclopropyl-1H-benzo[d]imidazol-5-yl)ethynyl)-1-(pyrrolidin-3-yl)-1H-pyrazolo[4,3-c]pyridin-7-yl)ethanone

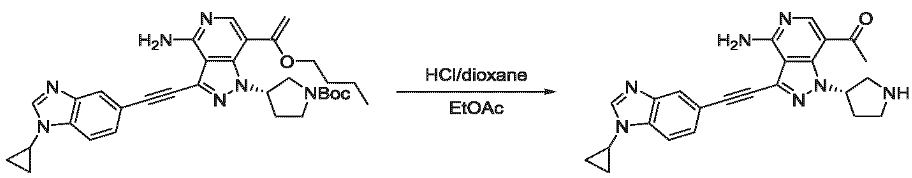


At room temperature, HCl/1,4-dioxane (4 M, 8 mL) was added to a solution of (*S*)-tert-butyl 3-(4-amino-7-(1-butoxyvinyl)-3-((1-cyclopropyl-1H-benzo[d]imidazol-5-yl)ethynyl)-1H-pyrazolo[4,3-c]pyridin-1-yl)pyrrolidine-1-carboxylate (837 mg, 1.44 mmol) in ethyl acetate (4 mL), and the mixture was stirred at room temperature for 1 hour. The reaction liquid was concentrated under reduced pressure to obtain the title compound hydrochloride (664 mg, 100%).

MS *m*/*z* (ESI): 426.2 $[M+H]^+$.

Step VIII: Preparation of (*S*)-1-(3-(7-acetyl-4-amino-3-((1-cyclopropyl-1H-benzo[d]imidazol-5-yl)ethynyl)-1H-pyrazolo[4,3-c]pyridin-1-yl)pyrrolidin-1-yl)prop-2-en-1-one

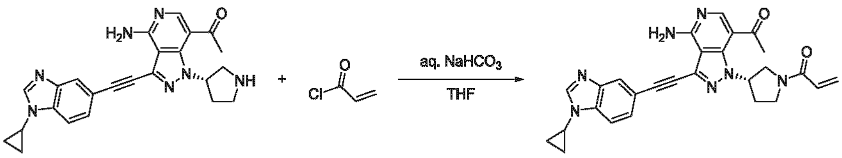


In an ice bath, a suspension of acryloyl chloride (101 mg, 1.15 mmol, 91 μL) in THF (5 mL) was dropwise added to (*S*)-1-(4-amino-3-((1-cyclopropyl-1H-benzo[d]imidazol-5-yl)ethynyl)-1-(pyrrolidin-3-yl)-1H-pyrazolo[4,3-c]pyridin-7-yl) ethanone hydrochloride (664 mg, 1.44 mmol), a saturated sodium bicarbonate solution (8.05 g), and THF (15 mL) was stirred in the ice bath for 15 minutes. The reaction liquid was concentrated under reduced pressure, the residue was diluted with 10 mL of a mixed solvent of dichloromethane and methanol (V/V = 1:1) and washed by adding water (5 mL), the organic layer was dried over anhydrous sodium sulfate, filtered, and concentrated, and the residue was separated by prep-HPLC to obtain the title compound (44 mg, 6%).

MS *m*/*z* (ESI): 480.2 $[M+H]^+$.

$^1$H NMR (400 MHz, DMSO-$d_6$) $\delta$ 8.60-8.54 (m, 1H), 8.35 (s, 1H), 8.05 (s, 1H), 7.71 (d, *J* = 8.4 Hz, 1H), 7.60 (d, *J* = 8.3 Hz, 1H), 6.71-6.52 (m, 1H), 6.21-6.10 (m, 1H), 6.02-5.83 (m, 1H), 5.74-5.62 (m, 1H), 4.01-3.51 (m, 5H), 2.61 (s, 3H), 2.46-2.27 (m, 2H), 1.17-1.01 (m, 4H).

**Example 38**

(*S*)-1-(3-(4-amino-7-(cyclopropanecarbonyl)-3-((1-cyclopropyl-1H-benzo[d]imidazol-5-yl)ethynyl)-1H-pyrazolo[4,3-c] pyridin-1-yl)pyrrolidin-1-yl)prop-2-en-1-one

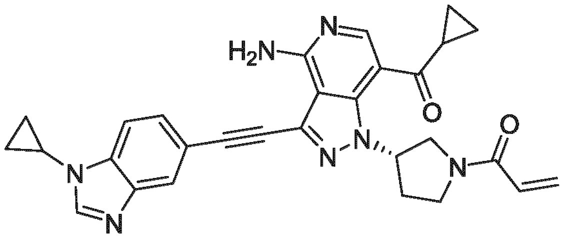

Step I: Preparation of (*S*)-tert-butyl 3-(4-amino-3-((1-cyclopropyl-1H-benzo[d]imidazol-5-yl)ethynyl)-7-vinyl-1H-pyrazolo[4,3-c]pyridin-1-yl)pyrrolidine-1-carboxylate

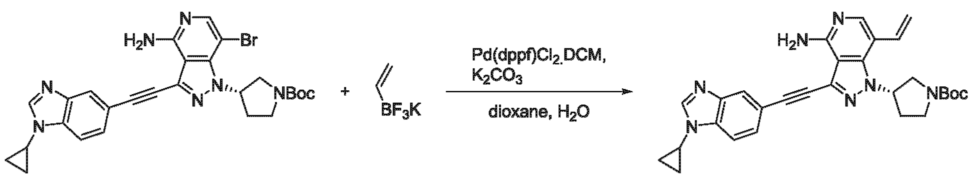


(*S*)-tert-butyl 3-(4-amino-7-bromo-3-((1-cyclopropyl-1H-benzo[d]imidazol-5-yl)ethynyl)-1H-pyrazolo[4,3-c]pyridin-1-yl)pyrrolidine-1-carboxylate (1.47 g, 2.61 mmol), potassium vinyltrifluoroborate (700 mg, 5.23 mmol), [1,1'-bis(diphenylphosphine)ferrocene]palladium dichloride dichloromethane complex (211 mg, 261 μmol), and potassium carbonate (1.08 g, 7.84 mmol) were added to a mixture of 1,4-dioxane (15 mL) and water (5 mL), which was warmed to 100°C under nitrogen protection and stirred for 1.5 hours. The reaction liquid was separated in water (50 mL) and DCM (50 mL). The organic layer was dried over anhydrous sodium sulfate, filtered, and concentrated, and the residue was separated and purified by silica gel column chromatography to obtain the title compound (0.88 g, 66%).

MS *m*/*z* (ESI): 510.2 $[M+H]^+$.

Step II: Preparation of (*S*)-tert-butyl 3-(4-amino-3-((1-cyclopropyl-1H-benzo[d]imidazol-5-yl)ethynyl)-7-formyl-1H-pyrazolo[4,3-c]pyridin-1-yl)pyrrolidine-1-carboxylate

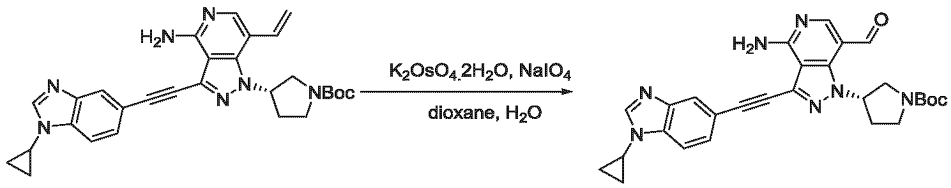


At room temperature, sodium periodate (1.92 g, 8.97 mmol) was added to a mixture of (*S*)-tert-butyl 3-(4-amino-3-((1-cyclopropyl-1H-benzo[d]imidazol-5-yl)ethynyl)-7-vinyl-1H-pyrazolo[4,3-c]pyridin-1-yl)pyrrolidine-1-carboxylate (880 mg, 1.73 mmol), potassium osmate dihydrate (41 mg, 112 μmol) in 1,4-dioxane (40 mL) and water (13 mL), and the mixture was stirred at room temperature for 16 hours. The reaction liquid was concentrated under reduced pressure, and the residue was separated in water (20 mL) and DCM (20 mL). The organic layer was dried over anhydrous sodium sulfate, filtered, and concentrated to obtain the title compound (826 mg, 94%).

MS *m*/*z* (ESI): 512.2 $[M+H]^+$.

Step III: Preparation of (3*S*)-tert-butyl 3-(4-amino-7-(cyclopropyl(hydroxy)methyl)-3-((1-cyclopropyl-1H-benzo[d]imidazol-5-yl)ethynyl)-1H-pyrazolo[4,3-c]pyridin-1-yl)pyrrolidine-1-carboxylate

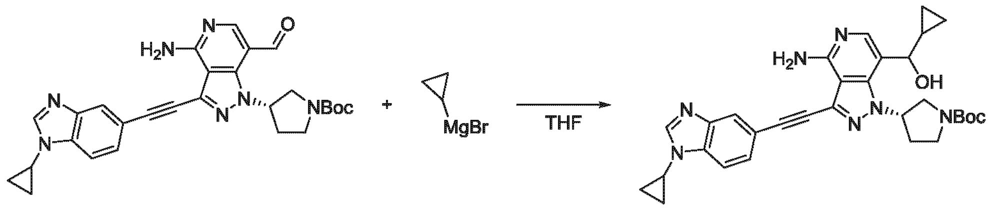


In an ice bath, cyclopropylmagnesium bromide (0.5 M, 6.6 mL) was added to a solution of (*S*)-tert-butyl 3-(4-amino-3-((1-cyclopropyl-1H-benzo[d]imidazol-5-yl)ethynyl)-7-formyl-1H-pyrazolo[4,3-c]pyridin-1-yl)pyrrolidine-1-carboxylate (423 mg, 827 μmol) in THF (5 mL), and the mixture was stirred at room temperature for 0.5 hours. The reaction liquid was quenched with a saturated ammonium chloride solution (5 mL) and extracted with ethyl acetate (10 mL). The organic layer was washed with a saturated sodium chloride solution (10 mL), dried over anhydrous sodium sulfate, filtered, and concentrated to obtain the title compound (168 mg, 37%).

MS *m*/*z* (ESI): 554.2 $[M+H]^+$.

Step IV: Preparation of (*S*)-tert-butyl 3-(4-amino-7-(cyclopropylcarbonyl)-3-((1-cyclopropyl-1H-benzo[d]imidazol-5-yl) ethynyl)-1H-pyrazolo[4,3-c]pyridin-1-yl)pyrrolidine-1-carboxylate

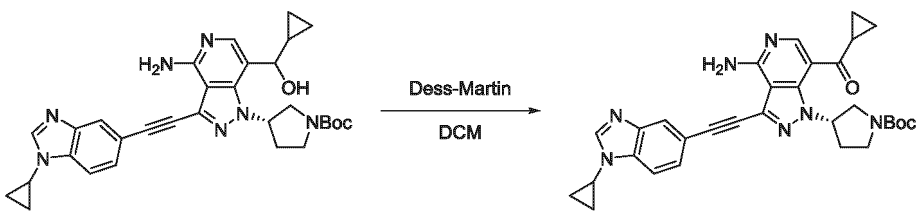


At room temperature, Dess-Martin periodinane (386 mg, 910 μmol) was added to a solution of (3*S*)-tert-butyl 3-(4-amino-7-(cyclopropyl(hydroxy)methyl)-3-((1-cyclopropyl-1H-benzo[d]imidazol-5-yl)ethynyl)-1H-pyrazolo[4,3-c]pyridin-1-yl)pyrrolidine-1-carboxylate (168 mg, 303 μmol) in DCM (5 mL), and the mixture was stirred at room temperature for 0.5 hours. The reaction liquid was quenched with a saturated sodium bicarbonate solution (5 mL) and extracted with

DCM (10 mL), the organic layers were combined, dried over anhydrous sodium sulfate, filtered, and concentrated under reduced pressure, and the residue was separated and purified by silica gel column chromatography to obtain the title compound (91 mg, 54%).

MS *m*/*z* (ESI): 552.2 $[M+H]^+$.

Step V: Preparation of (*S*)-(4-amino-3-((1-cyclopropyl-1H-benzo[d]imidazol-5-yl)ethynyl)-1-(pyrrolidin-3-yl)-1H-pyrazolo[4,3-c]pyridin-7-yl)(cyclopropyl)methanone

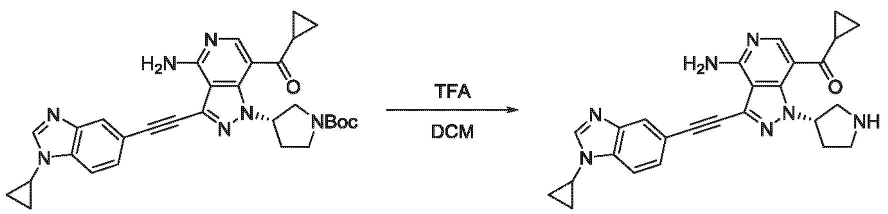


At room temperature, trifluoroacetic acid (1 mL) was added to a solution of (*S*)-tert-butyl 3-(4-amino-7-(cyclopropylcarbonyl)-3-((1-cyclopropyl-1H-benzo[d]imidazol-5-yl)ethynyl)-1H-pyrazolo[4,3-c]pyridin-1-yl)pyrrolidine-1-carboxylate (91 mg, 165 μmol), and the mixture was stirred at room temperature for 0.5 hours. The reaction liquid was concentrated under reduced pressure to obtain the title compound trifluoroacetate (93 mg, 100%).

MS *m*/*z* (ESI): 452.2 $[M+H]^+$.

Step VI: Preparation of (*S*)-1-(3-(4-amino-7-(cyclopropylcarbonyl)-3-((1-cyclopropyl-1H-benzo[d]imidazol-5-yl)ethynyl)-1H-pyrazolo[4,3-c]pyridin-1-yl)pyrrolidin-1-yl)prop-2-en-1-one

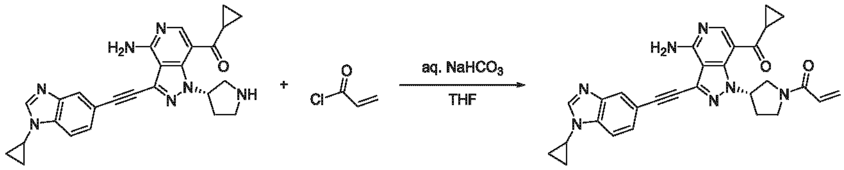


In an ice bath, a solution of acryloyl chloride (15 mg, 164 μmol, 13 μL) in THF (0.1 mL) was dropwise added to a mixture of (*S*)-(4-amino-3-((1-cyclopropyl-1H-benzo[d]imidazol-5-yl)ethynyl)-1-(pyrrolidin-3-yl)-1H-pyrazolo[4,3-c]pyridin-7-yl)(cyclopropyl)methanone trifluoroacetate (93 mg, 164 μmol), a saturated sodium bicarbonate solution (1.23 g), and THF (18 mL), and the mixture was stirred in the ice bath for 15 minutes. The reaction liquid was concentrated under reduced pressure, the residue was diluted with DCM (10 mL), washed by adding (10 mL), dried over anhydrous sodium sulfate, filtered, and concentrated, and the residue was separated by prep-HPLC to obtain the title compound (16 mg, 19%).

$^1H$ NMR (400 MHz, $CDCl_3$) $\delta$ 8.55 (d, *J* = 17.7 Hz, 1H), 8.04 (d, *J* = 7.6 Hz, 2H), 7.65-7.50 (m, 2H), 7.22-6.77 (m, 2H), 6.56-6.34 (m, 2H), 5.87-5.63 (m, 2H), 4.17-3.66 (m, 4H), 3.47-3.38 (m, 1H), 2.86-2.38 (m, 3H), 1.39-1.01 (m, 8H);

MS *m*/*z* (ESI): 506.2 $[M+H]^+$.

**Example 39**

(*S*)-1-(3-(4-amino-3-((1-cyclopropyl-1H-benzo[d]imidazol-5-yl)ethynyl)-7-propionyl-1H-pyrazolo[4,3-c]pyridin-1-yl)pyrrolidin-1-yl)prop-2-en-1-one

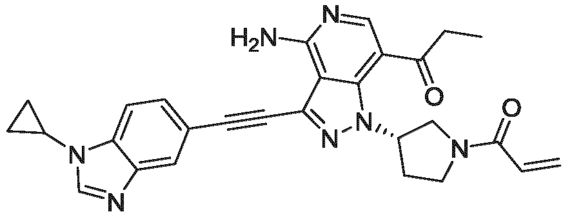

Preparation of (*S*)-1-(3-(4-amino-3-((1-cyclopropyl-1H-benzo[d]imidazol-5-yl)ethynyl)-7-propionyl-1H-pyrazolo [4,3-c]pyridin-1-yl)pyrrolidin-1-yl)prop-2-en-1-one according to Reference Example 38.

MS *m*/*z* (ESI): 494.2 $[M+H]^+$.

$^1H$ NMR (400 MHz, DMSO-$d_6$) $\delta$ 8.59-8.56 (m, 1H), 8.36 (s, 1H), 8.05 (s, 1H), 7.72 (d, *J* = 8.3 Hz, 1H), 7.61 (d, *J* = 8.4 Hz, 1H), 7.46-6.94 (m, 2H), 6.71-6.52 (m, 1H), 6.21-6.10 (m, 1H), 5.92-5.75 (m, 1H), 5.74-5.62 (m, 1H), 4.13-3.56 (m, 5H), 3.10-3.01 (m, 2H), 2.47-2.27 (m, 2H), 1.18-1.01 (m, 7H).

**Example 40**

(*S*)-1-(3-(4-amino-3-((1-cyclopropyl-1H-benzo[d]imidazol-5-yl)ethynyl)-7-isobutyl-1H-pyrazolo[4,3-c]pyridin-1-yl)pyrrolidin-1-yl)prop-2-en-1-one

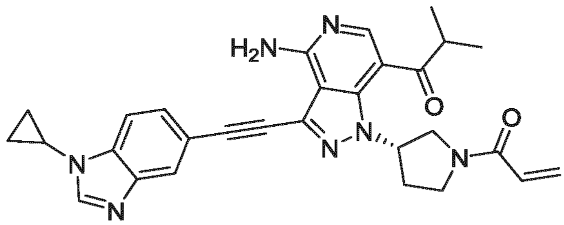

Preparation of (*S*)-1-(3-(4-amino-3-((1-cyclopropyl-1H-benzo[d]imidazol-5-yl)ethynyl)-7-isobutyl-1H-pyrazolo [4,3-c]pyridin-1-yl)pyrrolidin-1-yl)prop-2-en-1-one according to Reference Example 38.

MS *m*/*z* (ESI): 508.2 $[M+H]^+$.

$^1H$ NMR (400 MHz, $CDCl_3$) $\delta$ 8.23-8.06 (m, 3H), 7.69-7.50 (m, 2H), 6.55-6.36 (m, 2H), 5.77-5.58 (m, 2H), 4.21-3.74 (m, 4H), 3.49-3.32 (m, 2H), 2.83-2.37 (m, 2H), 1.31-1.24 (m, 10H).

**Example 41**

(*S*)-1-(3-(4-amino-3-((1-cyclopropyl-1H-benzo[d]imidazol-5-yl)ethynyl)-7-pivaloyl-1H-pyrazolo[4,3-c]pyridin-1-yl)pyrrolidin-1-yl)prop-2-en-1-one

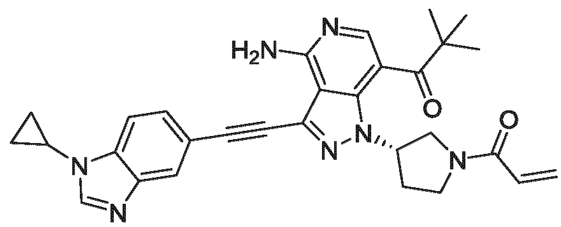

Preparation of (*S*)-1-(3-(4-amino-3-((1-cyclopropyl-1H-benzo[d]imidazol-5-yl)ethynyl)-7-pivaloyl-1H-pyrazolo [4,3-c]pyridin-1-yl)pyrrolidin-1-yl)prop-2-en-1-one according to Reference Example 38.

MS *m*/*z* (ESI): 522.2 $[M+H]^+$.

**Example 42**

(*S*)-1-(3-(4-amino-3-((1-cyclopropyl-1H-benzo[d]imidazol-5-yl)ethynyl)-7-(1-methyl-1H-pyrazol-3-yl)-1H-pyrazolo[4,3-c]pyridin-1-yl)pyrrolidine-1-methyl)prop-2-en-1-one

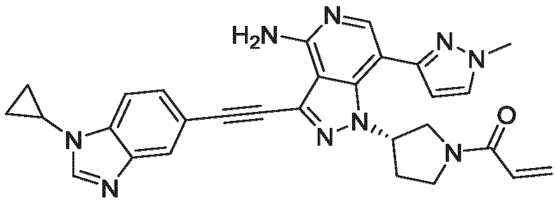

Preparation of (*S*)-1-(3-(4-amino-3-((1-cyclopropyl-1H-benzo[d]imidazol-5-yl)ethynyl)-7-(1-methyl-1H-pyrazol-3-yl)-1H-pyrazolo[4,3-c]pyridin-1-yl)pyrrolidine-1-methyl)prop-2-en-1-one according to Reference Example 13.

MS *m*/*z* (ESI): 518.2 $[M+H]^+$.

$^1H$ NMR (400 MHz, $CDCl_3$) $\delta$ 8.03 (d, *J* = 16.8 Hz, 2H), 7.73 (d, *J* = 15.2 Hz, 1H), 7.64-7.52 (m, 2H), 7.49 (t, *J* = 2.5 Hz, 1H), 7.03 (s, 1H), 6.79 (s, 1H), 6.54-6.30 (m, 3H), 5.73-5.64 (m, 1H), 5.51-5.37 (m, 1H), 4.08-3.38 (m, 8H), 2.75-2.20 (m, 2H), 1.25-1.18 (m, 2H), 1.12-1.05 (m, 2H).

**Example 43**

(*S*)-1-(3-(4-amino-3-((6-chloro-1-cyclopropyl-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one

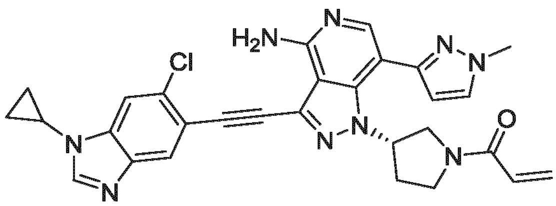

Preparation of (*S*)-1-(3-(4-amino-3-((6-chloro-1-cyclopropyl-1H-benzo[d]imidazol-5-yl)ethynyl)-7-(1-methyl-1H-pyrazol-3-yl)-1H-pyrazolo[4,3-c]pyridin-1-yl)pyrrolidin-1-yl)prop-2-en-1-one according to Reference Example 13.

MS *m*/*z* (ESI): 552.2 $[M+H]^+$.

$^1H$ NMR (400 MHz, DMSO) $\delta$ 8.41 (s, 1H), 8.15 (s, 1H), 7.93 (s, 1H), 7.85-7.73 (m, 2H), 6.71 (s, 2H), 6.68-6.47 (m, 2H), 6.14 (dt, *J* = 16.7, 1.8 Hz, 1H), 5.68 (td, *J* = 10.1, 2.4 Hz, 1H), 5.51 (s, 1H), 3.95-3.77 (m, 4H), 3.74-3.51 (m, 4H), 2.41-2.14 (m, 1H), 1.23 (s, 1H), 1.18-1.03 (m, 4H).

**Example 44**

(*S*)-1-(3-(7-acetyl-4-amino-3-((6-chloro-1-cyclopropyl-1H-benzo[d]imidazol-5-yl)ethynyl)-1H-pyrazolo[4,3-c]pyridin-1-yl)pyrrolidin-1-yl)prop-2-en-1-one

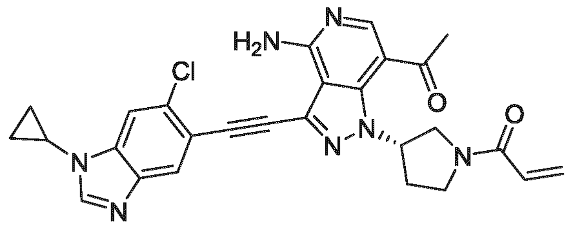

Preparation of (*S*)-1-(3-(7-acetyl-4-amino-3-((6-chloro-1-cyclopropyl-1H-benzo[d]imidazol-5-yl)ethynyl)-1H-pyrazolo[4,3-c]pyridin-1-yl)pyrrolidin-1-yl)prop-2-en-1-one according to Reference Example 37.
MS *m*/*z* (ESI): 514.2 $[M+H]^+$.
$^1$H NMR (400 MHz, DMSO-$d_6$) $\delta$ 8.52 (s, 1H), 8.35 (s, 1H), 8.10 (s, 1H), 7.86 (s, 1H), 7.15 (s, 1H), 6.69-6.43 (m, 2H), 6.09 (d, *J* = 17.1 Hz, 1H), 5.87 (d, *J* = 31.9 Hz, 1H), 5.62 (dd, *J* = 17.6, 11.1 Hz, 1H), 5.26 (d, *J* = 4.6 Hz, 1H), 4.08-3.94 (m,2H), 3.91-3.68 (m, 3H), 3.60-3.40 (m, 3H), 2.55 (s, 2H), 2.28 (d, *J* = 6.9 Hz, 1H), 1.93 (dd, *J* = 14.7, 7.1 Hz, 2H).

**Example 45**

(*S*)-1-(3-(4-amino-3-((6-chloro-1-cyclopropyl-1H-benzo[d]imidazol-5-yl)ethynyl)-7-propionyl-1H-pyrazolo[4,3-c]pyridin-1-yl)pyrrolidin-1-yl)prop-2-en-1-one

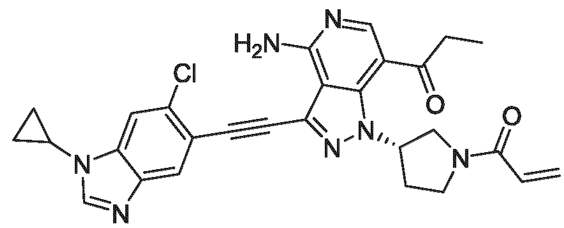

Preparation of (*S*)-1-(3-(4-amino-3-((6-chloro-1-cyclopropyl-1H-benzo[d]imidazol-5-yl)ethynyl)-7-propionyl-1H-pyrazolo[4,3-c]pyridin-1-yl)pyrrolidin-1-yl)prop-2-en-1-one according to Reference Example 38.
MS *m*/*z* (ESI): 528.2 $[M+H]^+$.
$^1$H NMR (400 MHz, DMSO-$d_6$) $\delta$ 8.58 (d, *J* = 2.1 Hz, 1H), 8.41 (d, *J* = 5.0 Hz, 1H), 8.16 (s, 1H), 7.93 (s, 1H), 6.71-6.52 (m, 1H), 6.21-6.11 (m, 1H), 5.81 (s, 1H), 5.78-5.63 (m, 1H), 3.91-3.73 (m, 2H), 3.69-3.51 (m, 1H), 3.07 (q, *J* = 7.2 Hz, 2H), 2.49-2.41 (m, 1H), 2.01 (t, *J* = 7.6 Hz, 1H), 1.97 (s, 1H), 1.46 (s, 1H), 1.24 (s, 3H), 1.20-1.07 (m, 2H), 1.12 (s, 2H), 1.08 (s, 1H), 0.89-0.81 (m, 1H).

**Example 46**

(*S*)-1-(3-(4-amino-3-((6-chloro-1-cyclopropyl-1H-benzo[d]imidazol-5-yl)ethynyl)-7-(cyclopropanecarbonyl)-1H-pyrazolo[4,3-c]pyridin-1-yl)pyrrolidin-1-yl)prop-2-en-1-one

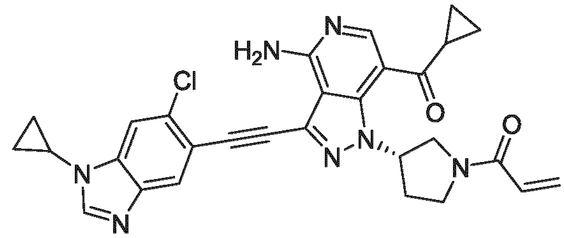

Preparation of (*S*)-1-(3-(4-amino-3-((6-chloro-1-cyclopropyl-1H-benzo[d]imidazol-5-yl)ethynyl)-7-(cyclopropanecarbonyl)-1H-pyrazolo[4,3-c]pyridin-1-yl)pyrrolidin-1-yl)prop-2-en-1-one according to Reference Example 38.
MS *m*/*z* (ESI): 540.2 $[M+H]^+$.
$^1$H NMR (400 MHz, DMSO) $\delta$ 8.74 (d, *J* = 2.2 Hz, 1H), 8.42 (s, 1H), 8.17 (s, 1H), 7.93 (d, *J* = 2.8 Hz, 1H), 6.64 (dd, *J*

= 16.8, 10.3 Hz, 1H), 6.62-6.51 (m, 1H), 6.16 (dt, *J* = 16.8, 2.8 Hz, 1H), 5.75 (s, 1H), 5.69 (ddd, *J* = 13.0, 10.3, 2.4 Hz, 2H), 4.22-3.95 (m, 1H), 3.94 (d, *J* = 4.5 Hz, 1H), 3.63-3.43 (m, 3H), 2.82 (s, 1H), 2.47-2.25 (m, 3H), 1.99 (dt, *J* = 15.0, 7.1 Hz, 2H), 1.47 (d, *J* = 7.7 Hz, 1H), 1.20-0.96 (m, 2H), 0.98-0.73 (m, 2H).

**Example 47**

(*S*)-1-(3-(4-amino-3-((6-chloro-1-cyclopropyl-1H-benzo[d]imidazol-5-yl)alkynyl)-7-isobutyl-1H-pyrazolo[4,3-c]pyridin-1-yl)pyrrolidin-1-yl)prop-2-en-1-one

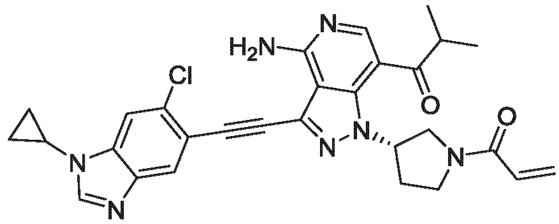

Preparation of (*S*)-1-(3-(4-amino-3-((6-chloro-1-cyclopropyl-1H-benzo[d]imidazol-5-yl)alkynyl)-7-isobutyl-1H-pyrazolo[4,3-c]pyridin-1-yl)pyrrolidin-1-yl)prop-2-en-1-one according to Reference Example 38.

MS *m*/*z* (ESI): 542.2 $[M+H]^+$.

$^1H$ NMR (400 MHz, DMSO-$d_6$) $\delta$ 8.58 (d, *J* = 2.7 Hz, 1H), 8.42 (s, 1H), 8.16 (s, 1H), 7.93 (s, 1H), 6.64 (dd, *J* = 16.9, 10.4 Hz, 1H), 6.15 (dt, *J* = 16.7, 2.7 Hz, 1H), 5.68 (ddd, *J* = 12.6, 10.3, 2.5 Hz, 2H), 4.17-3.91 (m, 1H), 3.87-3.64 (m, 3H), 3.64-3.50 (m, 2H), 2.45 (d, *J* = 6.8 Hz, 1H), 2.37-2.20 (m, 1H), 2.07-1.88 (m, 1H), 1.23 (s, 3H), 1.20-0.97 (m, 8H).

**Example 48**

(*S*)-1-(3-(4-amino-3-((1-cyclopropyl-6-fluoro-1H-benzo[d]imidazol-5-yl)ethynyl)-7-propionyl-1H-pyrazolo[4,3-c]pyridin-1-yl)pyrrolidin-1-yl)prop-2-en-1-one

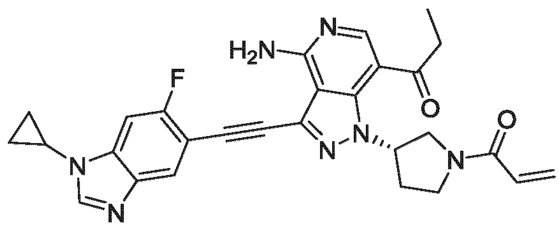

Preparation of (*S*)-1-(3-(4-amino-3-((1-cyclopropyl-6-fluoro-1H-benzo[d]imidazol-5-yl)ethynyl)-7-propionyl-1H-pyrazolo[4,3-c]pyridin-1-yl)pyrrolidin-1-yl)prop-2-en-1-one according to Reference Example 38.

MS *m*/*z* (ESI): 512.2 $[M+H]^+$.

$^1H$ NMR (400 MHz, DMSO) $\delta$ 8.58 (d, *J* = 2.1 Hz, 1H), 8.38 (s, 1H), 8.11 (dd, *J* = 6.4, 2.3 Hz, 1H), 7.69 (d, *J* = 9.5 Hz, 1H), 7.21 (s, 1H), 6.71-6.53 (m, 2H), 6.16 (dd, *J* = 16.7, 2.4 Hz, 1H), 5.88 (s, 1H), 5.80 (s, 1H), 5.76 (s, 1H), 5.69 (dd, 10.3, 2.4 Hz, 1H), 5.32 (t, *J* = 4.7 Hz, 1H), 4.08 (dd, *J* = 11.3, 6.7 Hz, 1H), 3.90-3.73 (m, 1H), 3.57-3.49 (m, 1H), 3.07 (q, *J* = 7.4 Hz, 2H), 2.31 (s, 1H), 2.05-1.97 (m, 3H), 1.50-1.42 (m, 2H), 0.89-0.81 (m, 2H).

**Example 49**

(*S*)-1-(3-(4-amino-7-(cyclopropanecarbonyl)-3-((1-cyclopropyl-6-fluoro-1H-benzo[d]imidazol-5-yl)ethynyl)-1H-pyrazolo[4,3-c]pyridin-1-yl)pyrrolidin-1-yl)prop-2-en-1-one

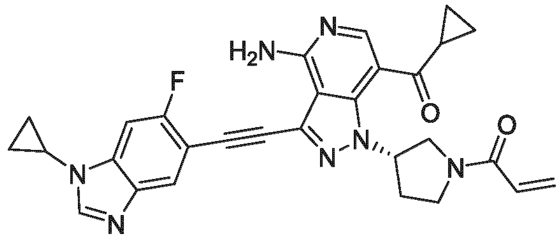

Preparation of (*S*)-1-(3-(4-amino-7-(cyclopropanecarbonyl)-3-((1-cyclopropyl-6-fluoro-1H-benzo[d]imidazol-5-yl)ethynyl)-1H-pyrazolo[4,3-c]pyridin-1-yl)pyrrolidin-1-yl)prop-2-en-1-one according to Reference Example 38.
MS *m*/*z* (ESI): 524.2 $[M+H]^+$.

**Example 50**

(*S*)-1-(3-(4-amino-3-((1-cyclopropyl-6-fluoro-1H-benzo[d]imidazol-5-yl)alkynyl)-7-isobutyl-1H-pyrazolo[4,3-c]pyridin-1-yl)pyrrolidin-1-yl)prop-2-en-1-one

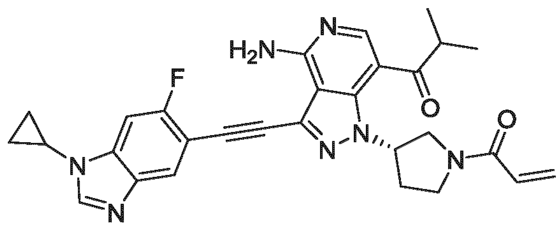

Preparation of (*S*)-1-(3-(4-amino-3-((1-cyclopropyl-6-fluoro-1H-benzo[d]imidazol-5-yl)alkynyl)-7-isobutyl-1H-pyrazolo[4,3-c]pyridin-1-yl)pyrrolidin-1-yl)prop-2-en-1-one according to Reference Example 38.
MS *m*/*z* (ESI): 526.2 $[M+H]^+$.
$^1$H NMR (400 MHz, DMSO) $\delta$ 8.58 (d, *J* = 2.5 Hz, 1H), 8.39 (s, 1H), 8.20-8.00 (m, 1H), 7.69 (d, *J* = 9.5 Hz, 1H), 6.70-6.51 (m, 1H), 6.15 (dd, *J* = 16.7, 3.0 Hz, 1H), 5.76-5.64 (m, 1H), 4.11-3.95 (m, 1H), 3.84-3.64 (m, 3H), 3.63-3.49 (m, 3H), 2.45 (d, *J* = 6.1 Hz, 1H), 2.35-2.27 (m, 2H), 2.00 (q, *J* = 7.5 Hz, 1H), 1.46 (s, 1H), 1.24 (s, 4H), 1.17-1.02 (m, 4H), 0.86 (t, *J* = 6.4 Hz, 1H).

**Example 51**

(*S*)-1-(3-(4-amino-3-((1-cyclopropyl-6-fluoro-1H-benzo[d]imidazol-5-yl)ethynyl)-7-methyl-1H-pyrazolo[4,3-c]pyridin-1-yl)pyrrolidin-1-yl)prop-2-en-1-one

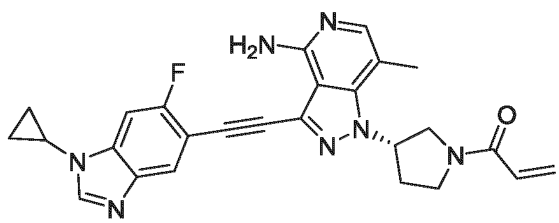

Preparation of (*S*)-1-(3-(4-amino-3-((1-cyclopropyl-6-fluoro-1H-benzo[d]imidazol-5-yl)ethynyl)-7-methyl-1H-pyrazolo[4,3-c]pyridin-1-yl)pyrrolidin-1-yl)prop-2-en-1-one according to Reference Example 3.
MS *m*/*z* (ESI): 470.2 $[M+H]^+$.
$^1$H NMR (400 MHz, DMSO-$d_6$) $\delta$ 8.38 (s, 1H), 8.18 (s, 1H), 8.09 (dd, *J* = 6.4, 2.0 Hz, 1H), 7.68 (s, 1H), 6.70-6.51 (m, 1H), 6.36 (s, 2H), 5.94 (dp, *J* = 33.2, 5.6 Hz, 1H), 5.69 (ddd, *J* = 16.4, 10.4, 2.4 Hz, 1H), 4.14-3.46 (m, 5H), 2.45 (d, *J* = 6.0 Hz, 1H), 2.36 (s, 3H),2.34 (d, *J* = 6.4 Hz, 1H), 1.22 (s, 1H), 1.15-1.03 (m, 4H).

**Example 52**

(*S*)-1-(3-(4-amino-7-cyclopropyl-3-((1-cyclopropyl-6-fluoro-1H-benzo[d]imidazol-5-yl)alkynyl)-1H-pyrazolo[4,3-c]pyridin-1-yl)pyrrolidin-1-yl)prop-2-en-1-one

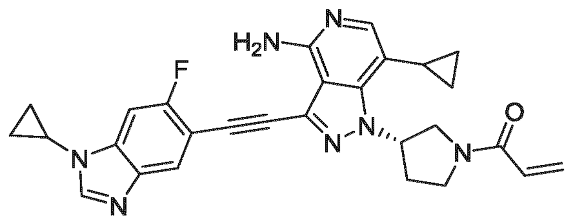

Preparation of (*S*)-1-(3-(4-amino-7-cyclopropyl-3-((1-cyclopropyl-6-fluoro-1H-benzo[d]imidazol-5-yl)alkynyl)-1H-pyrazolo[4,3-c]pyridin-1-yl)pyrrolidin-1-yl)prop-2-en-1-one according to Reference Example 6.
MS *m*/*z* (ESI): 496.2 $[M+H]^+$.
$^1$H NMR (400 MHz, DMSO-$d_6$) $\delta$ 8.37 (s, 1H), 8.18 (s, 1H), 8.06 (dd, *J* = 6.4, 2.4 Hz, 1H), 7.57 (s, 1H), 6.66 (ddd, *J* = 34.8, 16.8, 10.4 Hz, 1H), 6.38 (s, 2H), 6.22-6.06 (m, 1H), 5.71 (ddd, *J* = 20.4, 10.4, 2.4 Hz, 1H), 4.17 (dd, *J* = 10.8, 7.2 Hz, 1H), 4.03-3.90 (m, 2H), 3.87-3.79 (m, 2H), 2.42 (dd, *J* = 18.0, 7.2 Hz, 1H), 2.17 (s, 1H), 1.23 (s, 2H), 1.15-1.04 (m, 4H), 0.98 (d, *J* = 8.0 Hz, 2H), 0.76 (d, *J* = 12.4 Hz, 2H).

**Example 53**

(*S*)-1-(3-(7-acetyl-4-amino-3-((1-cyclopropyl-2-methyl-1H-benzo[d]imidazol-5-yl)ethynyl)-1H-pyrazolo[4,3-c]pyridin-1-yl)pyrrolidin-1-yl)prop-2-en-1-one

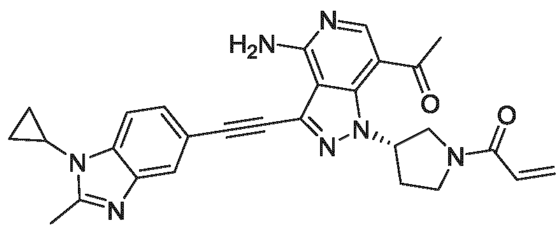

Preparation of (*S*)-1-(3-(7-acetyl-4-amino-3-((1-cyclopropyl-2-methyl-1H-benzo[d]imidazol-5-yl)ethynyl)-1H-pyrazolo[4,3-c]pyridin-1-yl)pyrrolidin-1-yl)prop-2-en-1-one according to Reference Example 4.
MS *m*/*z* (ESI): 494.3 $[M+H]^+$.
$^1$H NMR (400 MHz, $CDCl_3$) $\delta$ 8.45-8.37 (m, 1H), 7.84 (s, 1H), 7.50-7.37 (m, 2H), 6.61-6.24 (m, 4H), 6.10-6.01 (m, 1H), 5.66-5.57 (m, 1H), 4.14-3.61 (m, 4H), 3.24-3.14 (m, 1H), 2.80-2.25 (m, 8H), 1.30-0.98 (m, 4H).

**Example 54**

(*S*)-1-(3-(7-acetyl-4-amino-3-((6-chloro-1-cyclopropyl-2-methyl-1H-benzo[d]imidazol-5-yl)ethynyl)-1H-pyrazolo[4,3-c]pyridin-1-yl)pyrrolidin-1-yl)prop-2-en-1-one

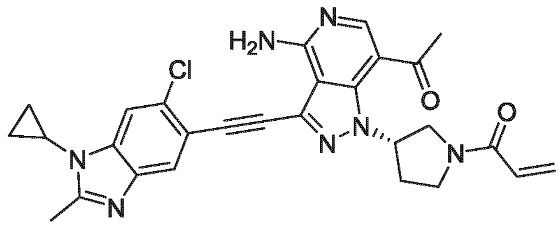

Preparation of (*S*)-1-(3-(7-acetyl-4-amino-3-((6-chloro-1-cyclopropyl-2-methyl-1H-benzo[d]imidazol-5-yl)ethynyl)-1H-pyrazolo[4,3-c]pyridin-1-yl)pyrrolidin-1-yl)prop-2-en-1-one according to Reference Example 4.
MS *m/z* (ESI): 528.2 $[M+H]^+$.

**Example 55**

(*S*)-1-(3-(7-acetyl-4-amino-3-((1-cyclopropyl-6-fluoro-2-methyl-1H-benzo[d]imidazol-5-yl)ethynyl)-1H-pyrazolo[4,3-c]pyridin-1-yl)pyrrolidin-1-yl)prop-2-en-1-one

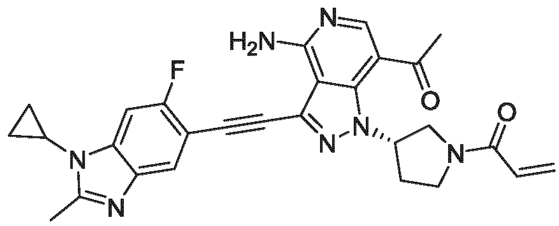

Preparation of (*S*)-1-(3-(7-acetyl-4-amino-3-((1-cyclopropyl-6-fluoro-2-methyl-1H-benzo[d]imidazol-5-yl)ethynyl)-1H-pyrazolo[4,3-c]pyridin-1-yl)pyrrolidin-1-yl)prop-2-en-1-one according to Reference Example 4.
MS *m/z* (ESI): 512.2 $[M+H]^+$.
$^1$H NMR (400 MHz, DMSO-$d_6$) $\delta$ 8.58 (d, *J* = 2.0 Hz, 1H), 7.93 (dd, *J* = 6.4, 2.4 Hz, 1H), 7.54 (d, *J* = 9.6 Hz, 1H), 6.71-6.53 (m, 2H), 6.22-6.12 (m, 1H), 6.02-5.86 (m, 1H), 5.72-5.64 (m, 1H), 4.12-3.50 (m, 5H), 2.61 (d, *J* = 7.6 Hz, 6H), 2.45 (d, *J* = 7.6 Hz, 1H), 2.33 (s, 1H), 1.28-1.16 (m, 5H).

**Example 56**

(*S*)-1-(3-(7-acetyl-4-amino-3-((1-ethyl-1H-benzo[d]imidazol-5-yl)ethynyl)-1H-pyrazolo[4,3-c]pyridin-1-yl)pyrrolidine-1-methyl)prop-2-en-1-one

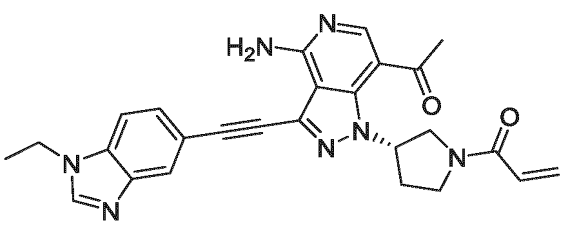

Preparation of (*S*)-1-(3-(7-acetyl-4-amino-3-((1-ethyl-1H-benzo[d]imidazol-5-yl)ethynyl)-1H-pyrazolo[4,3-c]pyridin-1-yl)pyrrolidine-1-methyl)prop-2-en-1-one according to Reference Example 4.
MS *m/z* (ESI): 468.2 $[M+H]^+$.
$^1$H NMR (400 MHz, DMSO) $\delta$ 8.74 (d, *J* = 2.4 Hz, 1H), 8.07 (d, *J* = 8.1 Hz, 2H), 7.22 (s, 1H), 7.14 (dd, *J* = 7.1, 1.7 Hz, 1H), 6.67 (s, 1H), 6.16 (dt, *J* = 16.9, 2.7 Hz, 1H), 5.69 (dd, *J* = 12.9, 2.4 Hz, 1H), 5.33 (t, *J* = 4.7 Hz, 2H), 3.16 (s, 1H), 2.82 (s, 1H), 2.43 (d, *J* = 6.6 Hz, 1H), 1.57 (s, 2H), 1.46 (s, 3H), 1.11-1.02 (m, 2H), 0.94 (t, *J* = 7.3 Hz, 2H), 0.90-0.82 (m, 3H).

**Example 57**

(*S*)-1-(3-(4-amino-3-((1-ethyl-1H-benzo[d]imidazol-5-yl)ethynyl)-7-propionyl-1H-pyrazolo[4,3-c]pyridin-1-yl)pyrrolidin-1-yl)prop-2-en-1-one

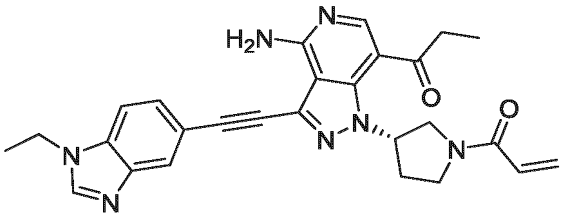

Preparation of (*S*)-1-(3-(4-amino-3-((1-ethyl-1H-benzo[d]imidazol-5-yl)ethynyl)-7-propionyl-1H-pyrazolo[4,3-c]pyridin-1-yl)pyrrolidin-1-yl)prop-2-en-1-one according to Reference Example 5.

MS *m/z* (ESI): 482.2 $[M+H]^+$.

$^1$H NMR (400 MHz, DMSO) $\delta$ 8.50 (d, *J* = 2.1 Hz, 1H), 8.33 (s, 1H), 7.98 (s, 1H), 7.66 (d, *J* = 8.4 Hz, 1H), 7.50 (d, *J* = 8.4 Hz, 1H), 6.72-6.56 (m, 1H), 6.09 (dt, *J* = 16.7, 3.2 Hz, 1H), 5.77 (d, *J* = 16.7 Hz, 1H), 5.72-5.52 (m, 1H), 4.25 (q, *J* = 7.3 Hz, 2H), 4.10-3.90 (m, 1H), 3.84-3.59 (m, 2H), 3.64-3.44 (m, 2H), 3.00 (q, *J* = 7.3 Hz, 2H), 2.31 (t, 6.9 Hz, 2H), 1.93 (q, *J* = 7.3 Hz, 1H), 1.36 (t, *J* = 7.2 Hz, 3H), 1.07 (t, *J* = 7.2 Hz, 3H).

**Example 58**

(*S*)-1-(3-(4-amino-7-(cyclopropanecarbonyl)-3-((1-ethyl-1H-benzo[d]imidazol-5-yl)ethynyl)-1H-pyrazolo[4,3-c]pyridin-1-yl)pyrrolidin-1-yl)prop-2-en-1-one

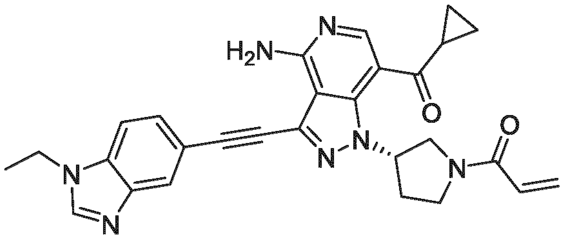

Preparation of (*S*)-1-(3-(4-amino-7-(cyclopropanecarbonyl)-3-((1-ethyl-1H-benzo[d]imidazol-5-yl)ethynyl)-1H-pyrazolo[4,3-c]pyridin-1-yl)pyrrolidin-1-yl)prop-2-en-1-one according to Reference Example 5.

MS *m/z* (ESI): 494.2 $[M+H]^+$.

$^1$H NMR (400 MHz, DMSO) $\delta$ 8.57 (d, *J* = 2.7 Hz, 1H), 8.40 (s, 1H), 8.05 (d, *J* = 1.6 Hz, 1H), 7.74 (d, *J* = 8.4 Hz, 1H), 7.58 (dd, *J* = 8.4, 1.5 Hz, 1H), 6.61 (dd, 16.7, 10.3 Hz, 1H), 6.16 (dt, *J* = 16.8, 2.6 Hz, 1H), 5.75-5.62 (m, 2H), 4.32 (q, *J* = 7.3 Hz, 2H), 4.11-3.93 (m, 1H), 3.79 (d, *J* = 6.4 Hz, 1H), 3.77-3.64 (m, 1H), 3.59 (t, *J* = 6.9 Hz, 1H), 2.49-2.41 (m, 1H), 2.34-2.24 (m, 1H), 2.00 (q, *J* = 7.3 Hz, 2H), 1.43 (t, *J* = 7.2 Hz, 4H), 1.24 (d, *J* = 3.1 Hz, 3H), 0.89-0.82 (m, 1H).

**Example 59**

(*S*)-1-(3-(4-amino-3-((1-ethyl-1H-benzo[d]imidazol-5-yl)acetylene)-7-isobutyl-1H-pyrazolo[4,3-c]pyridin-1-yl)pyrrolidin-1-yl)prop-2-en-1-one

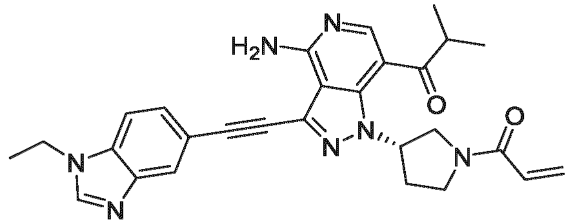

Preparation of (*S*)-1-(3-(4-amino-3-((1-ethyl-1H-benzo[d]imidazol-5-yl)acetylene)-7-isobutyl-1H-pyrazolo[4,3-c]pyridin-1-yl)pyrrolidin-1-yl)prop-2-en-1-one according to Reference Example 5.

MS *m/z* (ESI): 496.2 $[M+H]^+$.

$^1$H NMR (400 MHz, DMSO) $\delta$ 8.57 (d, *J* = 2.7 Hz, 1H), 8.40 (s, 1H), 8.05 (d, *J* = 1.6 Hz, 1H), 7.74 (d, *J* = 8.4 Hz, 1H), 7.58 (dd, *J* = 8.4, 1.5 Hz, 1H), 6.61 (dd, 16.7, 10.3 Hz, 1H), 6.16 (dt, *J* = 16.8, 2.6 Hz, 1H), 5.75-5.62 (m, 2H), 4.32 (q, *J* = 7.3 Hz, 2H), 4.11-3.93 (m, 1H), 3.79 (d, *J* = 6.4 Hz, 1H), 3.77-3.64 (m, 1H), 3.59 (t, *J* = 6.9 Hz, 1H), 2.49-2.41 (m, 1H), 2.34-2.24 (m, 1H), 2.00 (q, *J* = 7.3 Hz, 2H), 1.43 (t, *J* = 7.2 Hz, 3H), 1.17 (d, *J* = 6.7 Hz, 6H), 0.89-0.82 (m, 1H).

**Example 60**

(*S*)-1-(3-(4-amino-3-((1-ethyl-6-fluoro-1H-benzo[d]imidazol-5-yl)ethynyl)-7-propionyl-1H-pyrazolo[4,3-c]pyridin-1-yl)pyrrolidin-1-yl)prop-2-en-1-one

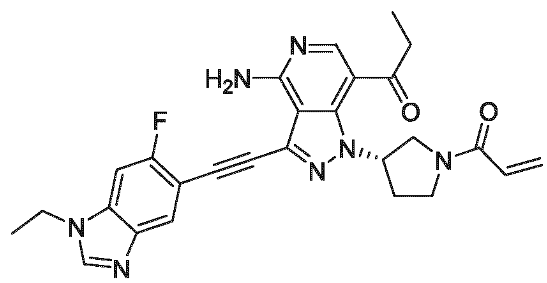

Preparation of (*S*)-1-(3-(4-amino-3-((1-ethyl-6-fluoro-1H-benzo[d]imidazol-5-yl)ethynyl)-7-propionyl-1H-pyrazolo[4,3-c]pyridin-1-yl)pyrrolidin-1-yl)prop-2-en-1-one according to Reference Example 5.

MS *m/z* (ESI): 500.2 $[M+H]^+$.

$^1$H NMR (400 MHz, DMSO) $\delta$ 8.51 (d, *J* = 2.1 Hz, 1H), 8.35 (s, 1H), 8.03 (dd, *J* = 6.4, 2.4 Hz, 1H), 7.73 (d, *J* = 9.9 Hz, 1H), 6.55 (dd, *J* = 16.8, 10.3 Hz, 1H), 6.09 (dd, *J* = 16.7, 4.4, 1H), 5.62 (dd, *J* = 15.3, 10.3, 1H), 4.22 (q, *J* = 7.2 Hz, 2H), 3.75 (dd, 13.5, 8.0 Hz, 2H), 3.55 (dd, *J* = 12.0, 6.3 Hz, 1H), 3.05-2.95 (m, 1H), 2.37 (t, J = 6.7 Hz, 1H), 2.32-2.20 (m, 1H), 1.93 (q, J = 7.2 Hz, 1H), 1.36 (q, *J* = 8.3 Hz, 3H), 1.17 (d, *J* = 3.1 Hz, 4H), 1.11-1.03 (m, 2H), 0.82-0.75 (m, 1H).

**Example 61**

(*S*)-1-(3-(4-amino-7-(cyclopropanecarbonyl)-3-((1-ethyl-6-fluoro-1H-benzo[d]imidazol-5-yl)ethynyl)-1H-pyrazolo[4,3-c]pyridin-1-yl)pyrrolidin-1-yl)prop-2-en-1-one

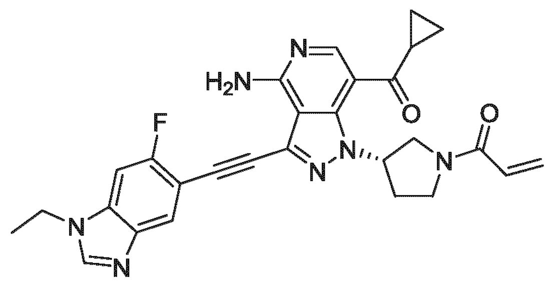

Preparation of (*S*)-1-(3-(4-amino-7-(cyclopropanecarbonyl)-3-((1-ethyl-6-fluoro-1H-benzo[d]imidazol-5-yl)ethynyl)-1H-pyrazolo[4,3-c]pyridin-1-yl)pyrrolidin-1-yl)prop-2-en-1-one according to Reference Example 5.
MS *m/z* (ESI): 512.2 $[M+H]^+$.
$^1H$ NMR (400 MHz, DMSO) $\delta$ 8.67 (d, *J* = 2.4 Hz, 1H), 8.35 (d, *J* = 2.5 Hz, 1H), 8.03 (d, *J* = 6.5 Hz, 1H), 7.73 (dd, *J* = 9.9, 2.6 Hz, 1H), 6.63-6.44 (m, 1H), 6.08 (d, *J* = 16.8 Hz, 1H), 5.61 (t, *J* = 11.8 Hz, 2H), 4.22 (q, *J* = 7.1 Hz, 2H), 3.70 (q, *J* = 8.8 Hz, 2H), 2.75 (s, 1H), 2.36 (d, *J* = 6.5 Hz, 1H), 2.22 (s, 1H), 1.92 (s, 3H), 1.34 (td, *J* = 7.3, 2.5 Hz, 3H), 0.99 (d, *J* = 13.5 Hz, 4H), 0.78 (d, *J* = 7.3 Hz, 1H).

**Example 62**

(*S*)-1-(3-(4-amino-3-((1-ethyl-6-fluoro-1H-benzo[d]imidazol-5-yl)ethynyl)-7-(thiazol-4-yl)-1H-pyrazolo[4,3-c]pyridin-1-yl)pyrrolidin-1-yl)prop-2-en-1-one

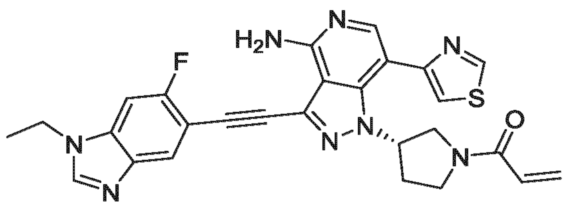

Preparation of (*S*)-1-(3-(4-amino-3-((1-ethyl-6-fluoro-1H-benzo[d]imidazol-5-yl)ethynyl)-7-(thiazol-4-yl)-1H-pyrazolo[4,3-c]pyridin-1-yl)pyrrolidin-1-yl)prop-2-en-1-one according to Reference Example 9.
MS *m/z* (ESI): 527.2 $[M+H]^+$.
H NMR (400 MHz, DMSO-$d_6$) $\delta$ 9.29 (t, *J* = 2.2 Hz, 1H), 8.41 (s, 1H), 8.08 (dd, *J* = 6.4, 2.4 Hz, 1H), 7.95 (t, *J* = 1.7 Hz, 1H), 7.84-7.75 (m, 2H), 6.75 (s, 2H), 6.56 (ddd, *J* = 39.4, 16.7, 10.3 Hz, 1H), 6.13 (dt, *J* = 16.8, 2.3 Hz, 1H), 5.67 (td, *J* = 10.3, 2.4 Hz, 1H), 5.12 (dd, *J* = 7.0, 4.0 Hz, 1H), 4.29 (q, *J* = 7.2 Hz, 2H), 3.94-3.82 (m, 1H), 3.81-3.62 (m, 2H), 3.61-3.54 (m, 1H), 2.37-2.06 (m, 2H), 1.42 (t, *J* = 7.3 Hz, 3H).

**Example 63**

(*S*)-1-(3-(7-acetyl-4-amino-3-((1-methyl-1H-benzo[d]imidazol-5-yl)ethynyl)-1H-pyrazolo[4,3-c]pyridin-1-yl)pyrrolidine-1-methyl)prop-2-en-1-one

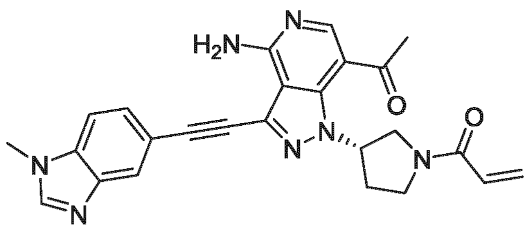

Preparation of (*S*)-1-(3-(7-acetyl-4-amino-3-((1-methyl-1H-benzo[d]imidazol-5-yl)ethynyl)-1H-pyrazolo[4,3-c]pyridin-1-yl)pyrrolidine-1-methyl)prop-2-en-1-one according to Reference Example 4.
MS *m/z* (ESI): 454.2 $[M+H]^+$.

**Example 64**

(*S*)-1-(3-(4-amino-3-((1-methyl-1H-benzo[d]imidazol-5-yl)ethynyl)-7-propionyl-1H-pyrazolo[4,3-c]pyridin-1-yl)pyrrolidin-1-yl)prop-2-en-1-one

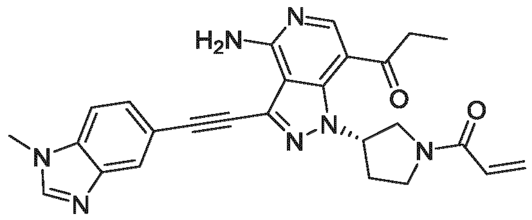

Preparation of (*S*)-1-(3-(4-amino-3-((1-methyl-1H-benzo[d]imidazol-5-yl)ethynyl)-7-propionyl-1H-pyrazolo[4,3-c]pyridin-1-yl)pyrrolidin-1-yl)prop-2-en-1-one according to Reference Example 5.

MS *m*/*z* (ESI): 468.2 $[M+H]^+$.

$^1$H NMR (400 MHz, DMSO) $\delta$ 8.50 (d, *J* = 2.2 Hz, 1H), 8.25 (s, 1H), 7.98 (s, 1H), 7.62 (d, *J* = 8.4 Hz, 1H), 7.52 (d, *J* = 8.3 Hz, 1H), 6.59 (dd, *J* = 17.0, 10.4 Hz, 1H), 6.55-6.43 (m, 1H), 6.19-5.90 (m, 1H), 5.77 (d, *J* = 8.3 Hz, 1H), 5.72-5.52 (m, 1H), 4.01 (dd, *J* = 11.3, 6.9 Hz, 1H), 3.81 (s, 3H), 3.79-3.50 (m, 3H), 3.03-2.83 (m, 2H), 2.36-2.12 (m, 2H), 1.93 (q, *J* = 7.3 Hz, 1H), 1.39 (s, 1H), 1.07 (t, *J* = 7.2 Hz, 2H).

**Example 65**

(*S*)-1-(3-(4-amino-7-(cyclopropanecarbonyl)-3-((1-methyl-1H-benzo[d]imidazol-5-yl)ethynyl)-1H-pyrazolo[4,3-c]pyridin-1-yl)pyrrolidin-1-yl)prop-2-en-1-one

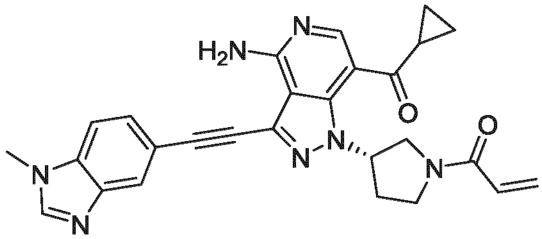

Preparation of (*S*)-1-(3-(4-amino-7-(cyclopropanecarbonyl)-3-((1-methyl-1H-benzo[d]imidazol-5-yl)ethynyl)-1H-pyrazolo[4,3-c]pyridin-1-yl)pyrrolidin-1-yl)prop-2-en-1-one according to Reference Example 5.

MS *m*/*z* (ESI): 480.2 $[M+H]^+$.

$^1$H NMR (400 MHz, DMSO) $\delta$ 8.66 (d, *J* = 2.4 Hz, 1H), 8.25 (s, 1H), 7.98 (s, 1H), 7.62 (d, *J* = 8.5 Hz, 1H), 7.52 (d, *J* = 8.0 Hz, 1H), 7.15 (s, 1H), 6.54 (dd, 16.6, 10.1 Hz, 2H), 6.14-6.04 (m, 1H), 5.70 (s, 1H), 5.67-5.56 (m, 2H), 5.26 (t, *J* = 4.8 Hz, 1H), 3.81 (s, 3H), 3.77-3.67 (m, 1H), 2.75 (s, 1H), 2.36 (d, *J* = 6.7 Hz, 1H), 2.26-2.18 (m, 1H), 1.39 (d, *J* = 8.0 Hz, 2H), 0.82-0.75 (m, 3H).

**Example 66**

(*S*)-1-(3-(4-amino-7-isobutyl-3-((1-methyl-1H-benzo[d]imidazol-5-yl)alkynyl)-1H-pyrazolo[4,3-c]pyridin-1-yl)pyrrolidin-1-yl)prop-2-en-1-one

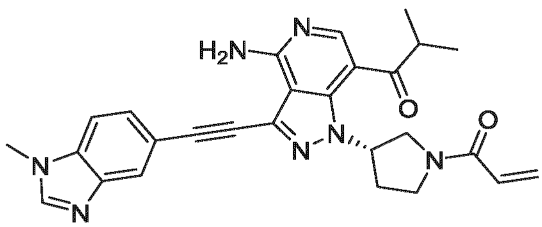

Preparation of (*S*)-1-(3-(4-amino-7-isobutyl-3-((1-methyl-1H-benzo[d]imidazol-5-yl)alkynyl)-1H-pyrazolo[4,3-c]pyridin-1-yl)pyrrolidin-1-yl)prop-2-en-1-one according to Reference Example 5.

MS *m*/*z* (ESI): 482.2 $[M+H]^+$.

$^1H$ NMR (400 MHz, DMSO) $\delta$ 8.50 (d, $J$ = 2.6 Hz, 1H), 8.25 (s, 1H), 7.98 (d, $J$ = 1.7 Hz, 1H), 7.62 (d, $J$ = 8.4 Hz, 1H), 7.52 (dd, $J$ = 8.4, 1.4 Hz, 1H), 7.16 (s, 1H), 6.54 (dd, 16.7, 10.3 Hz, 1H), 6.08 (dt, $J$ = 16.8, 2.6 Hz, 1H), 5.69-5.54 (m, 1H), 4.03-3.86 (m, 1H), 3.81 (s, 3H), 3.71 (s, 1H), 3.79-3.57 (m, 1H), 3.56-3.47 (m, 1H), 2.42-2.34 (m, 1H), 2.27-2.17 (m, 1H), 1.98-1.88 (m, 1H), 1.17 (d, $J$ = 3.1 Hz, 2H), 1.10 (d, $J$ = 6.7 Hz, 6H).

**Example 67**

(*S*)-1-(3-(7-acetyl-4-amino-3-((6-chloro-1-methyl-1H-benzo[d]imidazol-5-yl)ethynyl)-1H-pyrazolo[4,3-c]pyridin-1-yl)pyrrolidin-1-yl)prop-2-en-1-one

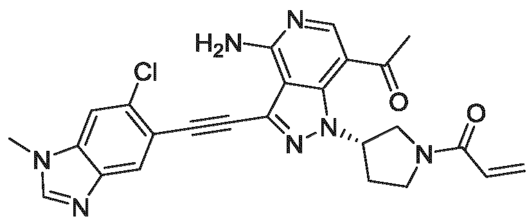

Preparation of (*S*)-1-(3-(7-acetyl-4-amino-3-((6-chloro-1-methyl-1H-benzo[d]imidazol-5-yl)ethynyl)-1H-pyrazolo[4,3-c]pyridin-1-yl)pyrrolidin-1-yl)prop-2-en-1-one according to Reference Example 4.
MS *m*/*z* (ESI): 488.2 $[M+H]^+$.

**Example 68**

(*S*)-1-(3-(4-amino-3-((6-chloro-1-methyl-1H-benzo[d]imidazol-5-yl)ethynyl)-7-(cyclopropanecarbonyl)-1H-pyrazolo[4,3-c]pyridin-1-yl)pyrrolidin-1-yl)prop-2-en-1-one

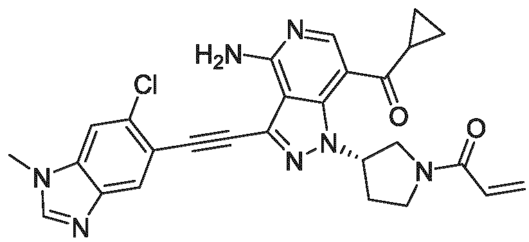

Preparation of (*S*)-1-(3-(4-amino-3-((6-chloro-1-methyl-1H-benzo[d]imidazol-5-yl)ethynyl)-7-(cyclopropanecarbonyl)-1H-pyrazolo[4,3-c]pyridin-1-yl)pyrrolidin-1-yl)prop-2-en-1-one according to Reference Example 5.
MS *m*/*z* (ESI): 514.2 $[M+H]^+$.
$^1H$ NMR (400 MHz, DMSO) $\delta$ 8.67 (d, $J$ = 2.3 Hz, 1H), 8.30 (s, 1H), 8.09 (s, 1H), 7.92 (s, 1H), 7.15 (s, 1H), 6.60 (s, 1H), 6.60-6.44 (m, 1H), 6.13-6.04 (m, 1H), 5.26 (t, $J$ = 4.8 Hz, 1H), 3.80 (s, 3H), 2.75 (s, 1H), 2.37 (d, $J$ = 7.3 Hz, 1H), 2.24 (s, 1H), 1.93 (q, $J$ = 7.3 Hz, 3H), 1.00 (d, 6.6 Hz, 3H), 0.79 (t, $J$ = 6.6 Hz, 3H).

**Example 69**

(*S*)-1-(3-(4-amino-3-((2-cyclopropyl-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one

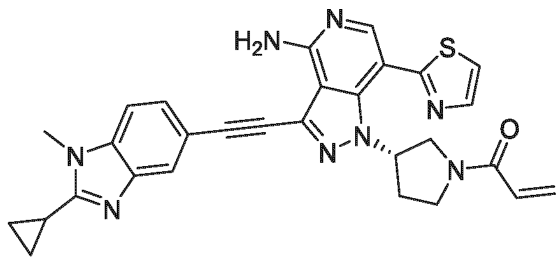

Preparation of (*S*)-1-(3-(4-amino-3-((2-cyclopropyl-1-methyl-1H-benzo[d]imidazol-5-yl)ethynyl)-7-(thiazol-2-yl)-1H-pyrazolo[4,3-c]pyridin-1-yl)pyrrolidin-1-yl)prop-2-en-1-one according to Reference Example 9.

MS *m/z* (ESI): 535.2 $[M+H]^+$.

$^1$H NMR (400 MHz, DMSO-$d_6$) $\delta$ 8.06 (d, *J* = 2.0 Hz, 1H), 8.00 (t, *J* = 3.2 Hz, 1H), 7.86-7.82 (m, 2H), 7.65 (d, *J* = 10.0 Hz, 1H), 7.34 (d, *J* = 9.6 Hz, 1H), 7.04 (s, 2H), 6.58 (ddd, *J* = 36.0, 16.8, 10.4 Hz, 2H), 6.14 (dd, *J* = 16.8, 2.0 Hz, 1H), 5.77-5.64 (m, 1H), 3.97 (s, 3H), 3.81-3.72 (m, 2H), 3.65-3.58 (m, 2H), 2.36 (td, *J* = 8.0, 4.0 Hz, 2H), 1.18-0.96 (m, 5H).

**Example 70**

(*S*)-1-(3-(7-acetyl-4-amino-3-((2-cyclopropylbenzo[d]oxazol-5-yl)alkynyl)-1H-pyrazolo[4,3-c]pyridin-1-yl)pyrrolidine-1-methyl)prop-2-en-1-one

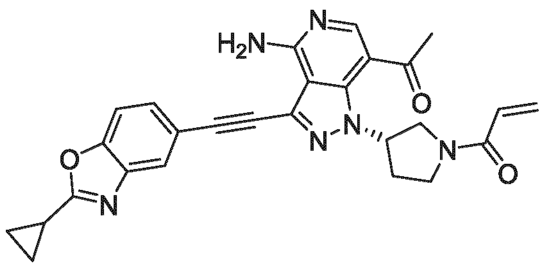

Preparation of (*S*)-1-(3-(7-acetyl-4-amino-3-((2-cyclopropylbenzo[d]oxazol-5-yl)alkynyl)-1H-pyrazolo[4,3-c]pyridin-1-yl)pyrrolidine-1-methyl)prop-2-en-1-one according to Reference Example 4.

MS *m/z* (ESI): 481.2 $[M+H]^+$.

$^1$H NMR (400 MHz, $CDCl_3$) $\delta$ 8.46 (d, *J* = 15.4 Hz, 1H), 7.83 (d, *J* = 0.9 Hz, 1H), 7.57-7.43 (m, 2H), 6.88-6.55 (m, 2H), 6.55-6.32 (m, 2H), 6.14-6.06 (m, 1H), 5.74-5.64 (m, 1H), 4.20-3.69 (m, 4H), 2.77-2.22 (m, 6H), 1.33-1.27 (m, 2H), 1.25-1.18 (m, 2H).

**Example 71**

(*S*)-1-(3-(7-acetyl-4-amino-3-(pyrazolo[1,5-a]pyridine-6-ethynylene)-1H-pyrazolo[4,3-c]pyridin-1-yl)pyrrolidine-1-methyl)prop-2-en-1-one

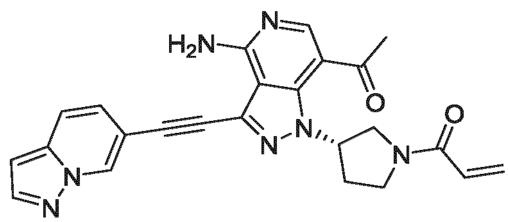

Step I: Preparation of 6-((trimethylsilyl)ethynyl)pyrazolo[1,5-a]pyridine

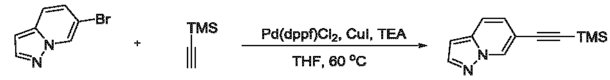


Under nitrogen protection, 6-bromopyrazolo[1,5-a]pyridine (2 g, 10.15 mmol), trimethylethynylsilane (1.5 g, 15.23 mmol), Pd(dppf)$Cl_2$ (742 mg, 1.02 mmol), CuI (387 mg, 2.03 mmol), and TEA (2.05 g, 20.3 mmol) were suspended in THF (50 mL) and heated to 60°C, and the reaction was stirred for 2 hours. After the reaction was complete, the reaction liquid was cooled and then filtered, extracted with ethyl acetate (50 mL × 3), and washed with a saturated aqueous sodium chloride solution (50 mL x 3), the organic phases were collected, dried over anhydrous sodium sulfate, filtered, and concentrated, and the residue was separated and purified by silica gel column chromatography to obtain the title compound (1.89 g, 87%).

MS *m*/*z* (ESI): 215.1 $[M+H]^+$.

Step II: Preparation of 6-ethynylpyrazolo[1,5-a]pyridine

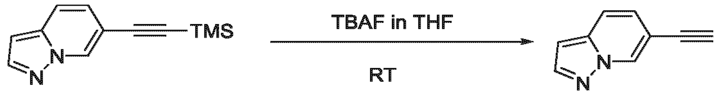


Under nitrogen protection, 6-((trimethylsilyl)ethynyl)pyrazolo[1,5-a]pyridine (1.89 g, 8.82 mmol) and TBAF(3.46 g, 13.23 mmol) were dissolved in tetrahydrofuran (50 mL), and the reaction was stirred at room temperature for 1 hour. After the reaction was complete, the reaction liquid was extracted with ethyl acetate (50 mL x 3), and washed with a saturated aqueous sodium chloride solution (50 mL x 3), the organic phases were collected, dried over anhydrous sodium sulfate, filtered, and concentrated, and the residue was separated and purified by silica gel column chromatography to obtain the title compound (780 mg, 62%).

MS *m*/*z* (ESI): 143.0 $[M+H]^+$.

Step III: Preparation of tert-butyl-(*S*)-3-(4-amino-7-bromo-3-(pyrazolo[1,5-a]pyridin-6-ylethynyl)-1H-pyrazolo[4,3-c]pyridin-1-yl)pyrrolidine-1-carboxylate

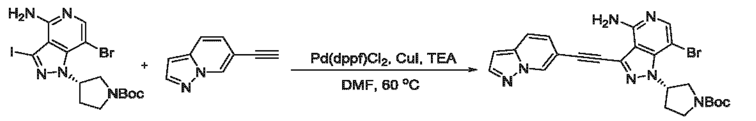


Under nitrogen protection, tert-butyl-(*S*)-3-(4-amino-5-iodo-7H-pyrrolo[2,3-d]pyrimidin-7-yl)pyrrolidine-1-carboxylate (500 mg, 0.98 mmol), 6-ethynylpyrazolo[1,5-a]pyridine (168 mg, 1.18 mmol), Pd(dppf)$Cl_2$(72 mg, 0.01 mmol), CuI (38 mg, 0.02 mmol), and TEA (199 mg, 1.97 mmol) were suspended in DMF (20 mL) and heated to 60°C, and the reaction was stirred for 2 hours. After the reaction was complete, the reaction liquid was cooled and then filtered, extracted with ethyl acetate (50 mL x 3), and washed with a saturated aqueous sodium chloride solution (50 mL x 3), the organic phases were collected, dried over anhydrous sodium sulfate, filtered, and concentrated, and the residue was separated and purified by silica gel column chromatography to obtain the title compound (450 mg, 88%).

MS *m*/*z* (ESI): 522.1 $[M+H]^+$.

Step IV: Preparation of tert-butyl-(*S*)-3-(4-amino-7-(1-butoxyvinyl)-3-(pyrazolo[1,5-a]pyridin-6-ylethynyl)-1H-pyrazolo[4,3-c]pyridin-1-yl)pyrrolidine-1-carboxylate

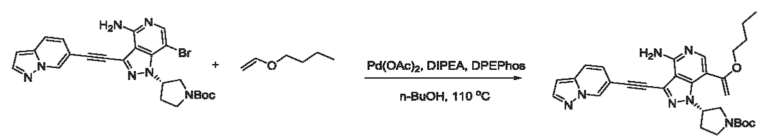

Under nitrogen protection, tert-butyl-(*S*)-3-(4-amino-7-bromo-3-(pyrazolo[1,5-a]pyridin-6-ylethynyl)-1H-pyrazolo[4,3-c]pyridin-1-yl)pyrrolidine-1-carboxylate (200 mg, 0.38 mmol), 1-(vinyloxo)butane (115 mg, 1.15 mmol), palladium acetate (9 mg, 0.04 mmol), N,N-diisopropylethylamine (148 mg, 1.15 mmol), and bis(2-diphenylphosphorusphenyl)ether (41 mg, 0.08 mmol) were suspended in n-butanol (10 mL) and heated to 110°C, and the reaction was stirred for 2 hours. After the reaction was complete, the reaction liquid was cooled and then filtered, the filtrate was extracted with ethyl acetate (30 mL x 3), and washed with a saturated aqueous sodium chloride solution (20 mL x 3), the organic phases were collected, dried over anhydrous sodium sulfate, filtered, and concentrated, and the residue was separated and purified by silica gel column chromatography to obtain the title compound (100 mg, 48%).

MS *m/z* (ESI): 542.1 [M+H]$^+$.

Step V: Preparation of (*S*)-1-(3-(7-acetyl-4-amino-3-(pyrazolo[1,5-a]pyridin-6-ylethynyl)-1H-pyrazolo[4,3-c]pyridin-1-yl) pyrrolidin-1-yl)prop-2-en-1-one

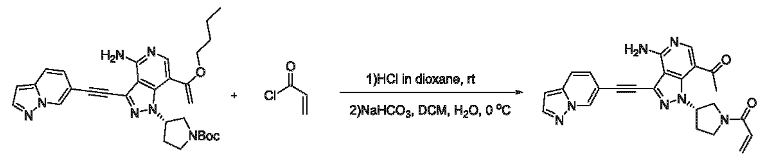


Under nitrogen protection, tert-butyl-(*S*)-3-(4-amino-7-(-butoxyvinyl)-3-(pyrazolo[1,5-a]pyridin-6-ylethynyl)-1H-pyrazolo[4,3-c]pyridin-1-yl)pyrrolidine-1-carboxylate (100 mg, 0.18 mmol) were dissolved in a solution of hydrogen chloride in dioxane (4 M, 10 mL), and the reaction was stirred at room temperature for 1 hour. The reaction liquid was dried and concentrated and then suspended, together with sodium bicarbonate (76 mg, 0.9 mmol), in a mixed solution of dichloromethane (10 mL) and water (20 mL), a solution of acryloyl chloride (20 mg, 0.22 mmol) in dichloromethane (5 mL) was dropwise added in an ice-water bath, and the reaction was stirred for 10 minutes. After the reaction was complete, the reaction liquid was extracted with dichloromethane (15 mL x 3) and washed with a saturated aqueous sodium chloride solution (10 mL x 3), the organic phases were collected, dried over anhydrous sodium sulfate, filtered, and concentrated, and the residue was separated and purified by silica gel column chromatography to obtain the title compound (44 mg, 56%).

MS *m/z* (ESI): 440.2 [M+H]$^+$.

$^1$H NMR (400 MHz, DMSO-$d_6$) $\delta$ 9.33 (s, 1H), 8.58 (d, *J* = 2.4 Hz, 1H), 8.14 (d, *J* = 2.4 Hz, 1H), 7.77 (d, *J* = 9.2 Hz, 1H), 7.41 (d, *J* = 9.2 Hz, 2H), 6.76-6.53 (m, 2H), 6.16 (ddd, *J* = 16.8, 5.2, 2.4 Hz, 1H), 6.01-5.85 (m, 1H), 5.69 (ddd, *J* = 16.4, 10.4, 2.4 Hz, 1H), 4.12-3.50 (m, 5H), 2.62 (s, 3H), 2.45 (q, *J* = 7.6 Hz, 1H), 2.33 (dt, *J* = 11.6, 6.4 Hz, 1H).

**Example 72**

(*S*)-1-(3-(4-amino-7-propionyl-3-(pyrazolo[1,5-a]pyridine-6-ethynylene)-1H-pyrazolo[4,3-c]pyridin-1-yl)pyrrolidine-1-methyl)prop-2-en-1-one

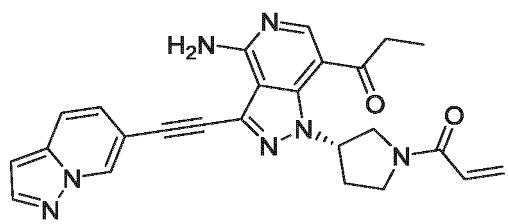

Step I: Preparation of tert-butyl-(*S*)-3-(4-amino-3-(pyrazolo[1,5-a]pyridin-6-ylethynyl)-7-vinyl-1H-pyrazolo[4,3-c]pyridin-1-yl)pyrrolidine-1-carboxylate

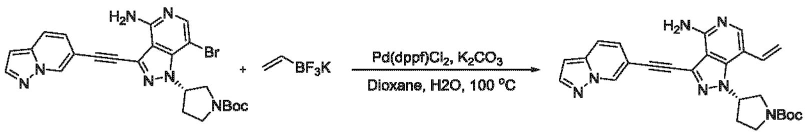


Under nitrogen protection, tert-butyl-(*S*)-3-(4-amino-7-bromo-3-(pyrazolo[1,5-a]pyridin-6-ylethynyl)-1H-pyrazolo[4,3-c]pyridin-1-yl)pyrrolidine-1-carboxylate (1.8 g, 3.45 mmol), potassium vinylfluoroborate (692 mg, 5.17 mmol), Pd(dppf)$Cl_2$ (252 mg, 0.34 mmol), and potassium carbonate (952 mg, 6.89 mmol) were suspended in a mixed solvent of 1,4-dioxane (50 mL) and water (10 mL) and heated to 100°C, and the reaction was stirred for 16 hours. After the reaction was complete, the reaction liquid was cooled and then filtered, the filtrate was extracted with ethyl acetate (30 mL x 3), and washed with a saturated aqueous sodium chloride solution (20 mL x 3), the organic phases were collected, dried over anhydrous sodium sulfate, filtered, and concentrated, and the residue was separated and purified by silica gel column chromatography to obtain the title compound (780 mg, 48%).

MS *m/z* (ESI): 470.2 $[M+H]^+$.

Step II: Preparation of tert-butyl-(*S*)-3-(4-amino-7-formyl-3-(pyrazolo[1,5-a]pyridin-6-ylethynyl)-1H-pyrazolo[4,3-c]pyridin-1-yl)pyrrolidine-1-carboxylate

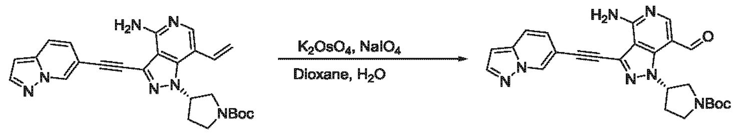


Under nitrogen protection, tert-butyl-(*S*)-3-(4-amino-3-(pyrazolo[1,5-a]pyridin-6-ylethynyl)-7-vinyl-1H-pyrazolo [4,3-c]pyridin-1-yl)pyrrolidine-1-carboxylate (373 mg, 0.79 mmol) and potassium osmate (12 mg, 0.04 mmol) were dispersed in a mixed solvent of 1,4-dioxane (50 mL) and water (10 mL). The reaction was stirred at room temperature for 10 minutes, sodium periodate (849 mg, 3.94 mmol) was then added, and the stirred reaction was continued for 2 hours. After the reaction was complete, the reaction liquid was cooled and then filtered, the filtrate was extracted with ethyl acetate (30 mL x 3), and washed with a saturated aqueous sodium chloride solution (20 mL x 3), the organic phases were collected, dried over anhydrous sodium sulfate, filtered, and concentrated, and the residue was separated and purified by silica gel column chromatography to obtain the title compound (100 mg, 27%).

MS *m/z* (ESI): 472.2 $[M+H]^+$.

Step III: Preparation of tert-butyl-(3*S*)-3-(4-amino-7-(1-hydroxypropyl)-3-(pyrazolo[1,5-a]pyridin-6-ylethynyl)-1H-pyrazolo[4,3-c]pyridin-1-yl)pyrrolidine-1-carboxylate

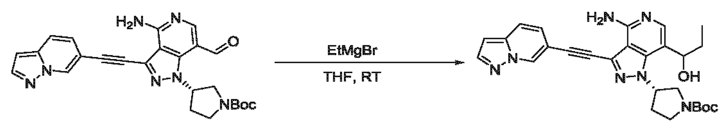


Under nitrogen protection, tert-butyl-(*S*)-3-(4-amino-7-formyl-3-(pyrazolo[1,5-a]pyridin-6-ylethynyl)-1H-pyrazolo[4,3-c]pyridin-1-yl)pyrrolidine-1-carboxylate (100 mg, 0.21 mmol) and ethylmagnesium bromide (0.2 mL, 0.63 mmol, 3M in THF) were dissolved in dry tetrahydrofuran (10 mL), and the reaction was stirred at room temperature for 1 hour. After the reaction was complete, the reaction liquid was cooled and then filtered, the filtrate was extracted with ethyl acetate (30 mL x 3), and washed with a saturated aqueous sodium chloride solution (20 mL x 3), the organic phases were collected, dried over anhydrous sodium sulfate, filtered, and concentrated, and the residue was separated and purified by silica gel column chromatography to obtain the title compound (82 mg, 77%).

MS *m/z* (ESI): 502.2 $[M+H]^+$.

Step IV: Preparation of tert-butyl-(*S*)-3-(4-amino-7-propionyl-3-(pyrazolo[1,5-a]pyridin-6-ylethynyl)-1H-pyrazolo[4,3-c] pyridin-1-yl)pyrrolidine-1-carboxylate

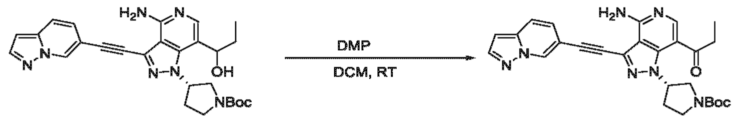


Under nitrogen protection, tert-butyl-(3*S*)-3-(4-amino-7-(1-hydroxypropyl)-3-(pyrazolo[1,5-a]pyridin-6-ylethynyl)-1H-pyrazolo[4,3-c]pyridin-1-yl)pyrrolidine-1-carboxylate (82 mg, 0.16 mmol) and Dess-Martin periodinane (104 mg, 0.26 mmol) were dissolved in dichloromethane (10 mL), and the reaction was stirred at room temperature for 1 hour. After the reaction was complete, the reaction liquid was cooled and then filtered, the filtrate was extracted with dichloromethane (30 mL x 3), and washed with a saturated aqueous sodium chloride solution (20 mL x 3), the organic phases were collected, dried over anhydrous sodium sulfate, filtered, and concentrated, and the residue was separated and purified by silica gel column chromatography to obtain the title compound (80 mg, 98%).
MS *m*/*z* (ESI): 500.2 $[M+H]^+$.

Step V: Preparation of (*S*)-1-(3-(4-amino-7-propionyl-3-(pyrazolo[1,5-a]pyridin-6-ylethynyl)-1H-pyrazolo[4,3-c]pyridin-1-yl)pyrrolidin-1-yl)prop-2-en-1-one

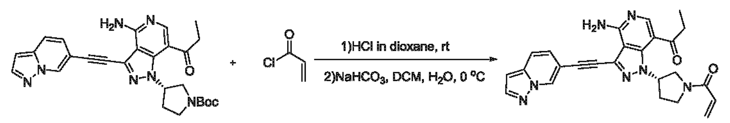


Under nitrogen protection, tert-butyl-(*S*)-3-(4-amino-7-propionyl-3-(pyrazolo[1,5-a]pyridin-6-ylethynyl)-1H-pyrazolo[4,3-c]pyridin-1-yl)pyrrolidine-1-carboxylate (80 mg, 0.16 mmol) was dissolved in a solution of hydrogen chloride in dioxane (4 M, 10 mL), and the reaction was stirred at room temperature for 1 hour. The reaction liquid was dried and concentrated and then suspended, together with sodium bicarbonate (76 mg, 0.9 mmol), in a mixed solution of dichloromethane (10 mL) and water (20 mL), a solution of acryloyl chloride (17 mg, 0.19 mmol) in dichloromethane (5 mL) was dropwise added in an ice-water bath, and the reaction was stirred for 10 minutes. After the reaction was complete, the reaction liquid was extracted with dichloromethane (15 mL x 3) and washed with a saturated aqueous sodium chloride solution (10 mL x 3), the organic phases were collected, dried over anhydrous sodium sulfate, filtered, and concentrated, and the residue was separated and purified by silica gel column chromatography to obtain the title compound (33 mg, 45%).
MS *m*/*z* (ESI): 454.1 $[M+H]^+$.
$^1$H NMR (400 MHz, DMSO-*d*$_6$) $\delta$ 9.33 (s, 1H), 8.58 (d, *J* = 2.4 Hz, 1H), 8.14 (d, *J* = 2.4 Hz, 1H), 7.77 (dd, *J* = 9.2, 1.2 Hz, 1H), 7.40 (dd, *J* = 9.2, 1.2 Hz, 1H), 7.21 (s, 1H), 6.74-6.51 (m, 2H), 6.16 (ddd, *J* = 16.8, 4.8, 2.4 Hz, 1H), 5.91-5.60 (m, 2H), 4.09-3.55 (m, 5H), 3.06 (q, *J* = 7.2 Hz, 2H), 2.32 (dd, *J* = 9.6, 5.6 Hz, 2H), 1.14 (t, *J* = 7.2 Hz, 3H).

## Example 73

(*S*)-1-(3-(4-amino-7-(cyclopropanecarbonyl)-3-(pyrazolo[1,5-a]pyridine-6-ethynylene)-1H-pyrazolo[4,3-c]pyridin-1-yl) pyrrolidine-1-methyl)prop-2-en-1-one

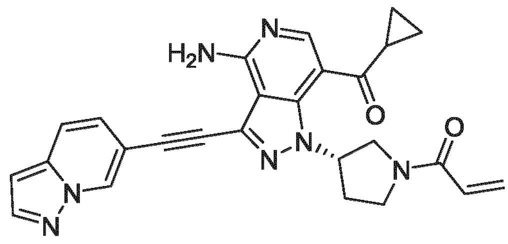

Preparation of (*S*)-1-(3-(4-amino-7-(cyclopropanecarbonyl)-3-(pyrazolo[1,5-a]pyridine-6-ethynylene)-1H-pyrazolo[4,3-c]pyridin-1-yl)pyrrolidine-1-methyl)prop-2-en-1-one according to Reference Example 72.
MS *m*/*z* (ESI): 466.2 [M+H]$^+$.

**Example 74**

(*S*)-1-(3-(7-acetyl-4-amino-3-((3-cyclopropylpyrazolo[1,5-a]pyridin-6-yl)alkynyl)-1Hpyrazolo[4,3-c]pyridin-1-yl)pyrrolidin-1-yl)prop-2-en-1-one

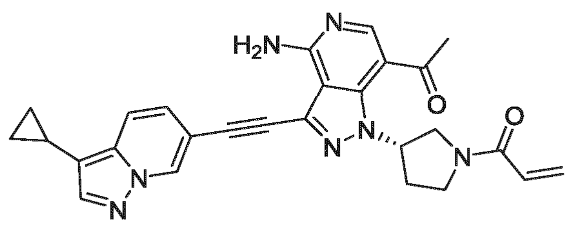

Preparation of (*S*)-1-(3-(7-acetyl-4-amino-3-((3-cyclopropylpyrazolo[1,5-a]pyridin-6-yl)alkynyl)-1Hpyrazolo [4,3-c]pyridin-1-yl)pyrrolidin-1-yl)prop-2-en-1-one according to Reference Example 71.
MS *m*/*z* (ESI): 480.2 [M+H]$^+$.

**Example 75**

(*S*)-1-(3-(7-acetyl-4-amino-3-((2-cyclopropylpyrazolo[1,5-a]pyridin-6-yl)alkynyl)-1Hpyrazolo[4,3-c]pyridin-1-yl)pyrrolidin-1-yl)prop-2-en-1-one

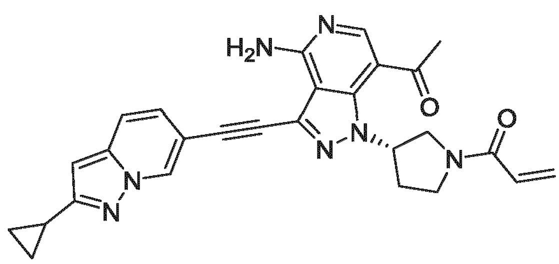

Preparation of (*S*)-1-(3-(7-acetyl-4-amino-3-((2-cyclopropylpyrazolo[1,5-a]pyridin-6-yl)alkynyl)-1Hpyrazolo [4,3-c]pyridin-1-yl)pyrrolidin-1-yl)prop-2-en-1-one according to Reference Example 71.
MS *m*/*z* (ESI): 480.2 [M+H]$^+$.

**Example 76**

(*S*)-1-(3-(4-amino-3-((3-chloropyrazolo[1,5-a]pyridin-6-yl)alkynyl)-7-isobutyl-1H-pyrazolo[4,3-c]pyridin-1-yl)pyrrolidin-1-yl)prop-2-en-1-one

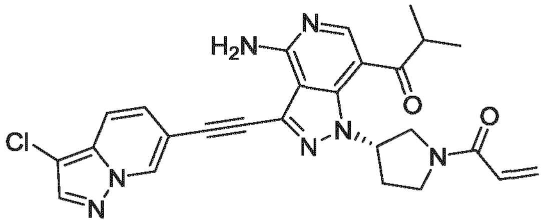

Preparation of (*S*)-1-(3-(4-amino-3-((3-chloropyrazolo[1,5-a]pyridin-6-yl)alkynyl)-7-isobutyl-1H-pyrazolo[4,3-c]pyridin-1-yl)pyrrolidin-1-yl)prop-2-en-1-one according to Reference Example 72.
MS *m*/*z* (ESI): 502.2 $[M+H]^+$.
$^1$H NMR (400 MHz, DMSO-$d_6$) $\delta$ 9.39 (s, 1H), 8.58 (d, *J* = 2.8 Hz, 1H), 8.32 (s, 1H), 7.72 (d, *J* = 9.2 Hz, 1H), 7.60-7.51 (m, 1H), 6.60 (ddd, *J* = 29.6, 16.8, 10.4 Hz, 2H), 6.15 (dt, *J* = 16.8, 2.8 Hz, 1H), 5.69 (ddd, *J* = 15.2, 9.2, 3.6 Hz, 2H), 4.10-3.57 (m, 6H), 2.35-2.23 (m, 2H), 1.17 (d, *J* = 6.8 Hz, 6H).

**Example 77**

(*S*)-1-(3-(7-acetyl-4-amino-3-(imidazo[1,2-a]pyridine-7-ethynylene)-1H-pyrazolo[4,3-c]pyridin-1-yl)pyrrolidine-1-methyl)prop-2-en-1-one

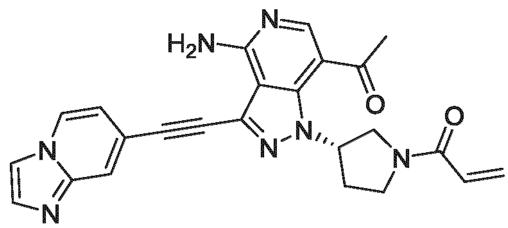

Preparation of (*S*)-1-(3-(7-acetyl-4-amino-3-(imidazo[1,2-a]pyridine-7-ethynylene)-1H-pyrazolo[4,3-c]pyridin-1-yl)pyrrolidine-1-methyl)prop-2-en-1-one according to Reference Example 71.
MS *m*/*z* (ESI): 440.2 $[M+H]^+$.
$^1$H NMR (400 MHz, DMSO-$d_6$) $\delta$ 8.58-8.49 (m, 2H), 8.00 (d, *J* = 8.0 Hz, 2H), 7.66 (s, 1H), 7.06 (dd, *J* = 7.2, 1.6 Hz, 2H), 6.55 (ddd, *J* = 30.8, 16.8, 10.4 Hz, 1H), 6.09 (ddd, *J* = 16.8, 5.2, 2.4 Hz, 1H), 5.95-5.77 (m, 1H), 5.62 (ddd, *J* = 16.4, 10.4, 2.4 Hz, 1H), 4.07-3.45 (m, 5H), 2.55 (s, 3H), 2.38 (d, *J* = 7.2 Hz, 1H), 2.27 (q, *J* = 6.4 Hz, 1H).

**Example 78**

(*S*)-1-(3-(4-amino-3-(imidazo[1,2-a]pyridine-7-ethynylene)-7-propionyl-1H-pyrazolo[4,3-c]pyridin-1-yl)pyrrolidine-1-methyl)prop-2-en-1-one

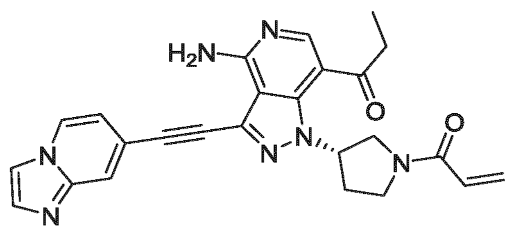

Preparation of (*S*)-1-(3-(4-amino-3-(imidazo[1,2-a]pyridine-7-ethynylene)-7-propionyl-1H-pyrazolo[4,3-c]pyridin-1-yl)pyrrolidine-1-methyl)prop-2-en-1-one according to Reference Example 72.
MS *m*/*z* (ESI): 454.2 $[M+H]^+$.

**Example 79**

(*S*)-1-(3-(4-amino-7-(cyclopropanecarbonyl)-3-(imidazo[1,2-a]pyridine-7-ethynylene)-1H-pyrazolo[4,3-c]pyridin-1-yl) pyrrolidine-1-methyl)prop-2-en-1-one

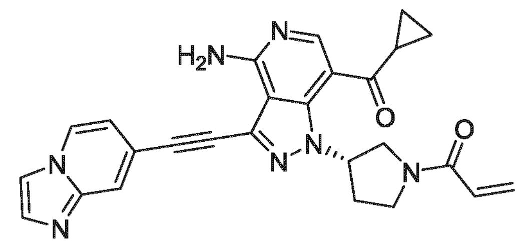

Preparation of (*S*)-1-(3-(4-amino-7-(cyclopropanecarbonyl)-3-(imidazo[1,2-a]pyridine-7-ethynylene)-1H-pyrazolo[4,3-c]pyridin-1-yl)pyrrolidine-1-methyl)prop-2-en-1-one according to Reference Example 72.
MS *m*/*z* (ESI): 466.2 $[M+H]^+$.
$^1H$ NMR (400 MHz, DMSO) $\delta$ 8.74 (d, *J* = 2.4 Hz, 1H), 8.07 (d, *J* = 8.1 Hz, 2H), 7.22 (s, 2H), 7.14 (dd, *J* = 7.1, 1.7 Hz, 1H), 6.67 (s, 1H), 6.16 (dt, *J* = 16.9, 2.7 Hz, 1H), 5.69 (dd, *J* = 12.9, 2.4 Hz, 1H), 5.33 (t, *J* = 4.7 Hz, 2H), 3.16 (s, 1H), 2.82 (s, 1H), 2.43 (d, *J* = 6.6 Hz, 3H), 1.57 (s, 2H), 0.94 (t, *J* = 7.3 Hz, 2H), 0.90-0.82 (m, 3H).

**Example 80**

(*S*)-1-(3-(7-acetyl-4-amino-3-((3-cyclopropylimidazo[1,2-a]pyridin-7-yl)alkynyl)-1H-pyrazolo[4,3-c]pyridin-1-yl)pyrrolidin-1-yl)prop-2-en-1-one

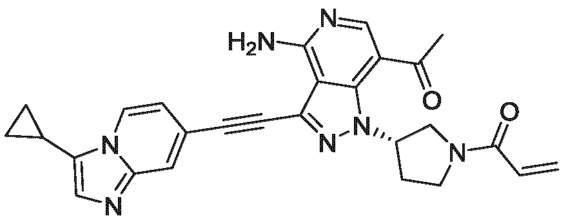

Preparation of (*S*)-1-(3-(7-acetyl-4-amino-3-((3-cyclopropylimidazo[1,2-a]pyridin-7-yl)alkynyl)-1H-pyrazolo [4,3-c]pyridin-1-yl)pyrrolidin-1-yl)prop-2-en-1-one according to Reference Example 71.
MS *m*/*z* (ESI): 480.2 $[M+H]^+$.

**Example 81**

(S)-1-(3-(7-acetyl-4-amino-3-((2-cyclopropylimidazo[1,2-a]pyridin-7-yl)alkynyl)-1H-pyrazolo[4,3-c]pyridin-1-yl)pyrrolidin-1-yl)prop-2-en-1-one

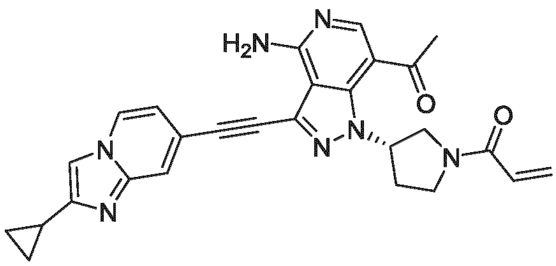

Preparation of (*S*)-1-(3-(7-acetyl-4-amino-3-((2-cyclopropylimidazo[1,2-a]pyridin-7-yl)alkynyl)-1H-pyrazolo [4,3-c]pyridin-1-yl)pyrrolidin-1-yl)prop-2-en-1-one according to Reference Example 71.

MS *m*/*z* (ESI): 480.2 [M+H]$^+$.

## Example 82

(*S*)-1-(3-(7-acetyl-4-amino-3-((2-methyl-2H-indazol-6-yl)alkynyl)-1H-pyrazolo[4,3-c]pyridin-1-yl)pyrrolidine-1-methyl) prop-2-en-1-one

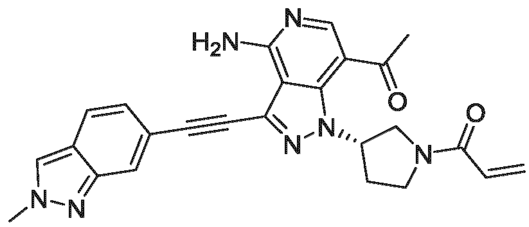

Step I: Preparation of 2-methyl-6-((trimethylsilyl)ethynyl)-2H-indazole

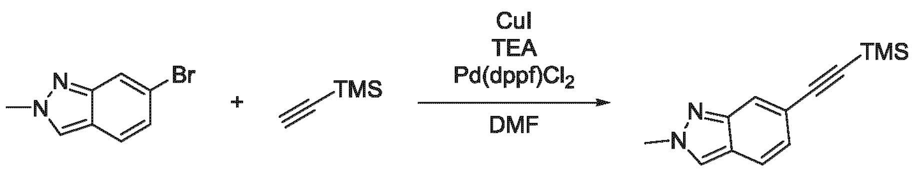


A solution of trimethylethynylsilane (1.86 g, 18.95 mmol, 2.7 mL), 6-bromo-2-methyl-2H-indazole (0.50 g, 2.37 mmol), CuI (67.7 mg, 355 μmol), TEA (1.92 g, 18.95 mmol, 2.6 mL), and Pd(dppf)$Cl_2$ (172 mg, 237 μmol) in DMF (4 mL) was warmed to 80°C and stirred for 1 hour. The reaction liquid was separated in water (20 mL) and ethyl acetate (40 mL), the organic layer was washed with water (10 mL) and saturated water (10 mL), dried over anhydrous sodium sulfate, filtered, and concentrated, and the residue was separated and purified by silica gel column chromatography to obtain the title compound (526 mg, 97%).

MS *m*/*z* (ESI): 229.1 [M+H]$^+$.

Step II: Preparation of 6-ethynyl-2-methyl-2H-indazole

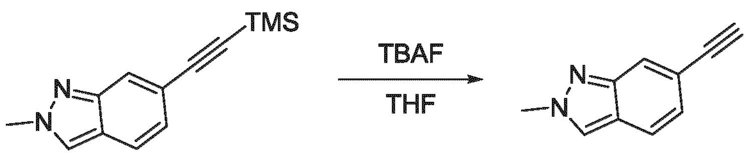


At room temperature, TBAF (1 M, 2.76 mL) was added to a solution of 2-methyl-6-((trimethylsilyl)ethynyl)-2H-indazole (0.526 g, 2.30 mmol) in THF (2 mL), and the mixture was stirred at room temperature for 1 hour. The reaction liquid was concentrated under reduced pressure, and the residue was separated and purified by silica gel column chromatography to obtain the title compound (273 mg, 76%).

MS *m*/*z* (ESI): 157.1 [M+H]$^+$.

Step III: Preparation of (*S*)-tert-butyl 3-(4-amino-7-bromo-3-((2-methyl-2H-indazol-6-yl)ethynyl)-1H-pyrazolo[4,3-c]pyridin-1-yl)pyrrolidine-1-carboxylate

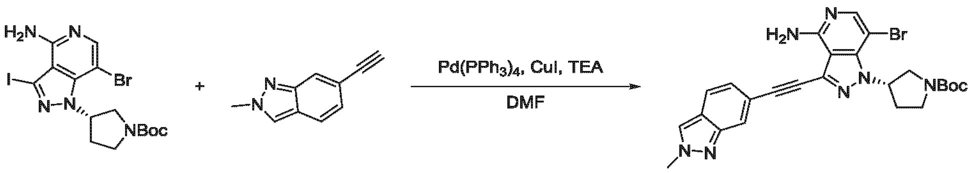


$Pd(PPh_3)_4$ (84.1 mg, 73 μmol) and CuI (41.6 mg, 218 μmol) were respectively added to a solution of (*S*)-tert-butyl 3-(4-amino-7-bromo-3-iodo-1H-pyrazolo[4,3-c]pyridin-1-yl)pyrrolidine-1-carboxylate (740 mg, 1.46 mmol), 6-ethynyl-2-methyl-2H-indazole (0.273 g, 1.75 mmol) and TEA (737 mg, 7.28 mmol, 1 mL) in DMF (7 mL), and after displacement with nitrogen three times, the solution was warmed to 65°C and stirred for 1 hour. The reaction liquid was poured into 50 mL of ice water, whereupon a solid was precipitated out. After filtration, the solid was dissolved with DCM (50 mL), dried over anhydrous sodium sulfate, filtered, and concentrated, and the residue was separated and purified by silica gel column chromatography to obtain the title compound (0.546 g, 70%).

MS *m*/*z* (ESI): 536.1 $[M+H]^+$.

Step IV: Preparation of (*S*)-tert-butyl 3-(4-amino-7-(1-butoxyvinyl)-3-((2-methyl-2H-indazol-6-yl)ethynyl)-1H-pyrazolo [4,3-c]pyridin-1-yl)pyrrolidine-1-carboxylate

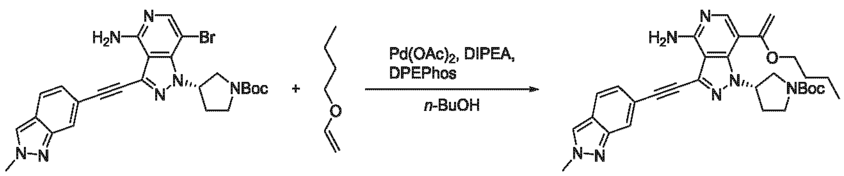


A solution of (*S*)-tert-butyl 3-(4-amino-7-bromo-3-((2-methyl-2H-indazol-6-yl)ethynyl)-1H-pyrazolo[4,3-c]pyridin-1-yl)pyrrolidine-1-carboxylate (0.546 g, 1.02 mmol), vinyl n-butyl ether (306 mg, 3.05 mmol, 395 μL), $Pd(OAc)_2$ (22.9 mg, 102 μmol), DIPEA (316 mg, 2.44 mmol, 425 μL), and DPEPhos (110 mg, 204 μmol) in n-butanol (15 mL) was warmed to 100°C under nitrogen protection and stirred for 2 hours. The reaction liquid was concentrated under reduced pressure, and the residue was separated in water (20 mL) and DCM (20 mL). The organic layer was dried over anhydrous sodium sulfate, filtered, and concentrated, and the residue was separated and purified by silica gel column chromatography to obtain the title compound (483 mg, 85%).

MS *m*/*z* (ESI): 556.3 $[M+H]^+$.

Step V: Preparation of (*S*)-1-(4-amino-3-((2-methyl-2H-indazol-6-yl)ethynyl)-1-(pyrrolidin-3-yl)-1H-pyrazolo[4,3-c]pyridin-7-yl)ethanone

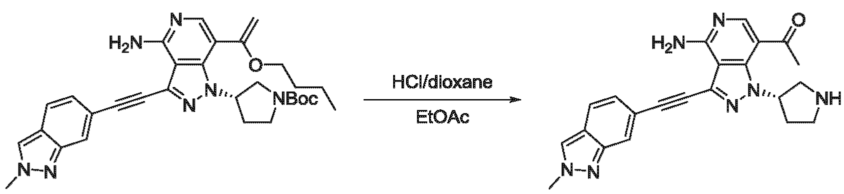


At room temperature, HCl/1,4-dioxane (4 M, 4 mL) was added to a solution of (*S*)-tert-butyl 3-(4-amino-7-(1-butoxyvinyl)-3-((2-methyl-2H-indazol-6-yl)ethynyl)-1H-pyrazolo[4,3-c]pyridin-1-yl)pyrrolidine-1-carboxylate (483 mg, 0.87 mmol) in ethyl acetate (2 mL), and the mixture was stirred at room temperature for 1 hour. The reaction liquid was concentrated under reduced pressure to obtain the title compound hydrochloride (378 mg, 99%).

MS *m*/*z* (ESI): 400.2 $[M+H]^+$.

Step VI: Preparation of (*S*)-1-(3-(7-acetyl-4-amino-3-((2-methyl-2H-indazol-6-yl)ethynyl)-1H-pyrazolo[4,3-c]pyridin-1-yl)pyrrolidin-1-yl)prop-2-en-1-one

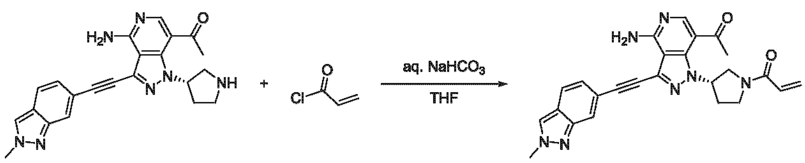


In an ice bath, a solution of acryloyl chloride (81 mg, 894 μmol, 71 μL) in THF (5 mL) was dropwise added to a suspension of (*S*)-1-(4-amino-3-((2-methyl-2H-indazol-6-yl)ethynyl)-1-(pyrrolidin-3-yl)-1H-pyrazolo[4,3-c]pyridin-7-yl) ethanone hydrochloride (0.378 g, 867 μmol), a saturated sodium bicarbonate solution (4.86 g), and THF (20 mL) and stirred in the ice bath for 15 minutes. The reaction liquid was concentrated under reduced pressure, the residue was diluted with DCM (20 mL), washed by adding water (10 mL), dried over anhydrous sodium sulfate, filtered, and concentrated, and the residue was separated and purified by silica gel column chromatography to obtain the title compound (68 mg, 17%).
MS *m*/*z* (ESI): 454.2 $[M+H]^+$.
$^1H$ NMR (400 MHz, DMSO-$d_6$) δ 8.60-8.55 (m, 1H), 8.43 (s, 1H), 8.08-8.02 (m, 1H), 7.79 (dd, *J* = 8.6, 1.0 Hz, 1H), 7.27-7.20 (m, 1H), 6.71-6.52 (m, 1H), 6.21-6.10 (m, 1H), 6.02-5.83 (m, 1H), 5.74-5.62 (m, 1H), 4.21 (s, 3H), 4.11-3.54 (m, 4H), 2.61 (s, 3H), 2.46-2.25 (m, 2H).

### Example 83

(*S*)-1-(3-(4-amino-3-((2-methyl-2H-indazol-6-yl)alkynyl)-7-propionyl-1H-pyrazolo[4,3-c]pyridin-1-yl)pyrrolidine-1-methyl)prop-2-en-1-one

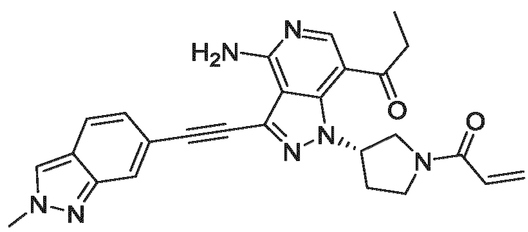

Preparation of (*S*)-1-(3-(4-amino-3-((2-methyl-2H-indazol-6-yl)alkynyl)-7-propionyl-1H-pyrazolo[4,3-c]pyridin-1-yl)pyrrolidine-1-methyl)prop-2-en-1-one according to Reference Example 82.
MS *m*/*z* (ESI): 468.2 $[M+H]^+$.
$^1H$ NMR (400 MHz, $CDCl_3$) δ 8.49 (s, 1H), 8.00 (s, 1H), 7.94 (s, 1H), 7.72-7.65 (m, 1H), 7.25-7.20 (m, 1H), 6.66-6.31 (m, 4H), 6.12-6.00 (m, 1H), 5.74-5.64 (m, 1H), 4.26 (s, 3H), 4.20-3.68 (m, 4H), 3.11-2.95 (m, 2H), 2.85-2.17 (m, 2H), 1.30-1.26 (m, 3H).

### Example 84

(S)-1-(3-(4-amino-7-(cyclopropanecarbonyl)-3-((2-methyl-2H-indazol-6-yl)ethynyl)-1H-pyrazolo[4,3-c]pyridin-1-yl)pyrrolidin-1-yl)prop-2-en-1-one

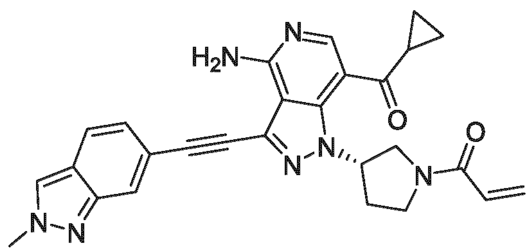

Preparation of (S)-1-(3-(4-amino-7-(cyclopropanecarbonyl)-3-((2-methyl-2H-indazol-6-yl)ethynyl)-1H-pyrazolo[4,3-c]pyridin-1-yl)pyrrolidin-1-yl)prop-2-en-1-one according to Reference Example 82.
MS *m*/*z* (ESI): 480.2 $[M+H]^+$.

$^1H$ NMR (400 MHz, $CDCl_3$) $\delta$ 8.59-8.51 (m, 1H), 8.01 (s, 1H), 7.95 (s, 1H), 7.69 (d, J = 8.6 Hz, 1H), 7.25-7.19 (m, 1H), 7.17-6.81 (m, 2H), 6.54-6.35 (m, 2H), 5.86-5.76 (m, 1H), 5.73-5.64 (m, 1H), 4.27 (s, 3H), 4.17-3.66 (m, 4H), 2.74-2.31 (m, 3H), 1.35-1.10 (m, 4H).

## Example 85

(S)-1-(3-(7-acetyl-4-amino-3-((2-ethyl-2H-indazol-6-yl)alkynyl)-1H-pyrazolo[4,3-c]pyridin-1-yl)pyrrolidine-1-methyl) prop-2-en-1-one

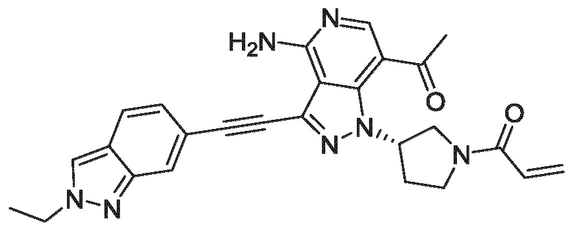

Step I: Preparation of 6-bromo-2-ethyl-2H-indazole

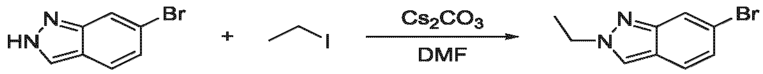


At room temperature, cesium carbonate (1.99 g, 6.09 mmol) was added to a mixture of 6-bromoindazole (1 g, 5.08 mmol) and iodoethane (1.19 g, 7.61 mmol, 609 μL) in DMF (10 mL) and stirred at 70°C for 1.5 hours. The reaction liquid was separated in water (50 mL) and ethyl acetate (80 mL), the organic layer was washed with a saturated sodium chloride solution (50 mL), dried over anhydrous sodium sulfate, filtered, and concentrated, and the residue was separated and purified by silica gel column chromatography to obtain the title compound (0.382 g, 33%).

MS *m*/*z* (ESI): 225.0 $[M+H]^+$.

Step II: Preparation of 2-ethyl-6-((trimethylsilyl)ethynyl)-2H-indazole

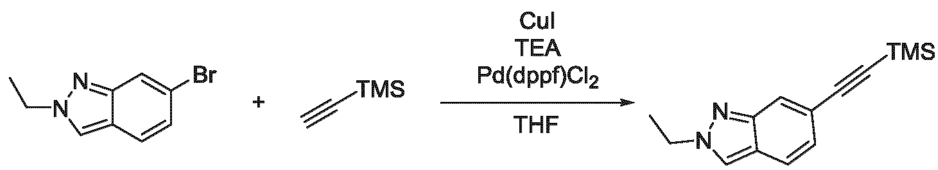


A solution of trimethylethynylsilane (833 mg, 8.49 mmol, 1.2 mL), 6-bromo-2-ethyl-2H-indazole (0.382 g, 1.70 mmol), CuI (48.5 mg, 255 μmol), TEA (859 mg, 8.49 mmol, 1.2 mL), and $Pd(dppf)Cl_2$ (123 mg, 170 μmol) in THF (10 mL) was warmed to 60°C and stirred for 2 hours. The reaction liquid was concentrated under reduced pressure, and the residue was separated and purified by silica gel column chromatography to obtain the title compound (253 mg, 86%).

MS *m*/*z* (ESI): 243.1 $[M+H]^+$.

Step III: Preparation of 2-ethyl-6-ethynyl-2H-indazole

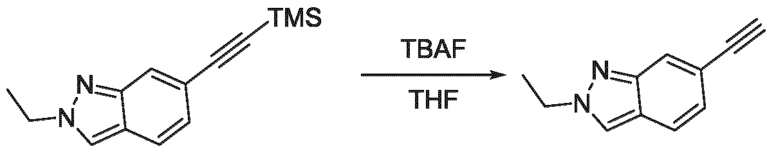


At room temperature, TBAF (1 M, 1.74 mL) was added to a solution of 2-ethyl-6-((trimethylsilyl)ethynyl)-2H-indazole (0.352 g, 1.45 mmol) in THF (1 mL), and the mixture was stirred at room temperature for 1 hour. The reaction liquid was concentrated under reduced pressure, and the residue was separated and purified by silica gel column chromatography to obtain the title compound (0.245 g, 99%).
MS *m/z* (ESI): 171.1 $[M+H]^+$.

Step IV: Preparation of (S)-tert-butyl 3-(4-amino-7-bromo-3-((2-ethyl-2H-indazol-6-yl)ethynyl)-1H-pyrazolo[4,3-c]pyridin-1-yl)pyrrolidine-1-carboxylate

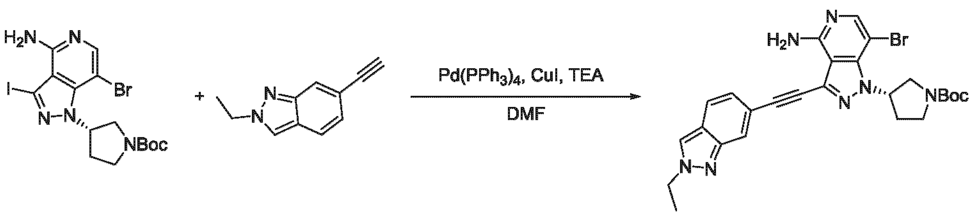


$Pd(PPh_3)_4$ (166 mg, 144 μmol) and CuI (41.1 mg, 216 μmol) were respectively added to a solution of (*S*)-tert-butyl 3-(4-amino-7-bromo-3-iodo-1H-pyrazolo[4,3-c]pyridin-1-yl)pyrrolidine-1-carboxylate (731 mg, 1.44 mmol), 2-ethyl-6-ethynyl-2H-indazole (0.245 g, 1.44 mmol), TEA (728 mg, 7.2 mmol, 1 mL) in DMF (8 mL), and after displacement with nitrogen three times, the solution was warmed to 65°C and stirred for 1 hour. The reaction liquid was poured into 50 mL of ice water, whereupon a solid was precipitated out. After filtration, the solid was dissolved with DCM (50 mL), dried over anhydrous sodium sulfate, filtered, and concentrated, and the residue was separated and purified by silica gel column chromatography to obtain the title compound (0.511 g, 64%).
MS *m/z* (ESI): 550.2 $[M+H]^+$.

Step V: Preparation of (*S*)-tert-butyl 3-(4-amino-7-(1-butoxyvinyl)-3-((2-ethyl-2H-indazol-6-yl)ethynyl)-1H-pyrazolo[4,3-c]pyridin-1-yl)pyrrolidine-1-carboxylate

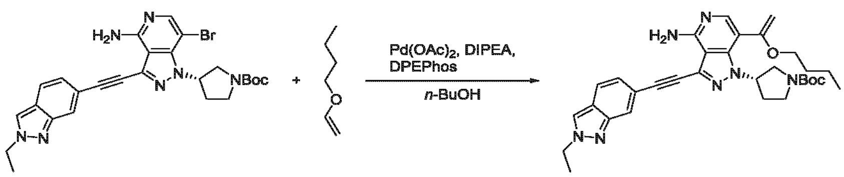


A solution of (*S*)-tert-butyl 3-(4-amino-7-bromo-3-((2-ethyl-2H-indazol-6-yl)ethynyl)-1H-pyrazolo[4,3-c]pyridin-1-yl)pyrrolidine-1-carboxylate (511 mg, 928 μmol), vinyl n-butyl ether (465 mg, 4.64 mmol, 601 μL), $Pd(OAc)_2$ (20.9 mg, 92 μmol), DIPEA (288 mg, 2.23 mmol, 388 μL), DPEPhos (100 mg, 186 μmol) in n-butanol (15 mL) was warmed to 100°C under nitrogen protection and stirred for 2 hours. The reaction liquid was concentrated under reduced pressure, and the residue was separated in water (20 mL) and DCM (20 mL). The organic layer was dried over anhydrous sodium sulfate, filtered, and concentrated under reduced pressure, and the residue was separated and purified by silica gel column chromatography to obtain the title compound (0.520 g, 98%).
MS *m/z* (ESI): 570.3 $[M+H]^+$.

Step VI: Preparation of (*S*)-1-(4-amino-3-((2-ethyl-2H-indazol-6-yl)ethynyl)-1-(pyrrolidin-3-yl)-1H-pyrazolo[4,3-c]pyridin-7-yl)ethanone

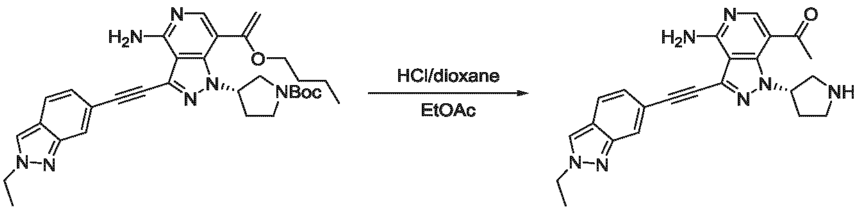


At room temperature, HCl/1,4-dioxane (4 M, 4 mL) was added to a solution of (*S*)-tert-butyl 3-(4-amino-7-(1-butoxyvinyl)-3-((2-ethyl-2H-indazol-6-yl)ethynyl)-1H-pyrazolo[4,3-c]pyridin-1-yl)pyrrolidine-1-carboxylate (520 mg, 913 μmol) in ethyl acetate (2 mL), and the mixture was stirred at room temperature for 1 hour. The reaction liquid was concentrated under reduced pressure to obtain the title compound hydrochloride (410 mg).
MS *m*/*z* (ESI): 414.2 $[M+H]^+$.

Step VI: Preparation of (*S*)-1-(3-(7-acetyl-4-amino-3-((2-ethyl-2H-indazol-6-yl)ethynyl)-1H-pyrazolo[4,3-c]pyridin-1-yl) pyrrolidin-1-yl)prop-2-en-1-one

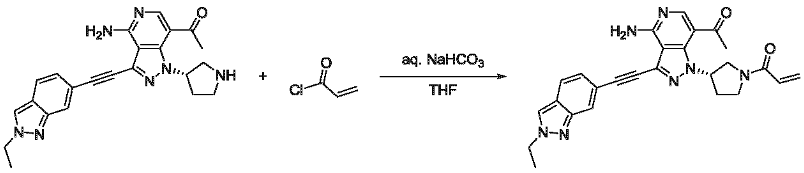


In an ice bath, a solution of acryloyl chloride (66 mg, 729 μmol, 58 μL) in THF (5 mL) was dropwise added to a suspension of (*S*)-1-(4-amino-3-((2-ethyl-2H-indazol-6-yl)ethynyl)-1-(pyrrolidin-3-yl)-1H-pyrazolo[4,3-c]pyridin-7-yl) ethanone hydrochloride (410 mg), a saturated sodium bicarbonate solution (5.10 g), and THF (20 mL) and stirred in the ice bath for 15 minutes. The reaction liquid was concentrated under reduced pressure, the residue was diluted with DCM (20 mL), washed by adding water (10 mL), dried over anhydrous sodium sulfate, filtered, and concentrated, and the residue was separated and purified by silica gel column chromatography to obtain the title compound (50 mg).
MS *m*/*z* (ESI): 468.2 $[M+H]^+$.
$^1H$ NMR (400 MHz, $CDCl_3$) $\delta$ 8.53-8.45 (m, 1H), 8.03-7.95 (m, 2H), 7.72-7.65 (m, 1H), 7.24-7.18 (m, 1H), 6.64-6.33 (m, 4H), 6.18-6.07 (m, 1H), 5.73-5.63 (m, 1H), 4.51 (q, *J* = 7.3 Hz, 2H), 4.18-3.70 (m, 4H), 2.83-2.35 (m, 5H), 1.66 (t, *J* = 7.3 Hz, 3H).

**Example 86**

(*S*)-1-(3-(4-amino-7-(cyclopropanecarbonyl)-3-((2-ethyl-2H-indazol-6-yl)ethynyl)-1H-pyrazolo[4,3-c]pyridin-1-yl)pyrrolidine-1-methyl)prop-2-en-1-one

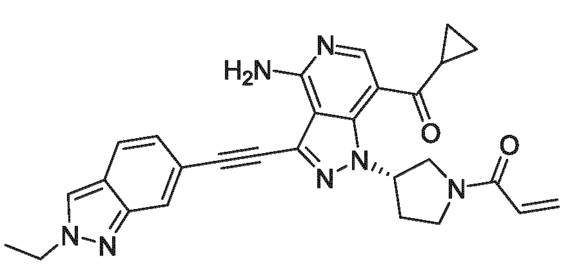

Preparation of (*S*)-1-(3-(4-amino-7-(cyclopropanecarbonyl)-3-((2-ethyl-2H-indazol-6-yl)ethynyl)-1H-pyrazolo [4,3-c]pyridin-1-yl)pyrrolidine-1-methyl)prop-2-en-1-one according to Reference Example 85.
MS *m*/*z* (ESI): 494.2 $[M+H]^+$.

Example 87

(*S*)-1-(3-(7-acetyl-4-amino-3-((2-cyclopropyl-2H-indazol-6-yl)ethynyl)-1H-pyrazolo[4,3-c]pyridin-1-yl)pyrrolidine-1-methyl)prop-2-en-1-one

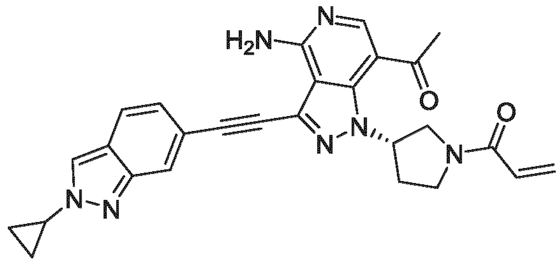

Step I: Preparation of N-(4-bromo-2-nitrobenzenemethyl)cyclopropylamine

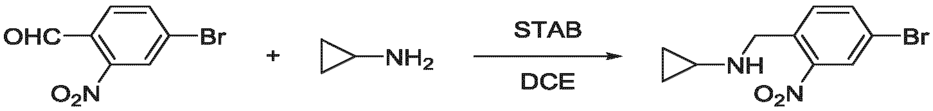


At room temperature, sodium borohydride acetate (691 mg, 3.26 mmol) was added to a solution of 4-bromo-2-nitrobenzaldehyde (0.50 g, 2.17 mmol) and cyclopropylamine (149 mg, 2.61 mmol, 181 μL) in DCE (10 mL) and stirred at room temperature for 16 hours. The reaction liquid was separated in a saturated sodium bicarbonate solution (20 mL) and DCM (20 mL), the organic layer was dried over anhydrous sodium sulfate, filtered, and concentrated under reduced pressure, and the residue was separated and purified by silica gel column chromatography to obtain the title compound (0.29 g, 49%).
MS *m*/*z* (ESI): 271.0 [M+H]$^+$.

Step II: Preparation of 6-bromo-2-cyclopropyl-2H-indazole

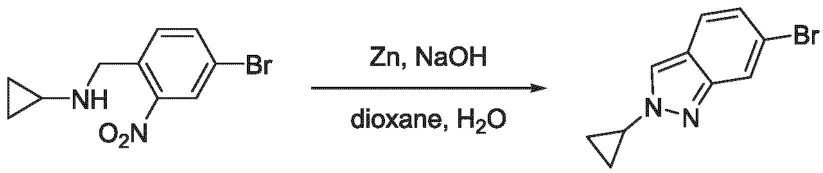


In an ice bath, an aqueous solution (10 mL) of NaOH (428 mg, 10.70 mmol) was dropwise added to a suspension of N-(4-bromo-2-nitrobenzenemethyl)cyclopropylamine (290 mg, 1.07 mmol) and zinc powder (278 mg, 4.28 mmol) in 1,4-dioxane (10 mL) and stirred at room temperature for 2 hours. The reaction liquid was separated in water (20 mL) and DCM (20 mL), the organic layer was washed with a saturated sodium chloride solution (10 mL), dried over anhydrous sodium sulfate, filtered, and concentrated, and the residue was separated and purified by silica gel column chromatography to obtain the title compound (98 mg, 39%).
MS *m*/*z* (ESI): 237.0 [M+H]$^+$.

Step III: Preparation of 2-cyclopropyl-6-((trimethylsilyl)ethynyl)-2H-indazole

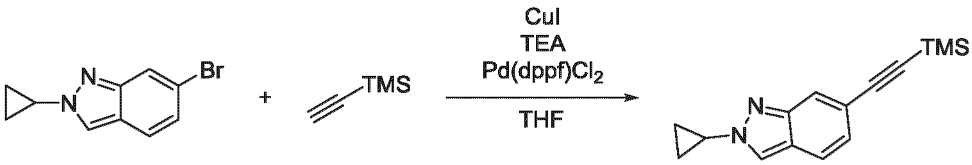


A solution of trimethylethynylsilane (203 mg, 2.07 mmol, 292 μL), 6-bromo-2-cyclopropyl-2H-indazole (98 mg, 413 μmol), CuI (11.8 mg, 62 μmol), TEA (209 mg, 2.07 mmol, 288 μL), and Pd(dppf)$Cl_2$ (30 mg, 41 μmol) in THF (12 mL) was warmed to 60°C and stirred for 2 hours. The reaction liquid was cooled and then filtered, and the filtrate was concentrated under reduced pressure, and the residue was separated and purified by silica gel column chromatography to obtain the title compound (102 mg, 97%).

MS *m*/*z* (ESI): 255.1 $[M+H]^+$.

Step IV: Preparation of 2-cyclopropyl-6-ethynyl-2H-indazole

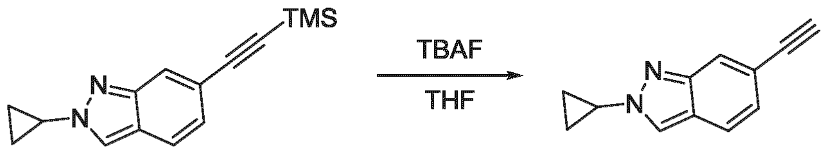


At room temperature, TBAF (1 M, 0.46 mL) was added to a solution of 2-cyclopropyl-6-((trimethylsilyl)ethynyl)-2H-indazole (98 mg, 385 μmol) in THF (0.5 mL), and the mixture was stirred at room temperature for 0.5 hours. The reaction liquid was concentrated under reduced pressure, and the residue was separated and purified by silica gel column chromatography to obtain the title compound (60 mg, 85%).

MS *m*/*z* (ESI): 183.1 $[M+H]^+$.

Step V: Preparation of (*S*)-tert-butyl 3-(4-amino-7-bromo-3-((2-cyclopropyl-2H-indazol-6-yl)ethynyl)-1H-pyrazolo[4,3-c]pyridin-1-yl)pyrrolidine-1-carboxylate

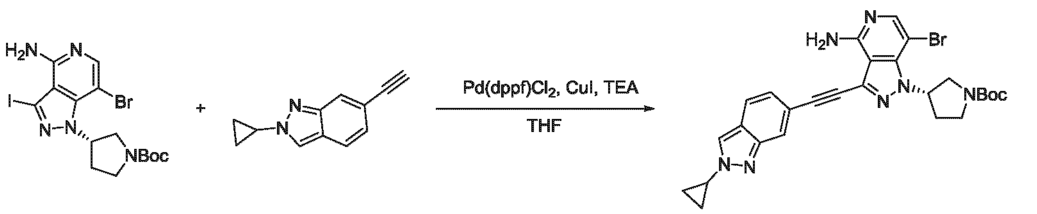


Pd(dppf)$Cl_2$ (23.9 mg, 33 μmol) and CuI (12.5 mg, 66 μmol) were respectively added to a solution of (*S*)-tert-butyl 3-(4-amino-7-bromo-3-iodo-1H-pyrazolo[4,3-c]pyridin-1-yl)pyrrolidine-1-carboxylate (167 mg, 329 μmol), 2-cyclopropyl-6-ethynyl-2H-indazole (60 mg, 329 μmol), and TEA (167 mg, 1.65 mmol, 230 μL) in THF (5 mL), and after displacement with nitrogen three times, the solution was warmed to 65°C and stirred for 2 hours. The reaction liquid was concentrated under reduced pressure, and the residue was separated and purified by silica gel column chromatography to obtain the title compound (158 mg, 85%).

MS *m*/*z* (ESI): 562.2 $[M+H]^+$.

Step VI: Preparation of (*S*)-tert-butyl 3-(4-amino-7-(1-butoxyvinyl)-3-((2-cyclopropyl-2H-indazol-6-yl)ethynyl)-1H-pyrazolo[4,3-c]pyridin-1-yl)pyrrolidine-1-carboxylate

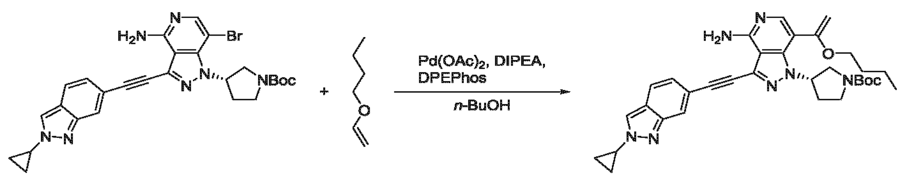


A solution of (*S*)-tert-butyl 3-(4-amino-7-bromo-3-((2-cyclopropyl-2H-indazol-6-yl)ethynyl)-1H-pyrazolo[4,3-c]pyridin-1-yl)pyrrolidine-1-carboxylate (158 mg, 281 μmol), vinyl n-butyl ether (232 mg, 2.32 mmol, 300 μL), $Pd(OAc)_2$(15 mg, 67 μmol), DIPEA (223 mg, 1.72 mmol, 300 μL), and DPEPhos (30 mg, 56 μmol) in n-butanol (3 mL) was warmed to 100°C under nitrogen protection and stirred for 1 hour. The reaction liquid was concentrated under reduced pressure, and the residue was separated in water (20 mL) and DCM (20 mL). The organic layer was dried over anhydrous sodium sulfate, filtered, and concentrated, and the residue was separated and purified by silica gel column chromatography to obtain the title compound (110 mg, 67%).

MS *m*/*z* (ESI): 582.3 $[M+H]^+$.

Step VII: Preparation of (*S*)-1-(4-amino-3-((2-cyclopropyl-2H-indazol-6-yl)ethynyl)-1-(pyrrolidin-3-yl)-1H-pyrazolo[4,3-c]pyridin-7-yl)ethanone

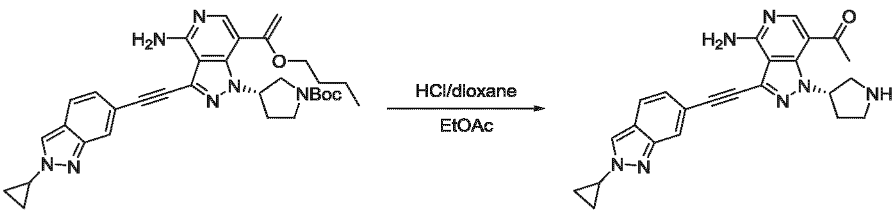


At room temperature, HCl/1,4-dioxane (4 M, 2 mL) was added to a solution of (*S*)-tert-butyl 3-(4-amino-7-(1-butoxyvinyl)-3-((2-cyclopropyl-2H-indazol-6-yl)ethynyl)-1H-pyrazolo[4,3-c]pyridin-1-yl)pyrrolidine-1-carboxylate (110 mg, 189 μmol) in ethyl acetate (2 mL), and the mixture was stirred at room temperature for 0.5 hours. The reaction liquid was concentrated under reduced pressure to obtain the title compound hydrochloride (87 mg).

MS *m*/*z* (ESI): 426.2 $[M+H]^+$.

Step VIII: Preparation of (*S*)-1-(3-(7-acetyl-4-amino-3-((2-cyclopropyl-2H-indazol-6-yl)ethynyl)-1H-pyrazolo[4,3-c]pyridin-1-yl)pyrrolidin-1-yl)prop-2-en-1-one

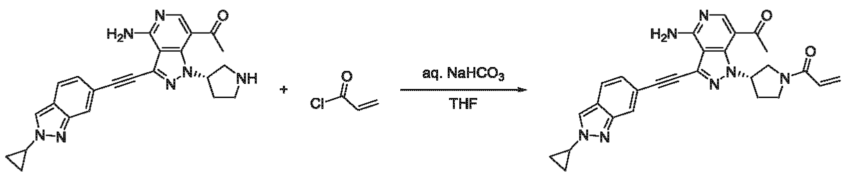


In an ice bath, a solution of acryloyl chloride (13.6 mg, 151 μmol, 12 μL) in THF (0.1 mL) was dropwise added to a suspension of (*S*)-1-(4-amino-3-((2-cyclopropyl-2H-indazol-6-yl)ethynyl)-1-(pyrrolidin-3-yl)-1H-pyrazolo[4,3-c]pyridin-7-yl)ethanone hydrochloride (87 mg, 188 μmol), a saturated sodium bicarbonate solution (1 g), and THF (5 mL), and stirred in the ice bath for 15 minutes. The reaction liquid was concentrated under reduced pressure, the residue was diluted with DCM (30 mL) and washed by adding water (10 mL), the organic layer was dried over anhydrous sodium sulfate, filtered, and concentrated, and the residue was separated and purified by silica gel column chromatography to obtain the title compound (13 mg, 13%).

MS *m*/*z* (ESI): 480.2 $[M+H]^+$.

$^1H$ NMR (400 MHz, $CDCl_3$) δ 8.62-8.38 (m, 1H), 8.03 (s, 1H), 7.97 (s, 1H), 7.69-7.62 (m, 1H), 7.24-7.17 (m, 1H), 6.92-6.54 (m, 2H), 6.53-6.33 (m, 2H), 6.16-6.05 (m, 1H), 5.74-5.64 (m, 1H), 4.19-3.64 (m, 5H), 2.85-2.33 (m, 5H), 1.43-1.30 (m, 2H), 1.25-1.16 (m, 2H).

**Example 88**

(*S*)-1-(3-(4-amino-7-(cyclopropanecarbonyl)-3-((2-cyclopropyl-2H-indazol-6-yl)ethynyl)-1H-pyrazolo[4,3-c]pyridin-1-yl)pyrrolidin-1-yl)prop-2-en-1-one

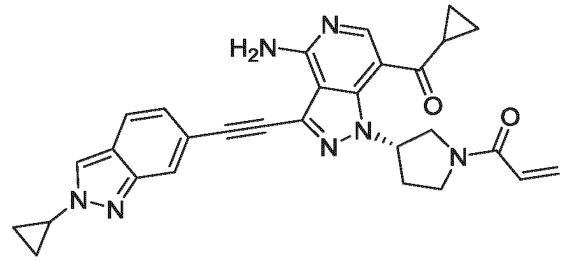

Preparation of (*S*)-1-(3-(4-amino-7-(cyclopropanecarbonyl)-3-((2-cyclopropyl-2H-indazol-6-yl)ethynyl)-1H-pyrazolo[4,3-c]pyridin-1-yl)pyrrolidin-1-yl)prop-2-en-1-one according to Reference Example 87.

MS *m*/*z* (ESI): 506.2 $[M+H]^+$.

**Example 89**

(*S*)-1-(3-(7-acetyl-4-amino-3-(benzo[c]isothiazole-6-ethynylene)-1H-pyrazolo[4,3-c]pyridin-1-yl)pyrrolidine-1-methyl)prop-2-en-1-one

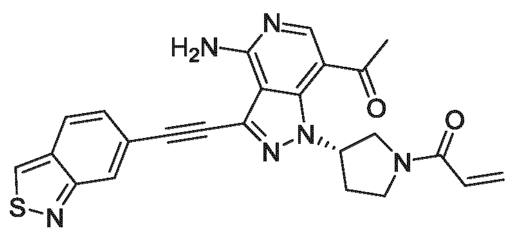

Step I: Preparation of 6-((trimethylsilyl)ethynyl)benzo[c]isothiazole

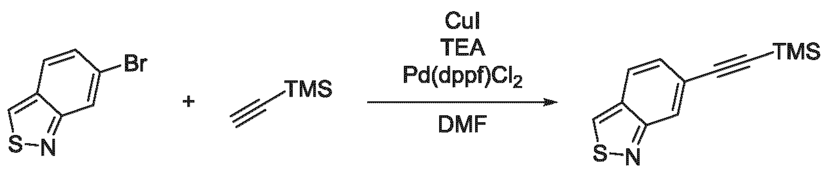


A solution of trimethylethynylsilane (688 mg, 7.01 mmol, 990 μL), 6-bromobenzo[C]isothiazole (300 mg, 1.40 mmol), CuI (40 mg, 210 μmol), TEA (1.13 g, 11.21 mmol, 1.6 mL), and Pd(dppf)$Cl_2$ (102 mg, 140 μmol) in DMF (3 mL) was warmed to 60°C and stirred for 1 hour. The reaction liquid was separated in water (10 mL) and ethyl acetate (20 mL), the organic layer was washed with water (10 mL) and a saturated sodium chloride solution (10 mL), dried over anhydrous sodium sulfate, filtered, and concentrated, and the residue was separated and purified by silica gel column chromatography to obtain the title compound (0.252 g, 78%).

MS *m*/*z* (ESI): 232.1 $[M+H]^+$.

Step II: Preparation of 6-ethynylbenzo[c]isothiazole

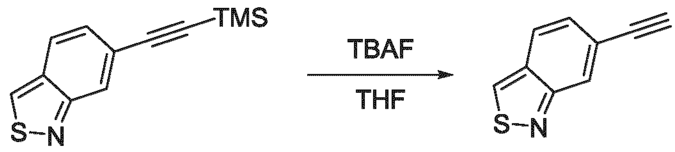


At room temperature, TBAF (1 M, 1.3 mL) was added to a solution of 6-((trimethylsilyl)ethynyl)benzo[c]isothiazole (0.252 g, 1.09 mmol) in THF (1 mL), and the mixture was stirred at room temperature for 0.5 hours. The reaction liquid was concentrated under reduced pressure, and the residue was separated and purified by silica gel column chromatography to obtain the title compound (0.166 g, 96%).

MS *m/z* (ESI): 160.1 [M+H]$^+$.

Step III: Preparation of (*S*)-tert-butyl 3-(4-amino-3-(benzo[c]isothiazol-6-ylethynyl)-7-bromo-1H-pyrazolo[4,3-c]pyridin-1-yl)pyrrolidine-1-carboxylate

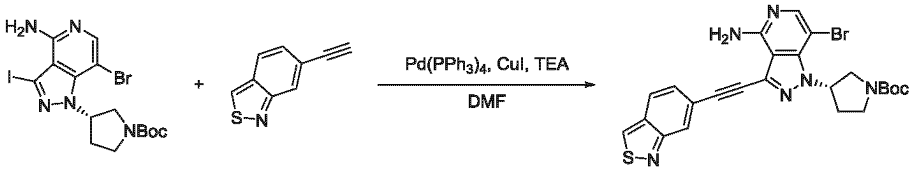


Pd(PPh3)$_4$ (50.2 mg, 43 μmol) and CuI (24.8 mg, 130 μmol) were respectively added to a solution of (*S*)-tert-butyl 3-(4-amino-7-bromo-3-iodo-1H-pyrazolo[4,3-c]pyridin-1-yl)pyrrolidine-1-carboxylate (442 mg, 869 μmol), 6-ethynylbenzo[c]isothiazole (166 mg, 1.04 mmol) and TEA (440 mg, 4.34 mmol, 606 μL) in DMF (4.5 mL), and after displacement with nitrogen three times, the solution was warmed to 65°C and stirred for 1 hour. The reaction liquid was poured into 30 mL of ice water, whereupon a solid was precipitated out. After filtration, the solid was dissolved with DCM (20 mL), dried over anhydrous sodium sulfate, filtered, and concentrated, and the residue was separated and purified by silica gel column chromatography to obtain the title compound (0.419 g, 89%).

MS *m/z* (ESI): 539.1 [M+H]$^+$.

Step IV: Preparation of (*S*)-tert-butyl 3-(4-amino-3-(benzo[c]isothiazol-6-ylethynyl)-7-(1-butoxyvinyl)-1H-pyrazolo[4,3-c]pyridin-1-yl)pyrrolidine-1-carboxylate

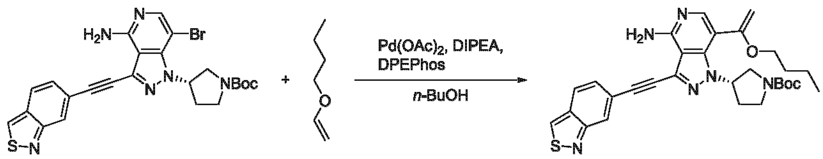


A suspension of (*S*)-tert-butyl 3-(4-amino-3-(benzo[c]isothiazol-6-ylethynyl)-7-bromo-1H-pyrazolo[4,3-c]pyridin-1-yl)pyrrolidine-1-carboxylate (0.419 g, 777 μmol), vinyl n-butyl ether (389 mg, 3.88 mmol, 503 μL), Pd(OAc)$_2$ (17.5 mg, 77 μmol), DIPEA (241 mg, 1.86 mmol, 325 μL), and DPEPhos (84 mg, 155 μmol) in n-butanol (12 mL) was warmed to 100°C under nitrogen protection and stirred for 4 hours. The reaction liquid was concentrated under reduced pressure, and the residue was separated in water (20 mL) and DCM (20 mL). The organic layer was dried over anhydrous sodium sulfate, filtered, and concentrated, and the residue was separated and purified by silica gel column chromatography to obtain the title compound (0.289 g, 67%).

MS *m/z* (ESI): 559.2 [M+H]$^+$.

Step V: Preparation of (*S*)-1-(4-amino-3-(benzo[c]isothiazol-6-ylethynyl)-1-(pyrrolidin-3-yl)-1H-pyrazolo[4,3-c]pyridin-7-yl)ethanone

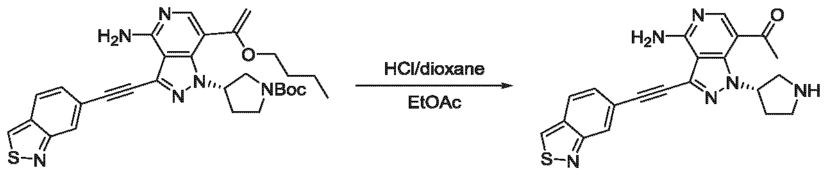


At room temperature, HCl/1,4-dioxane (4 M, 4 mL) was added to a solution of (*S*)-tert-butyl 3-(4-amino-3-(benzo[c]isothiazol-6-ylethynyl)-7-(1-butoxyvinyl)-1H-pyrazolo[4,3-c]pyridin-1-yl)pyrrolidine-1-carboxylate (0.289 g, 517 μmol) in ethyl acetate (2 mL), and the mixture was stirred at room temperature for 0.5 hours. The reaction liquid was concentrated under reduced pressure to obtain the title compound hydrochloride (0.227 g).

MS *m/z* (ESI): 403.1 $[M+H]^+$.

Step VI: Preparation of (*S*)-1-(3-(7-acetyl-4-amino-3-(benzo[c]isothiazol-6-ylethynyl)-1H-pyrazolo[4,3-c]pyridin-1-yl)pyrrolidin-1-yl)prop-2-en-1-one

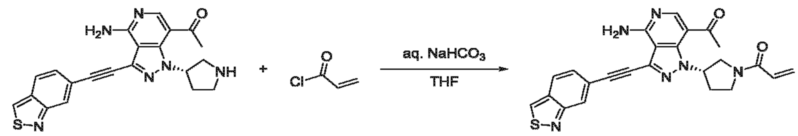


In an ice bath, a solution of acryloyl chloride (47 mg, 517 μmol, 41 μL) in THF (5 mL) was dropwise added to a mixture of (*S*)-1-(4-amino-3-(benzo[c]isothiazol-6-ylethynyl)-1-(pyrrolidin-3-yl)-1H-pyrazolo[4,3-c]pyridin-7-yl)ethanone hydrochloride (0.227 g, 517 μmol), a saturated sodium bicarbonate solution (2.90 g), and THF (40 mL) and stirred in the ice bath for 15 minutes. The reaction liquid was concentrated under reduced pressure to remove THF, the residue was diluted with DCM (20 mL), washed by adding water (10 mL), dried over anhydrous sodium sulfate, filtered, and concentrated, and the residue was separated and purified by silica gel column chromatography to obtain the title compound (74 mg, 30%).

MS *m/z* (ESI): 457.1 $[M+H]^+$.

$^1H$ NMR (400 MHz, DMSO-$d_6$) δ 9.86 (s, 1H), 8.59 (d, *J* = 2.3 Hz, 1H), 8.29-8.24 (m, 1H), 8.01 (d, *J* = 8.8 Hz, 1H), 7.48 (d, *J* = 8.9 Hz, 1H), 6.71-6.52 (m, 1H), 6.21-6.10 (m, 1H), 6.00-5.82 (m, 1H), 5.74-5.62 (m, 1H), 4.15-3.58 (m, 4H), 2.62 (s, 3H), 2.45-2.29 (m, 2H);

**Example 90**

(*S*)-1-(3-(4-amino-3-(benzo[c]isothiazole-6-ethynylene)-7-propionyl-1H-pyrazolo[4,3-c]pyridin-1-yl)pyrrolidine-1-methyl)prop-2-en-1-one

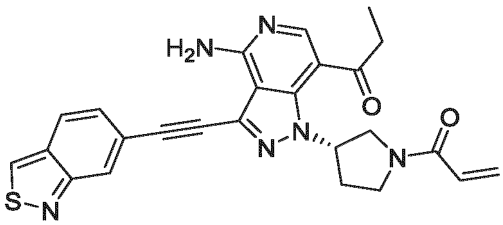

Preparation of (*S*)-1-(3-(4-amino-3-(benzo[c]isothiazole-6-ethynylene)-7-propionyl-1H-pyrazolo[4,3-c]pyridin-1-yl)pyrrolidine-1-methyl)prop-2-en-1-one according to Reference Example 89.

MS *m/z* (ESI): 471.2 $[M+H]^+$.

$^1H$ NMR (400 MHz, DMSO) δ 8.59 (d, *J* = 2.1 Hz, 1H), 8.27 (p, *J* = 1.2 Hz, 1H), 8.01 (dd, *J* = 9.0, 0.9 Hz, 1H), 7.48 (dd, *J* = 8.7, 1.4 Hz, 1H), 7.21 (s, 1H), 6.71-6.52 (m, 1H), 6.16 (dd, *J* = 16.8, 4.3, 1H), 5.88 (s, 1H), 5.82-5.63 (m, 2H), 4.09 (dd, *J* = 11.2, 6.8 Hz, 1H), 3.96 (dd, *J* = 11.2, 4.2 Hz, 1H), 3.90-3.73 (m, 1H), 3.12-3.02 (m, 2H), 2.45 (t, *J* = 6.8 Hz, 1H), 2.00 (q, *J* = 7.0 Hz, 2H), 1.46 (s, 1H), 1.18-1.10 (m,2H), 0.89-0.81 (m, 1H).

**Example 91**

(*S*)-1-(3-(4-amino-3-(benzo[c]isothiazole-6-ethynylene)-7-(cyclopropanecarbonyl)-1H-pyrazolo[4,3-c]pyridin-1-yl)pyrrolidine-1-methyl)prop-2-en-1-one

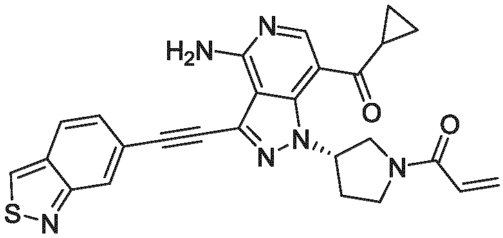

Preparation of (*S*)-1-(3-(4-amino-3-(benzo[c]isothiazole-6-ethynylene)-7-(cyclopropanecarbonyl)-1H-pyrazolo[4,3-c]pyridin-1-yl)pyrrolidine-1-methyl)prop-2-en-1-one according to Reference Example 89.
MS *m/z* (ESI): 483.2 $[M+H]^+$.
$^1H$ NMR (400 MHz, DMSO) $\delta$ 8.67 (d, *J* = 2.3 Hz, 1H), 8.34 (s, 1H), 8.20 (s, 1H), 7.95 (d, *J* = 8.8 Hz, 1H), 7.42 (dd, *J* = 8.9, 1.4 Hz, 1H), 7.15 (s, 1H), 6.54 (dd, 16.7, 10.3 Hz, 1H), 6.09 (dt, *J* = 16.8, 2.7 Hz, 1H), 5.70 (d, *J* = 8.0 Hz, 1H), 5.26 (t, *J* = 4.8 Hz, 1H), 3.99 (dd, *J* = 11.3, 6.6 Hz, 1H), 3.78-3.65 (m, 1H), 2.41-2.34 (m, 1H), 2.22 (d, *J* = 7.0 Hz, 1H), 1.93 (q, *J* = 7.4 Hz, 3H), 1.39 (s, 2H), 1.04-0.95 (m, 3H).

**Example 92**

(*S*)-1-(3-(7-acetyl-4-amino-3-((3-cyclopropylbenzo[c]isothiazol-6-yl)alkynyl)-1H-pyrazolo[4,3-c]pyridin-1-yl)pyrrolidine-1-methyl)prop-2-en-1-one

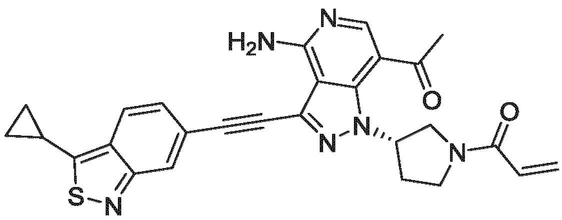

Preparation of (*S*)-1-(3-(7-acetyl-4-amino-3-((3-cyclopropylbenzo[c]isothiazol-6-yl)alkynyl)-1H-pyrazolo[4,3-c]pyridin-1-yl)pyrrolidine-1-methyl)prop-2-en-1-one according to Reference Example 89.
MS *m/z* (ESI): 497.2 $[M+H]^+$.

**Example 93**

(*S*)-1-(3-(7-acetyl-4-amino-3-(benzo[c][1,2,5]oxadiazole-5-ethynylene)-1H-pyrazolo[4,3-c]pyridin-1-yl)pyrrolidine-1-methyl)prop-2-en-1-one

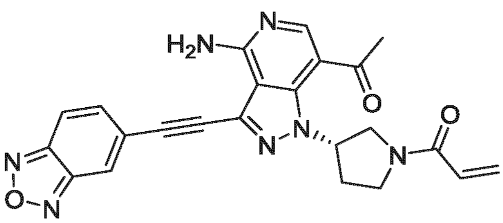

Preparation of (*S*)-1-(3-(7-acetyl-4-amino-3-(benzo[c][1,2,5]oxadiazole-5-ethynylene)-1H-pyrazolo[4,3-c]pyridin-1-yl)pyrrolidine-1-methyl)prop-2-en-1-one according to Reference Example 89.

MS *m*/*z* (ESI): 442.2 $[M+H]^+$.

$^1H$ NMR (400 MHz, DMSO-$d_6$) $\delta$ 8.64-8.60 (m, 1H), 8.60 (d, *J* = 2.3 Hz, 1H), 8.17 (dd, *J* = 9.3, 1.1 Hz, 1H), 7.81 (d, *J* = 9.3 Hz, 1H), 7.69-7.11 (m, 2H), 6.71-6.52 (m, 1H), 6.21-6.10 (m, 1H), 6.02-5.82 (m, 1H), 5.74-5.62 (m, 1H), 4.06-3.50 (m, 4H), 2.62 (s, 3H), 2.47-2.28 (m, 2H).

**Biological test and evaluation**

The present invention will be further described and explained below in conjunction with test examples, but these examples are not meant to limit the scope of the present invention.

I. Test enzymology experiment

Test example 1. Determination of inhibitory effects of compounds of the present invention on FGFR1-4 and FGFR2 V564F activities

1. Experimental purpose:

To measure the $IC_{50}$ values of the compounds against FGFR1, FGFR2, FGFR3, FGFR4, and FGFR2 V564F at ATP Km concentrations by TR-FRET method.

2. Experimental instruments and reagents:

2.1 Instruments:

| Name of instrument | Manufacturer | Specification and model |
|---|---|---|
| Centrifuge | Eppendorf | 5810R |
| Microcentrifuge | DALB SCIENTIFIC CO., LTD. | D1008 |
| Microplate reader | BioTek | H1MFD |

2.2 Reagents:

| Reagents and materials | Manufacturer | Item No. |
|---|---|---|
| FGFR1 | Carna | 08-133 |
| FGFR2 | Carna | 08-134 |
| FGFR3 | Carna | 08-135 |
| FGFR4 | Carna | 08-136 |
| FGFR2 V564F | SignalChem | F05-12FG-05 |
| ULigh™-JAK-1 (Tyr1023) Peptide | Perkinelmer | TRF0121-D |
| Europium-anti-phosphotyrosine (PT66) | PerkinElmer | AD0068 |

3. Experimental method:

TR-FRET (time-resolved fluorescence resonance energy transfer) method was used to detect the inhibitory activity of the compounds on FGFR1, FGFR2, FGFR3, FGFR4, and FGFR2 V564F kinase region fragments. The highest detection concentration of the compounds in 5 kinase experiments was 1000 nM, which was diluted in 3 folds, with a total of 11 concentrations (1000 nM to 0.017 nM). A kinase buffer (50 mM HEPES pH 7.5, 1 mM EGTA, 10 mM MgCl2, 2 mM DTT,

and 0.01% Tween-20) was used to prepare 4× enzyme solution, 4× compound solutions diluted in gradient, and a 2× ATP/Peptide substrate solution. To a 384-well plate, 2.5 μL of enzyme solution and 2.5 μL of diluted 5× compound solution were added, and 5 μL of 2× ATP/Peptide substrate solution was then added. After reacting at room temperature for 60 minutes for FGFR1, FGFR2, FGFR3, and FGFR2 V564F kinases, and 120 minutes for FGFR4 kinase, 10 μL of a 2× termination and detection mixed liquid prepared with 1× LANCE detection buffer with an EDTA concentration of 20 mM and a Europium-anti-phosphotyrosine (PT66) concentration of 2 nM was added to each well of the plate. After reacting at room temperature for 60 minutes for FGFR1, FGFR2, FGFR3, and FGFR4, and 30 minutes for FGFR2 V564F, the fluorescence signal value in each well of the plate was measured by a microplate reader.

Enzyme reaction system:

| Enzyme type | Reaction components | Volume | Final concentration | Reaction Time |
|---|---|---|---|---|
| FGFR1 | Compound | 2.5 μL | Starting with 1000 nM, 3-fold dilutions, 11 doses | 60 min |
| | Enzyme | 2.5 μL | 0.2 nM | |
| | Peptide/ATP substrate mixed liquid | 5 μL | Peptide final concentration: 20 nM<br>ATP final concentration: 30 | |
| | | | μM | |

| Enzyme type | Reaction components | Volume | Final concentration | Reaction Time |
|---|---|---|---|---|
| FGFR2 | Compound | 2.5 μL | Starting with 1000 nM, 3-fold dilutions, 11 doses | 60 min |
| | Enzyme | 2.5 μL | 0.2 nM | |
| | Peptide/ATP substrate mixed liquid | 5 μL | Peptide final concentration: 20 nM<br>ATP final concentration: 30 μM | |

| Enzyme type | Reaction components | Volume | Final concentration | Reaction Time |
|---|---|---|---|---|
| FGFR3 | Compound | 2.5 μL | Starting with 1000 nM, 3-fold dilutions, 11 doses | 60 min |
| | Enzyme | 2.5 μL | 0.2 nM | |
| | Peptide/ATP substrate mixed liquid | 5 μL | Peptide final concentration: 20 nM<br>ATP final concentration: 30 μM | |

| Enzyme type | Reaction components | Volume | Final concentration | Reaction Time |
|---|---|---|---|---|
| FGFR4 | Compound | 2.5 μL | Starting with 1000 nM, 3-fold dilutions, 11 doses | 120 min |
| | Enzyme | 2.5 μL | 1.25 nM | |
| | Peptide/ATP substrate mixed liquid | 5 μL | Peptide final concentration: 20 nM<br>ATP final concentration: 200 μM | |

| Enzyme type | Reaction components | Volume | Final concentration | Reaction Time |
|---|---|---|---|---|
| FGFR2 V564F | Compound | 2.5 μL | Starting with 1000 nM, 3-fold dilutions, 11 doses | 60 min |
| | Enzyme | 2.5 μL | 0.1 nM | |
| | Peptide/ATP substrate mixed liquid | 5 μL | Peptide final concentration: 20 nM<br>ATP final concentration: 20 μM | |

Enzyme reaction termination and detection mixed liquid system:

| Reaction components | Volume (μL) | Final concentration | Reaction time |
|---|---|---|---|
| 10X LANCE detection buffer | 10 μL | 1× | FGFR1, FGFR2, FGFR3, FGFR4: 60 min; FGFR2 V564F: 30 min |
| ddH2O | | - | |
| EDTA | | 10 mM | |
| Europium-anti-phosphotyrosine (PT66) | | 1nM | |

4. Experimental data processing method:

1) Inhibition rate (%): Calculation was performed on the raw data (665 nm/620 nm reading ratio) according to the following formula to obtain the inhibition rate. Inhibition rate % = [(mean value in positive control well - value in sample well) / (mean value in positive control well - mean value in negative control well)] × 100, wherein the positive control well was a reaction well without a compound and an enzyme, and the negative control well was a reaction wells without an enzyme.
2) Curve fitting: Log(inhibitor) vs. response - Variable slope (four parameters) in GraphPad Prism 6 was used for fitting equation analysis of the compound concentration and the corresponding inhibition rate, and the curve was fitted to obtain the $IC_{50}$ values of the compounds.

The fitting calculation equation was Y = Bottom + (Top - Bottom) / (1 + 10^((LogIC50-X) * HillSlope)).

5. Experimental results:

The $IC_{50}$ values of the compounds against the kinase activities of FGFR1-4 and FGFR2 V564F were as shown in the following tables:

Table 1

| Example No. | FGFR1 $IC_{50}$ (nM) | Example No. | FGFR1 $IC_{50}$ (nM) |
|---|---|---|---|
| 7 | 1.3 | 61 | 2.2 |
| 22 | 6.5 | 64 | 6.3 |
| 24 | 3.6 | 65 | 2.8 |
| 25 | 5.7 | 66 | 6.8 |
| 29 | 7.1 | 68 | 2.4 |
| 31 | 3.5 | 70 | 2.4 |
| 34 | 6.6 | 71 | 7.2 |
| 37 | 2.4 | 72 | 7.3 |

(continued)

| Example No. | FGFR1 $IC_{50}$ (nM) | Example No. | FGFR1 $IC_{50}$ (nM) |
|---|---|---|---|
| 38 | 2.8 | 73 | 2.3 |
| 39 | 6.1 | 79 | 4.5 |
| 44 | 4.4 | 82 | 1.4 |
| 46 | 5.2 | 83 | 1.7 |
| 49 | 3.9 | 84 | 1.0 |
| 54 | 5.5 | 85 | 2.1 |
| 55 | 4.4 | 87 | 1.6 |
| 57 | 5.6 | 89 | 2.7 |
| 58 | 3.0 | 91 | 3.9 |
| 60 | 4.7 | / | / |

Table 2

| Example No. | FGFR2 $IC_{50}$ (nM) | Example No. | FGFR2 $IC_{50}$ (nM) |
|---|---|---|---|
| 7 | 1.0 | 59 | 3.4 |
| 9 | 2.2 | 60 | 0.9 |
| 14 | 2.4 | 61 | 0.8 |
| 22 | 1.6 | 62 | 2.4 |
| 24 | 0.7 | 64 | 1.4 |
| 25 | 1.5 | 65 | 0.7 |
| 29 | 1.5 | 66 | 2.9 |
| 31 | 1.2 | 68 | 1.4 |
| 34 | 3.2 | 70 | 0.6 |
| 37 | 1.0 | 71 | 2.0 |
| 38 | 1.6 | 72 | 1.4 |
| 39 | 1.7 | 73 | 0.7 |
| 44 | 2.2 | 77 | 1.8 |
| 46 | 3.5 | 79 | 0.7 |
| 48 | 1.7 | 82 | 0.3 |
| 49 | 1.3 | 83 | 0.3 |
| 50 | 2.6 | 84 | 0.5 |
| 53 | 3.5 | 85 | 0.4 |
| 54 | 3.4 | 87 | 0.5 |
| 55 | 2.8 | 89 | 0.4 |
| 56 | 1.8 | 90 | 1.6 |
| 57 | 1.3 | 91 | 1.0 |
| 58 | 1.2 | A | 12.8 |

Table 3

| Example No. | FGFR3 $IC_{50}$ (nM) |
|---|---|
| 37 | 1.1 |

(continued)

| Example No. | FGFR3 $IC_{50}$ (nM) |
|---|---|
| 71 | 2.7 |
| 72 | 1.6 |
| 82 | 0.5 |

Table 4

| Example No. | FGFR2 V564F $IC_{50}$ (nM) | Example No. | FGFR2 V564F $IC_{50}$ (nM) |
|---|---|---|---|
| 7 | 0.3 | 56 | 0.7 |
| 9 | 1.0 | 57 | 0.5 |
| 14 | 1.3 | 58 | 0.5 |
| 20 | 2.8 | 59 | 1.1 |
| 22 | 0.8 | 60 | 0.4 |
| 24 | 0.4 | 61 | 0.4 |
| 25 | 0.6 | 62 | 1.2 |
| 29 | 3.6 | 64 | 0.6 |
| 31 | 0.8 | 65 | 0.5 |
| 34 | 2.2 | 66 | 0.8 |
| 37 | 0.5 | 67 | 1.6 |
| 38 | 1.0 | 68 | 0.3 |
| 39 | 0.6 | 70 | 1.3 |
| 40 | 1.9 | 71 | 2.8 |
| 42 | 3.9 | 72 | 2.1 |
| 43 | 1.1 | 73 | 0.9 |
| 44 | 0.4 | 77 | 1.4 |
| 45 | 0.7 | 79 | 0.4 |
| 46 | 1.1 | 82 | 0.3 |
| 47 | 1.0 | 83 | 0.3 |
| 48 | 0.8 | 84 | 0.3 |
| 49 | 1.0 | 85 | 0.5 |
| 50 | 1.0 | 87 | 0.6 |
| 51 | 3.3 | 89 | 0.7 |
| 52 | 1.8 | 90 | 3.1 |
| 53 | 1.9 | 91 | 1.1 |
| 54 | 0.9 | A | 6.1 |
| 55 | 1.0 | B | 18.4 |

6. Experimental conclusion:

The example compounds of the present invention had good inhibitory effects on both FGFR1-4 and FGFR2 V564F kinase activities.
Note: The structures of compounds A and B were as shown below.

| Compound | Structure | Pertinent patent |
|---|---|---|
| A | 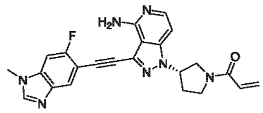 | WO 2021247971 |
| B | 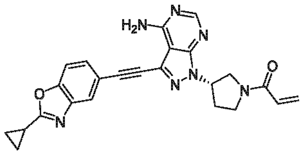 | WO 2022033532 |

**II. Cell activity experiment**

1. Experimental purpose:

To evaluate inhibitory effects of the compounds on the proliferation of NCI-H716, SNU-16 and BaF3-FGFR2-BICC1 V564F cells by cell proliferation inhibition experiments.

2. Experimental instruments and reagents:

2.1 Instruments:

| Name of instrument | Manufacturer | Specification and model |
|---|---|---|
| Full-function microplate reader | Bio Tek USA | H1MFD |
| Centrifuge | eppendorf | 5702R |
| Cell counter | Life | Countess II |
| $CO_2$ incubator | Thermo | 311 |
| Biosafety cabinet | Shanghai Boxun Industry Co., Ltd | BSC-1300IIA2 |

2.2 Reagents:

| Reagents and materials | Manufactu rer | Item No. |
|---|---|---|
| RPMI 1640 | GIBCO | 22400-089 |
| DMEM | GIBCO | 11995-065 |
| PBS | GIBCO | 10010-023 |
| FBS | GIBCO | 10099-141C |
| Trypsin (TE) | GIBCO | 25200-056 |
| CellTiter-Glo® Luminescent Cell Viability test kit (CTG) | Promega | G7573 |
| 96-well V-shaped Bottom Plate (Compoud plate) | Axygen | WIPP02280 |
| 96-well plate | Corning | 3610 |

2.3 Cell sources

| Cell lines | Source | Cell type | Culture conditions |
|---|---|---|---|
| NCI-H716 | ATCC | Human colon cancer cells (FGFR2 gene amplification and FGFR2-COL14A1 gene fusion) | 1640 containing 10% FBS |
| SNU-16 | ATCC | Human gastric cancer cells (FGFR2 gene amplification) | 1640 containing 10% FBS |
| BaF3 FGFR2-BICC1 V564F | Kyinno Biotechnology | Mouse pro-B cells over-expressing FGFR2-BICC1 fusion gene and having an amino acid mutation at position 564 of FGFR2 | 1640 containing 10% FBS |

3. Experimental method:

CellTiter-Glo method was used to detect inhibitory effects of the compounds on the proliferation of NCI-H716, SNU-16 and BaF3 FGFR2-BICC1 V564F cells. The highest detection concentration of the compounds was 1000 nM, which was diluted in 3 folds, with a total of 9 concentrations (1000 nM to 0.15 nM). After the cells were plated at an appropriate density, compounds were added the next day and incubated for 72 h before detection using the CellTiter-Glo Luminescebt detection kit.

4. Experimental data processing method:

1) Inhibition rate (%):

Inhibition rate % = [(mean value in positive control well - value in sample well) / (mean value in positive control well - mean value in negative control well)] × 100,

wherein the positive control well was a reaction well without a compound and an enzyme, and the negative control well was a reaction wells without an enzyme.

2) Curve fitting: Log(inhibitor) vs. response - Variable slope (four parameters) in GraphPad Prism 6 was used for fitting equation analysis of the compound concentration and the corresponding inhibition rate, and the curve was fitted to obtain the $IC_{50}$ values of the compounds.

The fitting calculation equation was Y = Bottom + (Top - Bottom) / (1 + 10^((Log$IC_{50}$-X) * HillSlope)).

5. Experimental results:

Table 5

| Example No. | NCI-H716 cell proliferation inhibition rate $IC_{50}$ (nM) | Example No. | NCI-H716 cell proliferation inhibition rate $IC_{50}$ (nM) |
|---|---|---|---|
| 7 | 1.26 | 68 | 1.42 |
| 8 | 1.74 | 70 | 1.60 |
| 9 | 1.82 | 71 | 1.50 |
| 18 | 1.82 | 72 | 1.97 |
| 24 | 2.09 | 77 | 0.92 |
| 37 | 0.95 | 79 | 1.20 |
| 38 | 0.91 | 82 | 0.21 |

(continued)

| Example No. | NCI-H716 cell proliferation inhibition rate $IC_{50}$ (nM) | Example No. | NCI-H716 cell proliferation inhibition rate $IC_{50}$ (nM) |
|---|---|---|---|
| 45 | 1.84 | 83 | 0.82 |
| 49 | 1.92 | 84 | 1.04 |
| 58 | 1.94 | 85 | 0.96 |
| 60 | 2.19 | 87 | 1.31 |
| 61 | 1.77 | 89 | 0.94 |
| 64 | 1.80 | 90 | 0.98 |
| 65 | 1.50 | 91 | 0.97 |

Table 6

| Examples | BaF3 FGFR2-BICC1 V564F inhibitory activity $IC_{50}$ (nM) | Examples | BaF3 FGFR2-BICC1 V564F inhibitory activity $IC_{50}$ (nM) |
|---|---|---|---|
| 7 | 0.9 | 60 | 1.7 |
| 8 | 0.4 | 61 | 1.0 |
| 9 | 1.6 | 64 | 1.9 |
| 24 | 0.8 | 65 | 1.4 |
| 31 | 1.4 | 66 | 2.8 |
| 34 | 2.9 | 67 | 1.6 |
| 37 | 2.0 | 68 | 0.5 |
| 38 | 1.9 | 70 | 2.6 |
| 39 | 2.2 | 71 | 2.2 |
| 44 | 0.8 | 72 | 1.5 |
| 45 | 0.6 | 77 | 2.9 |
| 46 | 0.2 | 79 | 1.1 |
| 47 | 0.5 | 82 | 0.5 |
| 48 | 1.4 | 83 | 0.7 |
| 49 | 0.4 | 84 | 0.1 |
| 50 | 2.1 | 85 | 0.4 |
| 53 | 4.9 | 87 | 0.3 |
| 54 | 2.2 | 89 | 1.6 |
| 55 | 2.2 | 90 | 3.2 |
| 56 | 3.5 | 91 | 0.8 |
| 57 | 2.5 | A | 7.4 |
| 58 | 0.4 | B | 11.5 |
| 59 | 4.0 | / | / |

6. Experimental conclusion:

The example compounds of the present invention had significant inhibitory effects on the proliferation of NCI-H716, SNU-16 and BaF3 FGFR2-BICC1 V564F cells.

**III. Pharmacokinetic experiment**

1. Experimental purpose:

To study the pharmacokinetic behavior of the compounds of the present invention in the rat body (plasma) after oral administration at a dose of 5 mg/kg using SD rats as test animals.

2. Experimental scheme

2.1 Test drugs:

the examples of the present invention, made in house.

2.2 Test animals:

3 SD rats/group, male, from Shanghai Jiesijie Experimental Animal Co., Ltd., animal production license number (SCXK (Shanghai) 2013-0006 N0.311620400001794).

2.3 Drug preparation:

PO, 10% solutol HS15-0.5% CMC-Na was dissolved by ultrasound and prepared into a clear solution or homogeneous suspension.

2.4 Administration scheme:

3 SD rats/group, male; p.o. respectively at a dose of 5 mg/kg and an administration volume of 10 mL/kg after the mice were fasted overnight.

2.5 Sample collection:

After oral administration to rats, at 0.25, 0.5, 1, 2, 4, 6, 8 and 24 hours, 0.2 mL of blood was collected from the jugular vein, placed in an EDTA-$K_2$ test tube, centrifuged at 6000 rpm for 6 min at 4°C to separate the plasma, which was stored at -80°C.

2.6 Sample treatment:

1) 100 uL of acetonitrile was added to 20 uL of plasma sample for pelleting, and it was mixed and centrifuged at 3500 × g for 5-20 min.
2) The treated supernatant solution was taken and subjected to LC/MS/MS to analyze the concentrations of the test compounds. LC/MS/MS analysis instrument: AB Sciex API 4000 Qtrap.

2.7 Liquid phase analysis:

• Liquid phase conditions: Shimadzu LC-20AD pump
• Chromatographic column: Waters Xbridge C18 5 μm, 4.6 × 50 mm mobile phase: liquid A was 0.1% aqueous formic acid solution, and liquid B was methanol
• Flow rate: 1.0 mL/min
• Elution time: 0-4.0 min, the eluent was as follows:

| Time/min | Liquid A | Liquid B |
|---|---|---|
| 0.2 | 90% | 10% |
| 1.2 | 2% | 98% |
| 3.0 | 2% | 98% |

| | | |
|---|---|---|
| 3.1 | 90% | 10% |
| 4.0 | Stop | |

2.8 Experimental results and data processing:

Table 7 Pharmacokinetic parameters

| Examples | Pharmacokinetic experiments (p.o., 5 mg/kg) | | | | | |
|---|---|---|---|---|---|---|
| | Time to peak | Plasma concentration | Area under the curve | Area under the curve | Half-life | Mean residence time |
| | $t_{max}$ (ng/mL) | $C_{max}$ (ng/mL) | $AUC_{0-t}$ (ng/mL×h) | $AUC_{0-\infty}$ (ng/mL×h) | $t_{1/2}$ (h) | MRT (h) |
| 38 | 2 | 604 | 2226 | 2273 | 1.1 | 2.99 |
| 39 | 2 | 1246 | 4189 | 4238 | 1.0 | 2.63 |
| 45 | 2 | 290 | 1442 | 1460 | 4.3 | 4.59 |
| 48 | 2 | 795 | 4116 | 4150 | 3.9 | 4.2 |
| 64 | 2 | 866 | 3395 | 3426 | 0.9 | 2.58 |
| 70 | 4 | 287 | 1513 | 1864 | 2.5 | 5.5 |
| 72 | 0.5 | 298 | 1149 | 1150 | 0.6 | 2.26 |
| 82 | 1 | 677 | 2795 | 2908 | 1.4 | 3.05 |
| 85 | 0.5 | 394 | 1490 | 1490 | 0.5 | 2.39 |
| 87 | 2 | 534 | 2243 | 2245 | 0.5 | 2.38 |

Experimental conclusion:

As could be seen from the results of the pharmacokinetic experiment on SD rats in the table, the example compounds of the present invention exhibited good metabolic properties and performed well on both exposure (AUC) and maximum blood drug concentration $C_{max}$. IV. *in vivo* pharmacodynamic study of compounds in subcutaneously transplanted tumor model of nude mice bearing mouse pro-B cell line Ba/F3 FGFR2-BICC-V564F

1.1 Experimental purpose

To evaluate the *in vivo* efficacy of the compounds in a subcutaneously transplanted tumor model of nude mice bearing mouse pro-B cell line $Ba/F_3$ FGFR2-BICC-V564F.

1.2 Experimental instruments and reagents

1.2.1 Instruments

1. Refrigerator (BCD-268TN, Haier)
2. Biosafety cabinet (BSC-1300II A2, Shanghai Boxun Industry & Commerce Co., Ltd. Medical Equipment Factory)
3. Clean bench (CJ-2F, Suzhou Fengshi Laboratory Animal Equipment Co., Ltd.)
4. Electronic pipette controller (Easypet 3, Eppendorf)
5. Thermostat water bath (HWS-12, Shanghai Yiheng Kexue Instruments Co., Ltd.)
6. $CO_2$ incubator (Thermo-311, Thermo)
7. Centrifuge (Centrifuge 5720R, Eppendorf)
8. Fully automated cell counter (Countess II, Life Technologies)
9. Vernier caliper (CD-6"AX, Mitutoyo, Japan)

10. Cell culture flask (T25/T75/T225, Corning)
11. Electronic scale (CPA2202S, Sartorius)
12. Electronic scale (BSA2202S-CW, Sartorius)
13. Ultrasonic cleaner (115F0032, Shanghai Kudos Ultrasonic Instrument Co., Ltd.)
14. Water purifier (Pacific TII, Thermo)
15. Magnetic stirrer (08-2G, Chijiu)

1.2.2 Reagents

1. RPMI-1640 medium (22400-089, Gibco)
2. Fetal bovine serum (FBS) (10099-141C, Gibco)
3. Phosphate buffer saline (PBS) (10010-023, Gibco)
4. Kolliphor HS15 (42966-1KG, Sigma-Aldrich)
5. Sodium carboxymethylcellulose (30036365, Sinopharm Chemical Reagent Co., Ltd.)

1.3 Experimental operations and data processing

1.3.1 Animals

BALB/c nude mice, 6-8 weeks old, ♀, purchased from Laboratory Animal Business Department, Shanghai Institute of Planned Parenthood Research

1.3.2 Cell culture and cell suspension preparation

a. Ba/$F_3$ FGFR2-BICC-V564F cells were taken from a cell bank, the cells were thawed with RPMI-1640 medium (RPMI-1640 + 10% FBS), and the thawed cells were placed in a cell culture flask (with the cell type, date, the name of the culture operator, etc., being marked on the wall of the flask), which were placed in a $CO_2$ incubator for culturing (wherein the temperature of the incubator was 37°C, and the $CO_2$ concentration was 5%).
b. Passage was carried out once every three days; and after passaging, the cells were placed in the $CO_2$ incubator, and culturing was continued. This process was repeated until the cell number met the pharmacodynamic requirements *in vivo.*
c. The cells in the exponential growth phase were collected, counted by a fully automated cell counter, resuspended to $3 \times 10^7$ cells/mL with PBS according to the counting results, and placed in an ice box for later use.

1.3.3 Cell inoculation

a. Before inoculation, the nude mice were marked with disposable universal ear tags for rats and mice.
b. During inoculation, the cell suspension was uniformly mixed, 0.1-1 mL of cell suspension was drawn by a 1 mL syringe, bubbles were eliminated, and the syringe was then placed on an ice pack for later use.
c. The nude mice were held with the left hand, the right back of the nude mice near the right shoulder (inoculation site) was disinfected with a 75% alcohol cotton ball, and after 30 seconds, inoculation was started.
d. The experimental nude mice were successively inoculated (0.1 mL of cell suspension was inoculated per mouse).

1.3.4 Tumor measurement, grouping and administration in tumor-bearing mice

a. According to the tumor growth, the tumor was measured on Days 10-14 after inoculation, and the tumor size was calculated.

$$\text{Calculation of tumor volume: tumor volume (mm}^3) = \text{length (mm)} \times \text{width (mm)} \times \text{width (mm)}/2$$

b. According to the weight of the tumor-bearing mice and the tumor size, the mice were grouped by a random grouping method.
c. According to the grouping results, the administration of the test drugs was started (administration method: oral administration; administration volume: 10 mL/kg; administration frequency: once/day; administration cycle: 14 days; vehicle: 10% Solutol HS15/0.5% CMC-Na).
d. The tumor was measured and the mice were weighed twice a week after the administration of the test drugs was started.
e. The animals were euthanized after the experiment was completed.
f. The data was processed by Excel and other softwares. Calculation of tumor growth inhibition rate TGI (%) of compounds: Where there was no tumor regression, TGI (%) = [1 - (average tumor volume at the end of administration in a treatment group - average tumor volume at the beginning of administration in the treatment group) / (average tumor volume at the end of treatment in the solvent control group - average tumor volume at the beginning of administration in the solvent control group)] × 100%. Where there was tumor regression, TGI (%) = [1 - (average tumor volume at the end of administration in a treatment group - average tumor volume at the beginning of administration in the treatment group) / average tumor volume at the beginning of administration in the treatment group] × 100%.

1.4 Experimental conclusion:

The compounds of the present invention had excellent effects on inhibiting tumors and good safety.

## Claims

**1.** A compound represented by general formula (I-1), or a stereoisomer or pharmaceutically acceptable salt thereof:

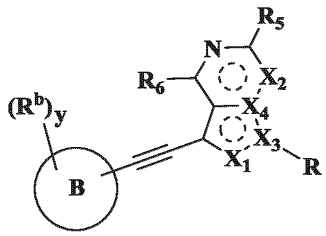

(I-1)

wherein $X_1$ is N or $CR_3$;
$X_2$ is N or $CR_4$; preferably, $X_2$ is $CR_4$;
$X_3$ is N or C;
$X_4$ is N or C;
$R_3$, $R_4$, $R_5$, and $R_6$ are each independently selected from hydrogen, deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more R‴; wherein the R ‴ is selected from deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5-to 14-membered heteroaryl, optionally substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;
preferably, $R_3$, $R_4$, $R_5$, and $R_6$ are each independently selected from hydrogen, deuterium, halogen, amino,

hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3-to 12-membered heterocyclyl-carbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5-to 14-membered heteroaryl;

$R^b$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ **haloalkoxy,** $C_{3-12}$ cycloalkyl, 3- **to** 12-membered heterocyclyl, $C_{6-14}$ aryl, and 5- to 14-membered heteroaryl;

alternatively, any two $R^b$ and adjacent atoms form $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

ring B is selected from $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

R is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, 3- to 12-membered heterocyclyl $C_{1-6}$ alkylene, $C_{3-12}$ cycloalkyl, 3- to 12-membered heterocyclyl, $C_{6-14}$ aryl, 5- to 14-membered heteroaryl, $-(CH_2)_{n2}NR_{a2}C(O)CR_{b2}=CR_{c2}$, $-O(CR_{a2}R_{b2})_{n2}R_{c2}$, $-S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}C(O)R_{c2}$, $-(CH_2)_{n2}S(O)_{m2}R_{c2}$, $-(CH_2)_{n2}NR_{a2}S(O)_{m2}R_{c2}$, or $-(CH_2)_{n2}S(O)_{m2}NR_{a2}R_{c2}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more R';

R' is selected from deuterium, halogen, amino, hydroxyl, cyano, mercapto, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, 5- to 14-membered heteroaryl, $-C(O)CR_a=CR_b(CH_2)_nR_c$, $-C(O)CR_a=CR_b(CH_2)_nNR_cR_d$, $-(CH_2)_nR_a$, $-(CR_aR_b)_nR_c$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, mercapto, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_{a2}$, $R_{b2}$, $R_{c2}$, and $R_{d2}$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to

14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl; n and n2 are 0, 1, 2, 3, 4, or 5; and

m and m2 are 0, 1, or 2;

y is 0, 1, 2, 3, 4, or 5.

**2.** The compound or the stereoisomer or pharmaceutically acceptable salt thereof according to claim 1, wherein the compound is further as represented by general formula (II-1):

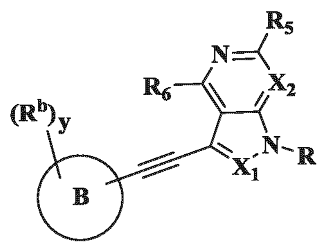

(II-1)

wherein $X_1$ is N or $CR_3$;

X2 is N or $CR_4$;

$R_3$, $R_4$, $R_5$, and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylamino-carbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;

preferably, $R_3$, $R_4$, $R_5$, and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R^b$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl,

$C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

ring B is selected from $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

R is selected from hydrogen, deuterium, halogen, amino, nitro, hydroxyl, cyano, alkyl, deuteroalkyl, haloalkyl, hydroxyalkyl, alkoxy, haloalkoxy, heterocyclylalkylene, alkenyl, alkynyl, cycloalkyl, heterocyclyl, aryl, heteroaryl, $-(CH_2)_nNR_{a2}C(O)CR_{b2}=CR_{c2}$, $-O(CR_{a2}R_{b2})_{n2}R_{c2}$, $-S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}C(O)R_{c2}$, $-(CH_2)_{n2}S(O)_{m2}R_{c2}$, $-(CH_2)_{n2}NR_{a2}S(O)_{m2}R_{c2}$, or $-(CH_2)_{n2}S(O)_{m2}NR_{a2}R_{c2}$, wherein the amino, alkyl, deuteroalkyl, haloalkyl, hydroxyalkyl, alkoxy, haloalkoxy, alkenyl, alkynyl, cycloalkyl, heterocyclyl, aryl, and heteroaryl can be optionally further substituted;

$R_{a2}$, $R_{b2}$, and $R_{c2}$ are each independently selected from hydrogen, deuterium, halogen, amino, nitro, hydroxyl, cyano, alkyl, deuteroalkyl, haloalkyl, hydroxyalkyl, alkoxy, haloalkoxy, alkenyl, alkynyl, cycloalkyl, heterocyclyl, aryl, or heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl, and heteroaryl can be optionally further substituted;

alternatively, any two of $R_{a2}$, $R_{b2}$, and $R_{c2}$ and adjacent atoms form cycloalkyl, heterocyclyl, aryl, or heteroaryl, wherein the cycloalkyl, heterocyclyl, aryl, and heteroaryl can be optionally further substituted;

m1 and n2 are each independently 0, 1, 2, 3, 4, or 5;

y is 0, 1, 2, 3, 4, or 5;

preferably provided that when ring B is

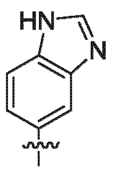 , 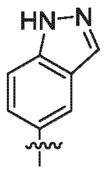 , 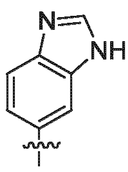 , 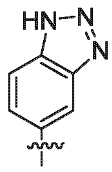 , 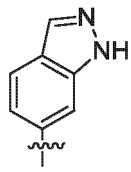 ,

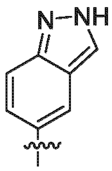 , 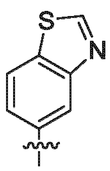 , 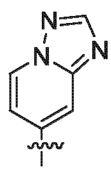 , 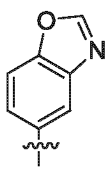 , or 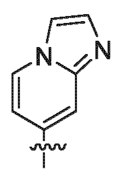 ,

$R_4$ is not hydrogen.

**3.** The compound or the stereoisomer or pharmaceutically acceptable salt thereof according to either claim 1 or 2, wherein

R is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, 3- to 12-membered heterocyclyl $C_{1-6}$ alkylene, $C_{3-12}$ cycloalkyl, 3- to 12-membered heterocyclyl, $C_{6-14}$ aryl, 5- to 14-membered heteroaryl, $-(CH_2)_{n2}NR_{a2}C(O)CR_{b2}=CR_{c2}$, $-O(CR_{a2}R_{b2})_{n2}R_{c2}$, $-S(CR_{a2}R_{b2})_{n2}R_{c2}$, $-(CR_{a2}R_{b2})_{n2}NR_{c2}R_{d2}$, $-(CH_2)_{n2}C(O)NR_{a2}R_{c2}$, $-(CH_2)_{n2}NR_{a2}C(O)R_{c2}$, $-(CH_2)_{n2}S(O)_{m2}R_{c2}$, $-(CH_2)_{n2}NR_{a2}S(O)_{m2}R_{c2}$, or $-(CH_2)_{n2}S(O)_{m2}NR_{a2}R_{c2}$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more R';

preferably, R is selected from 3- to 8-membered heterocyclyl $C_{1-6}$ alkylene, 3- to 8-membered heterocyclyl, or $-(CH_2)_{n2}NR_{a2}C(O)CR_{b2}=CR_{c2}$, wherein the 3- to 8-membered heterocyclyl $C_{1-6}$ alkylene, and 3- to 8-membered heterocyclyl are optionally further substituted with one or more R';

R' is selected from deuterium, halogen, amino, hydroxyl, cyano, mercapto, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, 5- to 14-membered heteroaryl, $-C(O)CR_a=CR_b(CH_2)_nR_c$, $-C(O)CR_a=CR_b(CH_2)_nNR_cR_d$, $-(CH_2)_nR_a$, $-(CR_aR_b)_nR_c$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, mercapto, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl; $R_{a2}$, $R_{b2}$, $R_{c2}$, and $R_{d2}$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;
$R_a$, $R_b$, $R_c$, and $R_d$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the amino, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, oxo, thio, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl; n and n2 are 0, 1, 2, 3, 4, or 5; and
m and m2 are 0, 1, or 2.

4. The compound or the stereoisomer or pharmaceutically acceptable salt thereof according to claim 1, wherein the compound is further as represented by general formula (I-2):

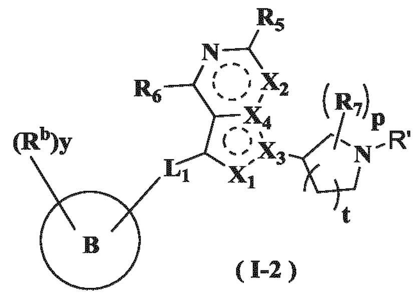

(I-2)

wherein $X_1$ is N or $CR_3$;
$X_2$ is N or $CR_4$; preferably, $X_2$ is $CR_4$;
$X_3$ is N or C;
$X_4$ is N or C;
$R_3$, $R_4$, $R_5$, and $R_6$ are each independently selected from hydrogen, deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more R'''; wherein the R‴ is selected from deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5-to 14-membered heteroaryl, optionally substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;

ring B is selected from $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R^b$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

alternatively, any two $R^b$ and adjacent atoms form $C_{3-12}$ cycloalkyl, 3- to 12-membered heterocyclyl, $C_{6-14}$ aryl, or 5- to 14-membered heteroaryl, wherein the $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_7$ is independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3-to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

R' is selected from deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, 5- to 14-membered heteroaryl, $-C(O)CR_a=CR_b(CH_2)_nR_c$, $-C(O)CR_a=CR_b(CH_2)_nNR_cR_d$, $-(CH_2)_nR_a$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to

14-membered heteroaryl;

$L_1$ is selected from a bond, $C_{1-3}$ alkylene, $C_{2-3}$ alkenylene, $C_{2-6}$ alkynylene, $C_{3-8}$ cycloalkylene, 3- to 8-membered heterocyclylene, -(C=C)-, $-(CR_{a1}=CR_{b1})_{n1}-$, $-O(CR_{a1}R_{b1})_{n1}-$ $-(CR_{a1}R_{b1})_{n1}(CO)-$, - $(CR_{a1}R_{b1})_{n1}NR_{c1}-$, or $-(CH_2)_{n1}C(O)NR_{a1}-$, wherein the $C_{1-3}$ alkylene, $C_{2-3}$ alkenylene, $C_{2-6}$ alkynylene, $C_{3-8}$ cycloalkylene, and 3- to 8-membered heterocyclylene can be optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-8}$ cycloalkyl, 3- to 8-membered heterocyclyl, $C_{6-10}$ aryl, and 5- to 10-membered heteroaryl;

$R_{a1}$, $R_{b1}$, and $R_{c1}$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

n1 is 0, 1, 2, 3, 4, or 5;

y is 0, 1, 2, 3, 4, or 5;

p is 0, 1, 2, 3, 4, or 5;

n is 0, 1, 2, 3, 4, or 5;

m is 0, 1, or 2; and

t is 0, 1, 2, 3, or 4;

provided that when ring B is

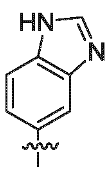 , 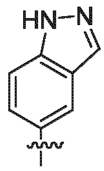 , 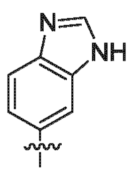 , 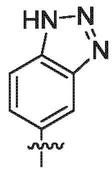 , 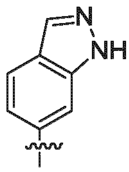 , 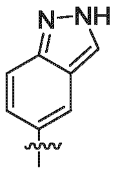 ,

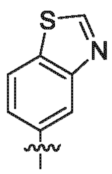 , 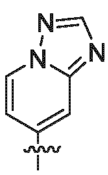 , 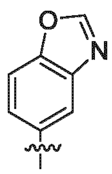 , or 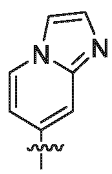 ,

$R_4$ is not hydrogen.

**5.** The compound or the stereoisomer or pharmaceutically acceptable salt thereof according to claim 1, wherein the compound is further as represented by general formula (IV):

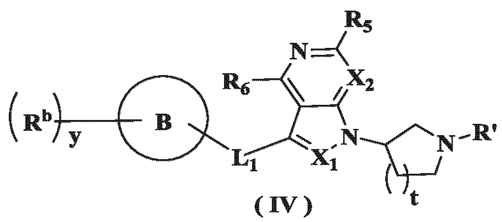

(IV)

wherein:

$L_1$ is -(C≡C)-;

wherein $X_1$ is N or $CR_3$;

$X_2$ is N or $CR_4$;

$R_3$, $R_4$, $R_5$, and $R_6$ are each independently selected from hydrogen, deuterium, halogen, amino, hydroxyl, cyano,

$C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylamino-carbonyl, 3- to 12-membered heterocyclyl-carbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl;

preferably, $R_3$ and $R_4$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3-to 12-membered heterocyclyl, $C_{6-14}$ aryl,

or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

preferably, $R_5$ and $R_6$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3-to 12-membered heterocyclyl, $C_{6-14}$ aryl,

or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

R' is selected from deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, 5- to 14-membered heteroaryl, $-C(O)CR_a=CR_b(CH_2)_nR_c$, $-C(O)CR_a=CR_b(CH_2)_nNR_cR_d$, $-(CH_2)_nR_a$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, or 5- to 14-membered heteroaryl, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

n is 0, 1, 2, 3, 4, or 5;

m is 0, 1, or 2; and

t is 0, 1, 2, 3, or 4.

**6.** The compound or the stereoisomer or pharmaceutically acceptable salt thereof according to any one of claims 1-5, wherein

ring B is selected from monocyclic aryl, 5- to 6-membered monocyclic heteroaryl, $C_{8-10}$ bicyclic aryl, or 8- to 10-membered bicyclic heteroaryl;

more preferably, phenyl, pyridyl, benzo 5- to 6-membered heterocyclyl, benzo 5- to 6-membered heteroaromatic ring group, pyrido 5- to 6-membered heterocyclyl, or pyrido 5- to 6-membered heteroaromatic ring group;

further preferably,

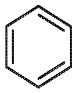, 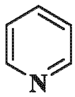, 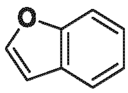, 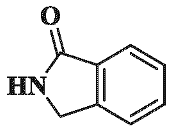, 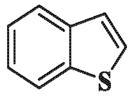, 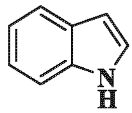,

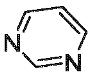, 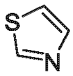, 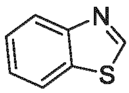, 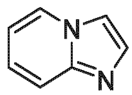, 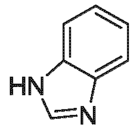, 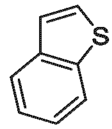, 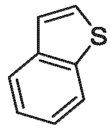, 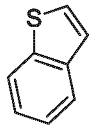, 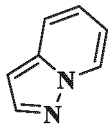,

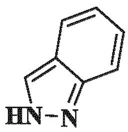, 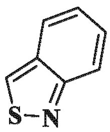, 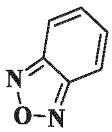, 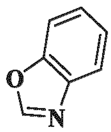, or 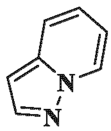;

more further preferably,

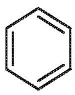, 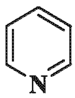, 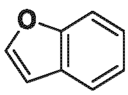, 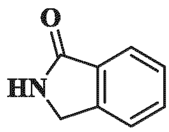, 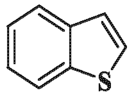, 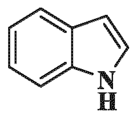,

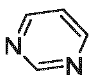, 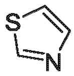, 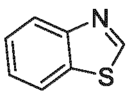, 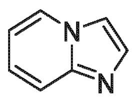, 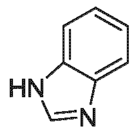, 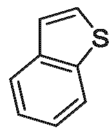, 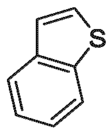, or 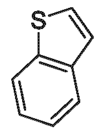

**7.** The compound or the stereoisomer or pharmaceutically acceptable salt thereof according to any one of claims 1-6, wherein

$R_3$, $R_4$, $R_5$, and $R_6$ are each independently selected from hydrogen, deuterium, halogen, amino, hydroxyl, cyano, aldehyde group, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{1-4}$ alkylcarbonyl, aminocarbonyl, $C_{1-4}$ alkylaminocarbonyl, $C_{1-4}$ alkylcarbonylamino, 3- to 8-membered cycloalkyl-carbonyl, 3- to 8-membered heterocyclyl-carbonyl, $C_{1-4}$ alkylamino, amino $C_{1-4}$ alkyl, $C_{3-8}$ cycloalkyl, 3- to 8-membered heterocyclyl, $C_{6-10}$ aryl, or 5- to 10-membered heteroaryl, wherein the amino, aldehyde group, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{1-4}$ alkylcarbonyl, aminocarbonyl, $C_{1-4}$ alkylaminocarbonyl, $C_{1-4}$ alkylcarbonylamino, 3- to 8-membered heterocyclyl-carbonyl, $C_{1-4}$ alkylamino, amino $C_{1-4}$ alkyl, $C_{3-8}$ cycloalkyl, 3- to 8-membered heterocyclyl, $C_{6-10}$ aryl, and 5- to 10-membered heteroaryl are optionally further substituted with one or more R‴; wherein the R‴ is selected from deuterium, halogen, amino, $C_{1-4}$ alkyl-substituted amino, hydroxyl, cyano, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{3-8}$ cycloalkyl, 3- to 8-membered heterocyclyl, $C_{1-4}$ alkyl-substituted 3-to 8-membered heterocyclyl, $C_{6-10}$ aryl, and 5- to 10-membered heteroaryl, optionally substituted with one or more substituents of deuterium, halogen, amino, $C_{1-4}$ alkyl-substituted amino, hydroxyl, cyano, $C_{1-4}$ alkyl, $C_{2-4}$ alkenyl, $C_{2-4}$ alkynyl, $C_{1-4}$ deuteroalkyl, $C_{1-4}$ haloalkyl, $C_{1-4}$ hydroxyalkyl, $C_{1-4}$ alkoxy, $C_{1-4}$ alkylthio, $C_{1-4}$ haloalkoxy, $C_{3-8}$ cycloalkyl, 3- to 8-membered heterocyclyl, $C_{1-4}$ alkyl-substituted 3- to 8-membered heterocyclyl, $C_{6-10}$ aryl, and 5- to 10-membered heteroaryl;

preferably, $R_3$, $R_4$, $R_5$, and $R_6$ are each independently selected from hydrogen, deuterium, halogen, amino,

hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 3-to 8-membered heterocyclyl-carbonyl, $C_{1-3}$ alkylamino, $C_{3-6}$ cycloalkyl, 3- to 8-membered heterocyclyl, $C_{6-10}$ aryl, or 5- to 10-membered heteroaryl, wherein the $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 3- to 8-membered heterocyclyl-carbonyl, $C_{1-3}$ alkylamino, $C_{3-6}$ cycloalkyl, 3-to 8-membered heterocyclyl, $C_{6-10}$ aryl, and 5- to 10-membered heteroaryl are optionally further substituted with one or more substituents of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 8-membered heterocyclyl, $C_{1-3}$ alkyl-substituted 3- to 8-membered heterocyclyl, $C_{6-10}$ aryl, and 5- to 10-membered heteroaryl;

more preferably, $R_3$, $R_4$, $R_5$, and $R_6$ are each independently selected from hydrogen, deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 4- to 6-membered heterocyclyl-carbonyl containing 1-3 atoms selected from N, O, or S, $C_{1-3}$ alkylamino, $C_{3-6}$ cycloalkyl, 4- to 6-membered heterocyclyl containing 1-3 atoms selected from N, O, or S, $C_{6-10}$ aryl, or 5- to 6-membered heteroaryl containing 1-3 atoms selected from N, O, or S, wherein the $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, 4- to 6-membered heterocyclyl-carbonyl containing 1-3 atoms selected from N, O, or S, $C_{1-3}$ alkylamino, $C_{3-6}$ cycloalkyl, 4- to 6-membered heterocyclyl containing 1-3 atoms selected from N, O, or S, $C_{6-10}$ aryl, and 5- to 6-membered heteroaryl containing 1-3 atoms selected from N, O, or S are optionally further substituted with one or more substituents of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 8-membered heterocyclyl, $C_{1-3}$ alkyl-substituted 3- to 8-membered heterocyclyl, $C_{6-10}$ aryl, and 5- to 10-membered heteroaryl; further preferably, $R_3$, $R_4$, $R_5$, and $R_6$ are each independently selected from hydrogen, deuterium, halogen, amino, $C_{1-3}$ alkyl, $C_{1-3}$ alkoxy, $C_{1-3}$ haloalkyl, $C_{3-6}$ cycloalkyl, cyano, $C_{1-3}$ deuteroalkyl, $C_{1-3}$ haloalkoxy, $C_{1-3}$ haloalkyl, aminocarbonyl, or $C_{1-3}$ alkylamino, wherein the amino, $C_{1-3}$ alkyl, $C_{1-3}$ deuteroalkyl, $C_{1-3}$ haloalkyl, $C_{1-3}$ alkoxy, $C_{1-3}$ haloalkoxy, aminocarbonyl, $C_{1-3}$ alkylamino, $C_{1-3}$ haloalkyl, $C_{1-3}$ alkylamino, and $C_{3-6}$ cycloalkyl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl.

**8.** The compound or the stereoisomer or pharmaceutically acceptable salt thereof according to any one of claims 1, 2, 6, and 7, wherein

the R is

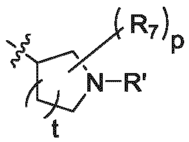

;

preferably, the R is

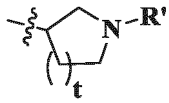

,

wherein $R_7$, R', and t are as defined in either claim 4 or 5;

preferably, R' is selected from deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, 5- to 6-membered heteroaryl, $-C(O)CR_a=CR_b(CH_2)_nR_c$, $-C(O)CR_a=CR_b(CH_2)_nNR_cR_d$, $-C(O)C{\equiv}C(CH_2)_nR_c$, $-(CH_2)_nR_a$, $-(CH_2)_nNR_aOR_b$, $-(CH_2)_nC(O)NR_aR_b$, $-(CH_2)_nNR_aC(O)R_b$, $-(CH_2)_nS(O)_mR_a$, $-(CH_2)_nS(O)_mNR_aR_b$, $-(CH_2)_nNR_aS(O)_mR_b$, $-(CH_2)_nNR_aR_b$, or $-(CH_2)_nNR_a(CH_2)_{n2}R_b$, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$

alkylthio, $C_{1-6}$ haloalkoxy, $C_{3-12}$ cycloalkyl, 3- to 12-membered heterocyclyl, $C_{6-14}$ aryl, and 5- to 14-membered heteroaryl are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{6-14}$ aryl, and 5- to 14-membered heteroaryl;

$R_a$, $R_b$, $R_c$, and $R_d$ are each independently selected from hydrogen, deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, or 5- to 6-membered heteroaryl, wherein the $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl are optionally further substituted with one or more substituents of deuterium, halogen, amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 6-membered heteroaryl;

n is 0, 1, 2, 3, 4, or 5;

m is 0, 1, or 2; and

t is 0, 1, 2, 3, or 4;

preferably, $R_7$ is independently selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, $C_{3-6}$ cycloalkyl, 3- to 6-membered heterocyclyl, $C_{6-10}$ aryl, or 5- to 10-membered heteroaryl, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, $C_{3-6}$ cycloalkyl, 3- to 6-membered heterocyclyl, $C_{6-10}$ aryl, and 5- to 10-membered heteroaryl are optionally further substituted with one or more substituents of deuterium, halogen, amino, hydroxyl, cyano, $C_{1-3}$ alkyl, $C_{2-4}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{3-6}$ cycloalkyl, 3- to 6-membered heterocyclyl, $C_{6-10}$ aryl, and 5- to 10-membered heteroaryl;

more preferably, the R is

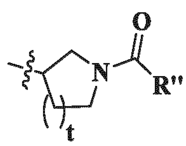

,

wherein R" is $-CR_a=CR_b(CH_2)_nR_c$, $-C\equiv C(CH_2)_nR_c$, or $-CR_a=CR_b(CH_2)_nNR_cR_d$;

further preferably, R" is selected from

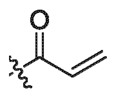
,
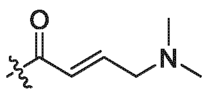
,
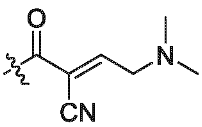
,

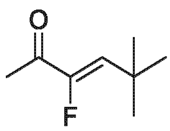
,
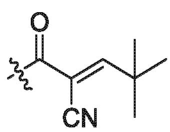
,
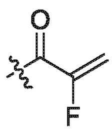
,
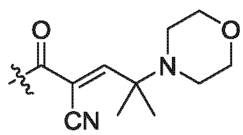
, and
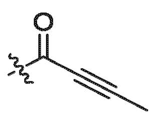
;

t is 0 or 1;

more further preferably, R is

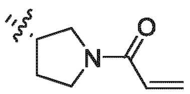
,
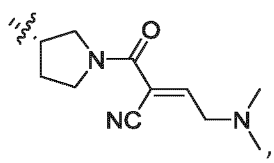
,
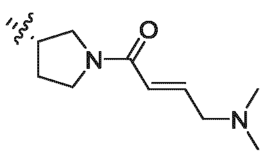
,

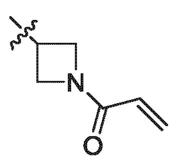, 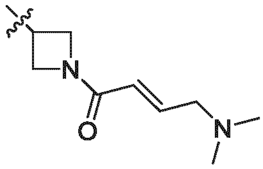, 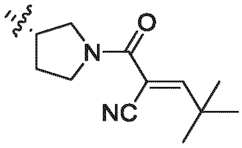, 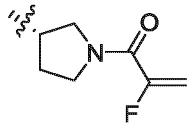,

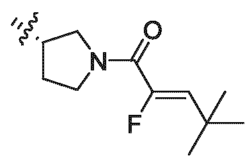, 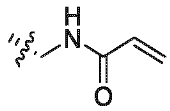, 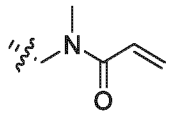, 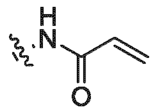, 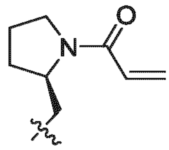,

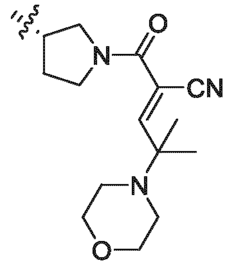, 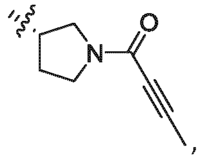, 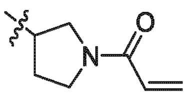, 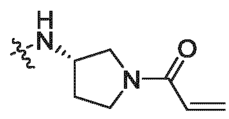, or 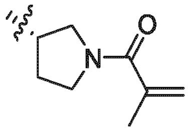

**9.** The compound or the stereoisomer or pharmaceutically acceptable salt thereof according to claim 4, wherein the compound is further as represented by general formula (II-3):

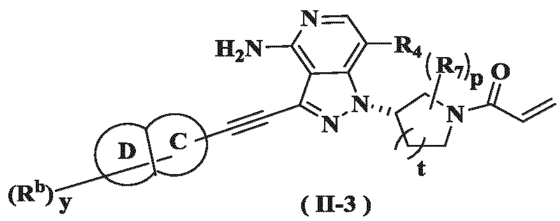

(II-3)

wherein

ring C is phenyl or 6-membered heteroaryl;

ring D is five-membered heteroaryl;

$R_4$ is selected from deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl; preferably, $R_4$ is selected from deuterium, halogen, amino, hydroxyl, cyano, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, or 5- to 10-membered heteroaryl, wherein the amino, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl,

aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, and 5- to 10-membered heteroaryl are optionally further substituted with one or more of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, and $C_{3-6}$ cycloalkyl;

more preferably, $R_4$ is selected from aldehyde group, $C_{1-3}$ alkylcarbonyl, $C_{1-3}$ alkyl, $C_{3-6}$ cycloalkyl, halogen, 5- to 6-membered heteroaryl containing 1-3 atoms selected from N, O, or S, or $C_{1-3}$ alkyl-substituted 5- to 6-membered heteroaryl containing 1-3 atoms selected from N, O, or S, wherein optionally, the aldehyde group is further substituted with one or more of $C_{1-6}$ alkyl and $C_{3-6}$ cycloalkyl; more further preferably, $R_4$ is selected from methyl, ethyl, cyclopropyl, chlorine, methylcarbonyl, isopropylcarbonyl, cyclobutylcarbonyl, ethylcarbonyl, cyclopropyl-carbonyl, tertbutylcarbonyl,

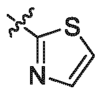, 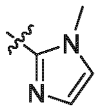, 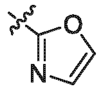, 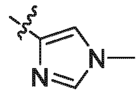, 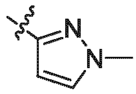, 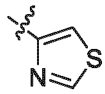, 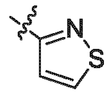,

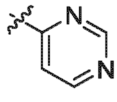, or 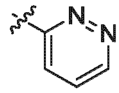;

$R_7$ is independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, and $C_{1-6}$ alkylamino are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, and $C_{1-6}$ haloalkoxy; preferably, $R_7$ is independently selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{1-3}$ alkylamino, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, and $C_{1-3}$ alkylamino are optionally further substituted with one or more substituents of deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, and $C_{1-3}$ haloalkoxy; more preferably, $R_7$ is independently selected from hydrogen, $-CH_2OCH_3$, or $-CHF_2$;

$R^b$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl;

p is 0, 1, 2, 3, 4, or 5;

t is 0, 1, 2, or 3.

**10.** The compound or the stereoisomer or pharmaceutically acceptable salt thereof according to claim 1, wherein the compound is further as represented by general formula (VII-1), (VII-2), (VII-3), (VII-4), (VII-5), (VII-6), or (VII-7):

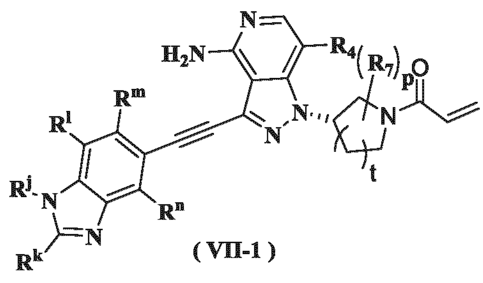

( VII-1 )

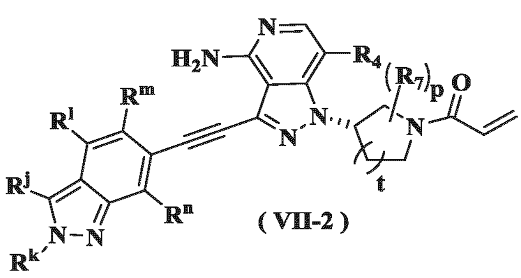

( VII-2 )

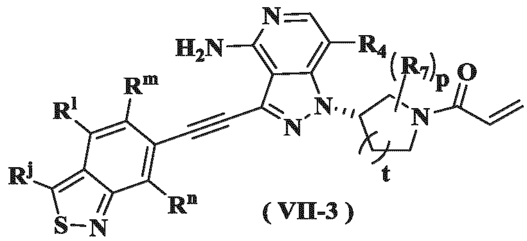

( VII-3 )

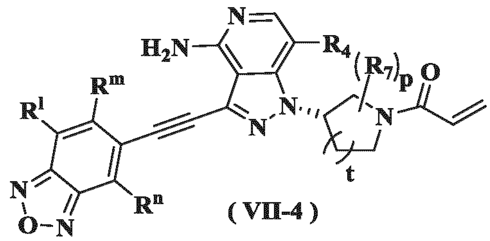

( VII-4 )

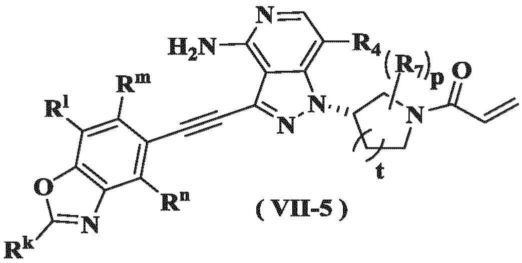

( VII-5 )

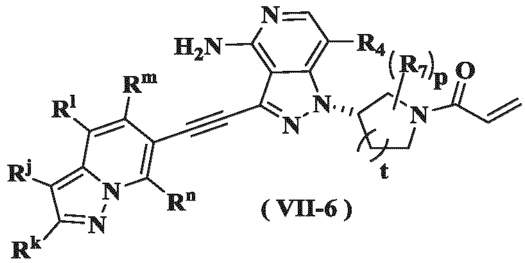

( VII-6 )

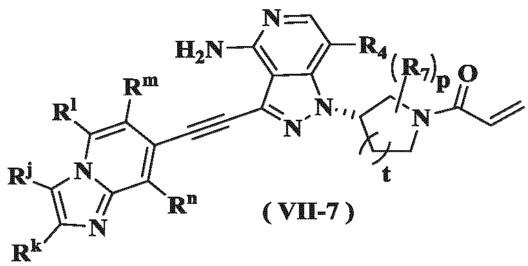

( VII-7 )

wherein

$R_4$ is selected from deuterium, halogen, amino, hydroxyl, cyano, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, or 5- to 14-membered heteroaryl, wherein the amino, aldehyde group, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylcarbonylamino, 3- to 12-membered cycloalkyl-carbonyl, 3- to 12-membered heterocyclyl-carbonyl, $C_{1-6}$ alkylamino, amino $C_{1-6}$ alkyl, $C_{3-12}$ cycloalkyl, 3- to 12-membered heterocyclyl, $C_{6-14}$ aryl, and 5- to 14-membered heteroaryl are optionally further substituted with one or more of deuterium, halogen, amino, $C_{1-6}$ alkyl-substituted amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3- to 12-membered heterocyclyl, $C_{1-6}$ alkyl-substituted 3- to 12-membered heterocyclyl, $C_{6-14}$ aryl, and 5- to 14-membered heteroaryl; preferably, $R_4$ is selected from deuterium, halogen, amino, hydroxyl, cyano, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, or 5- to 10-membered heteroaryl, wherein the amino, aldehyde group, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylcarbonylamino, 3- to 6-membered cycloalkyl-carbonyl, 3- to 6-membered heterocyclyl-carbonyl, $C_{1-3}$ alkylamino, amino $C_{1-3}$ alkyl, $C_{3-6}$ cycloalkyl, 3- to 6-membered heterocyclyl, $C_{6-10}$ aryl, and 5- to 10-membered heteroaryl are optionally further substituted with one or more of deuterium, halogen, amino, $C_{1-3}$ alkyl-substituted amino, hydroxyl, cyano, $C_{1-3}$ alkyl, $C_{2-3}$ alkenyl, $C_{2-3}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, and $C_{3-6}$ cycloalkyl;

more preferably, $R_4$ is selected from aldehyde group, $C_{1-3}$ alkylcarbonyl, $C_{1-3}$ alkyl, $C_{3-6}$ cycloalkyl, halogen, 5- to 6-membered heteroaryl containing 1-3 atoms selected from N, O, or S, or $C_{1-3}$ alkyl-substituted 5- to 6-membered heteroaryl containing 1-3 atoms selected from N, O, or S, wherein optionally, the aldehyde group is further

substituted with one or more of $C_{1-6}$ alkyl and $C_{3-6}$ cycloalkyl; more further preferably, $R_4$ is selected from $C_{1-3}$ alkylcarbonyl, $C_{1-3}$ alkyl, $C_{3-6}$ cycloalkyl, halogen, 5- to 6-membered heteroaryl containing 1-3 atoms selected from N, O, or S, or $C_{1-3}$ alkyl-substituted 5- to 6-membered heteroaryl containing 1-3 atoms selected from N, O, or S; still more further preferably, $R_4$ is selected from methyl, ethyl, cyclopropyl, chlorine, methylcarbonyl, isopropylcarbonyl, cyclobutylcarbonyl, ethylcarbonyl, cyclopropylcarbonyl, tertbutylcarbonyl,

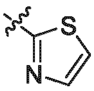, 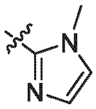, 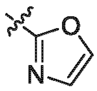, 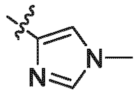, 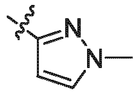, 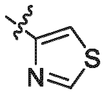, 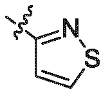,

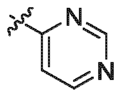, or 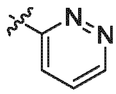;

or still more further preferably, $R_4$ is selected from methyl, ethyl, cyclopropyl, chlorine, methylcarbonyl,

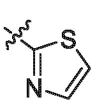, 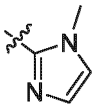, 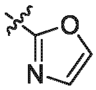, 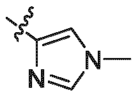, 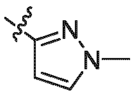,

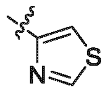, 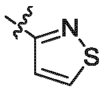, 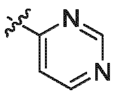, or 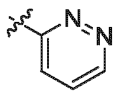;

$R_7$ is independently selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino, wherein the $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, and $C_{1-6}$ alkylamino are optionally further substituted with one or more substituents of deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, and $C_{1-6}$ haloalkoxy; preferably, $R_7$ is independently selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{1-3}$ alkylamino, wherein the $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, and $C_{1-3}$ alkylamino are optionally further substituted with one or more substituents of deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, and $C_{1-3}$ haloalkoxy; more preferably, $R_7$ is independently selected from hydrogen, $-CH_2OCH_3$, or $-CHF_2$;

$R^j$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl; preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, or $C_{3-6}$ cycloalkyl; further preferably selected from hydrogen, deuterium, halogen, $C_{1-3}$ alkyl, $C_{1-3}$ deuteroalkyl, or $C_{3-6}$ cycloalkyl; more further preferably selected from hydrogen, chlorine, methyl, ethyl, deuteromethyl, or cyclopropyl; still more further preferably selected from methyl, ethyl, deuteromethyl, or cyclopropyl;

$R^m$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino;

preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{1-3}$ alkylamino; further preferably selected from hydrogen, deuterium, or halogen; more further preferably fluorine;

$R^n$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, or $C_{1-6}$ alkylamino;
preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, or $C_{1-3}$ alkylamino; further preferably selected from hydrogen, deuterium, or halogen; more further preferably fluorine;
$R^k$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl; preferably selected from hydrogen, deuterium, halogen, amino, hydroxyl, cyano, $C_{1-3}$ alkyl, $C_{2-4}$ alkenyl, $C_{2-4}$ alkynyl, $C_{1-3}$ deuteroalkyl, $C_{1-3}$ haloalkyl, $C_{1-3}$ hydroxyalkyl, $C_{1-3}$ alkoxy, $C_{1-3}$ alkylthio, $C_{1-3}$ haloalkoxy, $C_{1-3}$ alkylcarbonyl, aminocarbonyl, $C_{1-3}$ alkylaminocarbonyl, $C_{1-3}$ alkylamino, or $C_{3-6}$ cycloalkyl; further preferably selected from hydrogen, deuterium, halogen, $C_{1-3}$ alkyl, $C_{1-3}$ deuteroalkyl, or $C_{3-6}$ cycloalkyl; more further preferably hydrogen, methyl, ethyl, or cyclopropyl; still more further preferably cyclopropyl;
$R^l$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuteroalkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{1-6}$ alkylcarbonyl, aminocarbonyl, $C_{1-6}$ alkylaminocarbonyl, $C_{1-6}$ alkylamino, or $C_{3-12}$ cycloalkyl; preferably hydrogen;
p is 0, 1, 2, 3, 4, or 5;
t is 0, 1, 2, or 3.

**11.** The compound or the stereoisomer or pharmaceutically acceptable salt thereof according to claim 1, wherein the structure of the specific compound is as follows:

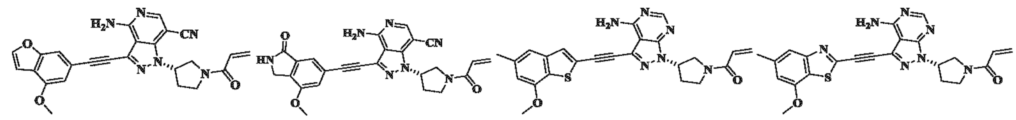
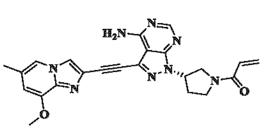
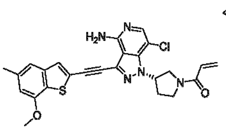
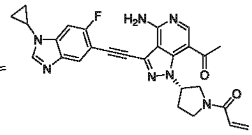
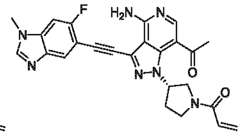
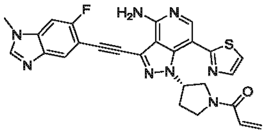
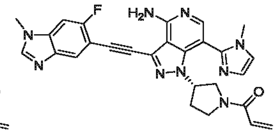
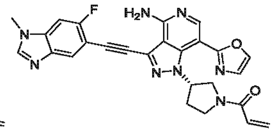
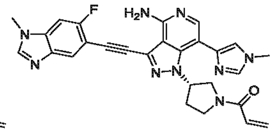
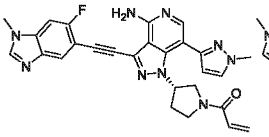
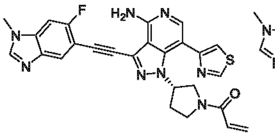
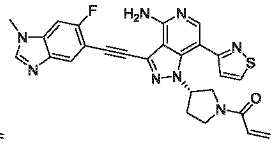
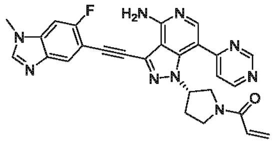
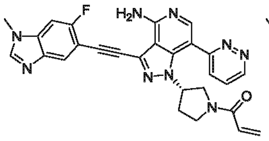
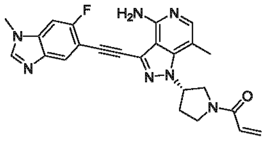
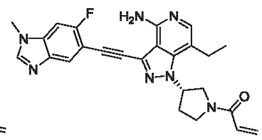
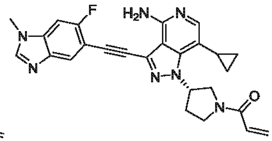
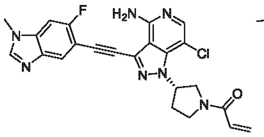
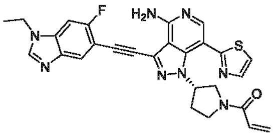
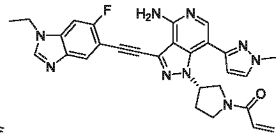
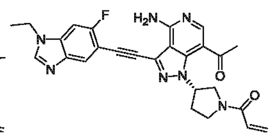
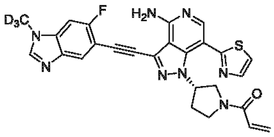
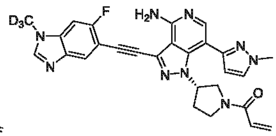
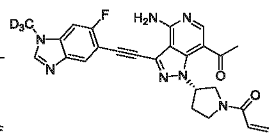
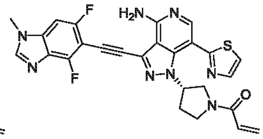
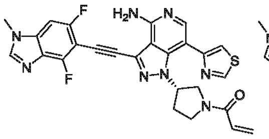
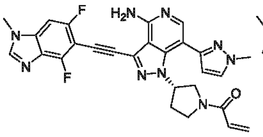
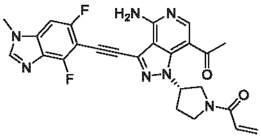
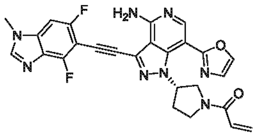

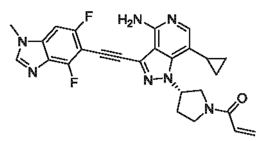
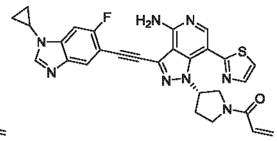
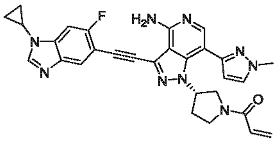
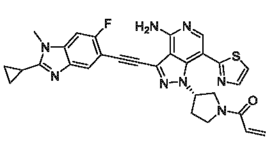
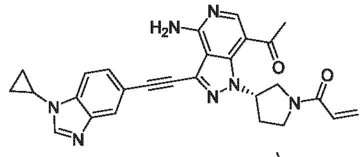
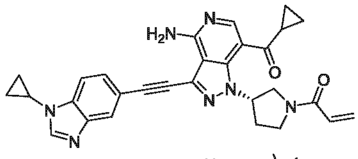
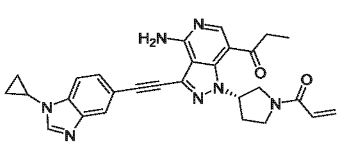
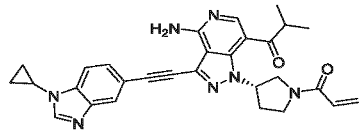
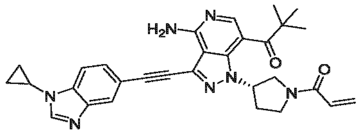
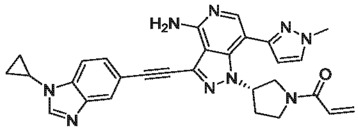
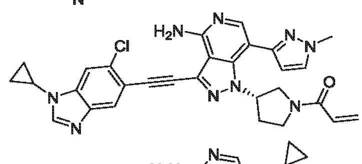
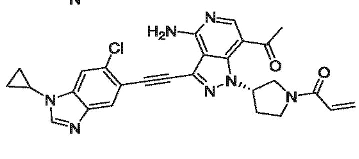
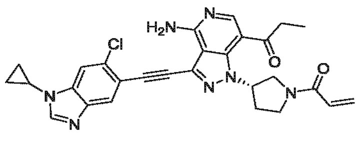
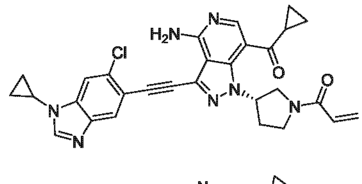
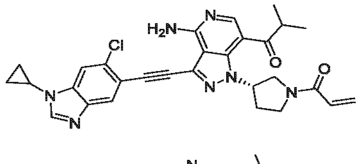
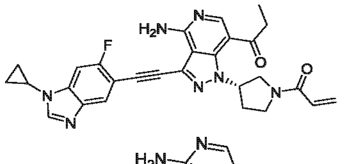
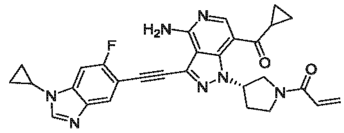
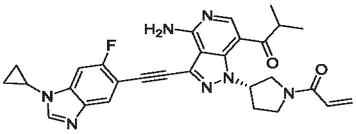
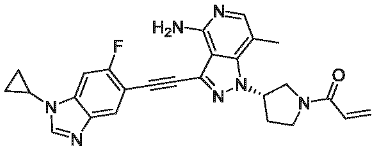
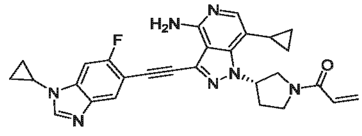
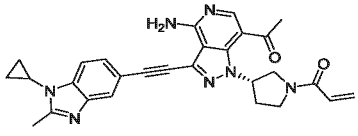
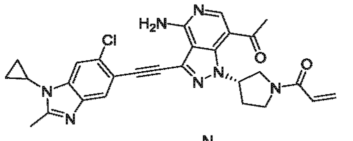
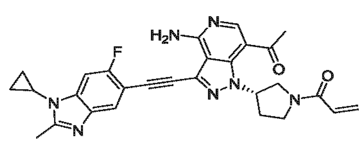
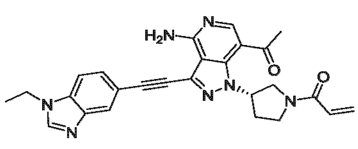
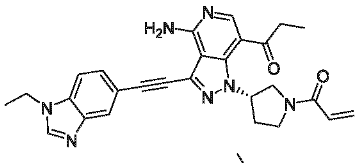
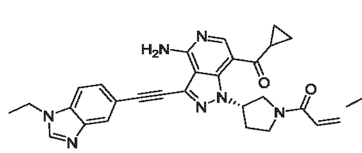
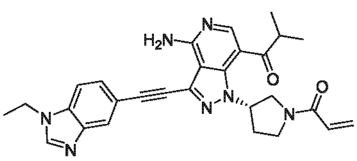
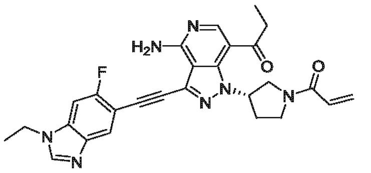
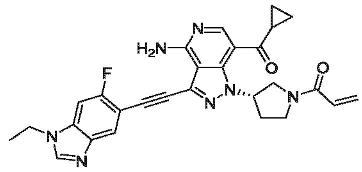
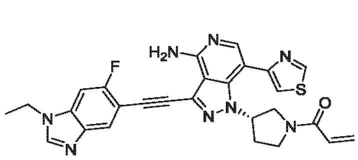
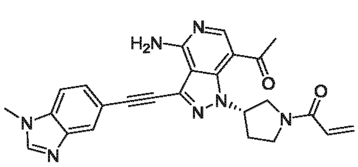
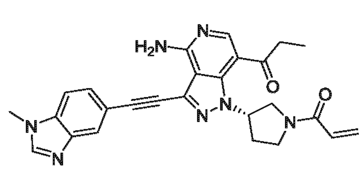
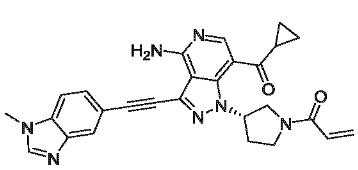
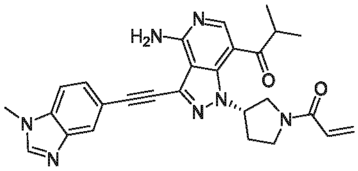

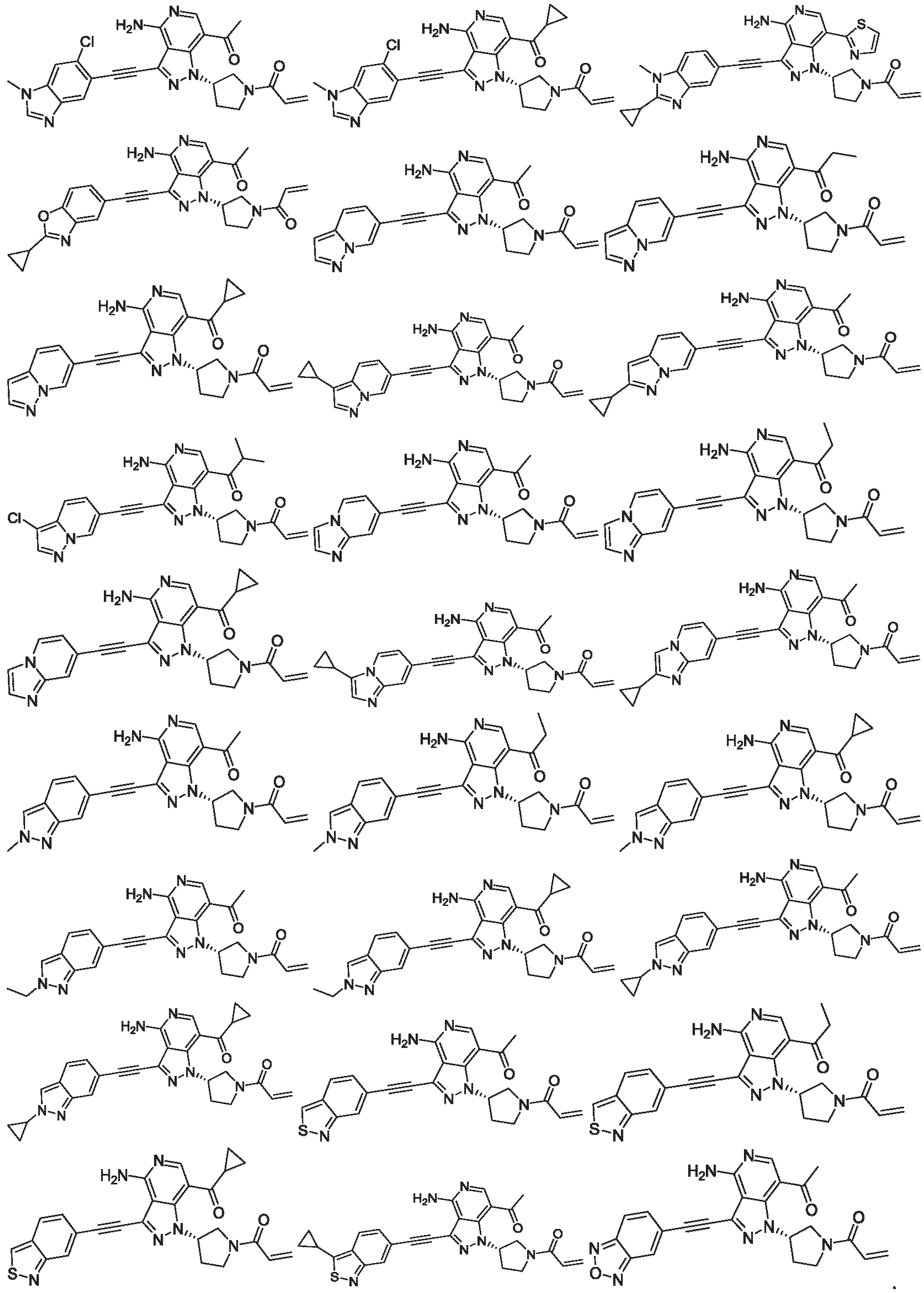

12. A method for preparing the compound represented by general formula (II-3) according to claim 9, wherein the method is method I or method II:

method I: the method comprises the following steps:

step I: under acidic conditions, a compound represented by formula (II-3-1) is subjected to a deprotection reaction as shown below;
step II: in an organic solvent, under alkaline conditions, a compound represented by formula (II-3-2) obtained in step I and acryloyl chloride are subjected to a condensation reaction,

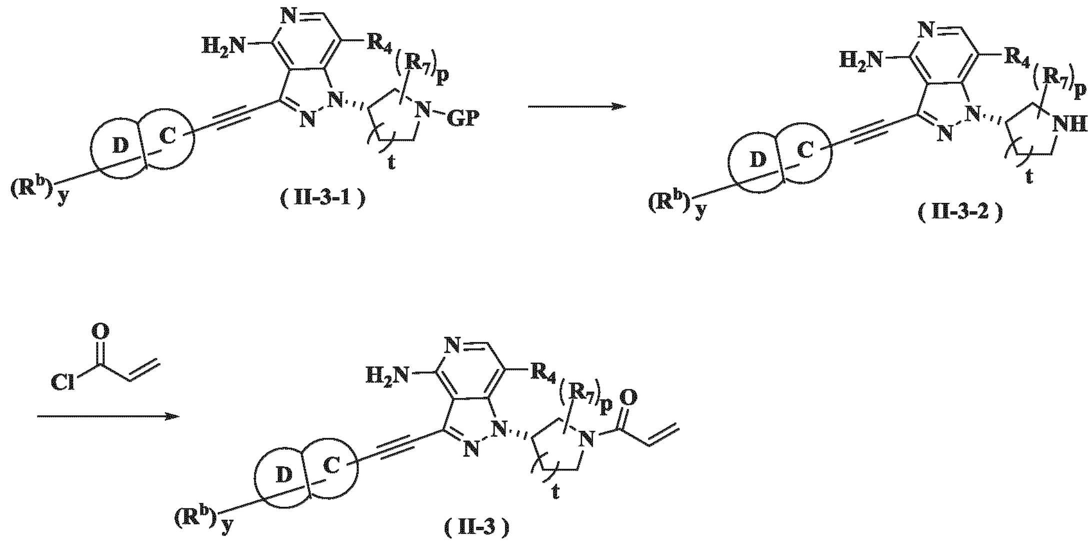

( II-3-1 ) ( II-3-2 ) ( II-3 )

method II comprises the following steps:

step I: under acidic conditions, a compound represented by formula (II-3-3) is subjected to a reaction as shown below to obtain a compound represented by formula (II-3-4);
step II: in an organic solvent, under alkaline conditions, the compound represented by formula (II-3-4) obtained in step I and acryloyl chloride are subjected to a condensation reaction,

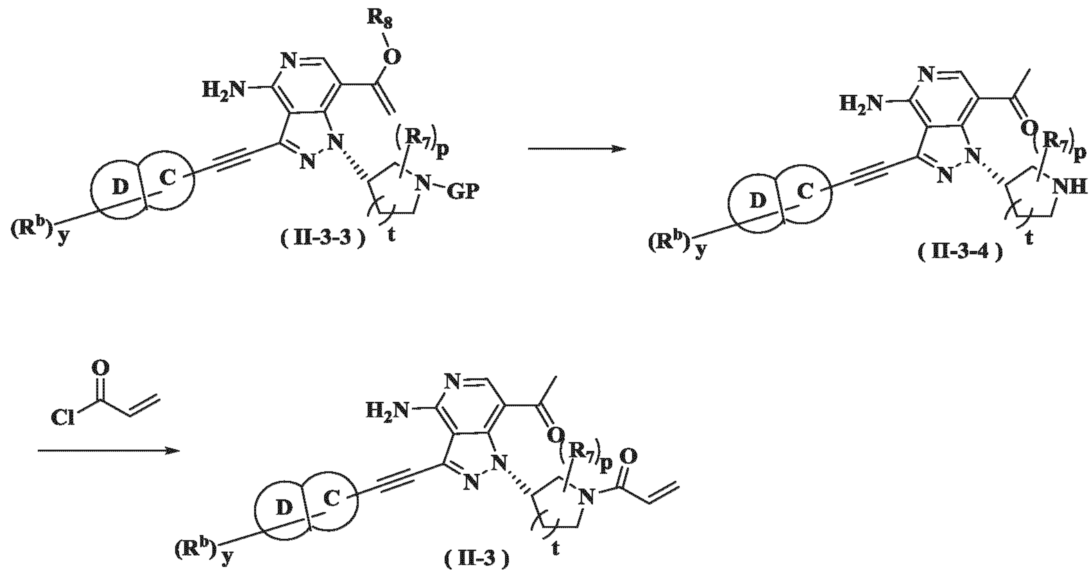

( II-3-3 ) ( II-3-4 ) ( II-3 )

wherein GP is an amino protecting group, preferably Boc;
$R_8$ is $C_{1-6}$ alkyl, preferably n-butyl.

**13.** A compound represented by general formula (II-3-1), (II-3-2), (II-3-3), or (II-3-4), or a

stereoisomer or pharmaceutically acceptable salt thereof,

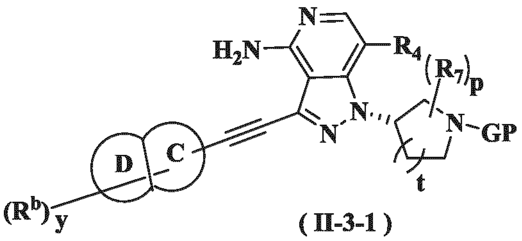
( II-3-1 )
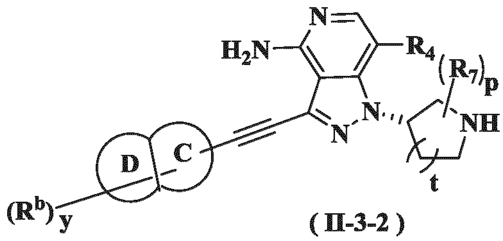
( II-3-2 )
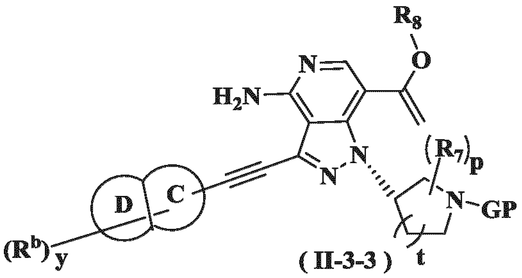
( II-3-3 )
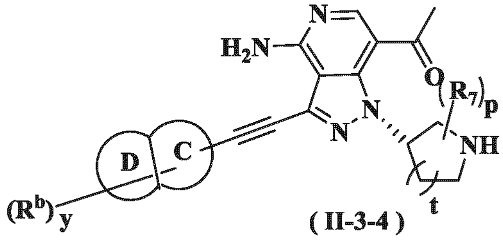
( II-3-4 )

wherein ring C, ring D, $R^b$, $R_4$, y, t, $R_7$, and p are as defined in claim 9, and $R_8$ and GP are as defined in claim 12; the following compounds are preferred:

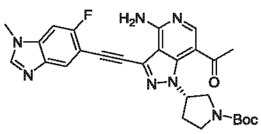 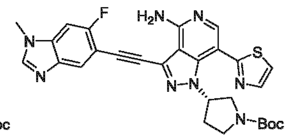 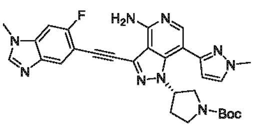 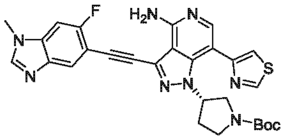
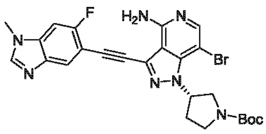 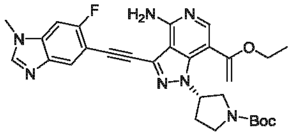 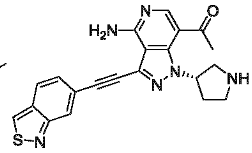 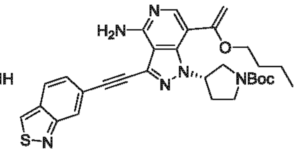
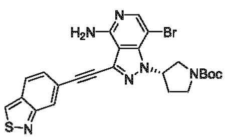 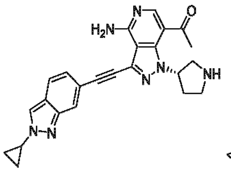 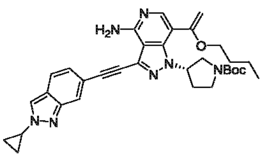 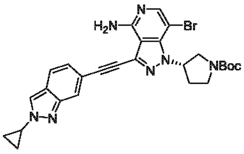
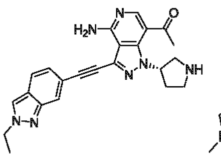 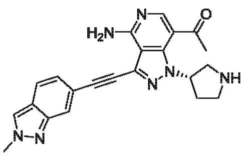 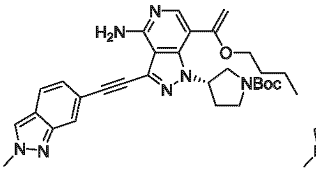 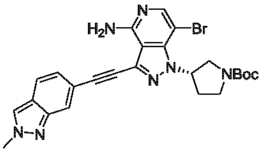
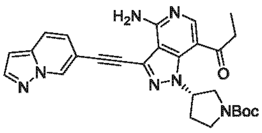 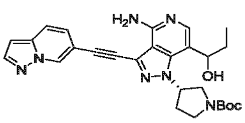 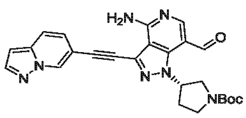 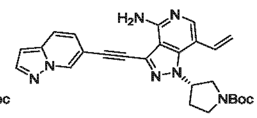
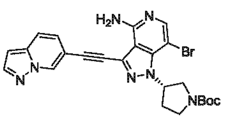 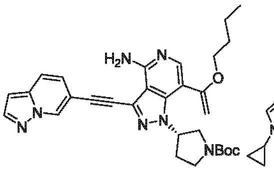 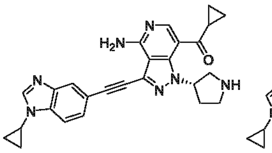 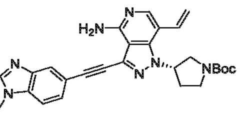
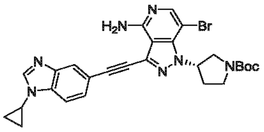 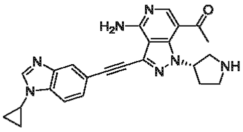 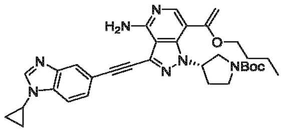 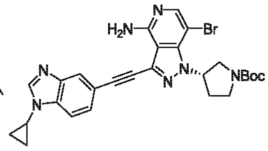

**14.** A pharmaceutical composition comprising a therapeutically effective dose of the compound or the stereoisomer or pharmaceutically acceptable salt thereof according to any one of claims 1-11, and one or more pharmaceutically acceptable carriers or excipients.

**15.** Use of the compound or the stereoisomer or pharmaceutically acceptable salt thereof according to any one of claims 1-11 or the pharmaceutical composition according to claim 14 in the preparation of a drug for treating and/or preventing FGFR inhibitor-related diseases, especially a drug for treating and/or preventing FGFR1-4 inhibitor-related diseases.

**16.** Use of the compound or the stereoisomer or pharmaceutically acceptable salt thereof according to any one of claims 1-11 or the pharmaceutical composition according to claim 14 in the preparation of a drug for treating and/or preventing cancer, achondroplasia, and other related diseases, wherein preferably, the cancer is selected from colorectal cancer, bladder cancer, gastric cancer, thyroid cancer, esophageal cancer, head and neck cancer, brain cancer, glioma, glioblastoma, hepatocellular carcinoma, lung cancer, melanoma, myeloma, pancreatic cancer, renal cell cancer, cervical cancer, urothelial cancer, prostate cancer, ovarian cancer, breast cancer, leukemia, or lymphoma.

## INTERNATIONAL SEARCH REPORT

International application No.
**PCT/CN2023/073557**

### A. CLASSIFICATION OF SUBJECT MATTER

C07D471/04(2006.01)i;C07D487/04(2006.01)i; A61K31/437(2006.01)i;A61K31/5025(2006.01)i;A61P35/00(2006.01)i

According to International Patent Classification (IPC) or to both national classification and IPC

### B. FIELDS SEARCHED

Minimum documentation searched (classification system followed by classification symbols)

IPC：C07D471/04，C07D487/04, A61K31/437，A61K31/5025，A61P35/-

Documentation searched other than minimum documentation to the extent that such documents are included in the fields searched

Electronic data base consulted during the international search (name of data base and, where practicable, search terms used)

CNTXT, WPABSC, DWPI, CNKI, REGISTRY(STN), CAPLUS(STN): 吡唑并(3, 4-d)嘧啶, 吡唑并, 嘧啶, 吡咯烷, 丙, 烯, 酮, 乙炔基, fgfr, pyrazolo+pyridin+, pyrazolo(4, 3-c)pyridin, 根据权利要求9的结构式进行的子结构检索, substructure search according to the structural formula of claim 9

### C. DOCUMENTS CONSIDERED TO BE RELEVANT

| Category* | Citation of document, with indication, where appropriate, of the relevant passages | Relevant to claim No. |
|---|---|---|
| X | WO 2021247971 A1 (KINNATE BIOPHARMA INC.) 09 December 2021 (2021-12-09) description pages 32-88, compounds 16-39, 43, 45, 47, 50-51, 53, 58-60, 65, 73-74, 79, 81, 83, 86, 88, 95, 97-98, 100, 103, 105, 109, 117, 130, 133, 136-139, 141, 148-150, 155-156, 159-160, 162, 165-167, 169, 171-172, 174, 176, 181-183, 185 of the table and corresponding embodiments, pages 318-326 “biological assessment” and claims | 1-16 |

☐ Further documents are listed in the continuation of Box C. ☑ See patent family annex.

* Special categories of cited documents:
- “A” document defining the general state of the art which is not considered to be of particular relevance
- “D” document cited by the applicant in the international application
- “E” earlier application or patent but published on or after the international filing date
- “L” document which may throw doubts on priority claim(s) or which is cited to establish the publication date of another citation or other special reason (as specified)
- “O” document referring to an oral disclosure, use, exhibition or other means
- “P” document published prior to the international filing date but later than the priority date claimed
- “T” later document published after the international filing date or priority date and not in conflict with the application but cited to understand the principle or theory underlying the invention
- “X” document of particular relevance; the claimed invention cannot be considered novel or cannot be considered to involve an inventive step when the document is taken alone
- “Y” document of particular relevance; the claimed invention cannot be considered to involve an inventive step when the document is combined with one or more other such documents, such combination being obvious to a person skilled in the art
- “&” document member of the same patent family

| Date of the actual completion of the international search | Date of mailing of the international search report |
|---|---|
| **09 May 2023** | **10 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/073557**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15-16**
because they relate to subject matter not required to be searched by this Authority, namely:

Although claims 15-16 relate to a method for treatment of the human or animal body (PCT Rule 39.1(iv)), a search is still carried out on the basis of the pharmaceutical use of the compound/pharmaceutical composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The present International Authority considers the claims to include two inventions, as shown below:

I: claims 1-12 and 14-16 and the compound part of formulas (II-3-2) and (II-3-4) in claim 13 relate to a compound of formula (I-1), a preparation method therefor, a pharmaceutical composition, pharmaceutical use and a preparation intermediate of the compound.

II: the compound part of formulas (II-3-1) and (II-3-3) in claim 13.

Although formulas (II-3-1) and (II-3-3) set forth in claim 13 are intermediates for preparing the compound of formula (I-1) involved in claims 1-12 and 14-16, the compound cannot be directly obtained from the intermediates. Therefore, the two groups of inventions lack unity of invention, and do not comply with the requirement of unity of invention (PCT Rule 13).

Form PCT/ISA/210 (continuation of first sheet) (July 2022)

**INTERNATIONAL SEARCH REPORT**

International application No.
**PCT/CN2023/073557**

**Box No. III Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2023/073557**

| Patent document cited in search report | Publication date (day/month/year) | Patent family member(s) | Publication date (day/month/year) |
|---|---|---|---|
| WO 2021247971 A1 | 09 December 2021 | CA 3181209 A1 | 09 December 2021 |
| | | EP 4161657 A1 | 12 April 2023 |
| | | AU 2021285974 A1 | 19 January 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210107872
- CN 202210557434
- CN 202211065464
- WO 2021247971 A
- WO 2022033532 A


### Non-patent literature cited in the description


- *Clin Cancer Res*, 01 January 2016, vol. 22 (1)